# EUROPEAN PATENT APPLICATION

(11) **EP 4 512 828 A2**
(43) Date of publication of application: **26.02.2025**
(21) Application number: 24210550.0
(22) Date of filing: 01.03.2021
(51) Int. Cl.: C07K 16/28

(54) **METHODS FOR EX VIVO ENRICHMENT AND EXPANSION OF TUMOR REACTIVE T CELLS AND RELATED COMPOSITIONS THEREOF**

(30) Priority: 27.02.2020 US 202062982704 P
(62) Divisional of application: 21713843.7
(71) Applicant: Turnstone Biologics Corp., La Jolla, CA 92037 (US)
(72) Inventor: LANGER, Timothy J., New York, 10010 (US); MILES, Brodie James, New York, 10010 (US)
(74) Representative: J A Kemp LLP

(57) **Abstract**

Provided herein are methods for manufacturing of tumor reactive T cells that includes ex vivo enrichment of, and expansion of, cells secreting chemokine (C-X-C motif) ligand 13 (CXCL13); cells surface positive for C-X-C chemokine receptor type 5 (CXCR5); and/or one or more of CD39, PD-1 and TIGIT. Also provided are populations of T cells produced by methods described herein and pharmaceutical compositions thereof.

## Description

### CROSS-REFERENCE TO RELATED APPLICATIONS

This application claims priority from U.S. provisional patent application No. 62/982,704 filed February 27, 2020 entitled "METHODS FOR EX VIVO ENRICHMENT AND EXPANSION OF TUMOR REACTIVE T CELLS AND RELATED COMPOSITIONS THEREOF", the content of which are incorporated by reference in its entirety.

### INCORPORATION BY REFERENCE OF SEQUENCE LISTING

The present application is being filed along with a Sequence listing in electronic format. The sequence listing is provided as a filed entitled 16517_2000840_SEQLIST.txt, created March 1, 2021, which is 12,537 bytes in size. The information in the electronic format of the Sequence Listing is incorporated by reference in its entirety.

### Field

The present disclosure provides methods for manufacturing of tumor reactive T cells that includes ex vivo enrichment of, and expansion of, cells secreting chemokine (C-X-C motif) ligand 13 (CXCL13); cells surface positive for C-X-C chemokine receptor type 5 (CXCR5); and/or one or more of CD39, PD-1 and TIGIT. The present disclosure also provides populations of T cells produced by methods described herein and pharmaceutical compositions thereof.

### Background

Clinical studies have demonstrated that T cells isolated from surgically resected tumor possess T-cell receptors (TCRs) that recognize neoantigens, and expanding these neoantigen reactive tumor infiltrating lymphocyte (TIL) populations and re-infusing them into the patient can in some cases result in a dramatic clinical benefit. However, a major obstacle to applications of such cells in cell therapy is the difficulty in obtaining such cells. For example, existing methods for producing TIL therapies for use in cancer is lengthy, involves a low number of reactive cells and is not suited for commercial applications. Improved methods are needed for obtaining and manufacturing cell compositions containing tumor-reactive T cells for therapeutic use. Provided herein are embodiments that meet such needs.

### Summary

According to certain embodiments described herein, methods for manufacturing tumor reactive T cells are provided. Such methods include, but are not limited to, the steps of (a) selecting cells secreting chemokine (C-X-C motif) ligand 13 (CXCL13) and/or surface positive for C-X-C chemokine receptor type 5 (CXCR5), from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells. Also provided herein are populations of T cells produced by methods described herein and pharmaceutical compositions thereof.

Provided herein is a method of manufacturing tumor-reactive T cells, the method comprising (a) selecting cells secreting chemokine (C-X-C motif) ligand 13 (CXCL13) and/or surface positive for C-X-C chemokine receptor type 5 (CXCR5), from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells.

In some of any of the embodiments, the method comprises selecting cells secreting CXCL 13. In some of any of the provided embodiments, the method comprises selecting cells surface positive for CXCR5.

In some of any of the provided embodiments, the selecting further comprises selecting cell surface positive for one or more further marker selected from, CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT, wherein the selecting cells secreting CXCL13 and/or surface positive for CXCR5 and the selecting cells surface positive for the one or more further marker is carried out simultaneously or sequentially in any order to obtain the selected cells. In some of any of the provided embodiments, the one or further marker is PD-1, CD39 and/or TIGIT.

In some of any of the provided embodiments, the method further comprises selecting T cells surface positive for a T cell marker selected from CD3, CD4 or CD8, wherein the selecting cells surface positive for the T cell marker and the selecting cells secreting CXCL13 and/or surface positive for CXCR5 is carried out simultaneously or sequentially in any order to obtain the selected cells. Optionally, selecting by positive or negative selection.

Provided herein is a method for manufacturing tumor-reactive T cells comprising (a) selecting cells surface positive for activation markers PD-1, CD39 and TIGIT from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells. Also provided herein is a method for manufacturing tumor-reactive T cells, the method comprising selecting cells surface positive at least two activation markers from the group consisting of PD-1, CD39 and TIGIT from an input sample comprising T cells from a subject that has a tumor to obtain selected cells from the sample; and performing an expansion by culture of the selected cells with one or more T-cell stimulating agent of lymphocytes under conditions to produce a population of expanded T cells.

Provided herein is a method for manufacturing tumor-reactive T cells comprising (a) selecting cells surface positive for PD-1, CD39 and TIGIT from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells. In some of any of the provided embodiments, the selecting further comprises selecting cell surface positive for one or more further marker selected from CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT, wherein the selecting for cells surface positive for PD-1/CD39/TIGIT and the selecting cells surface positive for the further marker is carried out simultaneously or sequentially in any order to obtain the selected cells.. In some of any of the provided embodiments, the selecting further comprises selecting cell surface positive for one or more further marker selected from CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT, wherein the selecting for cells surface positive for the activation markers and the selecting cells surface positive for the further marker is carried out simultaneously or sequentially in any order to obtain the selected cells. In some of any of the provided embodiments, the selecting further comprises selecting cells secreting chemokine (C-X-C motif) ligand 13 (CXCL13), wherein the selecting for cells surface positive for PD-1/CD39/TIGIT and the selecting cells secreting CXCL13 is carried out simultaneously or sequentially in any order to obtain the selected cells. In some of any of the provided embodiments, the selecting further comprises T cells surface positive for a T cell marker selected from CD3, CD4 or CD8, wherein the selecting cells surface positive for the T cell marker and the selecting cells surface positive for PD-1/CD39/TIGIT is carried out simultaneously or sequentially in any order to obtain the selected cells. In some of any of the provided embodiments, the selecting further comprises T cells surface positive for a T cell marker selected from CD3, CD4 or CD8, wherein the selecting cells surface positive for the T cell marker and the selecting cells surface positive for the activation markers is carried out simultaneously or sequentially in any order to obtain the selected cells. Optionally, selecting by positive or negative selection.

In some of any of the provided embodiments, the input sample comprising T cells is from the peripheral blood or from a tumor. In some of any of the provided embodiments, the input sample comprises tumor infiltrating lymphocytes. In some of any of the provided embodiments, the input sample comprising T cells is derived from a resected tumor. In some of any of the provided embodiments, the input sample comprising T cells is a single cell suspension processed by homogenization and/or enzymatic digestion of one or more tumor fragments from the resected tumor. In some of any of the provided embodiments, the input sample comprising T cells is a single cell suspension processed by homogenization and enzymatic digestion of one or more tumor fragments from the resected tumor.

In some of any of the provided embodiments, the enzymatic digestion is by incubation with a collagenase, optionally collagenase IV or collagenase I/II.

In some of any of the provided embodiments, the input sample comprises from at or about 10 x 10⁶ T cells per gram of tumor sample from the subject to at or about 100 × 10⁶ T cells per gram of the tumor sample from the subject. In some of any of the provided embodiments, the expanded T cell population is for use as a therapeutic cell composition. In some of any of the provided embodiments, performing the expansion to produce the expanded population of T cells is for 7 to 35 days. In some of any of the provided embodiments, performing the expansion to produce the expanded population of T cells is for 7 to 28 days, optionally 14 days to 28 days. In some of any of the provided embodiments, performing the expansion to produce the expanded population of T cells is for 7 to 21 days, optionally 7 to 14 days.

In some of any of the provided embodiments, the one or more T-cell stimulating agent of lymphocytes is an anti-CD3 agent and/or a recombinant cytokine selected from one or more of IL-2, IL-7, IL-15, IL-21, IL-25, and IL-23. In some of any of the provided embodiments, the one or more T-cell stimulating agent of lymphocytes is an anti-CD3 agent (e.g. OKT3) and/or a recombinant cytokine selected from one or more of IL-2, IL-7, IL-15, IL-21, IL-25, IL-23, IL-27, and IL-35. In some of any of the provided embodiments, at least one of the one or more T-cell stimulating agent is recombinant IL-2. In some any of the provided embodiments, culture with the one or more T-cell stimulating agent further comprises an apoptosis inhibitor. In some of any of the provided embodiments, the one or more T-cell stimulating agent is one or more first T-cell stimulating agent and the performing the expansion is a first expansion, wherein the method further comprises performing a second expansion by culture of the first expanded T cell population with one or more second T-cell stimulating agent under conditions to produce a second expanded population of T cells, wherein the second population of expanded T cells is for use as a therapeutic cell composition.

In some of any of the provided embodiments, one or more T cell stimulating agent of the first expansion and the one or more T cell stimulating agent of the second expansion are the same. In some of any of the provided embodiments, performing the first expansion is for 7 to 21 days, optionally 7 to 14 days. In some of any of the provided embodiments, performing the second expansion is 7 to 21 days, optionally 7 to 14 days

In some of any of the provided methods, the one or more T-cell stimulating agent is one or more first T-cell stimulating agent and the performing the expansion is a first expansion, wherein the method further comprises (c) co-culturing the first expanded T cell population in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor; and (d) performing a second expansion by culture of the reactive T cell population with one or more second T-cell stimulating agent under conditions to produce a second population of expanded T cells, wherein the second population of expanded T cells is for use as a therapeutic cell composition.

In some of any of the provided methods, the one or more T-cell stimulating agent is one or more first T-cell stimulating agent and the performing the expansion is a first expansion, wherein the method further comprises (c) co-culturing the first expanded T cell population in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor; (d) selecting, from the reactive T cell population, cells positive for one or more marker associated with reactive T cells comprising native T cell receptors reactive to mutation encoding peptides of the tumor, to produce an enriched population of the reactive T cells; and (e) performing a second expansion by culture of the enriched population of the reactive T cells with one or more second T-cell stimulating agent under conditions to produce a second population of expanded T cells, wherein the second population of expanded T cells is for use as a therapeutic cell composition.

In some of any of the provided embodiments, the one or more marker is a marker of a T cell exhaustion marker. In some of any of the provided embodiments, the one or more marker is cell surface CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT. In some of any of the provided embodiments, the one or marker is PD-1, CD39 and TIGIT. In some of any of the provided embodiments, the one or more marker is secreted CXCL13.

In some of any of the provided methods, the method further comprises selecting, optionally by positive selection or negative selection, T cells surface positive for a T cell marker selected from CD3, CD4 or CD8, wherein the selecting cells surface positive for the T cell marker and the selecting cells positive for a marker associated with reactive T cells is carried out simultaneously or sequentially in any order to obtain the enriched population of reactive T cells.

Provided herein is a method for manufacturing tumor-reactive T cells, the method comprising (a) processing a biological sample containing T cells obtained from a donor subject that has a tumor to produce an input sample comprising T cells, (b) performing a first expansion by culture of the input sample comprising T cells with one or more first T-cell stimulating agent of lymphocytes under conditions to produce a first population of expanded T cells, (c) selecting cells secreting chemokine (C-X-C motif) ligand 13 (CXCL13) and/or surface positive for C-X-C chemokine receptor type 5 (CXCR5) from the first population of expanded cells to produce a selected population, and (d) performing a second expansion by culture of the selected population with one or more second T-cell stimulating agent under conditions to produce a second expanded population of T cells, wherein the second population of expanded T cells is for use as a therapeutic cell composition.

Provided herein is a method for manufacturing tumor-reactive T cells, the method comprising (a) processing a biological sample containing T cells obtained from a donor subject that has a tumor to produce an input sample comprising T cells, (b) performing a first expansion by culture of the input sample comprising T cells with one or more first T-cell stimulating agent of lymphocytes under conditions to produce a first population of expanded T cells, (c) selecting cells secreting chemokine (C-X-C motif) ligand 13 (CXCL13) and/or surface positive for C-X-C chemokine receptor type 5 (CXCR5), from the first population of expanded cells to produce a selected population, (d) co-culturing the selected population in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor, and (e) performing a second expansion by culture of the reactive T cell population with one or more second T-cell stimulating agent under conditions to produce a second expanded population of T cells, wherein the second population of expanded T cells is for use as a therapeutic cell composition.

Provided herein is a method for manufacturing tumor-reactive T cells, the method comprising (a) processing a biological sample containing T cells obtained from a donor subject that has a tumor to produce an input sample comprising T cells, (b) performing a first expansion by culture of the input sample comprising T cells with one or more first T-cell stimulating agent of lymphocytes under conditions to produce a first population of expanded T cells, (c) co-culturing the first population of expanded cells in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor, (d) selecting cells secreting chemokine (C-X-C motif) ligand 13 (CXCL13) and/or surface positive for C-X-C chemokine receptor type 5 (CXCR5), from the reactive T cell population to produce a selected population; and (e) performing a second expansion by culture of the selected population with one or more second T-cell stimulating agent under conditions to produce a second expanded population of T cells, wherein the second population of expanded T cells is for use as a therapeutic cell composition.

In some of any of the provided embodiments, the method comprises selecting cells secreting CXCL13. In some of any of the provided embodiments, the method comprises selecting cells surface positive for CXCR5.

In some of any of the provided embodiments, the selecting further comprises selecting cell surface positive for one or more further marker selected from CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT, wherein the selecting cells secreting CXCL13 and/or surface positive for CXCR5 and the selecting cells surface positive for the one or more further marker is carried out simultaneously or sequentially in any order to produce the selected population. In some of any of the provided embodiments, the one or more further marker is PD-1, CD39 and/or TIGIT. In some of the provided embodiments, the one or more further marker is PD-1, CD39 and TIGIT.

In some of any of the provided methods, the method further comprising selecting, optionally by positive selection or negative selection, T cells surface positive for a T cell marker selected from CD3, CD4 or CD8, wherein the selecting cells surface positive for the T cell marker and the selecting cells secreting CXCL13 and/or surface positive for CXCR5 is carried out simultaneously or sequentially in any order to produce the selected cell population.

Provided herein is a method for manufacturing tumor-reactive T cells, the method comprising (a) processing a biological sample containing T cells obtained from a donor subject that has a tumor to produce an input sample comprising T cells, (b) performing a first expansion by culture of the sample comprising T cells with one or more first T-cell stimulating agent of lymphocytes under conditions to produce a first population of expanded T cells, (c) selecting cells surface positive for activation markers PD-1, CD39 and TIGIT from the first population of expanded cells to produce a selected population; and (d) performing a second expansion by culture of the selected population with one or more second T-cell stimulating agent under conditions to produce a second expanded population of T cells, wherein the second population of expanded T cells is for use as a therapeutic cell composition.

Provided herein is a method for manufacturing tumor-reactive T cells, the method comprising (a) processing a biological sample containing T cells obtained from a donor subject that has a tumor to produce an input sample comprising T cells (b) performing a first expansion by culture of the sample comprising T cells with one or more first T-cell stimulating agent of lymphocytes under conditions to produce a first population of expanded T cells; (c) selecting cells surface positive for at least two activation markers from the group consisting of PD-1, CD39 and TIGIT from the first population of expanded cells to produce a selected population; and (d)performing a second expansion by culture of the selected population with one or more second T-cell stimulating agent under conditions to produce a second expanded population of T cells, wherein the second population of expanded T cells is for use as a therapeutic cell composition.

Provided herein is a method for manufacturing tumor-reactive T cells, the method comprising (a) processing a biological sample containing T cells obtained from a donor subject that has a tumor to produce an input sample comprising T cells, (b) performing a first expansion by culture of the sample comprising T cells with one or more first T-cell stimulating agent of lymphocytes under conditions to produce a first population of expanded T cells, (c) selecting cells surface positive for activation markers PD-1, CD39 and TIGIT from the first population of expanded cells to produce a selected cell population, (d) co-culturing the selected cell population in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor, and (e) performing a second expansion by culture of the reactive T cell population with one or more second T-cell stimulating agent under conditions to produce a second expanded population of T cells, wherein the second population of expanded T cells is for use as a therapeutic cell composition.

Provided herein is A method for manufacturing tumor-reactive T cells, the method comprising, (a) processing a biological sample containing T cells obtained from a donor subject that has a tumor to produce an input sample comprising T cells, (b) performing a first expansion by culture of the sample comprising T cells with one or more first T-cell stimulating agent of lymphocytes under conditions to produce a first population of expanded T cells, (c) co-culturing the first population of expanded cells in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor, (d) selecting cells surface positive for activation markers PD-1, CD39 and TIGIT from the reactive T cell population to produce a selected cell population; and (e) performing a second expansion by culture of the selected cell population with one or more second T-cell stimulating agent under conditions to produce a second expanded population of T cells, wherein the second population of expanded T cells is for use as a therapeutic cell composition.

In some of any of the provided embodiments, the selecting further comprises selecting cell surface positive for one or more further marker selected from CD107a, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), TIM-3, or LAG-3, wherein the selecting for cells surface positive for PD-1/CD39/TIGIT and the selecting cells surface positive for the further marker is carried out simultaneously or sequentially in any order to produce the selected cell population. In some of any of the provided embodiments, the selecting further comprises selecting cells secreting chemokine (C-X-C motif) ligand 13 (CXCL13), wherein the selecting for cells surface positive for PD-1/CD39/TIGIT and the selecting cells surface positive for the further marker is carried out simultaneously or sequentially in any order to produce the selected cell population. In some of any of the provided embodiments, the selecting further comprises selecting cell surface positive for one or more further marker selected from CD107a, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), TIM-3, or LAG-3, wherein the selecting for cells surface positive for the activation markers and the selecting cells surface positive for the further marker is carried out simultaneously or sequentially in any order to produce the selected cell population

In some of any of the provided embodiments, the selecting further comprises selecting cells secreting chemokine (C-X-C motif) ligand 13 (CXCL13), wherein the selecting for cells surface positive for PD-1/CD39/TIGIT and the selecting cells surface positive for the further marker is carried out simultaneously or sequentially in any order to produce the selected cell population. In some of any of the provided methods further comprising selecting, optionally by positive selection or negative selection, T cells surface positive for a T cell marker selected from CD3, CD4 or CD8, wherein the selecting cells surface positive for the T cell marker and the selecting cells surface positive for PD-1/CD39/TIGIT is carried out simultaneously or sequentially in any order to produce the selected cell population.

In some of any of the provided embodiments, the biological sample is a peripheral blood sample or a tumor sample. In some of the provided embodiments, the input sample comprising T cells comprises tumor infiltrating lymphocytes (TILs).

In some of any of the provided embodiments, the input sample comprising T cells is derived from a resected tumor. In some of any of the provided embodiments, the one or more tumor fragments are 1-8 mm in diameter. In some of any of the provided embodiments, the one or more tumor fragments are seeded for the first expansion at about 1 tumor fragment per 2 cm² .

In some of any of the provided embodiments, the input sample comprising T cells is a single cell suspension processed by homogenization and/or enzymatic digestion of one or more tumor fragments from the resected tumor. In some of any of the provided embodiments, the input sample comprising T cells is a single cell suspension processed by homogenization and enzymatic digestion of one or more tumor fragments from a resected tumor. In some of any of the provided embodiments, the enzymatic digestion is by incubation with a collagenase, optionally collagenase IV or collagenase I/II.

In some of any of the provided embodiments, the input sample comprises from at or about 10 x 10⁶ T cells per gram of tumor sample from the subject to at or about 100 x 10⁶ T cells per gram of the tumor sample from the subject.

In some of any of the provided embodiments, the input sample comprising T cells is seeded for expansion at about 5 x 10⁵ to at or about 2 x 10⁶ total cells per 2 cm².

In some of any of the provided embodiments, performing the first expansion is for 1 to 14 days. In some of any of the provided embodiments, performing the first expansion is for at or about 1 day, at or about 2 days, at or about 3 days, at or about 4 days, at or about 5 days, at or about 6 days, at or about 7 days, at or about 8 days, at or about 9 days, at or about 10 days, at or about 11 days, at or about 12 days, at or about 13 days or at or about 14 days. In some of any of the provided embodiments, performing the second expansion is for 7 to 35 days. In some of any of the provided embodiments, performing the second expansion is 7 to 21 days, optionally 7 to 14 days.

In some of any of the provided embodiments, the one or more T cell stimulating agent of the first expansion and the one or more T cell stimulating agent of the second expansion are the same.

In some of any of the provided embodiments, the one or more first T-cell stimulating agent of lymphocytes is an anti-CD3 agent and/or a recombinant cytokine selected from one or more of IL-2, IL-7, IL-15, IL-21, IL-25, and IL-23. In some of any of the provided embodiments, the one or more first T-cell stimulating agent of lymphocytes for the first expansion is a recombinant cytokine selected from one or more of IL-2, IL-7, IL-15, IL-21, IL-25, IL-23, IL-27, and IL-35. In some of any of the provided embodiments, at least one of the one or more first T-cell stimulating agent is recombinant IL-2.

In some of any of the provided embodiments, the one or more first T cell stimulating agent comprises an anti-CD3 antibody, optionally OKT3. In some of any of the provided embodiments, the one or more first T cell stimulating agent does not comprise an anti-CD3 antibody. Optionally, wherein the concentration of the anti-CD3 antibody is at or about 50 ng/mL. In some of any of the provided embodiments, the one or more first T-cell stimulating agent further comprises an apoptosis inhibitor. In some of any of the provided embodiments, culture with the one or more first T-cell stimulating agent further comprises an apoptosis inhibitor In some of any of the provided embodiments, the one or more second T-cell stimulating agent of lymphocytes is an anti-CD3 agent and/or a recombinant cytokine selected from one or more of IL-2, IL-7, IL-15, IL-21, IL-25, and IL-23. In some of any of the provided embodiments, the one or more second T-cell stimulating agent of lymphocytes is an anti-CD3 agent and/or is a recombinant cytokine selected from one or more of IL-2, IL-7, IL-15, IL-21, IL-25, IL-23, IL-27, and IL-35.

In some of any of the provided embodiments, at least one of the one or more second T-cell stimulating agent is recombinant IL-2. In some of any of the provided embodiments, the concentration of recombinant IL-2 is from 100 IU/mL to 6000 IU/mL. In some of any of the provided embodiments, the concentration of recombinant IL-2 is from 300 IU/mL to 1000 IU/mL, optionally wherein the concentration of recombinant IL-2 is at or about 300 IU/mL. In some of any of the provided embodiments, the concentration of recombinant IL-2 is at or about 1000 IU/mL.

In some of any of the provided embodiments, the one or more second T cell stimulating agent comprises an anti-CD3 antibody, optionally OKT3, optionally wherein the concentration of the anti-CD3 antibody is at or about 50 ng/mL.

In some of any of the provided embodiments, the one or more second T-cell stimulating agent further comprises an apoptosis inhibitor. In some of any of the provided embodiments, culture with the one or more second T-cell stimulating agent further comprises an apoptosis inhibitor In some of any of the provided embodiments, the apoptosis inhibitor reduces apoptosis induced by CD95 (Fas), optionally wherein the apoptosis inhibitor specifically binds CD95 (Fas) or CD95 ligand (Fas ligand). In some of any of the provided embodiments, the apoptosis inhibitor is an antibody or antigen-binding fragment. Optionally, wherein the apoptosis inhibitor is an anti-Fas antibody or an anti-Fas ligand antibody. In some of any of the provided embodiments, the apoptosis inhibitor is a fusion protein comprising the extracellular domain of CD95 (Fas) or a specific binding fragment thereof that binds to CD95 ligand (Fas ligand) fused to an Fc immunoglobulin domain. Optionally, wherein the apoptosis inhibitor is APG101 or CAN008. In some of any of the provided embodiments, the apoptosis inhibitor inhibits caspase activation or activity, optionally wherein the caspase is a caspase 2, a caspase 8, a caspase 9, a caspase 10, a caspase 3, a caspase 6 or a caspase 7, optionally wherein the caspase is a caspase 3. In some of any of the provided embodiments, the apoptosis inhibitor is selected from the group consisting of NAIP (neuronal apoptosis inhibitory protein; BIRC1), cIAP1 and cIAP2 (cellular inhibitor of apoptosis 1 and 2; BIRC2 and BIRC3, respectively), XIAP (X-chromosome binding IAP; BIRC4), survivin (BIRC5), BRUCE (Apollon; BIRC6), livin (BIRC7) and Ts-IAP (testis-specific IAP; BIRC8). In some of any of the provided embodiments, the apoptosis inhibitor is emericasan. In some of any of the provided embodiments, the apoptosis inhibitor is selected from the group consisting of Emricasan (IDN-6556, PF-03491390), NAIP (neuronal apoptosis inhibitory protein; BIRC1), cIAP1 and cIAP2 (cellular inhibitor of apoptosis 1 and 2; BIRC2 and BIRC3, respectively), XIAP (X-chromosome binding IAP; BIRC4), survivin (BIRC5), BRUCE (Apollon; BIRC6), livin (BIRC7) and Ts-IAP (testis-specific IAP; BIRC8), Wedelolactone, NS3694, NSCI and Z- fluoromethyl ketone Z-VAD-FMK or a flouromethyl ketone variant thereof. In some of any of the provided embodiments, the apoptosis inhibitor is a pan-caspase inhibitor that inhibits activation or activity of two or more caspases. In some of any of the provided embodiments, the apoptosis inhibitor is Z-VAD-FMK, Z-FA-FMK, Z-VAD(OH)-FMK, Z-DEVD-FMK, Z-VAD(OM2)-FMK, or Z-VDVAD-FMK.

In some of any of the provided embodiments, the concentration of the apoptosis inhibitor is between at and about 0.5 µM and at or about 50 µM, between at or about 0.5 µM and at or about 25 µM, between at or about 0.5 µM and at or about 10 µM, between at or about 0.5 µM and at or about 5 µM, between at or about 0.5 µM and at or about 1 µM, between at or about 1 µM and at or about 100 µM, between at or about 1 µM and at or about 50 µM, between at or about 1 µM and at or about 25 µM, between at or about 1 µM and at or about 10 µM, between at or about 1 µM and at or about 5 µM, between at or about 5 µM and at or about 100 µM, between at or about 5 µM and at or about 50 µM, between at or about 5 µM and at or about 25 µM, between at or about 5 µM and at or about 10 µM, between at or about 10 µM and at or about 100 µM, between at or about 10 µM and at or about 50 µM, between at or about 10 µM and at or about 25 µM, between at or about 25 µM and at or about 100 µM, between at or about 25 µM and at or about 50 µM, or between at or about 50 µM and at or about 100 µM, each inclusive

In some of any of the provided embodiments, the antigen presenting cells are dendritic cells, mononuclear phagocytes, B lymphocytes, endothelial cells or thymic epithelium. In some of any of the provided embodiments, the antigen presenting cells are dendritic cells. In some of any of the provided embodiments, the antigen presenting cells are autologous to the subject.

In some of any of the provided embodiments, the one or more non-native peptide comprises an individual peptide or a pool of peptides. In some of any of the provided embodiments, the one or more non-native peptides are loaded on antigen presenting cells by transfection of in vitro transcribed synthesized minigene constructs encoding for the one or more non-native peptides in tandem, wherein the transcribed minigene constructs generate individual peptides. In some of any of the provided embodiments, the one or more non-native peptides are loaded on antigen presenting cells by peptide pulse, optionally by electroporation. In some of any of the provided embodiments, the one or more non-native peptide is 5-30 amino acids, optionally 12-25 amino acids, optionally at or about 25 amino acids in length.

In some of any of the provided embodiments, the one or more non-native peptides are a pool of peptides and the concentration of peptides in the pool of peptides for the peptide pulse is between at or about 0.001 µg/mL and at or about 40 µg/mL, 0.01 µg/mL and at or about 40 µg/mL, at or about 0.1 µg/mL and at or about 40 µg/mL, at or about 1 µg/mL and at or about 40 µg/mL, at or about 0.01 µg/mL and at or about 10 µg/mL or at or about 1 µg/mL and at or about 10 µg/mL; or the one or more non-native peptides is an individual peptide and the concentration of individual peptides for the peptide pulse is between at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.0001 µg/mL and at or about 1 µg/mL, at or about 0.0001 µg/mL and at or about 0.1 µg/mL, at or about 0.0001 µg/mL and at or about 0.1 µg/mL or at or about 0.0001 µg/mL and at or about 0.01 µg/mL.

In some of any of the provided embodiments, the concentration of individual peptides of the one or more non-native peptide, on average, is from at or about 0.00001 µg/mL to at or about 0.01 µg/mL. In some of any of the provided embodiments, the concentration of individual peptide of the one or more non-native peptide, on average, is from at or about 0.0001 µg/mL and at or about 0.001 µg/mL.

In some of any of the provided embodiments, wherein the co-culture ratio of antigen presenting cells to T Cells is between 20:1 and 1:1, between 15:1 and 1:1, between 10:1 and 1:1, between 5:1 and 1:1, between 2.5:1 and 1:1, between 1:20 and 1:1, between 1:15 and 1:1, between 1:10 and 1:1, between 1:5 and 1:1, or between 1:2.5 and 1:1. In some of any of the provided embodiments, the co-culture ratio of dendritic cells to T Cells is between 5:1 and 1:5 or is between 3:1 and 1:3, optionally is or is about 1:1. In some of any of the provided embodiments, the co-culture ratio of antigen presenting cells to T cells is or is about 1:1.

In some of any of the provided embodiments, the co-culturing is for 2 hours to 24 hours. In some of any of the provided embodiments, the co-culturing is for at or about 6 hours.

In some of any of the provided embodiments, the selecting cells is performed using a florescence based cell sorter. In some of any of the provided embodiments, the fluorescence based cell sorter is an automated high-throughput flow cytometry sorter. Optionally, FX500 cell sorter or Miltenyi Tyto cell sorter. In some of any of the provided embodiments, the selection is by 1 run, 2 runs, 3 runs or 4 runs by the fluorescence based cell sorter. In some of any of the provided embodiments, the selection is performed at rate between 10,000 and 100,000 cells/ second using a florescent based disposable fluidics cell sorter.

In some of any of the provided embodiments, the culturing for expansion is for 7 to 35 days. In some of any of the provided embodiments, the culturing for expansion is 7 to 21 days, optionally 7 to 14 days. In some of any of the provided embodiments, the culturing is carried out in a closed system. In some of any of the provided embodiments, the culturing for the first expansion is for 7 to 21 days, optionally 7 to 14 days.

In some of any of the provided embodiments, the culturing for the first expansion is carried out in a closed system using a gas permeable culture vessel. In some of any of the provided embodiments, the culturing for the first expansion is carried out in a closed system using a bioreactor.. In some of any of the provided embodiments, the performing the first expansion is carried out in a closed system using a bioreactor. In some of any of the provided embodiments, the culturing for the second expansion is for 7 to 21 days, optionally 7 to 14 days. In some of any of the provided embodiments, the culturing for the second expansion is performed in a gas permeable culture vessel. In some of any of the provided embodiments, the culturing for the second expansion is performed using a bioreactor. In some of any of the provided embodiments, the performing for the second expansion is performed in a gas permeable culture vessel. In some of any of the provided embodiments, the performing for the second expansion is performed using a bioreactor

In some of any of the provided embodiments, the tumor is a tumor of an epithelial cancer. In some of any of the provided embodiments, the tumor is a tumor of a melanoma, lung squamous, lung adenocarcinoma, bladder cancer, lung small cell cancer, esophageal cancer, colorectal cancer (CRC), cervical cancer, head and neck cancer, stomach cancer or uterine cancer. In some of any of the provided embodiments, the tumor is a melanoma. In some of any of the provided embodiments, the tumor is a colorectal cancer (CRC). In some of any of the provided embodiments, the tumor is a tumor of a non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.

In some of any of the provided embodiments, the method results in a fold-expansion of T cells or in a fold-expansion of tumor reactive T cells from the input sample that is at least at or about 2-fold, at least at or about 5-fold, at least at or about 10-fold, at least at or about 25-fold, at least at or about 50-fold, at least at or about 100-fold, at least at or about 250-fold, at least at or about 500-fold, at least at or about 750-fold, at least at or about 1000-fold, at least at or about 1500-fold, at least at or about 2000-fold, at least at or about 2500-fold, or at least at or about 3000-fold.

In some of any of the provided embodiments, the composition of tumor reactive cells produced by the method are able to produce IFNgamma at a concentration of greater than at or about 30 pg/mL, optionally greater than at or about 60 pg/mL, following antigen-specific stimulation.

In some of any of the provided methods, further comprising harvesting cells produced by the method for formulation as the therapeutic composition. In some of any of the provided methods, the method comprising formulating the harvested cells with a cryoprotectant.

Provided herein is a composition comprising tumor-reactive T cells produced by any of the provided methods. In some of any of the provided embodiments, wherein the composition comprises a cryoprotectant.

In some of any of the provided embodiments, the T cells are CD3+ T cells or comprise CD4+ T cells and/or CD8+ T cells. In some of any of the provided embodiments, the T cells comprise CD4+ T cells and CD8+ T cells, wherein the ratio of CD8+ T cells to CD4+ T cells is between at or about 1:100 and at or about 100:1, between at or about 1:50 and at or about 50:1, between at or about 1:25 and at or about 25:1, between at or about 1:10 and at or about 10:1, between at or about 1:5 and at or about 5:1, or between at or about 1:2.5 and at or about 2.5:1.

In some of any of the provided embodiments, the number of tumor reactive T cells, or of viable cells thereof, in the composition is between at or about 0.5 x 10⁸ and at or about 50 x 10⁹, between at or about 0.5 x 10⁸ and at or about 30 x 10⁹, between 0.5 x 10⁸ and at or about 12 x 10⁹, between at or about 0.5 x 10⁸ and at or about 60 x 10⁸, between at or about 0.5 x 10⁸ and at or about 15 x 10⁸, between at or about 0.5 x 10⁸ and at or about 8 x 10⁸, between at or about 0.5 x 10⁸ and at or about 3.5x 10⁸, between at or about 0.5 x 10⁸ and at or about 1 x 10⁸, between 1 x 10⁸ and at or about 50 x 10⁹, between at or about 1 x 10⁸ and at or about 30 x 10⁹, between 1 x 10⁸ and at or about 12 x 10⁹, between at or about 1 x 10⁸ and at or about 60 x 10⁸, between at or about 1 x 10⁸ and at or about 15 x 10⁸, between at or about 1 x 10⁸ and at or about 8 x 10⁸, between at or about 1 x 10⁸ and at or about 3.5x 10⁸, between at or about 3.5 x 10⁸ and at or about 50 x 10⁹, between at or about 3.5 x 10⁸ and at or about 30 x 10⁹, between at or about 3.5 x 10⁸ and at or about 12 x 10⁹, between at or about 3.5 x 10⁸ and at or about 60 x 10⁸, between at or about 3.5 x 10⁸ and at or about 15 x 108, between at or about 3.5 x 10⁸ and at or about 8 x 10⁸, between at or about 8 x 10⁸ and at or about 50 x 10⁹, between at or about 8 x 10⁸ and at or about 30 x 10⁹, between at or about 8 x 10⁸ and at or about 12 x 10⁹, between at or about 8 x 10⁸ and at or about 60 x 108, between at or about 8 x 10⁸ and at or about 15 x 10⁸, between at or about 15 x 10⁸ and at or about 50 x 10⁹, between at or about 15 x 10⁸ and at or about 30 x 10⁹, between at or about 15 x 10⁸ and at or about 12 x 10⁹, between at or about 15 x 10⁸ and at or about 60 x 10⁸, between at or about 60 x 10⁸ and at or about 50 x 10⁹, between at or about 60 x 10⁸ and at or about 30 x 10⁹, between at or about 60 x 10⁸ and at or about 12 x 10⁹, between at or about 12 x 10⁹ and at or about 50 x 10⁹, between at or about 12 x 109 and at or about 30 x 10⁹, or between at or about 30 x 10⁹ and at or about 60 x 10⁹, each inclusive.

In some of any of the provided embodiments, the number of tumor reactive T cells, or of viable cells thereof, in the composition is at least at or about 5 x 10⁸. In some of any of the provided embodiments, the number of tumor reactive T cells, or of viable cells thereof, in the composition is at least at or about 1 x 10⁹. In some of any of the provided embodiments, the number of tumor reactive T cells, or of viable cells thereof, in the composition is at least at or about 10 x 10⁹.In some of any of the provided embodiments, the provided compoisitions comprise a pharmaceutically acceptable excipient. Provided herein is a method of treatment, comprising administering any of the provided compoisitions to a subject having a cancer. In some of any of the provided embodiments, the cells of the administered composition are autologous to the subject.

In some of any of the provided embodiments, the therapeutically effective dose is between 1 x 10⁸ and 10 x 10⁹ T cells or viable cells thereof. In some of any of the provided embodiments, the therapeutically effective dose is between 5 x 10⁸ and 10 x 10⁹ T cells or viable cells thereof. In some of any of the provided embodiments, the therapeutically effective dose is between 5 x 10⁸ and 1 x 10⁹ T cells or viable cells thereof,

In some of any of the provided embodiments, the cancer is an epithelial cancer. In some of any of the provided embodiments, the cancer is melanoma, lung squamous, lung adenocarcinoma, bladder cancer, lung small cell cancer, esophageal cancer, colorectal cancer, cervical cancer, head and neck cancer, stomach cancer or uterine cancer. In some of any of the provided embodiments, the cancer is non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.

Provided herein is a composition, for use in treating a subject having cancer. Also provided herein is use of any of the provided compositions for manufacture of a medicament for treating a subject having a cancer.

In some of any of the provided embodiments, In some of any of the provided embodiments, the cells of the administered composition are autologous to the subject.

In some of any of the provided embodiments, the therapeutically effective dose is between 1 x 10⁸ and 10 x 10⁹ T cells or viable cells thereof. In some of any of the provided embodiments, the therapeutically effective dose is between 5 x 10⁸ and 10 x 10⁹ T cells or viable cells thereof. In some of any of the provided embodiments, the therapeutically effective dose is between 5 x 10⁸ and 1 x 10⁹ T cells or viable cells thereof,

In some of any of the provided embodiments, the cancer is an epithelial cancer. In some of any of the provided embodiments, the cancer is melanoma, lung squamous, lung adenocarcinoma, bladder cancer, lung small cell cancer, esophageal cancer, colorectal cancer, cervical cancer, head and neck cancer, stomach cancer or uterine cancer. In some of any of the provided embodiments, the cancer is non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.

### Brief Description of the Drawings

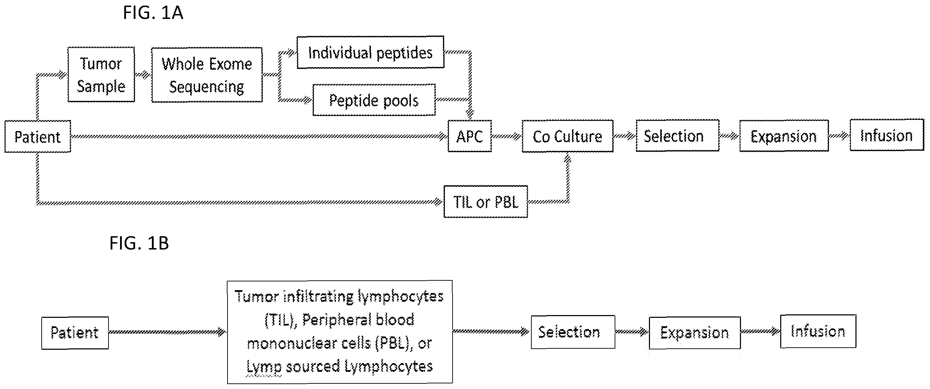

**FIG. 1A** and **FIG. 1B** are diagrams illustrating the T cell manufacturing process according to certain embodiments described herein.
**FIG. 1A** depicts a schematic of an exemplary process for manufacturing a T cell therapeutic composition in accord with the provided methods. In the exemplary process a tumor sample is obtained from a patient for identification and generation of peptides for use in co-culturing methods with autologous T cells obtained from the same subject. In some cases, a population of T cells from the patient, e.g. containing tumor infiltrating lymphocytes (TIL) or peripheral blood lymphocytes (PBL), is stimulated under conditions to expand the cells prior to co-culture with antigen presenting cells that have been contacted or exposed to peptide neoepitopes for presentation on a major histocompability complex. Following co-culture under conditions in which the antigen presenting cells present peptides in the context of a major histocompatibility complex, tumor-reactive T cells or T cells surface positive for one or more T cell activation marker (e.g. CD39, PD-1, and/or TIGIT) associated with tumor reactive T cells can be selected and cultured under conditions for expansion in accord with the provided methods, such as incubation with a T cell stimulatory agent(s) (e.g. IL-2 and/or anti-CD3/anti-CD28). In some embodiments of provided methods, the selection for tumor-reactive T cells is directly from a tumor sample, or a digested sample therefrom subjected to an initial (e.g. minimal) expansion, in which such methods do not involve a co-culture step with antigen presenting cells presenting peptide neoepiotpes. The steps can include incubation with T cell stimulating agents and/or other T cell adjuvants in accord with the provided methods. The culturing can be carried out in the presence of one or more recombinant cytokines (e.g. IL-2) to support proliferation and expansion of cells. The process can be carried out in the presence of serum-free media containing nutrients. One or more or all of the steps can be carried out in a closed system, such as without exposure of cells to the environment. Upon reaching a therapeutic dose or a threshold number of cells, the cells can be harvested and formulated, in some cases concentrated or cryopreserved, and used for administration to a subject such as by infusion.

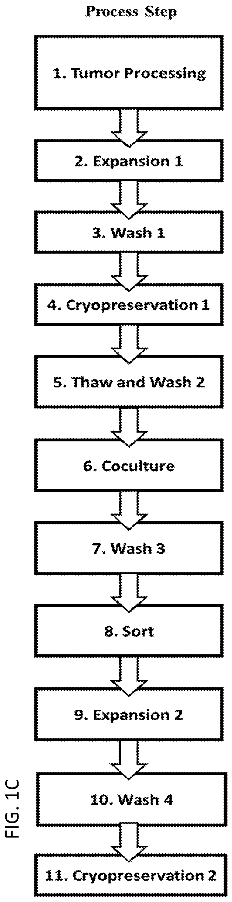

**FIG. 1B** depicts a schematic of an exemplary process for manufacturing a T cell therapeutic composition in accord with the provided methods. In the exemplary process, a biological sample containing T cells is used as a cellular source for the methods. The biological sample can include tumor infiltrating lymphocytes, peripheral blood mononuclear cells (e.g. apheresis), or lymph sourced lymphocytes. Tumor-reactive T cells or T cells surface positive for one or more T cell activation marker (e.g. CD39, PD-1, and/or TIGIT) associated with tumor reactive T cells can be selected directly from the sample and cultured under conditions for expansion in accord with the provided methods, including incubation with a T cell stimulating agent and/or T cell adjuvant in accord with the provided methods. The culturing can be carried out in the presence of one or more recombinant cytokines (e.g. IL-2) to support proliferation and expansion of cells. The process can be carried out in the presence of serum-free media containing nutrients. One or more or all of the steps can be carried out in a closed system, such as without exposure of cells to the environment. Upon reaching a therapeutic dose or a threshold number of cells, the cells can be harvested and formulated, in some cases concentrated or cryopreserved, and used for administration to a subject such as by infusion. **FIG. 1C** depicts a full process flow chart for the generation of a population of patient specific tumor-derived infiltrating T cells.

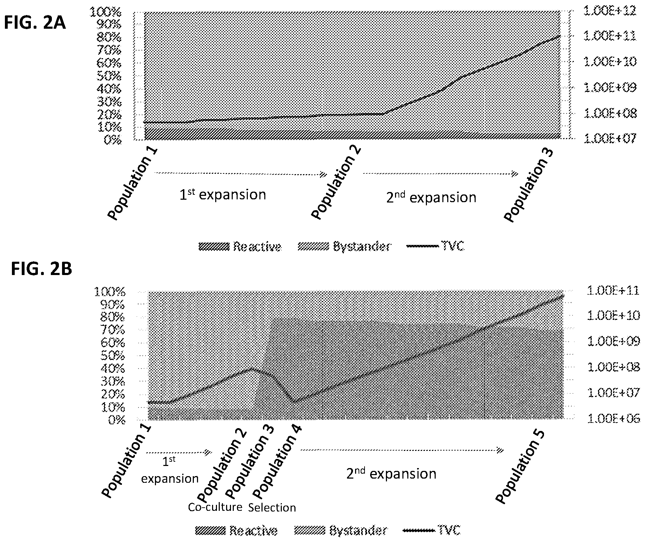

**FIG. 2A** depicts exemplary kinetics and T cell neoantigen reactivity in a typical TIL expansion process involving a bulk expansion of T cells with a first initial expansion and a second rapid expansion wherein reactivity remains low throughout the process, including within the final product. **FIG. 2B** further depicts the exemplary kinetics of a TIL expansion process as provided herein involving a first initial expansion, followed by an enrichment of tumor-reactive T cells by co-culture with neoantigen peptide-presenting antigen presenting cells, selection of tumor-reactive cells for T cell activation (upregulation) markers, and a second expansion of enriched reactive cells.

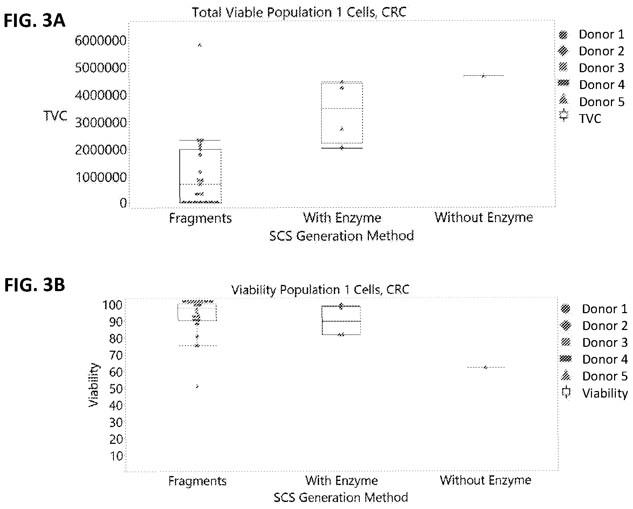

**FIG. 3A** depicts the generation of total viable Population 1 cells from patient derived CRC tumor tissue using fragment culture, homogenization with enzyme, and homogenization without enzyme. Digestion with and without enzyme both yielded more total cells than culture from fragments. Percent viability of these cells is shown in **FIG. 3B****.** Viabilities of cultures generated from fragments and digested with enzyme were higher than those derived using homogenization without enzyme.

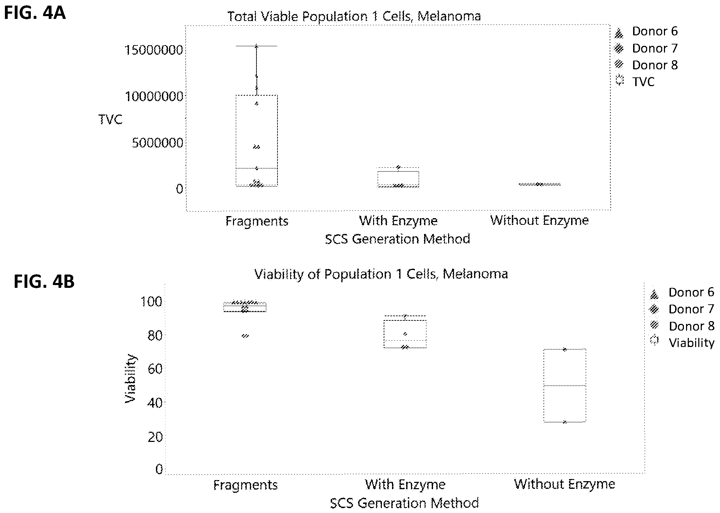

**FIG. 4A** depicts the generation of Population 1 cells from patient derived melanoma tumor tissue using fragment culture or homogenization with or without enzyme. Fragment culture yielded more total cells than cultures initiated from single cell suspensions. Percent viability of these cells is shown in **FIG. 4B****.** The population generated from fragments showed higher viability than cells from single cell suspensions.

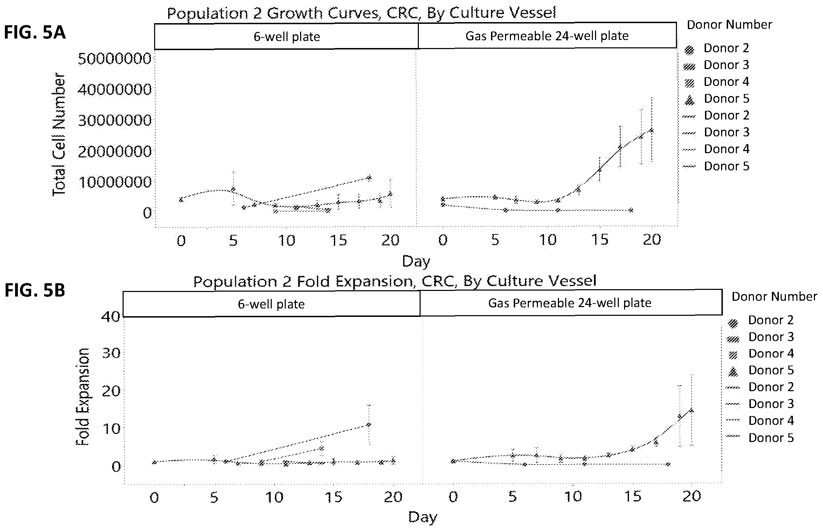

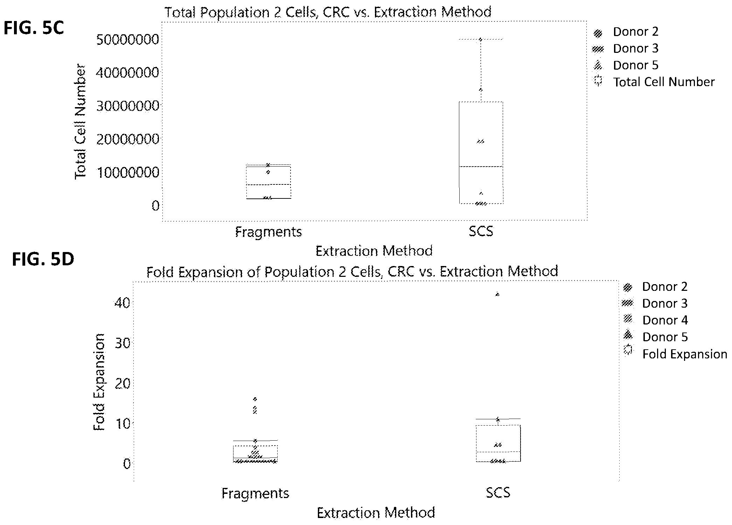

**FIG. 5** depicts growth curves **(****FIG. 5A****)** as well as fold expansion **(****FIG. 5B****)** of Population 2 cells derived from primary CRC tumors in either a conventional 6-well culture plate or a 24-well gas permeable culture plate. **FIG. 5** also depicts total cell number **(****FIG. 5C****)** as well as fold expansion **(****FIG. 5D****)** of Population 2 cells derived from primary CRC tumors contrasted by cellular extraction method, either fragment or single cell suspension culture.

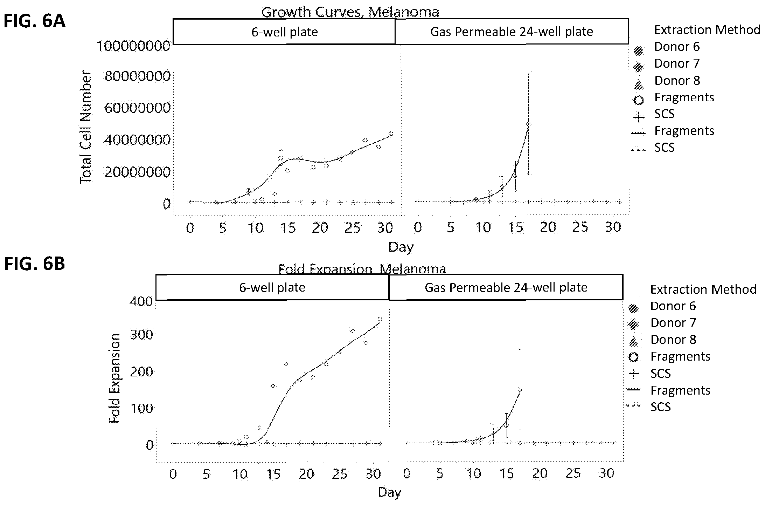

**FIG. 6** depicts growth curves **(****FIG. 6A****)** as well as fold expansion **(****FIG. 6B****)** of Population 2 cells derived from primary melanoma tumors in either a 6-well culture plate or a 24-well gas permeable culture plate.

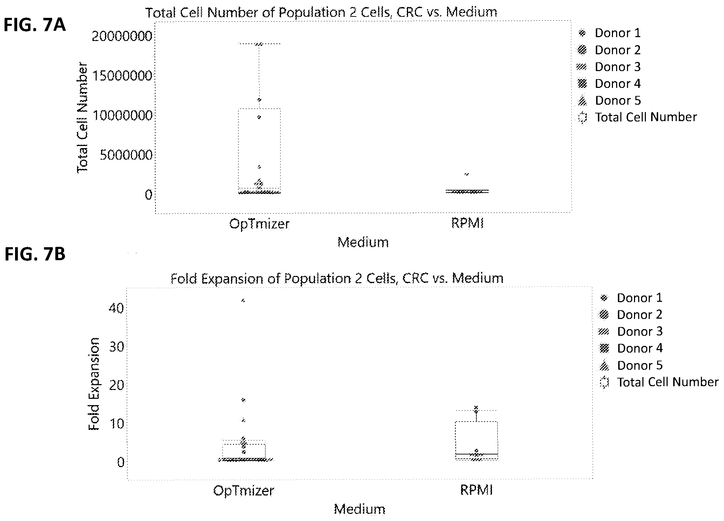

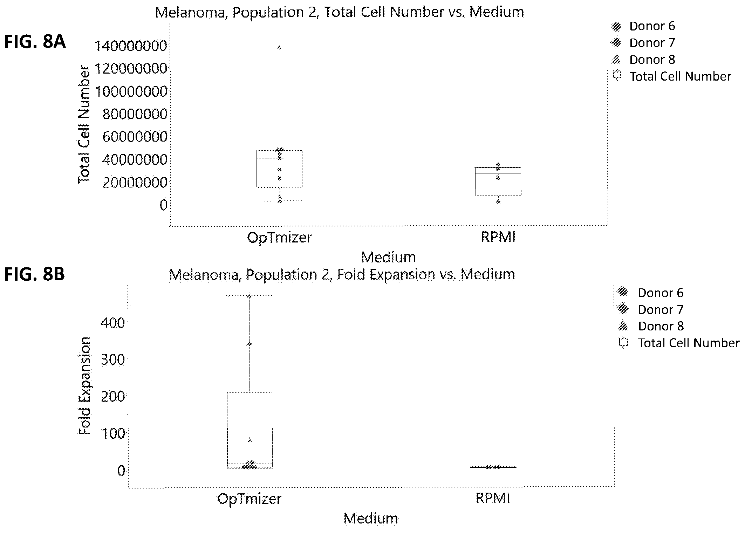

**FIG. 7** depicts total cell number **(****FIG. 7A****)** as well as fold expansion **(****FIG. 7B****)** of Population 2 cells derived from primary CRC tumors using serum free OpTmizer or RPMI media supplemented with 5% human serum. Similarly, **FIG. 8** depicts total cell number **(****FIG. 8A****)** as well as fold expansion **(****FIG. 8B****)** of Population 2 cells derived from primary melanoma tumors using serum free OpTmizer or RPMI media supplemented with 5% human serum.

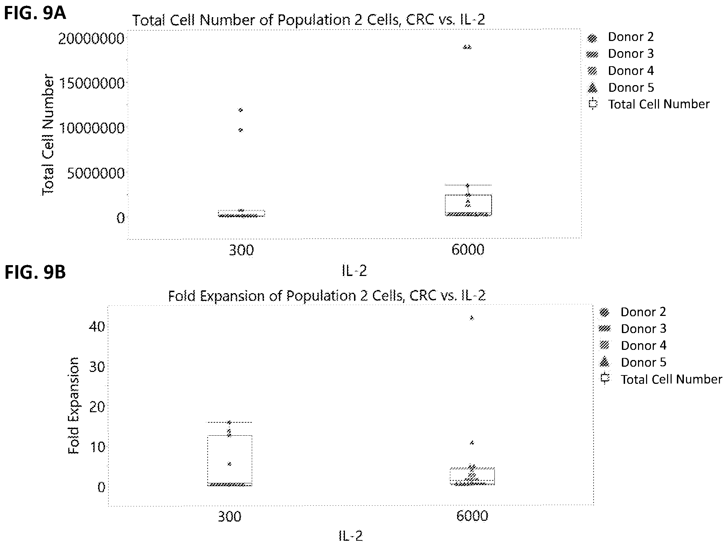

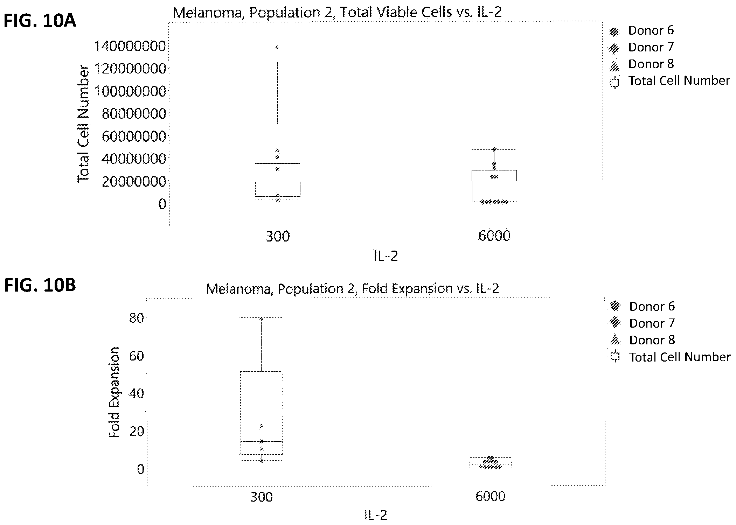

**FIG. 9** depicts total cell number **(****FIG. 9A****)** as well as fold expansion **(****FIG. 9B****)** of Population 2 cells derived from CRC tumors and cultured in media supplemented with either a low concentration (300 IU/mL) or a high concentration (6000 IU/mL) of recombinant human IL-2. These data are similarly depicted for melanoma tumor derived cells in **FIG. 10A-B**. A high concentration of IL-2 was not observed to be necessary for cellular expansion.

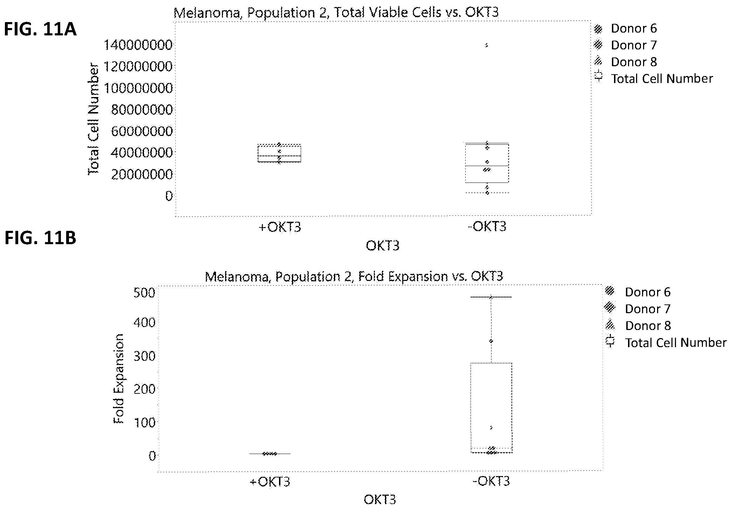

**FIG. 11A** depicts Population 2 total cell number and **FIG. 11B** depicts fold expansion from melanoma derived cell cultures that were unstimulated or stimulated with OKT3, an anti-CD3 monoclonal antibody, were observed to be largely similar.

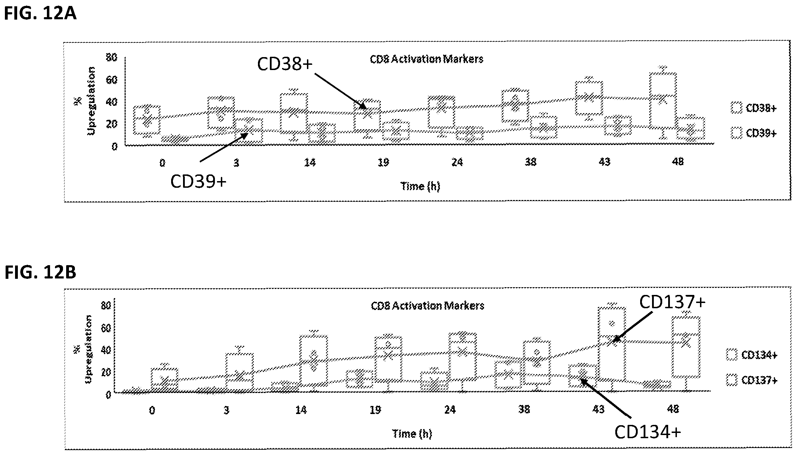

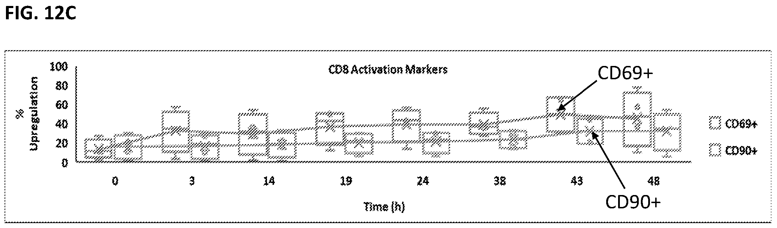

**FIG. 12A-C** depict percent upregulation of activation markers on CD8+ T cells, CD38 and CD39 **(****FIG. 12A****),** CD134 and CD137 **(****FIG. 12B****),** and CD69 and CD90 **(****FIG. 12C****),** between 0 and 48 hours after activation with OKT3.

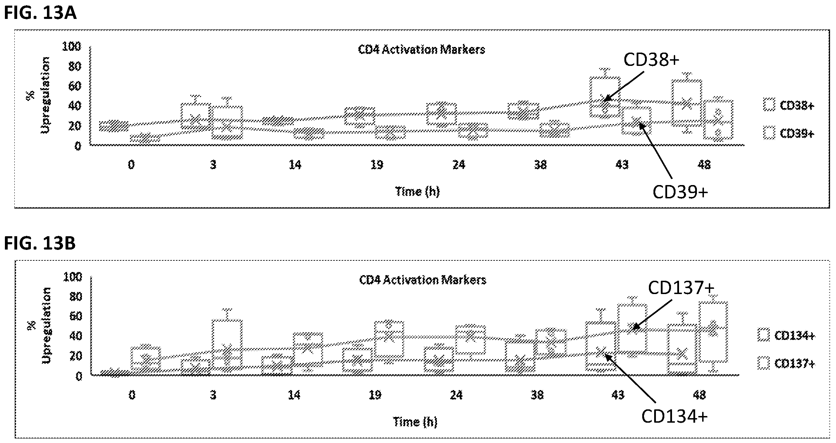

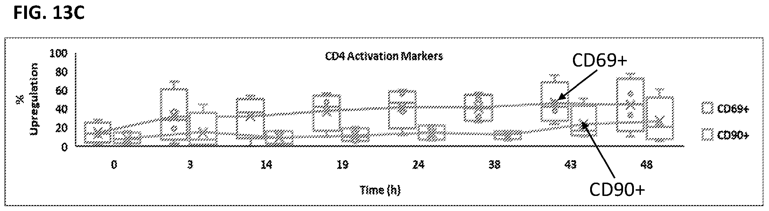

**FIG. 13A-C** depict percent upregulation of activation markers on CD4+ T cells, CD38 and CD39 **(****FIG. 13A****),** CD134 and CD137 **(****FIG. 13B****),** and CD69 and CD90 **(****FIG. 13C****),** between 0 and 48 hours after activation with OKT3.

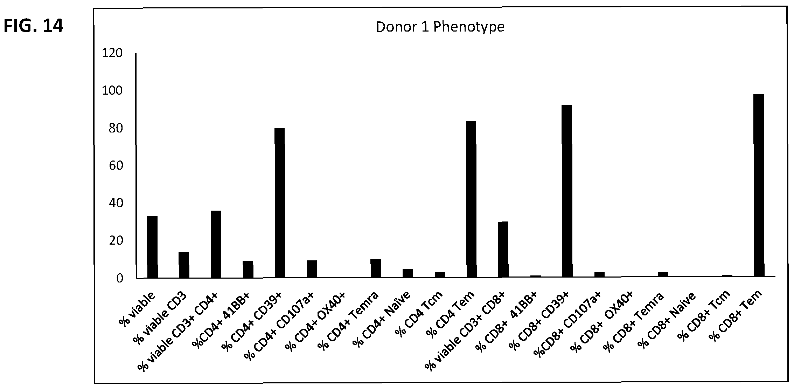

**FIG. 14** depicts expression of selected exemplary markers in a single cell suspension culture generated from a CRC tumor on Day 0.

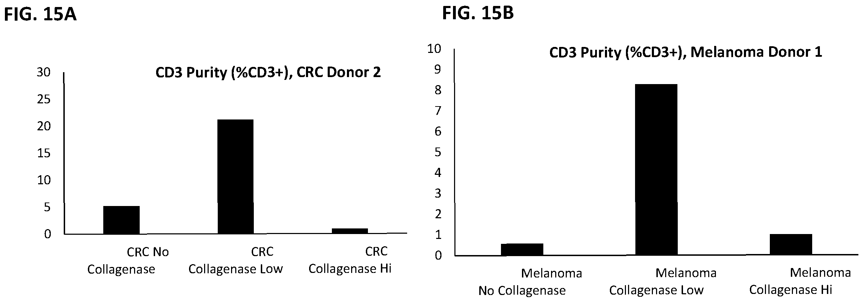

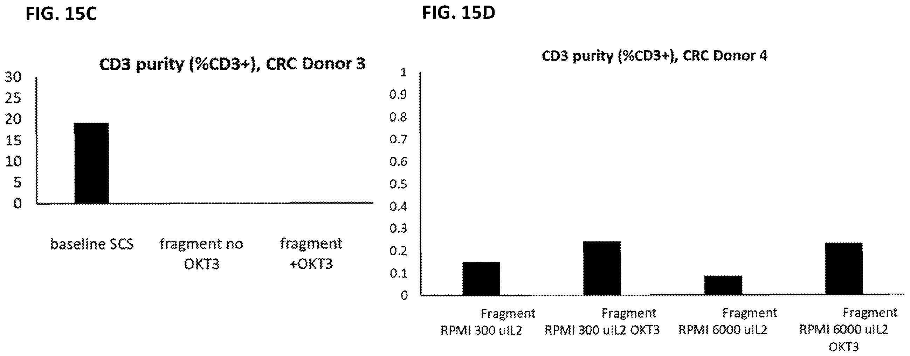

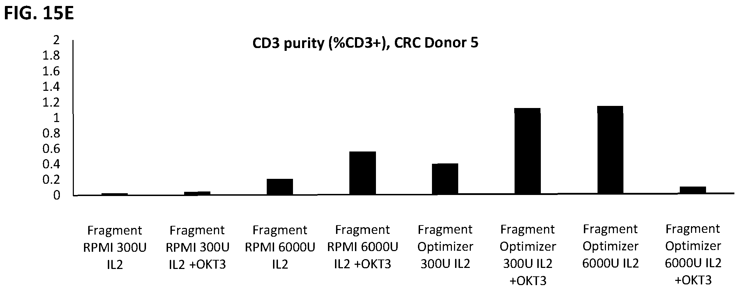

**FIG. 15A-E** depict CD3+ cell purity as a percent of Population 1 cells. **FIG. 15A** depicts the purity of cells from Day 0 SCS from a CRC tumor after homogenization without enzyme, with 1 mg/ml (low) enzyme, and 5 mg/ml (high) enzyme. These data are similarly shown for a melanoma derived culture in **FIG. 15B****.** **FIG. 15C** depicts the purity of CD3+ Population 1 cells from Day 0 (baseline SCS) and Day 6 from fragments cultured in the presence or absence of OKT3 stimulation. **FIG. 15D** shows the relative purity of CD3+ cells from a CRC donor on Day 11 using fragments cultured in media supplemented with either 6000 IU/mL (high) or 300 IU/mL (low) recombinant IL-2. **FIG. 15E** depicts Population 1 cells (Day 9) from fragments cultured in either serum free OpTmizer media or RPMI with either OKT3 stimulation and/or IL-2 at high or low concentrations. These observations support that SCSs from tumor biopsies of CRC patients may be more capable of providing a greater number of T cells for expansion than cells obtained from culture of tumor fragments.

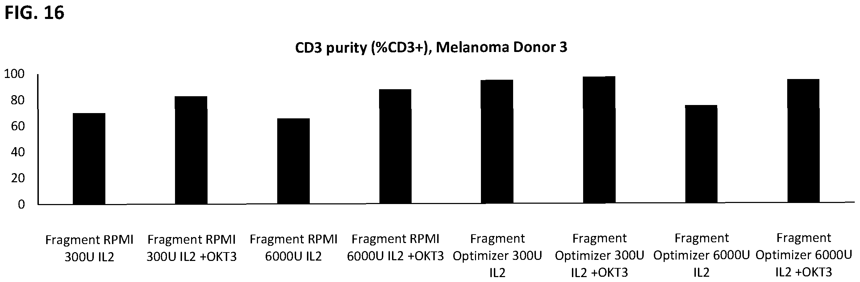

**FIG. 16** depicts the purity of CD3+ Population 1 cells derived from a melanoma patient as fragment cultures from Day 9 at high and low IL-2 concentrations and with serum containing RPMI medium or serum free OpTmizer.

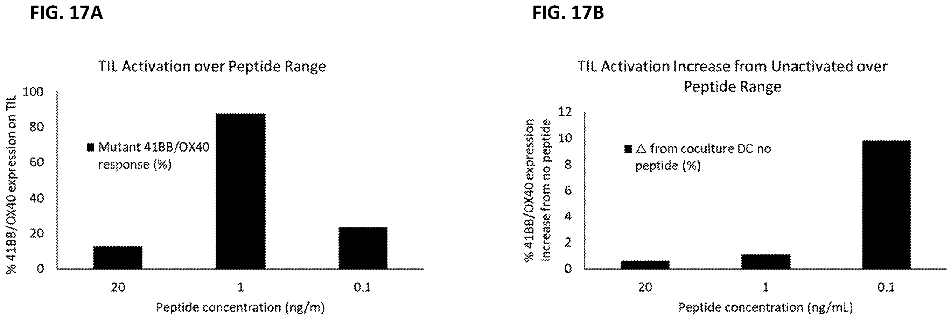

**FIG. 17A** depicts the generation of Population 3 cells following co-culture with dendritic cells loaded with peptide at concentrations from 0.1 ng/mL to 20 ng/mL. **FIG. 17B** depicts the fold increase in the same experiment from T cells which were co-cultured with unloaded dendritic cells **(****FIG. 17B****).**

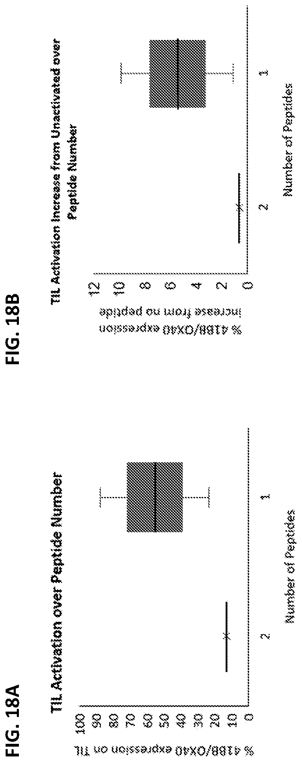

**FIG. 18A** compares stimulation with one peptide or two peptides reported as % 41BB/OX40 expression. **FIG. 18B** depicts stimulation with one peptide or two peptides reported as fold increase from T cells which were unactivated.

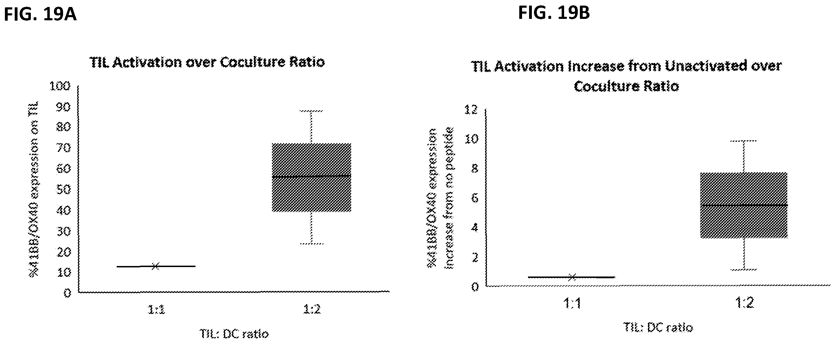

**FIG. 19A** compares two T cell to dendritic cell ratios, 1:1 and 1:2, reported as % 41BB/OX40 expression. **FIG. 19B** compares two T cell to dendritic cell ratios, 1:1 and 1:2, reported as fold increase from T cells which were unactivated.

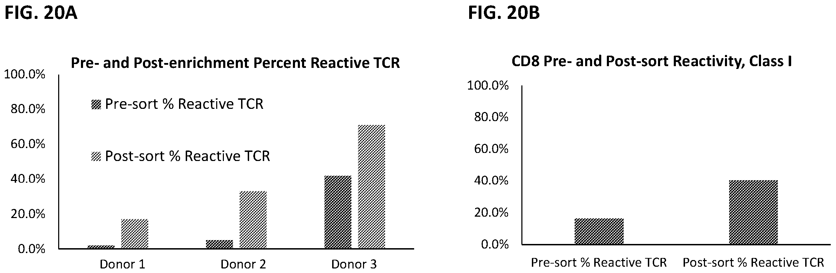

**FIG. 20A** depicts percent neoantigen reactive TCR before and after coculture with autologous neoantigen peptides and sorting of T cells sourced from the peripheral blood of three healthy donors. **FIG. 20B** depicts average class I reactivity pre- and post-coculture and sorting of CD8+ cells.

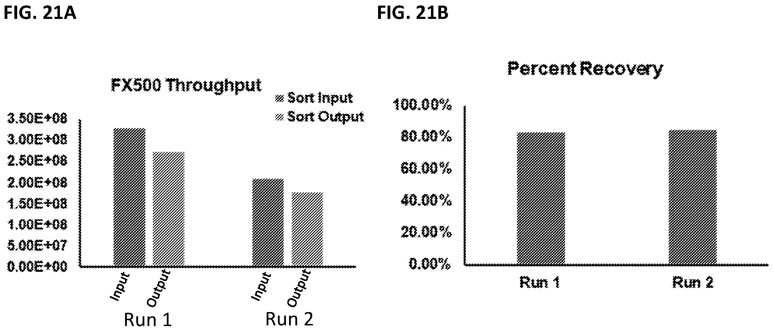

**FIG. 21A and FIG. 21B** depict recovery from cell sorting using the Sony FX500 as both total cell input and output for two independent runs **(****FIG. 21A****)** and percent recovery **(****FIG. 21B****).**

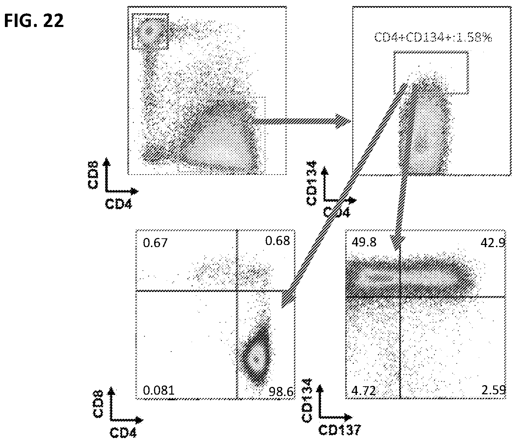

**FIG. 22** depicts purity and gating of a CD4+ population from cell sorting using Sony FX500. The results demonstrate a high recovery of cells after selection and sorting of cells positive for upregulation markers.

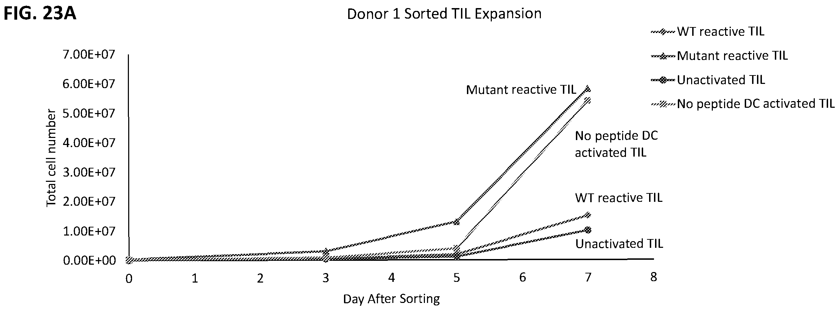

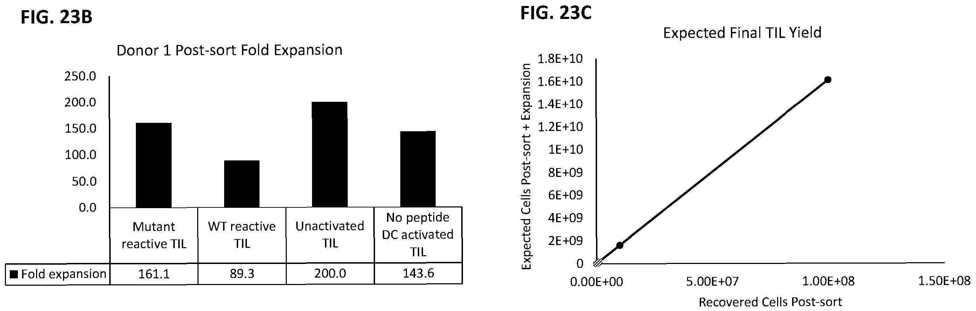

**FIG. 23A****-****FIG. 23C** depict expansion of tumor infiltrating T lymphocytes after sorting. **FIG. 23A** depicts total cell number and **FIG. 23B** depicts fold expansion, of Population 5 cells derived from Population 4 cells following co-culture with or without dendritic cells loaded with wild-type peptide, tumor associated peptide, or no peptide. Projected cell numbers after expansion of Population 4 cells into Population 5 cells at various cell recovery numbers post-sort are shown in **FIG. 23C****.**

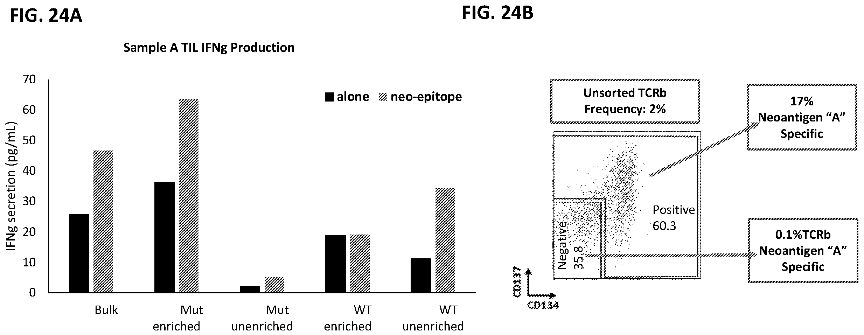

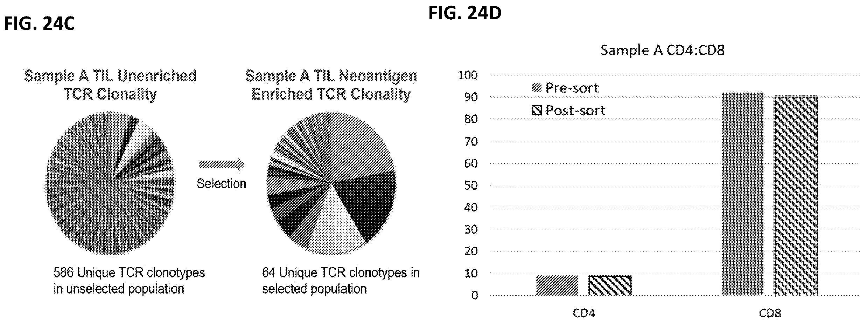

**FIG. 24A** depicts measured IFN-gamma secretion within a bulk co-culture, positive sorted (selected) population by expression of CD137 and/or CD134 from bulk co-culture cells (enriched), or negative sorted (unselected) population form bulk co-culture cells, following stimulation with mutant (mut) peptide or normal, wild-type (WT) peptide from an ovarian cancer patient. **FIG. 24B** depicts enrichment of neoantigen specific population of the tumor-reactive specific cells in the positive sort and negative sort compared to the bulk unsorted T cells. **FIG. 24C** depicts the number of TCR clonotypes present in the unselected and selected populations and demonstrates that the diversity of incoming TCRs is high in the unsorted T cell population and that there is enrichment of unique TCR clones in the selected population. **FIG. 24D** depicts the pre- and post-sort cell populations from Sample A which were observed to contain CD4+ and CD8+ cells, indicating that class I and class II reactive cells are present in the enriched population.

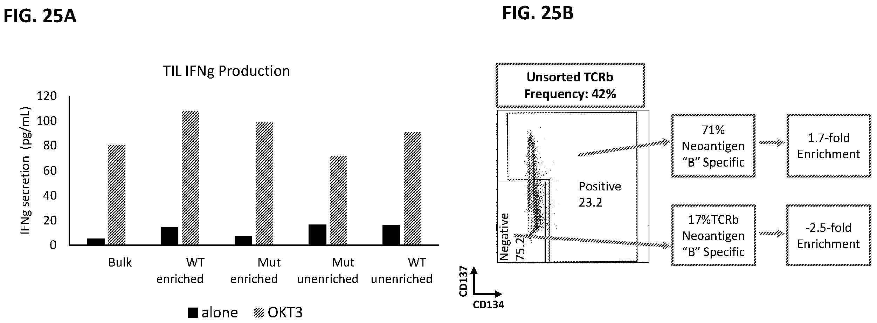

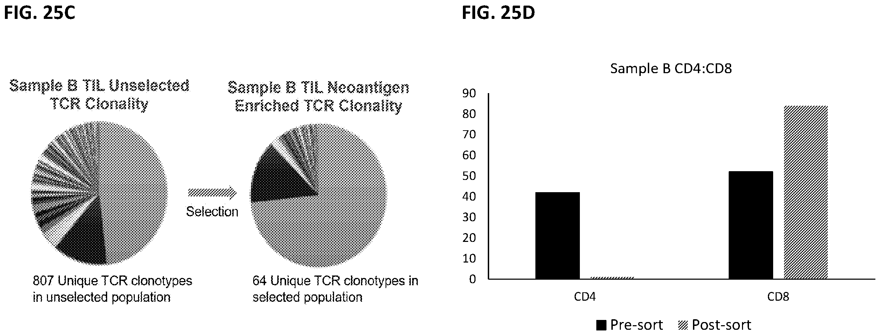

**FIG. 25A** depicts measured IFN-gamma secretion within a bulk co-culture, positive sorted (selected) population by expression of CD137 and/or CD134 from bulk co-culture cells (enriched), or negative sorted (unselected) population from bulk co-culture cells, following stimulation with anti-CD3 (OKT3) from colorectal cancer patient. **FIG. 25B** depicts enrichment of neoantigen specific population of the tumor-reactive specific cells in the positive sort and negative sort compared to the bulk unsorted T cells. **FIG. 25C** depicts the TCR clonality profile present in the unselected and selected populations. **FIG. 25D** depicts the pre- and post-sort cell populations which were observed to contain CD4+ and CD8+ cells, indicating that class I and class II reactive cells are present in the enriched population.

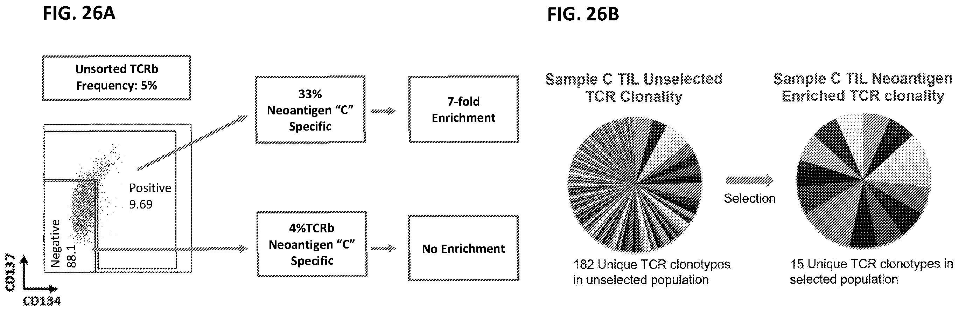

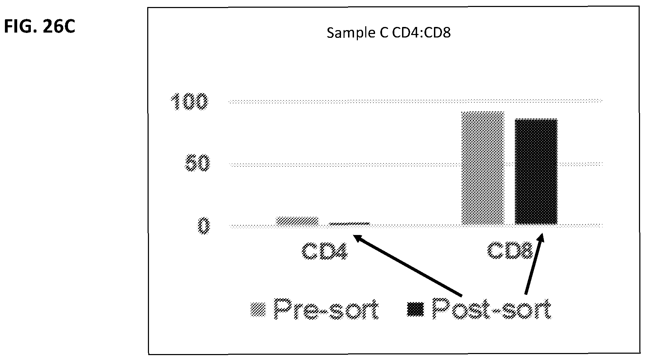

**FIG. 26A** depicts enrichment of neoantigen specific population of tumor-reactive specific cells in a bulk co-culture, positive sorted (selected) population by expression of CD137 and/or CD134 from bulk co-culture cells (enriched), or negative sorted (unselected) population fromm bulk co-culture cells. **FIG. 26B** depicts the TCR clonality profile present in the unselected and selected populations. **FIG. 26C** depicts Pre- (bulk) and post-sort cell populations, which were observed to contain both CD4+ class I reactive and CD8+ class II reactive cells.

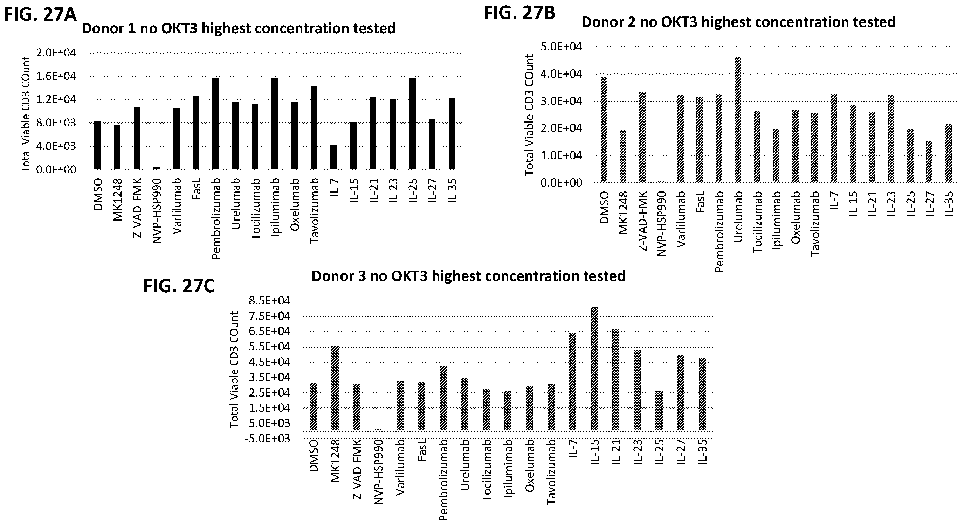

**FIGS. 27A-C** show total viable CD3+ cell count for cells grown in the presense of numerous T cell adjuvants with supplemental OKT3 stimulation. The results shown are for the following adjuvants: Tavolixizumab, Oxelumab, Ipilimumab, Tocilizumab, Urelumab, Pembrolizumab, Varlilumab, anti-GITR MK-1248, anto-human FasL at 10µg/mL; 25 µM for Z-VAD-FMK pan-caspase inhibitor; 250 nM for HSP inhibitor NVP-HSP990; and 1000 IU/mL for cytokines ((IL-7, IL-15, IL-21, IL-23, IL-25, IL-27, or IL-35).

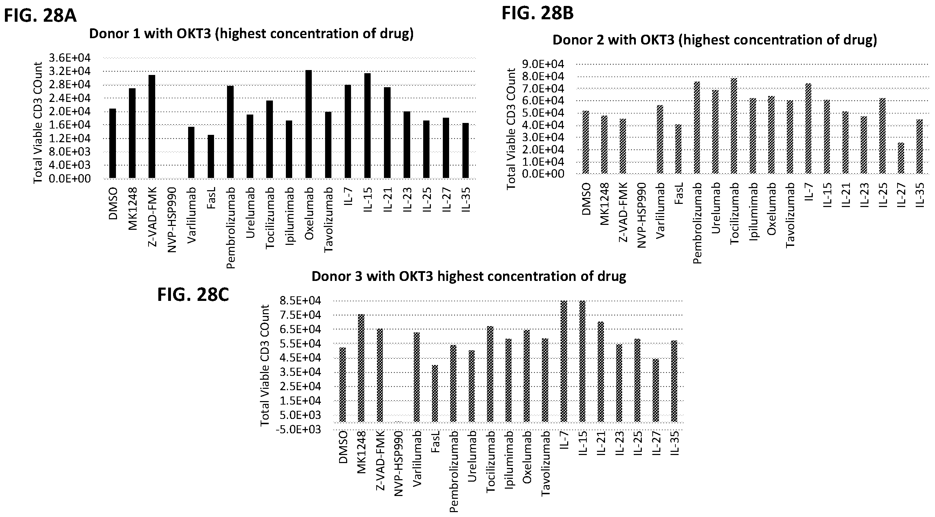

**FIGS. 28A-C** show total viable CD3+ cell count for cells grown in the presense of numerous T cell adjuvants without supplemental OKT3 stimulation. The results shown are for the following adjuvants: Tavolixizumab, Oxelumab, Ipilimumab, Tocilizumab, Urelumab, Pembrolizumab, Varlilumab, anti-GITR MK-1248, anto-human FasL at 10µg/mL; 25 µM for Z-VAD-FMK pan-caspase inhibitor; 250 nM for HSP inhibitor NVP-HSP990; and 1000 IU/mL for cytokine (IL-7, IL-15, IL-21, IL-23, IL-25, IL-27, or IL-35).

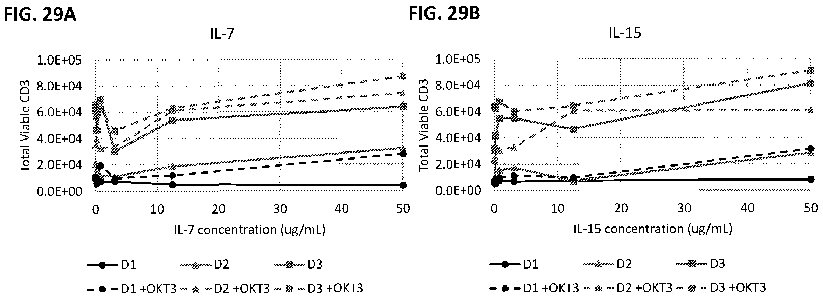

**FIG. 29** shows dose response curves for IL-7 **(****FIG. 29A****)** and IL-15 **(****FIG. 29B****).**

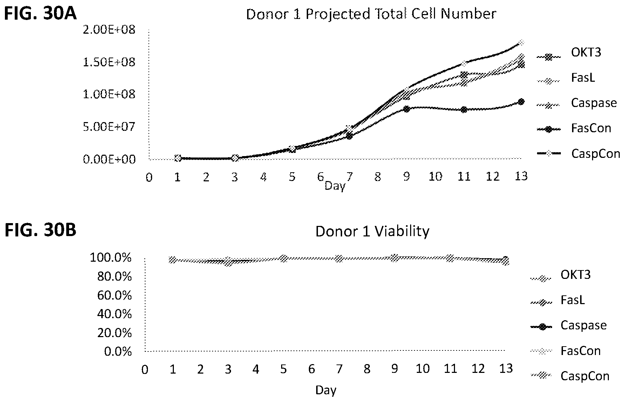

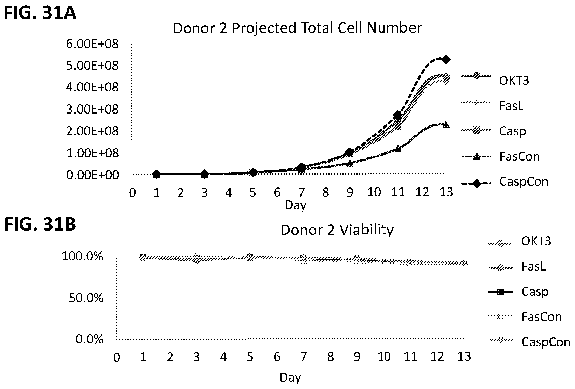

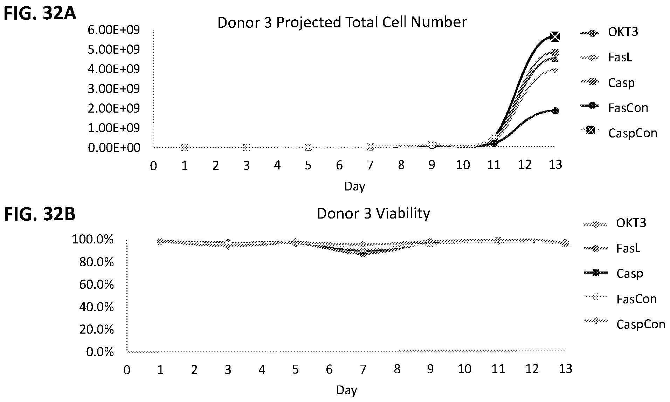

**FIG.30A-B**-**FIG.32A-B**show total cell number and and cell viability for cells derived from each of three healthy donors and grown in experimental conditions. It was observed that cells grown in the presense of continuous caspase inhibition showed superior growth despite inherent donor variability.

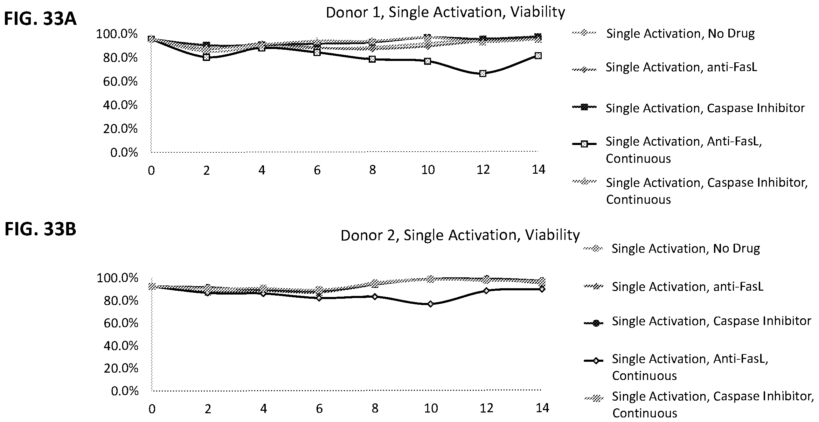

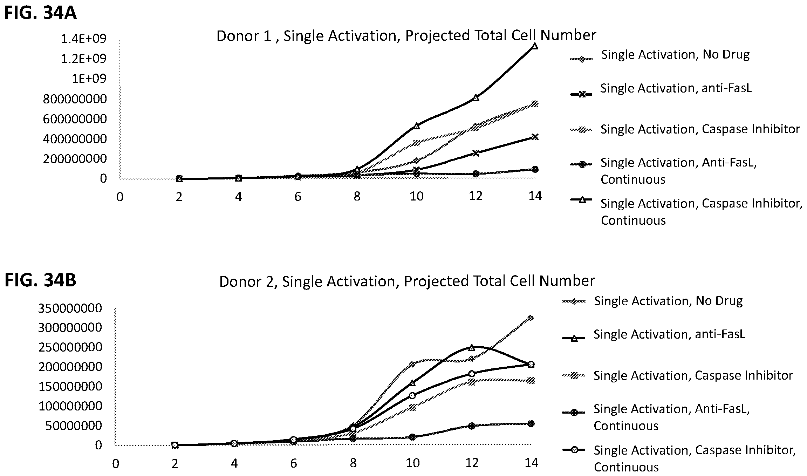

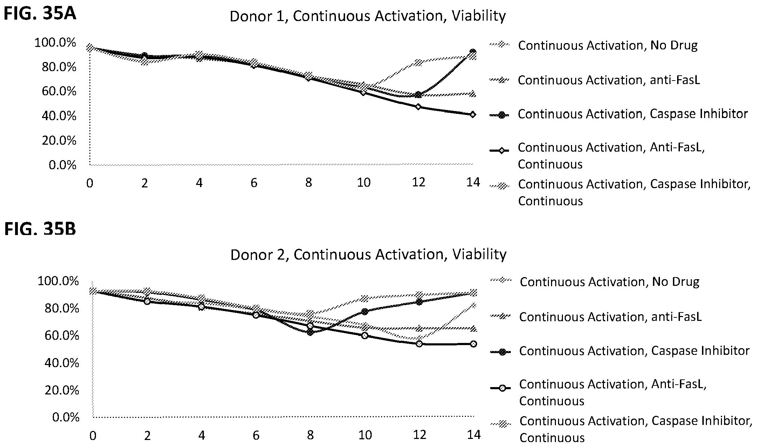

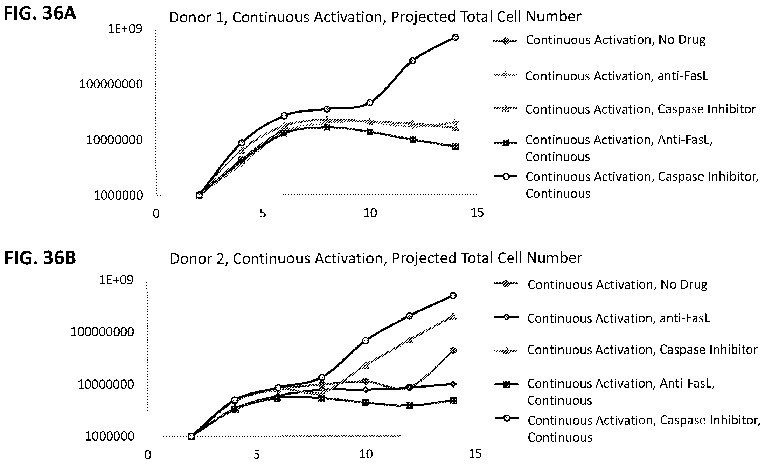

**FIGS. 33A****-B-** **FIG. 36A-B** show cell effects following continuous activation or transient activation with anti-CD3/anti-CD28 (transient activation) treatment groups for two donors. Cellular viability for a single activation with anti-CD3/anti-CD28 (transient activation) treatment groups for two donors are shown **in** **FIG. 33A-B**and total cell number for the same treatments are shown in **FIG. 34A-B**Cellular viability for the continuous activation with anti-CD3/anti-CD28 treatment groups for two donors are shown in **FIG 35A-B** and total cell number for the same treatments are shown in **FIG. 36A-B**

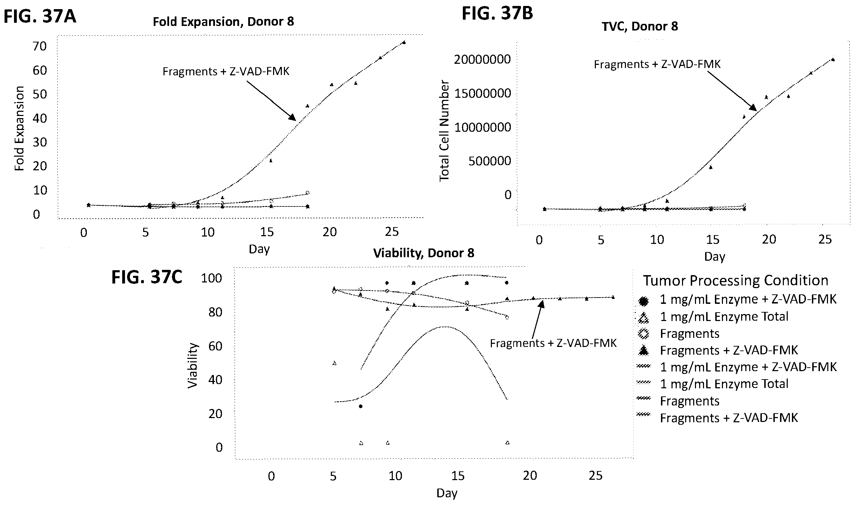

**FIG. 37A-C**shows the fold expansion (**FIG. 37A**), total viable cells (**FIG. 37B**) and percent viability (**FIG. 37C**), of both SCS and tumor fragment derived cultures grown in the presence or absence of pan-caspase inhibitor Z-VAD-FMK.

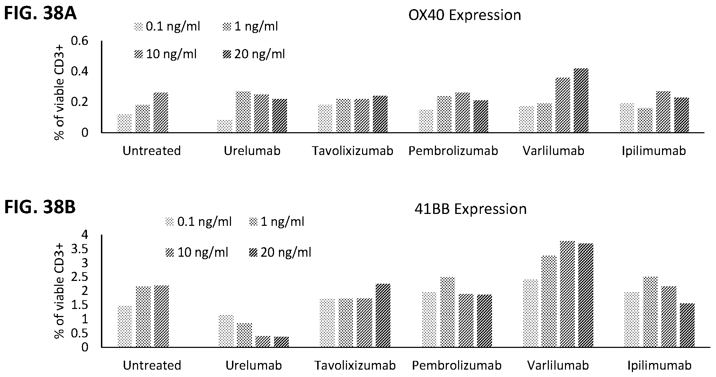

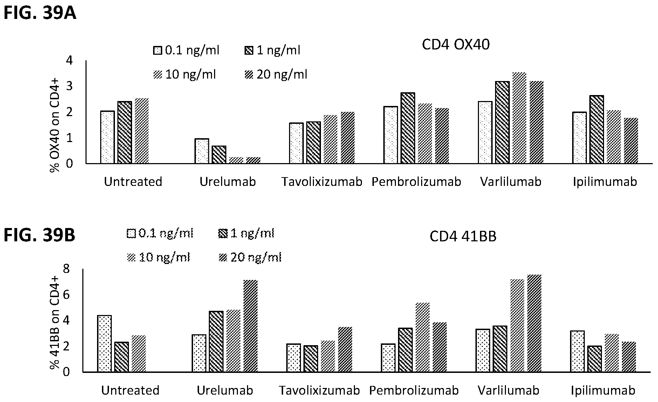

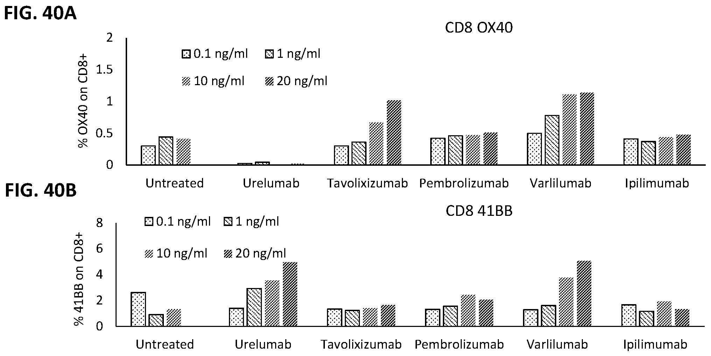

**FIGS. 38A-D** **-** **FIGS. 40A-D**show T cell phenotype of T cells following incubation with various T cell adjuvants. T cell phenotype is shown for CD3+ **(****FIG. 38A-D**), CD4+ (**FIG. 39A-D**) and CD8+ (**FIG. 40A-D**) cells grown in the presense of Ipilimumab (anti-CTLA4), Pembrolizumab (anti-PD1), Tavolixizumab (anti-TNFRSF4), Urelumab (anti-CD137), and Varlilumab (anti-CD27) at varying concentrations.

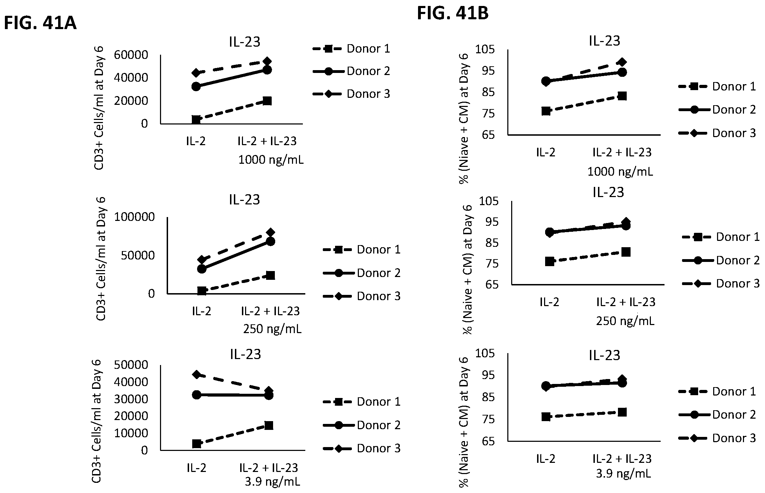

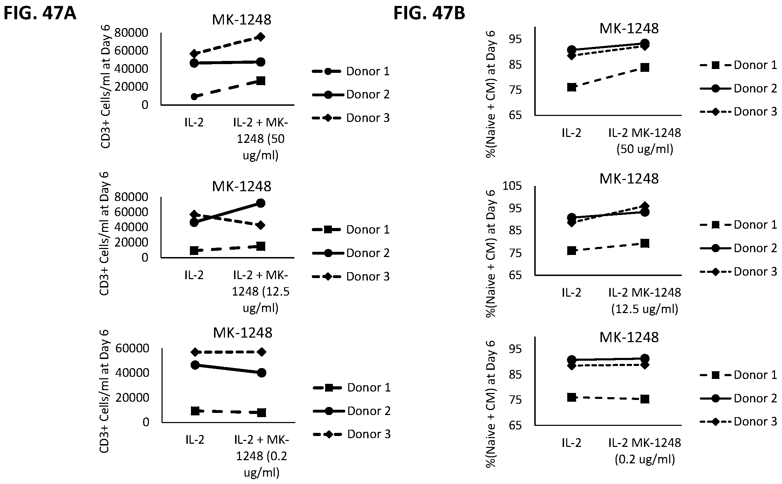

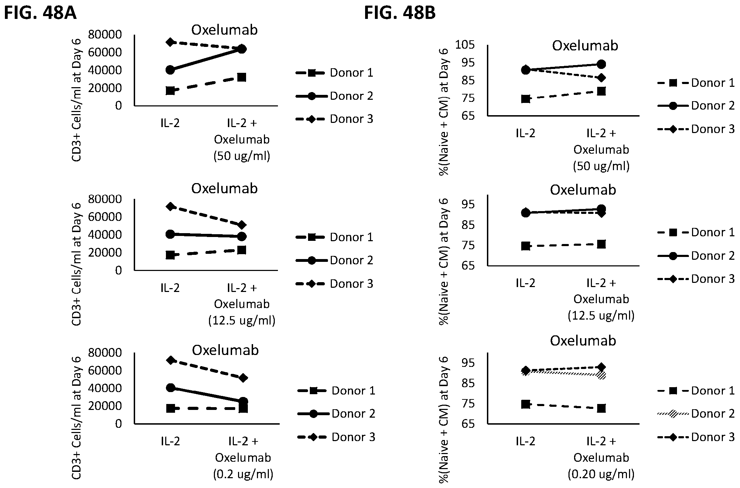

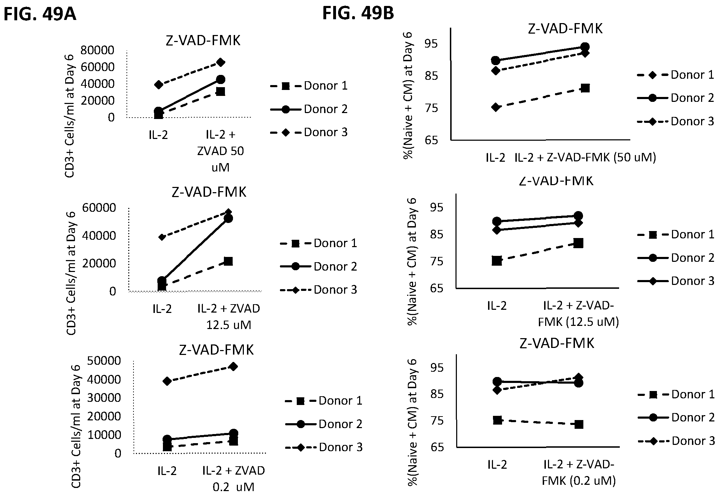

FIG. 41A-FIG.49A show total viable CD3+ cell count for cells grown in the presence of IL-2 with additional modulatory cytokines or other T cell adjuvant. The results shown are for the following adjuvants at three concentrations: Oxelumab **(****FIG. 48A****),** anti-GITR MK-1248 (**FIG. 47A**), Z-VAD-FMK pan-caspase inhibitor (**FIG. 49A**); and for cytokines IL-23, IL-21, IL-35, IL-27, IL-15, **IL-7(****FIG. 41A****,** **42A**, **43A**, **44A**, **45A**, and **46A**).
**FIG. 41B-FIG.49B** depict T cell phenotype as a function of naive and central memory cell populations in cells grown in the presence of three concentrations of Oxelumab (**FIG. 48B**), anti-GITR MK-1248 **(****FIG. 47B****),** Z-VAD-FMK pan-caspase inhibitor **(****FIG. 49B****);** and for cytokines IL-23, IL-21, IL-35, IL-27, IL-15, IL-7 **(****FIG. 41B****,** **42B****,** **43B****,** **44B****,** **45B****, and** **46B****).**

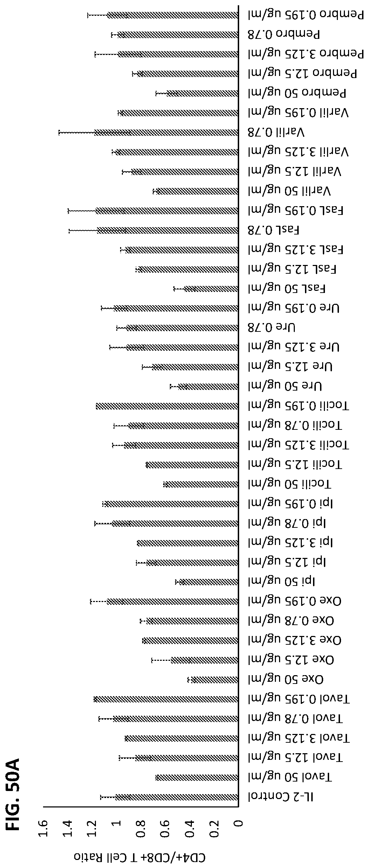

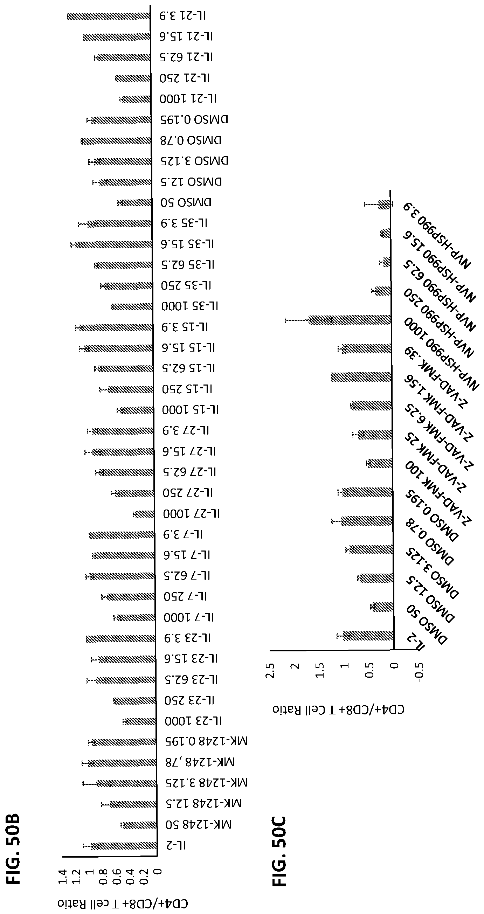

**FIG. 50A-50C** shows CD4+/CD8+ cell ratio as assessed at the end of the culture period by flow cytometry following culture of cells from a representative healthy donor grown in the presence of IL-2 with additional modulatory cytokines or other T cell adjuvant. None of the tested antibodies **(****FIG. 50A****),** cytokines **(****FIG. 50B****),** nor other modulators **(****FIG. 50C****),** substantially altered the CD4+/CD8+ T cell ratio from that which was observed with IL-2 alone.

### Detailed Description

Provided herein are method for manufacturing T cells. Such methods include, but are not limited to the steps of (1) selecting, from a population of cells containing T lymphocytes obtained from a donor subject, cells positive for Chemokine (C-X-C motif) ligand 13 (CXCL13) and/or positive for an exhaustion marker from among PD-1, CD39 and/or TIGIT; and (2) stimulating the population by incubation or culture of selected cells with one or more T-cell stimulating agents of lymphocytes to produce a population of expanded T cells. In some embodiments, the methods for selection and/or stimulation are performed in a closed system. In some embodiments, only a single expansion step is carried out in the method. In some embodiments, an initial expansion (e.g. first expansion) is carried out prior to selecting the cells. In some embodiments, the provided methods further can include a secondary stimulation to further expand cells in which the further stimulation is by incubation or culture with one or more T-cell stimulating agents. In some embodiments, after the first stimulation (first expansion) and prior to the second stimulation (second expansion), the method can further include: (i) co-culturing a population of T cells in the presence of antigen presenting cells that present one or more MHC-associated non-native peptide; (4) Separating antigen presenting cells from the population of T cells in a closed system, such as by selecting for T cells containing endogenous TCR that are reactive to peptides present on the APCs, such as based on upregulation markers or activation markers on T cells following their co-culture with the APCs/peptides. In some embodiments, one or more of the steps is carried out in a closed system. In some embodiments, all of the steps is carried out in a closed system.

In accordance with embodiments herein, methods or processes for manufacturing T cell preparations are provided which may be useful for treating patients with a pathological disease or condition. In contrast to known production methods, the methods and processes described herein can be completed in a significantly shorter time and recover a higher number of endogenous TCR-expressing T cells, thereby offering a significant advantage to bring cells into the clinic in therapeutic doses. Also provided herein are populations of T cells produced by methods described herein and pharmaceutical compositions thereof.

The provided methods relate to producing a T cell therapy reactive to tumor-associated antigens, such as neoantigens. Cancer cells accumulate lots of different DNA mutations as part of the tumorigenic process. These mutations can cause amino acid changes in protein coding regions. For a mutation to be recognized by the immune system the protein needs to be processed intracellularly and presented on the surface with the Major Histocompatibility Complex (MHC). Peptide neoantigens (also referred to herein as neoepitopes or peptide neoepitopes) are the mutant peptides presented by the MHC complex that can be recognized by a T-cell via TCR binding. In order for the immune system to recognize the mutation, it must be expressed on the surface of the cancer cell via the MHC complex and the T cell must have a TCR that recognizes the mutated peptide. These neoantigens may be presented by MHC class I and MHC class II, and are recognized by CD8+ and CD4+ T cells respectively.

In some embodiments, the method described may be used to manufacture T cells which express cell surface receptors. The cell surface receptor may be a T cell receptor (TCR) or novel group of TCRs. In particular embodiments of the provided methods, the population of T cells is or includes reactive T cells that express cell surface receptors, such as a T cell receptor (TCR), able to recognize peptide antigens on the surface of a target cells. Specifically, for an antigen to be recognized by the immune system the protein needs to be processed intracellularly to peptide fragments that are then presented on the surface with the Major Histocompatibility Complex (MHC). A TCR has two protein chains, which are designed to bind with specific peptides presented by a major histocompatibility complex (MHC) protein on the surface of certain cells. Since TCRs recognize peptides in the context of MHC molecules expressed on the surface of a target cell, TCRs have the potential to recognize antigens not only presented directly on the surface of target cells, e.g. cancer cells, but also presented by antigen-presenting cells, such as are present in tumor, inflammatory and infected microenvironments, and in secondary lymphoid organs. Reactive T cells expressing such cell surface receptors may be used to target and kill any target cell, including, but not limited to, infected cells, damaged cells, or dysfunctional cells. Thus, according to the embodiments described herein, the manufactured T cells expressing the cell surface receptor may be used to target and kill any target cell, including, but not limited to, infected cells, damaged cells, or dysfunctional cells. Examples of such target cells may include cancer cells, virally infected cells, bacterially infected cells, dysfunctionally activated inflammatory cells (e.g., inflammatory endothelial cells), and cells involved in dysfunctional immune reactions (e.g., cells involved in autoimmune diseases).

In some embodiments, a "T cell receptor" or "TCR" is a molecule that contains a variable α and β chains (also known as TCRα and TCRβ, respectively) or a variable γ and δ chains (also known as TCRy and TCRδ, respectively), or antigen-binding portions thereof, and which is capable of specifically binding to a peptide bound to an MHC molecule. In some embodiments, the TCR is in the αβ form. Typically, TCRs that exist in αβ and γδ forms are generally structurally similar, but T cells expressing them may have distinct anatomical locations or functions. A TCR can be found on the surface of a T cells (or T lymphocytes) where it is generally responsible for recognizing antigens bound to major histocompatibility complex (MHC) molecules.

In some aspects, the reactive T cells are tumor-reactive T cells that recognize a cancer neoantigen. The majority of neoantigens arise from passenger mutations, meaning they do not infer any growth advantage to the cancer cell. A smaller number of mutations actively promote tumor growth, these are known as driver mutations. Passenger mutations are likely to give rise to neoantigens that are unique to each patient and may be present in a subset of all cancer cells. Driver mutations give rise to neoantigens that are likely to be present in all the tumor cells of an individual and potentially shared. In some embodiments of the provided method, the population of T cells contain tumor-reactive T cells that can recognize neoantigens containing passenger and/or driver mutations.

In particular aspects, the provided methods can be used for the *ex vivo* production of a T cell therapy, including for the *ex vivo* expansion of autologous tumor-reactive T cells. In some aspects, neoantigens are ideal targets for immunotherapies because they represent disease-specific targets. For example, such antigens generally are not present in the body before the cancer developed and are truly cancer specific, not expressed on normal cells and are not subjected to off target immune toxicity. Thus, the unique repertoire of neoantigens specific to the patient can elicit a strong immune response specific to the cancer cells, avoiding normal cells. This is an advantage over other cell therapy targets that may not be disease-specific targets, since even low levels of target antigen on normal cells can lead to severe fatal autoimmune toxicity in the context of engineered therapies that target common antigens. For example an anti MAGE-A3-TCR program in melanoma patients was halted due to study related deaths attributed to cross reactivity with a similar target MAGE-A12, which is expressed at a low level in the brain. A significant challenge in cancer immunotherapy has been the identification of cancer targets.

Recent clinical studies have demonstrated that T cells isolated from surgically resected tumors possess TCRs that recognize neoantigens, and expanding these neoantigen reactive TIL populations and re-infusing them into the patient can in some cases result in a dramatic clinical benefit. This personalized therapy has generated remarkable clinical responses in certain patients with common epithelial tumors.

Existing methods for obtaining and generating tumor-reactive T cells are not entirely satisfactory. For example, direct isolation of tumor-reactive T cells from a subject without expansion is not feasible because therapeutically effective amounts of such cells cannot be obtained. As an alternative, attempts have been made to identify TCRs specific to a desired neoantigen for recombinant engineering of the TCR into T cells for use in adoptive cell therapy methods. Such approaches, however, produce only a single TCR against a specific neoantigen and thereby lack diversity to recognize a broader repertoire of multiple tumor-specific mutations. Other methods involve bulk expansion of T cells from a tumor source, which has the risk of expanding T cells that are not reactive to a tumor antigen and/or that may include a number of bystander cells that could exhibit inhibitory activity. For example, tumor regulatory T cells (Tregs) are a subpopulation of CD4+ T cells, which specialize in suppressing immune responses and could limit reactivity of a T cell product. These further approaches that have sought to expand tumor-reactive T cells *ex vivo* are not selective such that non-reactive T cells in the culture may preferentially expand over reactive T cells resulting in a final product that lacks satisfactory reactivity and/or in which the number of tumor-reactive T cells remains insufficient. Methods to produce tumor-reactive T cells for therapy are needed.

The provided embodiments relate to improved methods for identifying and expanding T cells *ex vivo,* including tumor-reactive T cells, for use in T cell therapy. In some embodiments, the provided methods improve or increase the growth and survival of T cells, such as tumor-reactive T cells, outside of the body. In particular embodiments, the methods enrich for expansion of reactive T cells compared to non-reactive T cells and promote their survival and growth in culture *ex vivo.* In some embodiments, the resulting methods can be carried out in a closed system. The methods in some embodiments are carried out in an automated or partially automated fashion.

The provided methods result in an enriched population of T cells reactive to patient specific mutations, such as based on selection of upregulation markers after presentation of mutant antigens and/or based on expansion of T cells enriched for tumor-reactive T cells following co-culture with antigen presenting cells presenting peptide neoepitopes. For example, the methods of culturing the cells include methods to proliferate and expand cells, particularly involving steps to enrich for proliferation and expansion of tumor-reactive T cells such as by selection of such cells, or based on certain selection markers that are associated with or indicative of tumor-reactive T cells.

Exemplary markers for selecting or enriching tumor reactive T cells in the provided methods include CXCL13 and/or one or more exhaustion marker such as one or more of PD-1, CD39 and TIGIT.

Chemokine (C-X-C motif) ligand 13 (CXCL13), also known as B lymphocyte chemoattractant (BLC) or B cell-attracting chemokine 1 (BCA-1), is a protein ligand that in humans is encoded by the CXCL13 gene. CXCL13 is a small chemokine belonging to the CXC chemokine family. As its name suggests, this chemokine is selectively chemotactic for B cells belonging to both the B-1 and B-2 subsets and elicits its effects by interacting with chemokine receptor CXCRS. CXCL13 and its receptor CXCRS control the organization of B cells within follicles of lymphoid tissues and is expressed highly in the liver, spleen, lymph nodes, and gut of humans. The gene for CXCL13 is located on human chromosome 4 in a cluster of other CXC chemokines. In T lymphocytes, CXCL13 expression is thought to reflect a germinal center origin of the T cell, particularly a subset of T cells called T follicular helper cells (or TFH cells). Hence, expression of CXCL13 in T-cell lymphomas, such as Angioimmunoblastic T-cell Lymphoma, may reflect a germinal center origin of the neoplastic T-cells.

Evidence also indicates that action of CXCL13 with its receptor CXCRS, via the CXCL13:CXCRS axis, orchestrates cell-cell interactions that regulate lymphocyte infiltration within the tumor microenvironment, thereby determining responsiveness to cytotoxic and immune-targeted therapies. In one study, a tumor-infiltrating lymphocyte (TIL) subset from non-small cell lung cancer that had an increased capacity for tumor recognition were characterized as having high PD-1 expression and constitutive CXCL13 secretion, which may mediate immune cell recruitment to tertiary lymphoid structure (Thommen et al., abstract In: Proceedings of the Fourth CRI-CIMT-EATI-AACR International Cancer Immunotherapy Conference: Translating Science into Survival; Sept 30-Oct 3, 2018; New York, NY. Philadelphia (PA): AACR; Cancer Immunol Res 2019;7(2 Suppl):Abstract nr B050; Thommen et al. (2018) Nature Medicine, 24:994-1004). CXCL13 can be expressed and produced from certain TIL subpopulations, such as TGFβ-dependent CD103+CD8+ subpopulation (Workel et al. (2019) Cancer Immunology Research, 7(5):784-796).

PD-1, CD39 and TIGIT are each checkpoint molecules that also can represent markers of exhausted T cells. They also are markers that are activation markers or upregulation markers in that their expression is increased upon tumor reactivity, which is a natural mechanism of immune suppression of the host immune response. For instance, the immune system is designed to shut itself off to avoid an overactive immune response in order to avoid inflammatory and autoimmune responses. In this way, an immune response is initially developed against cancer but this can be thwarted by the upregulation of certain checkpoint molecules, like PD-1, CD39 and TIGIT, that can inhibit the immune response. As these are markers that are upregulated on cells in which an immune response is being developed, it is contemplated by the provided methods that such markers serve as powerful markers for specifically enriching for tumor reactive T cells that express TCRs against tumor antigens or neoepitopes. By specifically selecting for tumor reactive cells based on these activation markers , the provided methods avoid bulk expansion of T cells from a tumor source that would include a number of bystander cells that are not tumor reactive or that could exhibit inhibitory activity, such as Tregs.

The provided methods contemplate that selection of cells during one or more steps of an ex vivo process for manufacturing tumor reactive T cells based on secreted CXCL13 and/or based on expression of one or more exhaustion marker PD-1, CD39 and/or TIGIT will result in an improved TIL therapy enriched in tumor reactive T cells with high potential for therapeutic efficacy against neoantigens for treating certain cancers. The provided methods results in a product containing tumor reactive T cells that can target many mutations and/or that contains hundreds of TCRs that are reactive to different tumor antigens. Thus, such tumor reactive T cells offer advantages compared to existing methods in which cells are transduced to express a single neoepitope reactive TCR.

In some embodiments, CXCL13 secretion and/or expression of one or more PD-1, CD39 and/or TIGIT will be used to enrich TIL immediately after tumor dissociation, either at the endpoint of tumor fragment culture or immediately after mechanical/enzymatic creation of a single cell suspension from tumor fragments. In some embodiments, CXCL13 producing cells will be isolated from either tumor fragment cultures or single cell suspensions generated through enzymatic digestion. In some embodiments, PD-1, CD39 and/or TIGIT expressing cells will be isolated from either tumor fragment cultures or single cell suspensions generated through enzymatic digestion. In some aspects, TIL will be selected based on CXCL13 production in combination with PD1, TIGIT, and CD39 enrichment or any combination thereof. In aspects of the provided methods, after selection, selected cells (e.g. cells positive for CXCL13 secretion or surface positive for one or more of PD1, TIGIT and CD39, e.g. PD1+/CD39+/TIGIT+) can be expanded in the presence of one or more T cell stimulating agent. In some embodimetns, the T cell stimulating agent can include any one or more recombinant cytokines IL-2, IL-7, IL-15, IL-21, IL-25, IL-23, IL-27 or IL-25, such as generally at least IL-2 or IL-15. In some embodiments, the T cell stimulating agent can further include an anti-CD3 antibody (e.g. OKT3). In some embodiments, the T cell stimulating agents include an anti-CD3 antibody (OKT3) and/or a recombinant cytokine such as IL-2, IL-7, IL-15, IL-21, IL-25, IL-23. In some embodiments, the stimulation or any culture or incubation of the cells can be further carried out with an apoptosis inhibitor, such as Fas decoys or caspase inhibitors or any combination thereof.

In some embodiments of the provided methods a source of potential tumor peptides is used to identify TCRs that are reactive to neoantigens in a process that includes expansion of the T cells reactive to the tumor neoantigenic peptides. Provided methods include *ex vivo* co-culture methods in which a population of T cells that have been expanded from T cells present in or from a biological sample (e.g. tumor fragments or peripheral blood or other source of T cells) is incubated in the presence of antigen-presenting cells that have been contacted with, or made to present, the neoantigenic peptides. In particular aspects, the T cells and antigen-presenting cells are autologous to the tumor-bearing subject from which the peptides were identified. The provided methods further include steps to separate, enrich for and/or select for tumor-reactive T cells from the co-culture prior to or in connection with their further *ex vivo* expansion.

In some embodiments, expanded, unenriched TIL can be co-cultured with antigen presenting cells (APCs), such as autologous dendritic cells, pulsed with pools of neo-antigen (mutated) peptides identified through whole exome sequencing. Activated neo-antigen specific TIL can then be enriched based on CXCL13 secretion and/or expression of one or more PD-1, CD39 and/or TIGIT. In some aspects, following co-culture with APCs presenting neo-antigen (mutated) peptides, tumor reactive cell populations can be selected or enriched based on CXCL13 production. In some aspects, following co-culture with APCs presenting neo-antigen (mutated) peptides, tumor reactive cell populations can be selected or enriched based on surface upregulation or expression of PD-1, TIGIT and/or CD39. In some aspects, following co-culture with APCs presenting neo-antigen (mutated) peptides, tumor reactive cell populations can be selected based on CXCL13 production in combination with PD1, TIGIT, and CD39 enrichment or any combination thereof. In aspects of the provided methods, selected, enriched neo-antigen reactive TIL (e.g. cells positive for CXCL13 secretion or surface positive for one or more of PD1, TIGIT and CD39, e.g. PD1+/CD39+/TIGIT+) can be expanded in the presence of one or more T cell stimulating agent, such as an anti-CD3 antibody (OKT3) and or a recombinant cytokine such as IL-2, IL-7, IL-15, IL-21, IL-25, IL-23. In some embodiments, the stimulation or any culture or incubation of the cells can be further carried out with an apoptosis inhibitor, such as Fas decoys or caspase inhibitors or any combination thereof.

Further, the provided methods include steps to reduce or limit the presence of bystander cells in the resulting product and/or to enrich for tumor reactive T cells. In particular aspects, the use of modulatory cytokines, such as one or more of recombinant IL-23, recombinant IL-25, recombinant IL-27 or recombinant IL-35, and/or immunosuppressive blocking agents (e.g. against TGFbeta or IDO), can help facilitate T cell functionality while putting breaks or reducing activity of undesired cells, such as suppressor Treg cells. In some aspects, such modulatory cytokines and/or immunosuppressive blocking agents may be particularly advantageous during isolation of TILs from a tumor as a result of suppressive factors in the tumor microenvironment. In some aspects, the provided use of such modulatory cytokines and/or immunosuppressive blocking agents also may be included during expansion of tumor reactive T cells after isolation or enrichment and co-culture with APCs/peptide neoepitopes. For example, in some embodiments, modulatory cytokines, such one or more of recombinant IL-23, recombinant IL-25, recombinant IL-27 and/or recombinant IL-35, and/or immunosuppressive blocking agents (e.g. against TGFbeta or IDO1) could prove beneficial in tumor cultures during initial stimulation and expansion of TIL, as well as expansion of isolated or enriched neo-antigen tumor reactive T cells. In other examples, modulatory cytokines, such as one or more of recombinant IL-23, recombinant IL-25, recombinant IL-27 or IL-35, and/or immunosuppressive blocking agents (e.g. against TGFbeta or IDO1), could prove beneficial during initial stimulation and expansion of TIL from suppressive tumor microenvironments as well as preventing immune suppression of neo-antigen tumor reactive T cells during expansion with stimulatory agents (such as IL-2). In further examples, the presence of such modulatory cytokines and/or immunosuppressive blocking agents could optimize TIL recovery during initial stimulation and expansion during tumor cell cultures.

FIG. 1A depicts a schematic of an exemplary process for manufacturing a T cell therapeutic composition in accord with the provided methods. In the exemplary process a tumor sample is obtained from a patient for identification and generation of peptides for use in co-culturing methods with antigen presenting cells (APCs) presenting the peptides and autologous antigen T cells obtained from the same subject. In some cases, TILs are enriched from the sample by selection for cells positive for one or more marker associated with tumor reactive cells (hereinafter "selection marker"), such as CXCL13 and/or an exhaustion marker such as PD-1/CD39/TIGIT. In some cases, a population of T cells from the patient, e.g. containing tumor infiltrating lymphocytes (TIL) or enriched to TILs, is stimulated under conditions to expand the cells, prior to co-culture with antigen presenting cells that have been contacted or exposed to peptide neoepitopes for presentation on a major histocompatibility complex. Following co-culture under conditions in which the antigen presenting cells present peptides in the context of a major histocompatibility complex, tumor-reactive T cells or T cells positive for one or more marker associated with tumor reactive cells (hereinafter "selection marker"), such as CXCL13, an exhaustion marker such as PD-1/CD39/TIGIT and/or a T cell activation marker (e.g. CD137 and/or CD134) can be selected and cultured under conditions for expansion in accord with the provided methods, such as incubation with a T cell stimulatory agent(s) (e.g. recombinant IL-2, anti-CD3). The culturing can be carried out in the presence of one or more recombinant cytokines (e.g. IL-2) to support proliferation and expansion of cells. The process can be carried out in the presence of serum-free media containing nutrients. One or more or all of the steps can be carried out in a closed system, such as without exposure of cells to the environment. Upon reaching a therapeutic dose or a threshold number of cells, the cells can be harvested and formulated, in some cases concentrated or cryopreserved, and used for administration to a subject such as by infusion. FIG. 1B depicts an exemplary process in which a cryopreservation step can be carried out after one or more of the steps.

In some embodiments as described, the provided methods are performed without a co-culturing step involving incubation of antigen presenting cells (APCs) presenting the tumor peptides and autologous antigen T cells obtained from the same subject.

The provided methods offer advantages compared to existing methods for producing and expanding TILs because the provided methods involve steps to enrich for tumor reactive cells, such as by selecting for T cells that are likely or suspecting of being enriched in tumor-reactive T cells. In some cases, the methods can further enrich for tumor reactive T cells by the co-culturing step with peptide-presenting APCs followed by selection of reactive T cell clones that have upregulated one or more selection marker associated with such cells. By virtue of this process, the initial small population of tumor reactive T cells expanded from the biological sample (e.g. tumor) are enriched for cells that are or likely to be tumor reactive cells before a subsequent second expansion step, thereby promoting preservation and expansion of cells of interest and limiting expansion of bystander T cells that are not reactive to a tumor antigen and/or that may include cells that exhibit inhibitory activity **(****FIG. 2A****).** This is in contrast to existing methods that involve passive expansion of bulk T cells in which all T cells from a tumor are subjected to a first initial expansion, e.g. with high IL-2 concentrations, followed by a second rapid expansion of T cells present after the initial expansion. In such other methods, while total viable cells (TVC) can be greatly expanded by these alternative processes, there is no step of actively ensuring that tumor reactive T cells are predominantly propagated **(****FIG. 2B****).** Further, the provided methods are carried out to maximize the numbers of tumor reactive cells that may be collected, for example by co-culturing all of the cells propagated after the first expansion with peptide-presenting APCs, and then by selecting from among all of the bulk cells after the co-culturing for cells positive for the one or more activation markers before the subsequent second expansion. In aspects of the provided methods, all steps of the method are carried out in a closed system.

The provided methods include one or more features that provide for or relate to an improved, more efficient and/or more robust process for producing a tumor-reactive T cell therapeutic composition ex vivo. In particular, the disclosure relates to methods that provide advantages over available methods for producing a TIL therapeutic cell composition. Such advantages include, for example, reduced cost, streamlining, improved enrichment of tumor-reactive T cells in the therapeutic composition, and increased efficacy of the therapeutic composition, including among different subjects and tumor conditions.

In aspects of the provided methods, expansion can be carried out with relatively lower concentrations of recombinant IL-2 during one or both expansion steps with success. Many existing methods use high concentrations of IL-2 of 6000 IU/mL for T cell expansion of TIL. However, high IL-2 concentrations can increase the cost of the process and may be limiting. In some cases, high IL-2 concentrations may lead to negative impacts on T cell differentiation by driving effector T cell differentiation over early memory T cells that may be more desirable in a therapeutic T cell composition. The provided methods can be carried out with concentrations that are several-fold lower than 6000 IU/mL, such as concentrations less than at or about 1000 IU/mL, for example from at or at about 300 IU/mL to at or about 1000 IU/mL. In particular embodiments, the concentration of IL-2 is at or about 300 IU/mL.

In embodiments of the provided methods, the population of T cells is obtained from a biological sample known to contain T cells. In some embodiments, the population of T cells is enriched from a biological sample from a subject, in particular a human subject. The biological sample can be any sample containing a bulk population of T cells. In some embodiments, the biological sample is or includes peripheral blood mononuclear cells. In some embodiments, the biological sample is a peripheral blood or serum sample. In some embodiments, the biological sample is a lymph node sample. In some embodiments, the biological sample is a tumor sample. In some aspects, the bulk T cells can include tumor-infiltrating T cells (TILs). In some embodiments, the subject is a human subject. In some embodiments the subject is a subject having a cancer, viral infection, bacterial infection, or is a subject with an inflammatory condition. In particular embodiments, the subject has a cancer.

In aspects of the provided methods, the starting source of cells (input sample) in the method can be tumor fragments (e.g. 1-8 mm diameter fragments) or can be a single cell suspension preparation from enzymatic digestion of tumor fragments. While certain sources may be superior for some tumor types, both fragments and single cell suspensions can support T cell expansion and enrichment of tumor-reactive T cells. In some cases, the tumor cell source can be chosen depending on the tumor type or cancer, such as to optimize or increase expansion and enrichment of tumor-reactive T cells from the tumor. In one example, the cancer is a melanoma and the starting population of lymphocytes are tumor fragments, such as from a resected tumor. In another example, the cancer is a colorectal cancer and the starting population of lymphocytes is a single cell suspension obtained by enzymatic digestion, e.g. collagenase, of tumor fragments.

In some embodiments, the methods include a step of co-culturing initially expanded T cells with autologous antigen presenting cells that have been loaded with peptide. In aspects of the method, relatively low concentrations of peptide or a peptide pool (containing a plurality of peptides, e.g. 2, 3, 4, 5, 6, 7, 8, 9, 10, 20, 30, 40, 50, 60, 70, 80, 90 or 100 more, or any value between any of the foregoing), such where each individual peptide is less than 20 ng/mL, and even as low as 0.1 ng/mL, can lead to an increase in activation of T cell during the culture. In some embodiments, this can lead to an improved enrichment of tumor-reactive T cells in the co-culture prior to enrichment of cells by selection therefrom. In some embodiments, the co-culturing step in the provided methods include a ratio of tumor-derived cells containing T cells to autologous APCs (e.g. dendritic cells) of at or about 1:5 to at or about 5:1, such as 1:3 to at or about 3:1, for example as at or about 1:1, and involves loading the APCs with an individual peptide or a pool of peptides. In some embodiments, the APCs are loaded with a concentration of peptide or peptide pool in which the individual peptide, or individual peptides of the pool of peptides on average, is less than at or about 20 ng/mL, such as from at or about 0.1 ng/mL to at or about 1 ng/mL, for example at or about 0.1 ng/mL.

In some embodiments, the provided methods include enriching T cells, such as CD3+ T cells or a CD4 and/or CD8 subset thereof, further based on one or more marker whose expression is upregulated on (e.g. compared to resting or non-activated T cells) or specific to reactive or activated T cells (hereinafter "reactive T cell marker" or T cell activation marker). Reactive T cells will express certain reactive markers when their endogenous TCR recognizes an antigen on a target cell or tissue, such as when a TCR recognizes a neoantigen on the tumor. Exemplary reactive T cell markers include one or more, such as two, three, four or more of, CD107, CD107a, CD39, CD103, CD137 (4-1BB), CD59, CD90, CD38, CD30, CD154, CD252, CD134 (OX40), CD258, CD256, PD-1, TIM-3 or LAG-3. The enrichment or selection for cells positive for one or more such upregulation marker on reactive or activated T cells can be carried out prior to or during one or more steps of the expansion method. In particular embodiments, the provided methods include enrichment or selection for cells positive for one or more upregulation marker on reactive or activated T cells after activation of a population of T cells by the co-culture incubation with peptide-presenting APCs (e.g. DC's). In some embodiments, the step of selecting cells positive for one or more upregulation marker on reactive or activated T cells from the co-culture can result in 2-fold or greater enrichment of antigen-specific tumor-reactive T cells and/or a substantial decrease in TCR clonality evidencing enrichment of TCR clonotypes consistent with enrichment of tumor-reactive T cells. Furthermore, such enriched T cells can exhibit an improved ability to produce IFN-gamma following antigen-specific stimulation compared to non-selected T cells or bulk T cells from the co-culture.

In some embodiments, the methods produce or expand T cells for use in adoptive cell therapy for treating a disease or condition in which cells or tissue associated with the disease or condition is known or suspected of expressing an antigen target recognized by the T cells. In some embodiments, the T cell therapy is autologous to the subject. In some embodiments, the T cell therapy is allogeneic to the subject.

All publications, including patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### I. EX VIVO EXPANSION OF TUMOR-REACTIVE T CELLS

The provided methods involve the *ex vivo* expansion and production of a T cell therapeutic composition, particularly for use in connection with treating cancer. In some embodiments, the method of manufacturing involves the growth and manipulation of patient cells outside of the body. In particular embodiments, the methods relate to methods for expanding T cells containing an endogenous TCR specific to a tumor-associated antigen (hereinafter "tumor reactive T cells"). For purposes of this disclosure, reference to tumor reactive T cells includes T cells that exhibit reactivity to a tumor antigen or that are likely or suspected of being tumor reactive T cells due to upregulation or positive surface expression of a protein expressed on the T cell that are only expressed when the T cells endogenous TCR recognizes a peptide expressed by the APCs, e.g. T cell activation marker. In some aspects, the frequency of these cells can be low and in order to expand these cells to a therapeutic dose *ex vivo* methods for enrichment and expansion are necessary.

The provided embodiments relate to processes for preparing a therapeutic TIL composition enriched for tumor reactive T cells that involves a direct selection of cells to yield a tumor reactive cell population that is further expanded. In the provided methods, a TIL tumor sample is obtained that contains or is expected to contain tumor reactive T cells (e.g. first population). In one embodiment, this population can be processed by digestion to create a single cell suspension (e.g. second population). In some embodiments, this second population is sorted to select for cells that are enriched for tumor reactive T cells (e.g. based on selection of cells positive for PD-1, CD39 and/or TIGIT), to yield a selected or sorted population of cells (e.g. third population), which then can be expanded to create a therapeutic composition containing an expanded population of tumor specific reactive cells (e.g. fourth population). In other embodiments, the first population is processed into fragments or digested to create a single cell suspension (e.g. second population), and then this second population is first subjected to an initial expansion (e.g. a minimal expansion of between 1-14 days) in which to yield an initial population of cells (e.g. third population) that is to be sorted/selected for tumor reactive T cells. In such an embodiment, this third population is sorted to select for cells that are enriched for tumor reactive T cells (e.g. based on selection of cells positive for PD-1, CD39 and/or TIGIT), to yield a selected or sorted population of cells (e.g. fourth population), which then can be expanded to create a therapeutic composition containing an expanded population of tumor specific reactive cells (e.g. fifth population).

In some embodiments, the sort and selection is carried out for tumor upregulation activation markers CD39, PD1, or TIGIT, or any combination thereof. Tumor containing reactive T cells that recognize the tumor upregulate activation markers such as, CD39, PD1 and TIGIT. In provided methods, the T cells are sorted directly after tumor digest or after a short period of cell culture for cells surface positive for CD39, PD1, and TIGIT to create a population of tumor neoantigen reactive T cells. This process removes the nonreactive and inhibitory 'bystander' cells, resulting in T cell product enriched in neoantigen reactive T cells. Cells are then expanded into clinically relevant numbers of tumor specific T cells. In some embodiments, the final expanded therapeutic composition is formulated with a cyroprotectant for cryopreservation.

In one aspect of provided methods, the tumor reactive T cells are directly sorted after tumor digest, and only a single expansion step is carried out, in which the population of expanded T cells is harvested as a therapeutic TIL composition. In such an example, tumor fragments are digested into a single cell suspension and provided as an input sample for sorting/selection for the tumor-reactive T cells thereof. Then, the selected cells are expanded and harvested as a therapeutic TIL composition. In some embodiments, the expansion is carried out for a period of time to achieve a therapeutic dose. In some embodiments, the expansion is carried to achieve a fold expansion of the cells of from at or about 200-fold to at or about 3000-fold. In some embodiments, the expansion is carried out to achieve a therapeutic dose of at or about or greater than at or abot 500 million total cells. In some embodiments, the expansion is carried out for 1-28 days, such as for at or about 7 to 28 days, 7 to 21 days, 7 to 14 day, such as at or about 7 days, 8 days, 9 days, 10 days, 11 days, 13 days, 14 days, 15 days, 16 days, 17 days, 18 days, 19 days, 20 days, 21 days, 22 days, 23 days, 24 days, 25 days, 26 days, 27 days or 28 days.

In some embodiments, provided herein is a method of manufacturing tumor-reactive T cells, the method comprising (a) selecting cells secreting chemokine (C-X-C motif) ligand 13 (CXCL13) and/or surface positive for C-X-C chemokine receptor type 5 (CXCR5), from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells. In some embodiments, the method includes harvesting the population of expanded T cells produced by the method for formulation as the therapeutic composition.

In some embodiments, provided herein is a method for manufacturing tumor-reactive T cells comprising (a) selecting cells surface positive for PD-1, CD39 and/or TIGIT from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells. In some embodiments, the method includes harvesting the population of expanded T cells produced by the method for formulation as the therapeutic composition.

In some embodiments, provided herein is a method for manufacturing tumor-reactive T cells comprising (a) selecting cells surface positive for PD-1 and CD39 from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells. In some embodiments, the method includes harvesting the population of expanded T cells produced by the method for formulation as the therapeutic composition.

In some embodiments, provided herein is a method for manufacturing tumor-reactive T cells comprising (a) selecting cells surface positive for PD-1 and TIGIT from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells. In some embodiments, the method includes harvesting the population of expanded T cells produced by the method for formulation as the therapeutic composition.

In some embodiments, provided herein is a method for manufacturing tumor-reactive T cells comprising (a) selecting cells surface positive for CD39 and TIGIT from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells. In some embodiments, the method includes harvesting the population of expanded T cells produced by the method for formulation as the therapeutic composition.

In some embodiments, provided herein is a method for manufacturing tumor-reactive T cells comprising (a) selecting cells surface positive for PD-1, CD39 and TIGIT from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells. In some embodiments, the method includes harvesting the population of expanded T cells produced by the method for formulation as the therapeutic composition.

In another apsect of the provided methods, at least two separate expansion steps are carried out in which both expansions are carried out after selecting or sorting the cells for the tumor-reactive T cells (e.g. for cells surface positive for PD-1, CD39 and/or TIGIT). Thus, the incubation for expansion and harvesting is split into two expansions. For example, provided methods include selecting cells surface positive for a marker of tumor reactive T cells (e.g. cell surface positive for PD-1, CD39 and/or TIGIT), and then involve a first expansion of the selected cells to yield a first population of expanded cells and then a further (second) expansion of the first population of expanded cells to yield a second population of expanded cells. In such an example, the second population of expanded T cells is harvested as a therapeutic TIL composition. In some embodiments, the first and second expansions together are carried out for a period of time to achieve a therapeutic dose. In some embodiments, the first expansion is carried to achieve a fold expansion of the cells of from at or about 2-fold to at or about 20-fold. In some embodiments, the first expansion is carried out for 1-14 days, such as for at or about or no more than 1 day, 2 days, 3 days, 4 days, 5 days, 6 days, 7 days, 8 days, 9 days, 10 days, 11 days, 12 days, 13 days or 14 days. In some embodiments, the second expansion is carried to achieve a fold expansion of the cells of from at or about 100-fold to at or about 3000-fold. In some embodiments, the second expansion is carried out for at or about 7 days to 21 days, such as for approximately 14 days. In some embodiments, the first and second expansions are carried out to achieve a therapeutic dose of at or about or greater than at or abot 500 million total cells.

In some embodiments, provided herein is a method for manufacturing tumor-reactive T cells comprising (a) selecting cells surface positive for PD-1, CD39 and/or TIGIT from an input sample comprising T cells from a subject that has a tumor to obtain selected cells from the sample; (b) performing a first expansion by culture of the selected cells with one or more T-cell stimulating agent of lymphocytes under conditions to produce a first population of expanded T cells, and (c) performing a second expansion by culture of the first population of expanded cells with one or more T-cell stimulating agent of lymphocytes under conditions to produce a second population of expanded T cells. In some embodiments, the method includes harvesting the second population of expanded T cells produced by the method for formulation as the therapeutic composition.

In some embodiments, provided herein is a method for manufacturing tumor-reactive T cells comprising (a) selecting cells surface positive for PD-1 and CD39 from an input sample comprising T cells from a subject that has a tumor to obtain selected cells from the sample; (b) performing a first expansion by culture of the selected cells with one or more T-cell stimulating agent of lymphocytes under conditions to produce a first population of expanded T cells, and (c) performing a second expansion by culture of the first population of expanded cells with one or more T-cell stimulating agent of lymphocytes under conditions to produce a second population of expanded T cells. In some embodiments, the method includes harvesting the second population of expanded T cells produced by the method for formulation as the therapeutic composition.

In some embodiments, provided herein is a method for manufacturing tumor-reactive T cells comprising (a) selecting cells surface positive for PD-1 and TIGIT from an input sample comprising T cells from a subject that has a tumor to obtain selected cells from the sample; (b) performing a first expansion by culture of the selected cells with one or more T-cell stimulating agent of lymphocytes under conditions to produce a first population of expanded T cells, and (c) performing a second expansion by culture of the first population of expanded cells with one or more T-cell stimulating agent of lymphocytes under conditions to produce a second population of expanded T cells. In some embodiments, the method includes harvesting the second population of expanded T cells produced by the method for formulation as the therapeutic composition.

In some embodiments, provided herein is a method for manufacturing tumor-reactive T cells comprising (a) selecting cells surface positive for CD39 and TIGIT from an input sample comprising T cells from a subject that has a tumor to obtain selected cells from the sample; (b) performing a first expansion by culture of the selected cells with one or more T-cell stimulating agent of lymphocytes under conditions to produce a first population of expanded T cells, and (c) performing a second expansion by culture of the first population of expanded cells with one or more T-cell stimulating agent of lymphocytes under conditions to produce a second population of expanded T cells. In some embodiments, the method includes harvesting the second population of expanded T cells produced by the method for formulation as the therapeutic composition.

In some embodiments, provided herein is a method for manufacturing tumor-reactive T cells comprising (a) selecting cells surface positive for PD-1, CD39 and TIGIT from an input sample comprising T cells from a subject that has a tumor to obtain selected cells from the sample; (b) performing a first expansion by culture of the selected cells with one or more T-cell stimulating agent of lymphocytes under conditions to produce a first population of expanded T cells, and (c) performing a second expansion by culture of the first population of expanded cells with one or more T-cell stimulating agent of lymphocytes under conditions to produce a second population of expanded T cells. In some embodiments, the method includes harvesting the second population of expanded T cells produced by the method for formulation as the therapeutic composition.

In another aspect of the provided methods, a first expansion is carried out prior to the selecting or sorting the cells for the tumor-reactive marker. In some embodiments, the first expansion is a minimal expansion that is carried out under conditions and for a period of time so that activation markers upregulated on cells of the collected tumor sample (e.g. PD-1, CD39 and/or TIGIT) are still present during the sorting step and have not been downregulated during the first expansion. In some embodiments, the initial expansion is carried out for 1-14 days, such as for at or about or no more than 1 day, 2 days, 3 days, 4 days, 5 days, 6 days, 7 days, 8 days, 9 days, 10 days, 11 days, 12 days, 13 days or 14 days. In such embodiments, the methods involve sorting or selecting the cells from the population of initially expanded T cells. After the selection of sorting for the tumor-reactive T cells (e.g. cells surface positive for PD-1, CD39 and/or TIGIT), the selected population of cells are further expanded in a second expansion to yield a second population of expanded cells. In such an example, the second population of expanded T cells is harvested as a therapeutic TIL composition. In some embodiments, the f second expansion is carried out for a period of time to achieve a therapeutic dose. In some embodiments, the second expansion is carried to achieve a fold expansion of the cells of from at or about 500-fold to at or about 1000-fold, such as at or about 750-fold. In some embodiments, the second expansion is carried out for at or about 7 days to 21 days, such as for approximately 14 days. In some embodiments, the second expansion is carried out to achieve a therapeutic dose of at or about or greater than at or abot 500 million total cells.

In some embodiments, provided herein is a method for manufacturing tumor-reactive T cells, the method comprising (a) processing a biological sample containing T cells obtained from a donor subject that has a tumor to produce an input sample comprising T cells, (b) performing a first expansion by culture of the input sample comprising T cells with one or more first T-cell stimulating agent of lymphocytes under conditions to produce a first population of expanded T cells, (c) selecting cells secreting chemokine (C-X-C motif) ligand 13 (CXCL13) and/or surface positive for C-X-C chemokine receptor type 5 (CXCRS) from the first population of expanded cells to produce a selected population, and (d) performing a second expansion by culture of the selected population with one or more second T-cell stimulating agent under conditions to produce a second expanded population of T cells, wherein the second population of expanded T cells is for use as a therapeutic cell composition. In some embodiments, the method includes harvesting the second population of expanded T cells produced by the method for formulation as the therapeutic composition.

In some embodiments, provided herein is a method for manufacturing tumor-reactive T cells, the method comprising (a) processing a biological sample containing T cells obtained from a donor subject that has a tumor to produce an input sample comprising T cells, (b) performing a first expansion by culture of the sample comprising T cells with one or more first T-cell stimulating agent of lymphocytes under conditions to produce a first population of expanded T cells, (c) selecting cells surface positive for PD-1, CD39 and/or TIGIT from the first population of expanded cells to produce a selected population; and (d) performing a second expansion by culture of the selected population with one or more second T-cell stimulating agent under conditions to produce a second expanded population of T cells, wherein the second population of expanded T cells is for use as a therapeutic cell composition. In some embodiments, the method includes harvesting the second population of expanded T cells produced by the method for formulation as the therapeutic composition.

In some embodiments, provided herein is a method for manufacturing tumor-reactive T cells, the method comprising (a) processing a biological sample containing T cells obtained from a donor subject that has a tumor to produce an input sample comprising T cells, (b) performing a first expansion by culture of the sample comprising T cells with one or more first T-cell stimulating agent of lymphocytes under conditions to produce a first population of expanded T cells, (c) selecting cells surface positive for PD-1 and CD39 from the first population of expanded cells to produce a selected population; and (d) performing a second expansion by culture of the selected population with one or more second T-cell stimulating agent under conditions to produce a second expanded population of T cells, wherein the second population of expanded T cells is for use as a therapeutic cell composition. In some embodiments, the method includes harvesting the second population of expanded T cells produced by the method for formulation as the therapeutic composition.

In some embodiments, provided herein is a method for manufacturing tumor-reactive T cells, the method comprising (a) processing a biological sample containing T cells obtained from a donor subject that has a tumor to produce an input sample comprising T cells, (b) performing a first expansion by culture of the sample comprising T cells with one or more first T-cell stimulating agent of lymphocytes under conditions to produce a first population of expanded T cells, (c) selecting cells surface positive for PD-1 and TIGIT from the first population of expanded cells to produce a selected population; and (d) performing a second expansion by culture of the selected population with one or more second T-cell stimulating agent under conditions to produce a second expanded population of T cells, wherein the second population of expanded T cells is for use as a therapeutic cell composition. In some embodiments, the method includes harvesting the second population of expanded T cells produced by the method for formulation as the therapeutic composition.

In some embodiments, provided herein is a method for manufacturing tumor-reactive T cells, the method comprising (a) processing a biological sample containing T cells obtained from a donor subject that has a tumor to produce an input sample comprising T cells, (b) performing a first expansion by culture of the sample comprising T cells with one or more first T-cell stimulating agent of lymphocytes under conditions to produce a first population of expanded T cells, (c) selecting cells surface positive for CD39 and TIGIT from the first population of expanded cells to produce a selected population; and (d) performing a second expansion by culture of the selected population with one or more second T-cell stimulating agent under conditions to produce a second expanded population of T cells, wherein the second population of expanded T cells is for use as a therapeutic cell composition. In some embodiments, the method includes harvesting the second population of expanded T cells produced by the method for formulation as the therapeutic composition.

In some embodiments, provided herein is a method for manufacturing tumor-reactive T cells, the method comprising (a) processing a biological sample containing T cells obtained from a donor subject that has a tumor to produce an input sample comprising T cells, (b) performing a first expansion by culture of the sample comprising T cells with one or more first T-cell stimulating agent of lymphocytes under conditions to produce a first population of expanded T cells, (c) selecting cells surface positive for PD-1, CD39 and TIGIT from the first population of expanded cells to produce a selected population; and (d) performing a second expansion by culture of the selected population with one or more second T-cell stimulating agent under conditions to produce a second expanded population of T cells, wherein the second population of expanded T cells is for use as a therapeutic cell composition. In some embodiments, the method includes harvesting the second population of expanded T cells produced by the method for formulation as the therapeutic composition.

In some aspects of the provided methods, the methods do not include a step of co-culturing a population of tumor-reactive T cells with antigen presenting cells that present one or more neoantigen peptide that corresponds to nonsynonymous somatic mutations associated in the tumor of a subject.

In other aspects of the provided methods, the methods do include a step of co-culturing a population of tumor-reactive T cells with antigen presenting cells that present one or more neoantigen peptide that corresponds to nonsynonymous somatic mutations associated in the tumor of a subject. In such other aspect of the provided methods, the methods include co-cultruing T cells digested directly from a tumor fragment from a subject, or after an initial (e.g. minimal expansion) of the cells therefrom, in the presence of antigen presenting cells that present one or more non-native peptide on a major histocompatibility complex (MHC), said one or more non-native peptides are peptides corresponding to nonsynonymous somatic mutations associated in the tumor of a subject. In some embodiments, prior to the co-culturing, tumor reactive T cells can be enriched or selected (e.g. based on selection of cells positive for PD-1, CD39 and/or TIGIT), and the selected T cell population is co-cultured with the antigen presenting cells presenting the peptide epitopes. Then, cells from the culture are expanded to create a therapeutic composition containing an expanded population of tumor specific reactive cells. In other embodiments, after the co-culturing, tumor reactive T cells can be enriched or selected (e.g. based on selection of cells positive for PD-1, CD39 and/or TIGIT) directly from the co-culture, and the selected cells are expanded to create a therapeutic composition containing an expanded population of tumor specific reactive cells.

In some embodiments, provided herein is a method for manufacturing tumor-reactive T cells, the method comprising (a) processing a biological sample containing T cells obtained from a donor subject that has a tumor to produce an input sample comprising T cells, (b) performing a first expansion by culture of the input sample comprising T cells with one or more first T-cell stimulating agent of lymphocytes under conditions to produce a first population of expanded T cells, (c) selecting cells secreting chemokine (C-X-C motif) ligand 13 (CXCL13) and/or surface positive for C-X-C chemokine receptor type 5 (CXCR5), from the first population of expanded cells to produce a selected population, (d) co-culturing the selected population in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor, and (e) performing a second expansion by culture of the reactive T cell population with one or more second T-cell stimulating agent under conditions to produce a second expanded population of T cells, wherein the second population of expanded T cells is for use as a therapeutic cell composition. In some embodiments, the method includes harvesting the second population of expanded T cells produced by the method for formulation as the therapeutic composition.

In some embodiments, provided herein is a method for manufacturing tumor-reactive T cells, the method comprising (a) processing a biological sample containing T cells obtained from a donor subject that has a tumor to produce an input sample comprising T cells, (b) performing a first expansion by culture of the input sample comprising T cells with one or more first T-cell stimulating agent of lymphocytes under conditions to produce a first population of expanded T cells, (c) co-culturing the first population of expanded cells in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor, (d) selecting cells secreting chemokine (C-X-C motif) ligand 13 (CXCL13) and/or surface positive for C-X-C chemokine receptor type 5 (CXCR5), from the reactive T cell population to produce a selected population; and (e) performing a second expansion by culture of the selected population with one or more second T-cell stimulating agent under conditions to produce a second expanded population of T cells, wherein the second population of expanded T cells is for use as a therapeutic cell composition. In some embodiments, the method includes harvesting the second population of expanded T cells produced by the method for formulation as the therapeutic composition.

Provided herein is a method for manufacturing tumor-reactive T cells, the method comprising (a) processing a biological sample containing T cells obtained from a donor subject that has a tumor to produce an input sample comprising T cells, (b) performing a first expansion by culture of the sample comprising T cells with one or more first T-cell stimulating agent of lymphocytes under conditions to produce a first population of expanded T cells, (c) selecting cells surface positive for PD-1, CD39 and/or TIGIT, such as PD-1, CD39 and TIGIT, from the first population of expanded cells to produce a selected cell population, (d) co-culturing the selected cell population in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor, and (e) performing a second expansion by culture of the reactive T cell population with one or more second T-cell stimulating agent under conditions to produce a second expanded population of T cells, wherein the second population of expanded T cells is for use as a therapeutic cell composition. In some embodiments, the method includes harvesting the second population of expanded T cells produced by the method for formulation as the therapeutic composition.

Provided herein is a method for manufacturing tumor-reactive T cells, the method comprising, (a) processing a biological sample containing T cells obtained from a donor subject that has a tumor to produce an input sample comprising T cells, (b) performing a first expansion by culture of the sample comprising T cells with one or more first T-cell stimulating agent of lymphocytes under conditions to produce a first population of expanded T cells, (c) co-culturing the first population of expanded cells in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor, (d) selecting cells surface positive for PD-1, CD39 and/or TIGIT, such as PD-1, CD39 and TIGIT, from the reactive T cell population to produce a selected cell population; and (e) performing a second expansion by culture of the selected cell population with one or more second T-cell stimulating agent under conditions to produce a second expanded population of T cells, wherein the second population of expanded T cells is for use as a therapeutic cell composition. In some embodiments, the method includes harvesting the second population of expanded T cells produced by the method for formulation as the therapeutic composition.

In some embodiments, the T-cell stimulating agents include anti-CD3 (e.g. anti-CD3 antibody, such as OKT3), anti-CD28 reagents (e.g. anti-CD28 antibody), such as an anti-CD3 antibody (e.g. OKT3) and an anti-CD28 antibody and/or one or more recombinant cytokine (e.g. IL-2, IL-7, IL-21 and/or IL-15) . In particular embodiments, the incubation or culture of T cells also is carried out with nutrient containing media so that the cells can survive outside of the body.

In some cases, the methods including incubation with recombinant IL-2 alone or in combination with one or more other recombinant cytokines (e.g. IL-7, IL-21 and/or IL-15) and, in some cases, one or more other T cell stimulating agents to provide a primary and secondary (costimulatory) signal to the cells. Standard methods for culturing T cells to provide a primary and secondary (costimulatory) signal to the cells, involve incubation with T cell stimulating agents provided by anti-CD3 (e.g. OKT3) and anti-CD28 reagents. In some embodiments, the T cell stimulatory agents include an anti-CD3 antibody (e.g. OKT3) and an anti-CD28 antibody. Typically such stimulations also include one or more additional recombinant cytokine (e.g. IL-2, IL-7, IL-21 and/or IL-15) and nutrient containing media so that the cells can survive outside of the body.

Provided methods for expansion of tumor-reactive T cells involve a first expansion involving culturing the selected or isolated population containing T cells (i.e. the first population of T cells) with a recombinant cytokine from one or more of (e.g. IL-2, IL-7, IL-21 and/or IL-15), typically generally including recombinant IL-2. In some cases, the T cell stimulatory agent(s) further provide a primary and secondary (costimulatory) signal to the cells, such as provided by anti-CD3 (e.g. OKT3) and anti-CD28 reagents, such as an anti-CD3 antibody (e.g. OKT3) and an anti-CD28 antibody. The initial or first expansion results in a second population of T cells that is enriched for T cells as a result of expansion or proliferation of T cells present in the first population.

In the provided methods, tumor reactive T cells are identified or enriched from the stimulated T cells expanded in the first step by one or more further steps that include *ex vivo* co-culture of the stimulated T cells (second population of T cells) with antigen presenting cells (APCs) and one or a plurality of peptides that include neoepitopes of a tumor antigen (APCs/peptide neoepitopes). In some embodiments, provided methods include *ex vivo* co-culture in which in which the second population of T cells are incubated with APCs, such as autologous APCs or artificial antigen presenting cells (aAPCs), that have been exposed to or contacted with one or more peptides, e.g. synthetic peptides, under conditions in which the APCs have been induced to present one or more peptides from a tumor-associated antigen. In some embodiments, the population of T cells are autologous T cells from a subject with a tumor and the source of synthetic peptides are tumor antigenic peptides from a tumor antigen of the same subject. In some embodiments, cells from the *ex vivo* co-culture are a population of cells (third population) that include tumor reactive T cells that recognize or are activated by a peptide presented on an MHC of an APC in the culture. In some embodiments, cells from the *ex vivo* co-culture represent a source of cells that are enriched for tumor reactive T cells.

In particular embodiments, a second expansion is performed on T cells enriched or isolated from the co-culture, such as after separation or selection of tumor reactive T cells or T cells that are surface positive for one or more T cell activation markers associated with tumor reactive T cells. The second expansion involves incubation to further stimulate T cells with a T cell stimulatory agent(s), such as anti-CD3 antibody (e.g. OKT3), anti-CD28 antibody, and recombinant cytokine(s) (e.g. IL-2, IL-7, IL-21 and/or IL-15). The T cells, such as tumor reactive T cells or T cells that are surface positive for one or more T cell activation markers associated with tumor reactive T cells, are allowed to expand for a certain number of days as desired and/or until a therapeutic dose or harvest dose is met. The composition of expanded T cells can then be harvested and formulated for administration to a subject for treatment of a cancer in the subject.

In embodiments of the provided methods, one or more of the steps can be carried out in serum-free media. In one embodiment, the serum free medium is OpTmizer CTS (LifeTech), Immunocult XF (Stemcell technologies), CellGro (CellGenix), TexMacs (Miltenyi), Stemline (Sigma), Xvivo15 (Lonza), PrimeXV (Irvine Scientific), or Stem XVivo (RandD systems). The serum-free medium can be supplemented with a serum substitute such as ICSR (immune cell serum replacement) from LifeTech. The level of serum substitute (e.g., ICSR) can be, e.g., up to 5%, e.g., about 1%, 2%, 3%, 4%, or 5%. In some embodiments, the serum-free media contains 0.5 mM to 5 mM of a dipeptide form of L-glutamine, such L-alanyl-L-glutamine (Glutamax^{™}). In some embodiments, the concentration of the dipeptide form of L-glutamine, such as L-alanyl-L-glutamine, is from or from about 0.5 mM to 5 mM, 0.5 mM to 4 mM, 0.5 mM to 3 mM, 0.5 mM to 2 mM, 0.5 mM to 1 mM, 1 mM to 5 mM, 1 mM to 4 mM, 1 mM to 3 mM, 1 mM to 2 mM, 2 mM to 5 mM, 2 mM to 4 mM, 2 mM to 3 mM, 3 mM to 5 mM, 3 mM to 4 mM or 4 mM to 5 mM, each inclusive. In some embodiments, the concentration of the dipeptide form of L-glutamine, such as L-alanyl-L-glutamine, is or is about 2 mM.

In connection with the provided methods, the methods result in enrichment of T cells containing an endogenous TCR specific to a tumor-associated antigen to maximize expansion of desired therapeutic cells. The T cells, such as tumor reactive T cells or T cells that are surface positive for one or more T cell upregulation markers or activation markers associated with tumor reactive T cells, are allowed to expand for a certain number of days as desired and/or until a therapeutic dose or harvest dose is met. The composition of expanded T cells can then be harvested and formulated for administration to a subject for treatment of a cancer in the subject.

### A. Expansion of T cells, e.g. First Expansion, by Stimulation of a Population of T cells

The provided methods include obtaining and enriching a population of T cells from a biological sample for use as a first or input sample containing T cells. In some cases, the first or input sample of T cells is one that is known or likely to contain T cells reactive to a tumor antigen or that are capable of being reactive to a tumor antigen, such as following an *ex vivo* co-culture with an autologous source of tumor antigen. For example, typically the first or input sample of T cells is from a biological sample from a tumor or from a subject known or likely to have a tumor. In particular embodiments, the first or input sample of T cells is further stimulated with one or more T cell stimulatory agent(s) (e.g. one or more recombinant cytokines, such as IL-2) to produce a second or stimulated population of T cells containing T cells that have expanded following the stimulation.

In some embodiments, the incubation with the T cell stimulatory agent(s) is carried out directly on an input or first sample of T cells selected or obtained from a biological sample from a subject (e.g. autologous T cells from the subject), wherein the input or first sample of T cells is incubated with the T cell stimulatory agent(s). In other embodiments, the input or first sample of T cells includes T cells that are likely to be or are suspected to be tumor reactive T cells, in which such cells are first selected from a population of T cells selected from a biological sample from a subject by selecting for cells positive for a marker associated with reactive T cells, such as one or more markers described in Section I.C. In such embodiments, the incubation with the T cell stimulatory agent(s) is carried out after the enriching for the population of T cell cells comprising tumor-reactive T cells. In the provided embodiments, the incubation with the T cell stimulatory agent(s) is carried out before the co-culturing of such T cells with the APCs/peptide neoepitopes.

In some cases, conditions for stimulating the T cells by culture with one or more T cell stimulatory agent(s) results in activation of the cells and expansion or outgrowth of T cells present in the first or input sample of T cells. In some embodiments, the conditions for stimulating the T cells with one or more T cell stimulatory agent(s) can include culturing the T cells under condition that results in bulk expansion of the T cells.

In the provided methods, the stimulated or expanded composition of T cells, such as a first expanded population, is then employed in subsequent downstream steps for enrichment and expansion of tumor reactive T cells, including steps that include co-culture of the stimulated T cells with antigen presenting cells (APCs) in the presence of T cell neoepitope (mutated) peptide antigens to produce, yield or to pull out T cells that are tumor reactive T cells. In particular embodiments, the provided methods also can include a step for selecting or enriching T cells reactive to a tumor antigen (tumor reactive T cells), after co-culturing T cells with APCs/peptide neoepitopes, for example by selecting for cells positive for a marker associated with reactive T cells, such as one or more markers described in Section I.C. The tumor reactive T cell populations can be cultured under conditions for expansion, such as to produce a therapeutic T cell composition.

In aspects of any of the provided methods, the input or first sample of T cells is incubated in the presence of a T cell stimulatory agent(s). In particular embodiments, the incubation is carried out under conditions in which the T cell stimulatory agent(s) activates or stimulates the cells or promotes expansion of T cells present in the input or first population of T cells.

In some embodiments, the T cell stimulatory agent(s) include a recombinant T cell stimulating cytokine, such as IL-2, IL-7, IL-15, IL-21, IL-25 and/or IL-23. In some embodiments, the T cell stimulating cytokine includes IL-2, alone or in combination with another cytokine from among IL-7, IL-15, IL-21, IL-25 and/or IL-23. In some embodiments, the T cell stimulating cytokine is one, two, three or more of IL-2, IL-7, IL-15 and IL-21. In some embodiments, the T cell stimulating cytokines are IL-7 and IL-15.

In some embodiments, the T cell stimulatory (agent(s) can include an agent or agents that engage CD3. The T cell stimulatory agent(s) can include an anti-CD3 antibody, such as OKT3. In some embodiments, the T cell stimulatory agent(s) can additionally include an agent that engages CD3, such as an anti-CD28 agent (presented by APCs or as a soluble antibody). For example, the T cell stimulatory agent(s) can include an anti-CD3 antibody (e.g. OKT3) and an anti-CD28 antibody. Thus, in aspects of the provided methods, a first or input sample of T cells is incubated with one or more T-cell stimulating agents of lymphocytes, such as but not limited to anti-CD3 antibody (e.g. OKT3) and anti-CD28 (presented by APCs or as soluble antibodies), to produce a second population of T cells that include activated or stimulated T cells. In some aspects, such the stimulation can occur before a co-culture in the presence of APCs and neoepitope (mutated) peptides. In particular embodiments, one or more recombinant cytokines also are present as additional T cell stimulatory agents during the incubation.

In some embodiments, the incubation with one or more T cell stimulatory agent(s), such as an anti-CD3 and/or recombinant cytokines, e.g. IL-2, can be continued for a time period sufficient to activate or stimulate the cells. In some embodiments, the incubation with the T cell stimulatory agent(s) is carried out for at or about 1 day, such as generally at or about 2 days, 3 days, 4 days, 5 days, 6 days, 7 days, 8 days, 9 days, 10 days, 11 days, 12 days, or any range of time between any of the foregoing. In some embodiments, the incubation is carried out for 7-10 days. In some embodiments, the incubation is for at or about 7 days. In some embodiments, the incubation is for at or about 8 days. In some embodiments, the incubation is for at or about 9 days. In some embodiments, the incubation is for at or about 10 days. In some embodiments, the incubation with the T cell stimulatory agent(s) is for 12 hours to 96 hours, such as 24 hours to 48 hours, and generally at or about 48 hours.

In some embodiments, the cells are washed one or more times during the culturing to remove agents present during the incubation or culturing and/or to replenish the culture medium with one or more additional agents. In some embodiments, the cells are washed during the incubation or culturing to reduce or remove the T cell stimulatory agent(s) prior to completion of the culturing.

In some embodiments, the methods of stimulating T cells provided herein include incubation with a T cell stimulatory agent(s) at a temperature suitable for the growth of human T lymphocytes, for example, at least about 25 degrees Celsius, generally at least about 30 degrees, and generally at or about 37 degrees Celsius. In some embodiments, the methods of culturing or incubation is carried out in serum-free media.

### 1. Sample Containing T cells

The provided methods include selecting or obtaining an input sample of T cells from a biological sample, which can be used as the source or input of T cells for stimulation with one or more T cell stimulatory agents(s) (e.g. recombinant IL-2 or other T cell stimulating cytokines and/or anti-CD3 ). In some embodiments, the T cells are from a biological sample from a subject that is known or likely to contain tumor reactive T cells. The collected biological sample contains or is suspected to contain lymphocytes that have endogenous TCRs that are reactive to mutations present on a tumor.

In aspects of any of the provided embodiments, a suitable biological sample from a subject, such as from a patient of interest, i.e., a patient suspected of having or known to have cancer, is obtained. In some embodiments, the sample is one that is known or suspected of containing T cells, such as T cells that may be or may likely express an endogenous T cell receptor (TCR) that is specific to, binds or recognizes a tumor-associated antigen. The biological sample may be derived from any initial source that would contain or is suspected of containing such T cells. In some aspects, biological sample sources of interest include, but are not limited to, many different physiological sources, e.g. tissue derived samples, e.g. homogenates, and blood or derivatives thereof.

Any of a variety of biological samples can be used as a source of potentially reactive T cells. Although the tumor and downstream lymph nodes may have the highest frequency of reactive T cells (Powell et al., Clin. Cancer. Res., 2014), other sample sources also can be used. In some cases the sample is a tumor sample, a tertiary lymphoid site, a draining lymph node, peripheral blood or bone marrow. In some embodiments, the biological sample is a tumor sample. In some embodiments, the biological sample is a lymph sample. In some embodiments, the biological sample is a peripheral blood sample.

The biological samples include tissue, fluid, and other samples taken directly from the subject to obtain an input sample, or can undergo one or more processing steps, such as separation, e.g. selection or enrichment, centrifugation, washing, and/or incubation, to obtain or produce an input sample. The input sample containing T cells can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In many embodiments, the sample may be derived from fluids in which the T cells of interest are at least suspected of being present. In many embodiments, a suitable initial source for the sample is blood. In some embodiments, the biological sample is a blood-derived sample. The blood-derived sample may be derived from whole blood or a fraction thereof, e.g. serum, plasma, etc., where in many embodiments the sample is derived from blood cells harvested from whole blood. In some aspects, the sample source contains mononuclear cells. For example, a biological sample is or contains peripheral blood mononuclear cells (PBMCs) or is derived from PBMCs.

In some embodiments in which the sample is a PBMC derived sample, the sample is generally a fluid PBMC derived sample. Any convenient methodology for producing a fluid PBMC sample may be employed. In many embodiments, the fluid PBMC derived sample is prepared by separating PBMCs from whole blood, i.e., collecting PBMCs, e.g., by centrifugation (such as by Ficoll-Hypaque density gradient centrifugation, where representative protocols for such separation procedures are disclosed in WO 98/15646 and U.S. Pat. No. 5,985,565).

In some embodiments, the sample is a tumor sample and thereby provides a source of tumor-infiltrating lymphocytes (TILs). In some aspects, TILs are T cells that have left the bloodstream of a subject and migrated into or infiltrated a tumor. In particular aspects, TILs are reactive to a tumor antigen.

A patient tumor sample may be obtained by any of a variety of methods in which the method obtains a sample that contains a mixture of tumor and TIL cells. In some embodiments, the tumor sample is obtained by surgical resection. In some embodiments, the tumor sample is obtained by needle biopsy. In general, the tumor sample may be from any solid tumor, including primary tumors, invasive tumors or metastatic tumors. The tumor sample may also be a liquid tumor, such as a tumor obtained from a hematological malignancy. The solid tumor may be of any cancer type, including, but not limited to, breast, pancreatic, prostate, colorectal, lung, brain, renal, stomach (gastrointestinal), and skin (including but not limited to squamous cell carcinoma, basal cell carcinoma, and melanoma). In particular embodiments, the tumor is any as described in Section IV. In some embodiments, the tumor sample is from the same tumor source as was used to identify a neoantigen for preparing peptide neoepitopes.

In provided embodiments, the obtained tumor sample is fragmented into small pieces of between at or about 1 mm³ and at or about 8 mm³ in size, such as between at or about 1 mm³ and at or about 6 mm³, between at or about 1 mm³ and at or about 4 mm³, between at or about 1 mm³ and at or about 2 mm³. In some embodiments, the tumor fragment is from about 2-3 mm³. In some embodiments, the tumor fragment is from about 1-2 mm³. In some embodiments, the tumor fragment is obtained by physical fragmentation, such as by dissection. In some embodiments, the tumor fragment is obtained by sharp dissection.

In some of any of the provided embodiments, the obtained tumor sample is fragmented into small pieces of between at or about 1 mm and at or about 8 mm in diameter, such as between at or about 1 mm and at or about 6 mmin diameter, between at or about 1 mm and at or about 4 mm in diameter, between at or about 1 mm and at or about 2 mm in diameter. In some embodiments, the tumor fragment is from about 2-3 mm in diameter. In some embodiments, the tumor fragment is from about 1-2 mm in diameter. In some embodiments, the tumor fragment is obtained by physical fragmentation, such as by dissection. In some embodiments, the tumor fragment is obtained by sharp dissection.

In some embodiments, the tumor sample is cryopreserved prior to fragmentation. In some embodiments, the tumor fragments are cryopreserved.

In some embodiments, obtained tumor fragments are used directly as an input sample of T cells in the provided methods. In some embodiments, the obtained tumor fragments are placed into culture media under conditions and with appropriate nutrients to mediate T cell activation and/or sustain T cell expansion, such as any of the conditions described in Subsection I.A.2 below for stimulation of T cells. In some embodiments 1 to 500 tumor fragments (e.g. each 1-8 mm in size) are placed in an appropriate culture vessel under conditions for expansion. In some embodiments, 10, 20, 30, 40, 50 or more fragments are cultured under conditions for expansion. The culture vessel can be a microwell, flask, tube, bag, plate or other closed system device. In some embodiments the culture vessel is a closed container that provides a gas-permeable surface area, such as a gas permeable flask. An exemplary culture vessel that provides a gas-permeable surface area include G-Rex plates or flasks. In some embodiments, 1 tumor fragment (about 1-8 mm in diameter) is placed for each about 2 cm² area of a culture vessel. The particular culture vessel can be chosen based on the number of tumor fragments available and/or the desired yield of cells. The choice of culture vessel (e.g. G-Rex) can be chosen by linearly scaling the number of fragments seeded to the surface area of the culture vessel. In some embodiments, the surface areas of the culture vessel is about 2 cm² (e.g. G-Rex 24 well plate) and about 1 tumor fragment (about 1-8 mm in diameter) is placed in the culture vessel. In some embodiments, the surface area of a culture vessel is about 10 cm² (e.g. G-Rex 10 or G-Rex 10M) and about 5 tumor fragments (each about 1-8 mm in diameter) are placed in the culture vessel. In some embodiments, the surface area of a culture vessel is about 100 cm² (e.g. G-Rex 100 M/100M-CS) and about 50 tumor fragments (each about 1-8 mm in diameter) are placed in the culture vessel. In some embodiments, the surface area of a culture vessel is about 500 cm² (e.g. G-Rex 500 M/500M-CS) and about 250 tumor fragments (each about 1-8 mm in diameter) are placed in the culture vessel. In aspects of the provided methods, increasing the size of the culture vessel, and hence the number of tumor fragments per vessel, may decrease variability as compared to methods involving smaller culture vessels and/or fewer fragments per vessel, such by pooling larger numbers of fragments to minimize inter-tumor variability among fragments.

In some embodiments, the tumor fragments are placed in culture media for stimulation of the cells using any of the conditions described in Subsection I.A.2 below. In some embodiments, the culture media is a serum-free media containing recombinant cytokine from IL-2, IL-7, IL-15, and/or IL-21, such as recombinant IL-12 or recombinant IL-7 and IL-15. The concentration of recombinant cytokine can include any as described. In particular embodiments, the culture media is a serum-free media containing recombinant IL-2, such as from at or about 300 IU/mL to at or about 1000 IU/mL, for example at or about 300 IU/mL. In some embodiments, the culture media is a serum-free media containing an anti-CD3 antibody and/or CD28 targeting agent (e.g. anti-CD28 antibody) and one or more recombinant cytokines (e.g. IL-2).

In some embodiments, tumor fragments are used as a source to prepare a single cell suspension for use as an input sample of T cells in the provided methods. In some embodiments, the provided methods involve obtaining cells from the tumor fragments, such as by enzymatic digestion of tumor fragments to obtain TILs. Enzymatic digestion can be carried out using a collagenase, such as a type IV collagenase or a type I/II collagenase. The enzyme, such as a collagenase, can be present in media for the enzymatic digestion at a concentration of from at or about 1 mg/mL to at or about 5 mg/mL, such as at or about 1 mg/mL, at or about 2 mg/mL, at or about 3 mg/mL, at or about 4 mg/mL or at or about 5 mg/mL, or any value between any of the foregoing. In some embodiments, the enzymatic digestion is with a media that includes type IV collagenase, such as from at or about 1 mg/mL to at or about 5 mg/mL. In some embodiments, the enzymatic digestion is with a media that includes type I/II collagenase, such as from at or about 1 mg/mL to at or about 5 mg/mL. In some embodiments, if a more gentle digestion is desired at or about 1 mg/mL collagenase is used. In some embodiments, if a more complete digestion is desired a higher concentration of collagenase is used, such as at or abut 5 mg/mL collagenase. In other embodiments, enzymes from the Miltenyi human tumor dissociation kit can be used (e.g. Cat. O. 130-095-929; Miltenyi Biotec). The enzymatic media containing the enzyme can be a serum-free media, such as any as described. In particular embodiments, enzymatic media includes collagenase, e.g., Roswell Park Memorial Institute (RPMI) 1640 buffer, 2 mM glutamate (e.g. GlutaMAX), 10 mg/mL gentamicin, 30 units/mL of DNase and 1.0 mg/mL of collagenase). In some embodiments, enzymatic media includes a serum free media (e.g. OpTmizer) containing 2 mM glutamate (e.g. GlutaMAX), 10 µg/mL gentamicin, an immune cell serum replacement (e.g. CTS Immune Cell Serum Replacement) and 1.0 mg/mL to 5.0 mg/mL of collagenase). In some embodiments, the collagenase is a type IV collagenase. In some embodiments, the collagenase is a type I/II collagenase.

The tumor fragment is then mechanically dissected to dissociate the TILs, e.g., using a tissue dissociator. An example of a tissue dissociator is GentleMACs^{™} (Miltenyi Biotec) to homogenize the tissue. Tumor digests may be produced by placing the tumor in enzymatic media and mechanically dissociating the tumor for approximately 1 minute, followed by incubation for 30 minutes at 37 °C in 5% CO₂, followed by repeated cycles of mechanical dissociation and incubation under the foregoing conditions until only small tissue pieces are present. At the end of this process, if the cell suspension contains a large number of red blood cells or dead cells, a density gradient separation using FICOLL can be performed to remove these cells. In some cases, separation can be achieved by centrifugation, in which case the cell pellet can be resuspended and strained through a e.g. 70 µm strainer to remove debris. Alternative methods known in the art may be used, such as those described in U.S. Patent Application Publication No. 2012/0244133 Al, the disclosure of which is incorporated by reference herein. Any of the foregoing methods may be used in any of the embodiments described herein for methods of obtaining TILs for use in the provided methods.

In some embodiments, a single cell suspension for use as an input sample of T cells comprises from at or about 1 x 10⁶ T cells per gram of tumor sample from the subject to at or about 1000 x 10⁶ T cells per gram of the tumor sample from the subject, such as 1 x 10⁶ to 500 x 10⁶ T cells per gram of the tumor sample from the subject, 1 x 10⁶ to 100 x 10⁶ T cells per gram of the tumor sample from the subject, 1 x 10⁶ to 50 x 10⁶ T cells per gram of the tumor sample from the subject, 1 x 10⁶ yo 10 x 10⁶ T cells per gram of the tumor sample from the subject, 10 x 10⁶ to 1000 x 10⁶ T cells per gram of the tumor sample from the subject, 10 x 10⁶ to 100 x 10⁶ T cells per gram of the tumor sample from the subject, 10 x 10⁶ to 500 x 10⁶ T cells per gram of the tumor sample from the subject, 10 x 10⁶ to 50 x 10⁶ T cells per gram of the tumor sample from the subject, 50 x 10⁶ to 1000 x 10⁶ T cells per gram of the tumor sample from the subject, 50 x 10⁶ to 500 x 10⁶ T cells per gram of the tumor sample from the subject, 50 x 10⁶ to 100 x 10⁶ T cells per gram of the tumor sample from the subject, 100 x 10⁶ to 1000 x 10⁶ T cells per gram of the tumor sample from the subject, 100 x 10⁶ to 500 x 10⁶ T cells per gram of the tumor sample from the subject, or 500 x 10⁶ to 1000 x 10⁶ T cells per gram of the tumor sample from the subject. In some embodiments, a single cell suspension for use as an input sample of T cells comprises from at or about or at least at or about 10 x 10⁶ T cells , 20 x 10⁶ T cells, 30 x 10⁶ T cells, 40 x 10⁶ T cells, 50 x 10⁶ T cells, 60 x 10⁶ T cells, 70 x 10⁶ T cells, 80 x 10⁶ T cells, 90 x 10⁶ T cells, 100 x 10⁶ T cells, each per gram of tumor sample from the subject In some embodiments, a single cell suspension for use as an input sample of T cells comprises from at or about 10 x 10⁶ T cells per gram of tumor sample from the subject to at or about 100 x 10⁶ T cells per gram of the tumor sample from the subject.

In some embodiments, digested cells from the tumor fragments are placed into culture media as a single cell suspension under conditions and with appropriate nutrients to mediate T cell activation and/or sustain T cell expansion, such as any of the conditions described in Subsection I.A.2 below for stimulation of T cells. The cells are seeded at a particular density suitable for the particular culture vessel. The culture vessel can be a microwell, flask, tube, bag or other closed system device. In some embodiments the culture vessel is a closed container that provides a gas-permeable surface area, such as a gas permeable flask. An exemplary culture vessel that provides a gas-permeable surface area include G-Rex plates or flasks. In some embodiments approximately 5 x 10⁵ to 2 x 10⁶ cells of an enzymatically digested single cell suspension are seeded for each about 2 cm² area of a culture vessel. The particular culture vessel can be chosen based on the number of cells available and/or the desired yield of cells. The choice of culture vessel (e.g. G-Rex) can be chosen by linearly scaling the number of cells seeded to the surface area of the culture vessel. In some embodiments, the surface areas of the culture vessel is about 2 cm² (e.g. G-Rex 24 well plate) and about 5 x 10⁵ to 2 x 10⁶ cells of an enzymatically digested single cell suspension is placed in the culture vessel. In some embodiments, the surface area of a culture vessel is about 10 cm² (e.g. G-Rex 10 or G-Rex 10M) and about 2.5 x 10⁶ to 1 x 10⁷ cells of an enzymatically digested single cell suspension are placed in the culture vessel. In some embodiments, the surface area of a culture vessel is about 100 cm² (e.g. G-Rex 100 M/100M-CS) and about 2.5 x 10⁷ to 1 x 10⁸ cells of an enzymatically digested single cell suspension are placed in the culture vessel. In some embodiments, the surface area of a culture vessel is about 500 cm² (e.g. G-Rex 500 M/500M-CS) and about 1.25 x 10⁸ to 5 x 10⁸ cells of an enzymatically digested single cell suspension are placed in the culture vessel.

In some embodiments, the culture media is a serum-free media containing recombinant IL-2. In some embodiments, one or more additional T cell stimulating agent can also be included. In some embodiments, the culture media is a serum-free media containing an anti-CD3 antibody and/or a CD28 targeting agent (e.g. anti-CD28 antibody) and one or more recombinant cytokines (e.g. IL-2, IL-7, IL-15 and/or IL-21).

The sample may be obtained from a variety of different subjects/patients/hosts. Generally such hosts are "mammals" or "mammalian," where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In many embodiments, the hosts will be humans.

In some aspects, the subject is a human. Accordingly, the cells in some embodiments are primary cells, e.g., primary human cells. In some embodiments, the sample is autologous to a subject to be treated, such as a subject who is a patient in need of a particular therapeutic intervention, such as the adoptive cell therapy for which cells are being isolated, processed, and/or expanded in accord with the provided methods. In some embodiments, the sample is allogenic to a subject to be treated.

In some of any of the provided embodiments, the biological sample is a lymph sourced sample or a tumor sourced sample, and wherein: the number of cells at the initiation of the culturing is between at or about 10 x 10⁶ and 100 x 10⁶ total viable cells, 20 x 10⁶ and 100 x 10⁶ total viable cells, or 12 x 10⁶ and 43 x 10⁶ total viable cells; or is at or about 10 x 10⁶ total viable cells, at or about 12 x 10⁶ total viable cells, 20 x 10⁶ total viable cells, 40 x 10⁶ total viable cells, 60 x 10⁶ total viable cells, or 100 x 10⁶ total viable cells, , or any value between any of the foregoing. In some embodiments, the percentage of tumor reactive T cells at the initiation of the culturing is between at or about 1% and at or about 90%, at or about 1% and at or about 75%, at or about 1% and at or about 50%, at or about 1% and at or about 25% or at or about 1% and at or about 14%.

### 2. Stimulation of T cells for Initial Expansion

In aspects of the provided methods, the T cells from the input sample (input or first population of T cells, such as present in a resected tumor fragment or a single cell suspension therefrom) are incubated or cultured in the presence of one or more T cell stimulatory agent(s) under conditions for stimulating the T cells, such as to expand the T cells. In some embodiments, the incubation or culturing with one or more T cell stimulatory agent(s) results in expansion (first expansion) or outgrowth of selected T cells, or a desired subset or subtype thereof or for viable cells thereof, for use in subsequent steps of the provided methods. Non-limiting examples of T cell stimulatory agent(s) and conditions for incubation or culture are described herein.

Thus, among the provided methods are methods of culturing T cells for manufacture of tumor reactive T cells in which T cells are cultured or incubated in the presence of a T cell stimulatory agent under conditions to expand T cells.

In some embodiments, the T cell stimulatory agent(s) include a recombinant T cell stimulating cytokine, such as IL-2, IL-7, IL-15, IL-21, IL-25, IL-23, IL-27 and/or IL-35. In some embodiments, the T cell stimulating cytokine includes IL-2, alone or in combination with another cytokine from among IL-7, IL-15, IL-21, IL-25, IL-23, IL-27 and/or IL-35. In some embodiments, the T cell stimulatory agent(s) include a recombinant T cell stimulating cytokine, such as IL-2, IL-7, IL-15, IL-21, IL-25 and/or IL-23. In some embodiments, the T cell stimulating cytokine includes IL-2, alone or in combination with another cytokine from among IL-7, IL-15, IL-21, IL-25 and/or IL-23. In some embodiments, the T cell stimulating cytokine is one, two, three or more of IL-2, IL-7, IL-15 and IL-21. In some embodiments, the T cell stimulating cytokine includes IL-2, alone or in combination with another cytokine from among IL-7, IL-15, and/or IL-21. In some embodiments, the T cell stimulating cytokine includes IL-2, alone or in combination with another cytokine from IL-25, IL-23, IL-27 and/or IL-35. In some embodiments, the T cell stimulating cytokines are IL-7 and IL-15.

In some embodiments, the choice of cytokine or combination of cytokines is within the level of a skilled artisan, so long as the cytokines or cytokines provide activity to stimulate the T cells to expand. The activity to stimulate tumor reactive T cells can be direct or indirect. In some embodiments, the one or more cytokines directly stimulate tumor reactive T cells to expand or proliferate. In some embodiments, the one or more cytokines suppress Tregulatory T cells, thereby indirectly stimulating or enhancing proliferation of desired tumor reactive T cells.

In some embodiments, the incubation with a T cell stimulatory agent(s) for the initial expansion does not include incubation with an agent or agents that engage CD3 and a costimulatory molecule, such as CD28. In some embodiments, the incubation with a T cell stimulatory agent(s) for the initial expansion does not include incubation with an anti-CD3 antibody, such as OKT3. In some embodiments, the incubation with a T cell stimulatory agent(s) for the initial expansion does not include incubation with an anti-CD3 (e.g. OKT3)/anti-CD28 antibody, presented by APC's, immobilized on a solid surface (e.g. bead), or as a soluble antibody. In some embodiment, the incubation with a T cell stimulatory agent(s) for the initial expansion does not include incubation with soluble anti-CD3, such as OKT3. In some embodiment, the incubation with a T cell stimulatory agent(s) for the initial expansion does not include incubation with an anti-CD3/anti-CD28, including such reagents immobilized on beads, e.g. as provided by Dynabeads. In some embodiments, the incubation with a T cell stimulatory agent(s) for the initial expansion does not include incubation with APCs, such as irradiated APCs. In some embodiments, the incubation with a T cell stimulatory agent(s) for the initial expansion does not include incubation with non-dividing PBMCs, such as irradiated PBMCs.

In some of any of the provided embodiments, the T cell stimulatory agent(s) is selected from an agent that initiates TCR/CD3 intracellular signaling and an agent that initiates signaling via a costimulatory receptor. In some of any of the provided embodiments, the agent that initiates TCR/CD3 intracellular signaling is an anti-CD3 antibody, such as OKT3. In some of any of the provided embodiments, the agent that initiates signaling via a costimulatory receptor comprises peripheral blood mononuclear cells (PBMCs), optionally non-dividing or irradiated PBMCs. In some of any of the provided embodiments, the agent that initiates signaling via a costimulatory receptor is an anti-CD28 antibody. In some of any of the provided embodiments, the T cell stimulatory agent(s) is an anti-CD3 antibody and an anti-CD28 antibody that each are soluble. In particular embodiments, one or more recombinant cytokines also are present as additional T cell stimulatory agents during the incubation. In some embodiments, the incubation with a T cell stimulatory agent(s) include incubation with at least one T cell stimulating recombinant cytokine (e.g. recombinant IL-2, IL-7, IL-21, IL-15, IL-25, IL-23, IL-27, and/or IL-35 ) and a further T cell stimulatory agent(s) that engage CD3 and/or a costimulatory molecule (e.g. CD28) on T cells.

In embodiments of the provided methods, the stimulating conditions include one or more agent, e.g., ligand, which turns on or initiates TCR/CD3 intracellular signaling cascade in a T cell and/or a costimulatory signal in a T cell. Such agents can include antibodies, such as those specific for a TCR component, e.g., anti-CD3, and/or costimulatory receptor, e.g. anti-CD28 or anti-4-1BB. In some embodiments, such agents are added to the culture medium as soluble antibodies. In other embodiments, such agents are bound to solid support such as a bead. In some embodiments, the T cell stimulatory agent(s) includes anti-CD3/CD28 conjugated magnetic beads (e.g., DYNABEADS^{®} M-450 CD3/CD28 T Cell Expander).

An anti-CD3 antibody can include any antibody directed against or that can specifically bind the CD3 receptor on the surface of T cells, typically human CD3 on human T cells. Anti-CD3 antibodies include OKT3, also known as muromonab. Anti-CD3 antibodies also include theUHCTI clone, also known as T3 and CD3E. Other anti-CD3 antibodies include, for example, otelixizumab, teplizumab, and visilizumab. The anti-CD3 antibody can be added as a soluble reagent or bound to a bead. In particular embodiments, the anti-CD3 antibody is soluble.

In particular embodiments, the T cell stimulatory agent(s) include an anti-CD3 antibody, which is added to the cell culture medium during the incubation. In some embodiments, the anti-CD3 antibody is added at a concentration ranging between at or about 0.1 ng/mL and 50 ng/mL, such between at or about 0.5 ng/mL and at or about 50 ng/mL, between at or about 0.5 ng/mL and at or about 30 ng/mL, between at or about 0.5 ng/mL and at or about 15 ng/mL, between at or about 0.5 ng/mL and at or about 5 ng/mL, between at or about 0.5 ng/mL and at or about 1 ng/mL, between at or about 1 ng/mL and at or about 50 ng/mL, between at or about 1 ng/mL and at or about 30 ng/mL, between at or about 1 ng/mL and at or about 15 ng/mL, between at or about 1 ng/mL and at or about 5 ng/mL, between at or about 5 ng/mL and at or about 50 ng/mL, between at or about 5 ng/mL and at or about 30 ng/mL, between at or about 5 ng/mL and at or about 15 ng/mL, between at or about 15 ng/mL and at or 50 ng/mL, between at or about 15 ng/mL and at or about 30 ng/mL or between at or about 30 ng/mL and at or about 50 ng/mL, each inclusive.

In particular embodiments, the anti-CD3 antibody is OKT3. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 µg/mL of OKT3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT3 antibody.

In some embodiments, the T cell stimulatory agent(s) includes incubation with an anti-CD3 antibody and incubation with a further agent that specifically binds to CD28 or stimulates or induces a CD28-mediated signal in cells. In some embodiments, the CD28-mediated signal can be initiated or provided by anti-CD28 antibody or antigen-binding fragment thereof. In some embodiments, the CD28-mediated signal can be provided by antigen-presenting feeder cells (APCs), such as peripheral blood mononuclear cells (PBMC).

In some embodiments, the T cell stimulatory agent(s) can include adding to the population of T cells feeder cells, such as non-dividing peripheral blood mononuclear cells (PBMC). In some aspects, the non-dividing feeder cells can comprise gamma- irradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells. In some embodiments, the resulting population of cells contains at least about 5, 10, 20, or 40 or more PBMC feeder cells for each T lymphocyte in the initial population to be expanded. In some embodiments, the ratio of T cells to PBMCs and/or antigen-presenting cells is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500.

In some embodiments, the stimulation does not include incubation with PBMCs or other feeder cells, such as non-divided or irradiated PBMCs or other non-dividing or irradiated feeder cells.

In some embodiments, the T cell stimulatory agent(s) can include adding to the population of cells an anti-CD28 antibody or antigen-binding fragment thereof. An anti-CD28 antibody can include any antibody directed against or that can specifically bind the CD28 receptor on the surface of T cells. Non-limiting examples of anti-CD28 antibodies include NA/LE (e.g. BD Pharmingen), IM1376 (e.g. Beckman Coulter), or 15E8 (e.g. Miltenyi Biotec). The anti-CD28 antibody can be added as a soluble reagent or bound to a bead. In particular embodiments, the anti-CD3 antibody is soluble. In some embodiments, the anti-CD28 antibody is added at a concentration ranging between at or about 1 ng/mL and 1000 ng/mL, between at or about 1 ng/mL and 500 ng/mL, between at or about 1 ng/mL and at or about 100 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 100 ng/mL, between at or about 100 ng/mL and at or about 1000 ng/mL, between at or about 100 ng/mL and at or about 500 ng/mL or between at or about 500 ng/mL and at or about 1000 ng/mL.

In some embodiments, the T cell stimulatory agent(s) include one or more recombinant cytokine. In some embodiments, the cytokine is added or is exogenous to the culture media. Thus, in some embodiments, one or more further recombinant cytokine also is included during the culturing. In some embodiments, the recombinant cytokine can include one or more of IL-2, IL-7, IL-15, IL-21, IL-25, IL-23, IL-27 and/or IL-35. In some embodiments, the recombinant cytokine can include one or more of IL-2, IL-7, IL-15, IL-21, IL-25 and/or IL-23. In some embodiments, the culturing and incubation is carried out in the presence of recombinant IL-2, IL-15 and IL-7. In some embodiments, the culturing is carried out in the presence of a IL-2. In some embodiments, the culturing is carried out in the presence of IL-15 and IL-17, which, in some aspects does not additionally include IL-2. In particular embodiments, the recombinant cytokine(s) is human.

The recombinant cytokine generally is a recombinant human protein. In particular embodiments, the recombinant cytokine is present in the cell culture medium during the incubation at a concentration of at least or at least about 0.5 IU/mL, at least or at least about 1.0 IU/mL, at least or at least about 5 IU/mL, at least at or about or at or about 10 IU/mL, at least at or about or at or about 100 IU/mL, at least at or about or at or about 1000 IU/mL, at least at or about or at or about 1500 IU/mL, at least at or about or at or about 2000 IU/mL, at least at or about or at or about 2500 IU/mL, at least at or about or at or about 3000 IU/mL, at least at or about or at or about 3500 IU/mL, at least at or about or at or about 4000 IU/mL, at least at or about or at or about 4500 IU/mL, at least at or about or at or about 5000 IU/mL, at least at or about or at or about 5500 IU/mL, at least at or about or at or about 6000 IU/mL, at least at or about or at or about 6500 IU/mL, at least at or about or at or about 7000 IU/mL, at least at or about or at or about 7500 IU/mL, or at least at or about or at or about 8000 IU/mL. In an embodiment, the cell culture medium comprises between at or about 10 IU/mL and at or about 100 IU/mL, at or about 100 IU/mL and at or about 1000 IU/mL, at or about 1000 and at or about 2000 IU/mL, between at or about 2000 and at or about 3000 IU/mL, between at or about 3000 and 4000 at or about IU/mL, between at or about 4000 and at or about 5000 IU/mL, between at or about 5000 and at or about 6000 IU/mL, between at or about 6000 and at or about 7000 IU/mL, between at or about 7000 and at or about 8000 IU/mL, each inclusive.

In some embodiments, recombinant IL-2 is present in the cell culture medium. In some aspects, IL-2 is the only recombinant cytokine added to the culture. In some aspects, recombinant IL-2 and one other recombinant modulatory cytokine from IL-7, IL-15, IL-21, IL-23, IL-25, IL-27 or IL-35 is added to the culture. IL-2 is a cytokine that supports T cell recovery and proliferation. IL-2 also supports the homeostasis of T cells, thereby supporting their phenotype, differentiation status, and immune memory. In some cases, induction of regulatory T cells in the tumor microenvironment may lead to low bioavailability of IL-2. Recombinant IL-2 has been regularly used in broad expansion of T cells in various contexts. Recombinant IL-2 is commercially available. In particular embodiments, recombinant IL-2 is GMP grade (e.g. MACS GMP Recombinant Human IL-2, Miltenyi Biotec).

Recombinant IL-2 can be included in cell culture media during various stages of the provided process. In some cases, recombinant IL-2 can be included in the initial T cell expansion (first expansion), such as to promote TIL outgrowth and allow their proliferation from solid tumor. IL-2 also can be included in antigen-presenting cell co-culture as described in Section I.B.2, such as to allow for peak activation of neo-antigen reactive T prior to their separation or selection. In some cases, recombinant IL-2 can also be included in cultures to expand tumor-reactive T cells during the second expansion phase, such as described in Section I.D.

In some embodiments, recombinant IL-2 is added to the culture medium at a concentration between at or about 10 IU/mL and at or about 1000 IU/mL, such as between at or about 10 IU/mL and at or about 600 IU/mL, between at or about 10 IU/mL and at or about 400 IU/mL, between at or about 10 IU/mL and at or about 200 IU/mL, between at or about 10 IU/mL and at or about 100 IU/mL, between at or about 10 IU/mL and at or about 50 IU/mL, between at or about 50 IU/mL and at or about 1000 IU/mL, between at or about 50 IU/mL and at or about 600 IU/mL, between at or about 50 IU/mL and at or about 400 IU/mL, between at or about 50 IU/mL and at or about 200 IU/mL, between at or about 50 IU/mL and at or about 100 IU/mL, between at or about 100 IU/mL and at or about 1000 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 1000 IU/mL, between at or about 200 IU/mL and at or about 600 IU/mL, between at or about 200 IU/mL and at or about 400 IU/mL, between at or about 400 IU/mL and at or about 1000 IU/mL, between at or about 400 IU/mL and at or about 600 IU/mL or between at or about 600 IU/mL and at or about 1000 IU/mL. In some embodiments, recombinant IL-2 is present in an amount that is between 50 and 400 IU/mL.

In some embodiments, the initial expansion is carried out in the presence of recombinant IL-2 added at a concentration of between 200 IU/mL and at or about 1000 IU/mL. In some embodiments, recombinant IL-2 Is added to the culture medium at a concentration of at or about 200 IU/mL, at or about 300 IU/mL, at or about 400 IU/mL, at or about 500 IU/mL, at or about 600 IU/mL, at or about 700 IU/mL, at or about 800 IU/mL, at or about 900 IU/mL, at or about 1000 IU/mL, or any concentration between any of the foregoing. In some embodiments, recombinant IL-2 Is added to the culture medium at a concentration of at or about 300 IU/mL. In some embodiments, recombinant IL-2 is added to the culture medium at a concentration of at or about 600 IU/mL. In some embodimetns, recombinant IL-2 is added to the culture medium at a concentration of at or about 1000 IU/mL. In some embodiments, at least one other recombinant modulatory cytokine from IL-7, IL-15, IL-21, IL-23, IL-25, IL-27 or IL-35 is added to the culture meduium.

In some embodiments, the incubation is carried out with a higher dose IL-2. In some aspects, IL-2 is the only recombinant cytokine added to the culture.

In some embodiments, the recombinant IL-2 is added to the culture medium at a concentration between at or about 1000 IU/mL at or about 8000 IU/mL, such as between at or about 1000 IU/mL and at or about 7000 IU/mL, between at or about 1000 IU/mL and at or about 6000 IU/mL, between at or about 1000 IU/mL and at or about 5000 IU/mL, between at or about 1000 IU/mL and at or about 4000 IU/mL, between at or about 1000 IU/mL and at or about 2000 IU/mL, 2000 IU/mL at or about 8000 IU/mL, between at or about 2000 IU/mL and at or about 7000 IU/mL, between at or about 2000 IU/mL and at or about 6000 IU/mL, between at or about 2000 IU/mL and at or about 5000 IU/mL, between at or about 2000 IU/mL and at or about 4000 IU/mL, 4000 IU/mL at or about 8000 IU/mL, between at or about 4000 IU/mL and at or about 7000 IU/mL, between at or about 4000 IU/mL and at or about 6000 IU/mL, between at or about 4000 IU/mL and at or about 5000 IU/mL, between at or about 5000 IU/mL at or about 8000 IU/mL, between at or about 5000 IU/mL and at or about 7000 IU/mL, between at or about 5000 IU/mL and at or about 6000 IU/mL, between at or about 6000 IU/mL at or about 8000 IU/mL, between at or about 6000 IU/mL and at or about 7000 IU/mL or between at or about 7000 IU/mL and at or about 8000 IU/mL. In some embodiments, recombinant IL-2 is present in an amount that is or is about 6000 IU/mL.

In some embodiments, recombinant IL-15 is present in the cell culture medium. IL-15 is a cytokine that is involved in memory T cell homeostasis and activation. In some cases, IL-15 can promote effector functions of antigen-experienced T cells in the absence of antigen and prevent their differentiation into an exhausted phenotype. IL-15 also plays a role in T cell proliferation. Recombinant IL-15 is commercially available. In particular embodiments, recombinant IL-15 is GMP grade (e.g. MACS GMP Recombinant Human IL-15, Miltenyi Biotec).

Recombinant IL-15 can be included in cell culture media during various stages of the provided process. In some cases, recombinant IL-15 can be included in the initial T cell expansion (first expansion), such as to promote TIL expansion to promote their outgrowth and allow their proliferation and/or stabilize phenotype from solid tumor. Recombinant IL-15 also can be included in antigen-presenting cell co-culture as described in Section I.B.2, such as to allow for peak activation of neo-antigen reactive T prior to their separation or selection. In some cases, recombinant IL-15 can also be included in cultures to expand tumor-reactive T cells during the second expansion phase, such as described in Section I.D. In some cases, recombinant IL-15 can be combined with recombinant IL-7 to provide for activation, survival and/or expansion of tumor-reactive T cells in the provided methods. In some such embodiments, the combination of recombinant IL-7 and IL-15 is an alternative to the use of recombinant IL-2 in the culture, and the culture media does not additionally contain recombinant IL-2.

In some embodiments, the recombinant IL-15 is added to the culture medium at a concentration between at or about 10 IU/mL and 500 IU/mL, such as between at or about 10 IU/mL and at or about 400 IU/mL, between at or about 10 IU/mL and at or about 300 IU/mL, between at or about 10 IU/mL and at or about 200 IU/mL, between at or about 10 IU/mL and at or about 100 IU/mL, between at or about 10 IU/mL and at or about 70 IU/mL, between at or about 10 IU/mL and at or about 50 IU/mL, between at or about 10 IU/mL and at or about 30 IU /mL, between at or about 30 IU/mL and 500 IU/mL, between at or about 30 IU/mL and at or about 400 IU/mL, between at or about 30 IU/mL and at or about 300 IU/mL, between at or about 30 IU/mL and at or about 200 IU/mL, between at or about 30 IU/mL and at or about 100 IU/mL, between at or about 30 IU/mL and at or about 70 IU/mL, between at or about 30 IU/mL and at or about 50 IU/mL, between at or about 50 IU/mL and at or about 400 IU/mL, between at or about 50 IU/mL and at or about 500 IU/mL, between at or about 50 IU/mL and at or about 300 IU/mL, between at or about 50 IU/mL and at or about 200 IU/mL, between at or about 50 IU/mL and at or about 100 IU/mL, between at or about 50 IU/mL and at or about 70 IU/mL, between at or about 70 IU/mL and at or about 500 IU/mL, between at or about 70 IU/mL and at or about 400 IU/mL, between at or about 70 IU/mL and at or about 300 IU/mL, between at or about 70 IU/mL and at or about 200 IU/mL, between at or about 70 IU/mL and at or about 100 IU/mL, between at or about 100 IU/mL and at or about 500 IU/mL, between at or about 100 IU/mL and at or about 400 IU/mL, between at or about 100 IU/mL and at or about 300 IU/mL, between at or about 100 IU/mL and at or about 200 IU/mL, between at or about 200 IU/mL and at or about 500 IU/mL, between at or about 200 IU/mL and at or about 400 IU/mL, between at or about 200 IU/mL and at or about 300 IU/mL, between at or about 300 IU/mL and at or about 500 IU/mL, between at or about 200 IU/mL and at or about 400 IU/mL, or between at or about 400 IU/mL and at or about 500 IU/mL. In some embodiments, the IL-15 is added to the culture medium in an amount between at or about 100 IU/mL and at or about 200 IU/mL. In some embodiments, the IL-15 is added to the culture medium at or about 180 IU/mL.

In some embodiments, the incubation is carried out with a higher dose IL-15.

In some embodiments, the recombinant IL-15 is added to the culture medium at a concentration between at or about 500 IU/mL and at or about 5000 IU/mL, such as between at or about 500 IU/mL and at or about 4000 IU/mL, between at or about 500 IU/mL and at or about 2000 IU/mL, between at or about 500 IU/mL and at or about 1500 IU/mL, between at or about 500 IU/mL and at or about 1000 IU/mL, between at or about 500 IU/mL and at or about 750 IU/mL, between at or about 750 IU/mL and at or about 5000 IU/mL, between at or about 750 IU/mL and at or about 4000 IU/mL, between at or about 750 IU/mL and at or about 2000 IU/mL, between at or about 750 IU/mL and at or about 1500 IU/mL, between at or about 750 IU/mL and at or about 1000 IU/mL, between at or about 1000 IU/mL and at or about 5000 IU/mL, between at or about 1000 IU/mL and at or about 4000 IU/mL, between at or about 1000 IU/mL and at or about 2000 IU/mL, between at or about 1000 IU/mL and at or about 1500 IU/mL, between at or about 1500 IU/mL and at or about 5000 IU/mL, between at or about 1500 IU/mL and at or about 4000 IU/mL, between at or about 1500 IU/mL and at or about 2000 IU/mL, between at or about 2000 IU/mL and at or about 5000 IU/mL, such as between at or about 2000 IU/mL and at or about 4000 IU/mL, or between at or about 4000 IU/mL and at or about 5000 IU/mL. In some embodiments, the recombinant IL-15 is added to the cell culture media at a concentration of at or about 500 IU/mL, at or about 600 IU/mL, at or about 700 IU/mL, at or about 800 IU/mL, at or about 900 IU/mL, at or about 1000 IU/mL, at or about 1100 IU/mL, at or about 1200 IU/mL, at or about 1300 IU/mL, at or about 1400 IU/mL, at or about 1500 IU/mL, at or about 1600 IU/mL, at or about 1700 IU/mL, at or about 1800 IU/mL, at or about 1900 IU/mL or at or about 2000 IU/mL, or any concentration between any of the foregoing. In some embodiments, IL-15 is added to the culture medium at a concentration of at or about 1000 IU/mL.

In some embodiments, the initial expansion (e.g. first expansion) is carried out in the presence of recombinant IL-15 added at a concentration of 500 IU/mL to 2000 IU/mL (e.g. at or about 1000 IU/mL). In some embodiments, the initial expansion (e.g. first expansion) is carried out in the presence of recombinant IL-15 added at a concentration of at or about 1000 IU/mL. In some embodiments, at least one other recombinant modulatory cytokine from IL-2, IL-7, IL-21, IL-23, IL-25, IL-27 or IL-35 is added to the culture meduium.

In some embodiments, recombinant IL-15 and IL-2 are added to the culture medium. In some embodiments, recombinant IL-15 is added at a concentration of 500 IU/mL to 2000 IU/mL (e.g. at or about 1000 IU/mL) and recombinant IL-2 is added at a concentration of 200 IU/mL to 1000 IU/mL (e.g. at or about 300 IU/mL). In some embodiments, the initial expansion (e.g. first expansion) is carried out in the presence of recombinant IL-15 added at 1000 IU/mL and recombinant IL-2 added at 300 IU/mL. In some embodiments, at least one other recombinant modulatory cytokine from IL-7, IL-21, IL-23, IL-25, IL-27 or IL-35 is added to the culture meduium.

In some embodiments, recombinant IL-7 is added to the culture medium. In some aspects, recombinant IL-7 is added to the culture media with one or both of IL-2 or IL-15. In some aspects, recombinant IL-7 and recombinant IL-2 are added to the culture media. In some aspects, recombinant IL-7 and recombinant IL-15 are added to the culture media. In some aspects, recombinant IL-7 (e.g. in combination with one or both of IL-2 and IL-15) and one other recombinant modulatory cytokine from IL-23, IL-25, IL-27 or IL-35 is added to the culture meduium. IL-7 is a cytokine that is involved in promoting T cell maintenance and homeostasis. In some cases, IL-7 can boost memory T cell survival and proliferation, particularly the central memory compartment. Recombinant IL-7 is commercially available. In particular embodiments, recombinant IL-7 is GMP grade (e.g. MACS GMP Recombinant Human IL-7, Miltenyi Biotec).

Recombinant IL-7 can be included in cell culture media during various stages of the provided process. In some cases, recombinant IL-7 can be included in the initial T cell expansion (first expansion), such as to promote TIL expansion to promote their outgrowth and allow their proliferation and/or stabilize phenotype from solid tumor. IL-7 also can be included in antigen-presenting cell co-culture as described in Section I.B.2, such as to allow for peak activation of neo-antigen reactive T prior to their separation or selection. In some cases, recombinant IL-7 can also be included in cultures to expand tumor-reactive T cells during the second expansion phase, such as described in Section I.D. Inclusion of recombinant IL-7 in the process can maintain or support expansion of memory T cell subsets in the process. In some cases, recombinant IL-7 can be combined with recombinant IL-15 to provide for activation, survival and/or expansion of tumor-reactive T cells in the provided methods. In some such embodiments, the combination of recombinant IL-7 and IL-15 is an alternative to the use of recombinant IL-2 in the culture, and the culture media does not additionally contain recombinant IL-2.

In some embodiments, the recombinant IL-7 is added to the culture medium at a concentration between at or about 100 IU/mL and at or about 2000 IU/mL, between at or about 100 IU/mL and at or about 1500 IU/mL, between at or about 100 IU/mL and at or about1000 IU/mL, between at or about 100 IU/mL and at or about 800 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 2000 IU/mL, between at or about 200 IU/mL and at or about 1500 IU/mL, between at or about 200 IU/mL and at or about1000 IU/mL, between at or about 200 IU/mL and at or about 800 IU/mL, between at or about 200 IU/mL and at or about 600 IU/mL, between at or about 200 IU/mL and at or about 400 IU/mL, between at or about 400 IU/mL and at or about 2000 IU/mL, between at or about 400 IU/mL and at or about 1500 IU/mL, between at or about 400 IU/mL and at or about1000 IU/mL, between at or about 400 IU/mL and at or about 800 IU/mL, between at or about 400 IU/mL and at or about 600 IU/mL, between at or about 600 IU/mL and at or about 2000 IU/mL, between at or about 600 IU/mL and at or about 1500 IU/mL, between at or about 600 IU/mL and at or about 1000 IU/mL, between at or about 600 IU/mL and at or about 800 IU/mL, between at or about 800 IU/mL and at or about 2000 IU/mL, between at or about 800 IU/mL and at or about 1500 IU/mL, between at or about 800 IU/mL and at or about 1000 IU/mL, between at or about 1000 IU/mL and at or about 2000 IU/mL, between at or about 1000 IU/mL and at or about 1500 IU/mL, between at or about 1500 IU/mL and at or about 2000 IU/mL. In some embodiments, the IL-7 is added to the culture medium in an amount between at or about 1000 IU/mL and at or about 2000 IU/mL. In some embodiments, the IL-7 is added to the culture medium at or about 600 IU/mL. In some embodiments, IL-7 is added to the culture medium at or about 1000 IU/mL.

In some embodiments, recombinant IL-7 and IL-2 are added to the culture medium. In some embodiments, recombinant IL-7 is added at a concentration of 400 IU/mL to 2000 IU/mL (e.g. at or about 600 IU/mL or 1000 IU/mL) and recombinant IL-2 is added at a concentration of 200 IU/mL to 1000 IU/mL (e.g. at or about 300 IU/mL). In some embodiments, the initial expansion (e.g. first expansion) is carried out in the presence of recombinant IL-7 added at 1000 IU/mL and recombinant IL-2 added at 300 IU/mL. In some embodiments, the first expansion is carried out in the presence of recombinant IL-7 added at 600 IU/mL and recombinant IL-2 added at 300 IU/mL. In some embodiments, at least one other recombinant modulatory cytokine from IL-15, IL-21, IL-23, IL-25, IL-27 or IL-35 is added to the culture medium.

In some embodiments, recombinant IL-15 and IL-7 are added to the culture medium. In some embodiments, recombinant IL-15 is added at a concentration of 500 IU/mL to 2000 IU/mL (e.g. at or about 1000 IU/mL) and recombinant IL-7 is added at a concentration of 400 IU/mL to 2000 IU/mL (e.g. at or about 600 IU/mL or 1000 IU/mL). In some embodiments, the initial expansion (e.g. first expansion) is carried out in the presence of recombinant IL-15 added at 1000 IU/mL and recombinant IL-7 added at 1000 IU/mL. In some embodiments, the first expansion is carried out in the presence of recombinant IL-15 added at 1000 IU/mL and recombinant IL-7 added at 600 IU/mL. In some embodiments, at least one other recombinant modulatory cytokine from IL-2, IL-21, IL-23, IL-25, IL-27 or IL-35 is added to the culture medium.

In some embodiments, recombinant IL-21 is added to the culture medium. In some aspects, recombinant IL-21 is added to the culture media with one or both of IL-2, IL-7, or IL-15. In some aspects, recombinant IL-21 and recombinant IL-2 are added to the culture media. In some aspects, recombinant IL-21 and recombinant IL-15 are added to the culture media. In some aspects, recombinant IL-21 (e.g. in combination with one or more IL-2, IL-7 and IL-15) and one other recombinant modulatory cytokine from IL-23, IL-25, IL-27 or IL-35 is added to the culture medium. IL-21 is a cytokine that supports a broad range of T cell activation without increasing regulatory T cell signaling. In some cases, IL-21 can support memory cell stabilization, effector function, and proliferation of antigen-experienced T cells. IL-21 can induce upregulation of effector molecules in both CD4 and CD8 T cells. Recombinant IL-21 is commercially available. In particular embodiments, recombinant IL-21 is GMP grade (e.g. MACS GMP Recombinant Human IL-21, Miltenyi Biotec).

Recombinant IL-21 can be included in cell culture media during various stages of the provided process. In some cases, recombinant IL-21 can be included in the initial T cell expansion (first expansion), such as to promote TIL outgrowth from solid tumor, including by stabilizing memory T cell activation, function and/or proliferation. In some aspects, the presence of IL-21 allows for improved recovery of TIL. Recombinant IL-21 also can be included in antigen-presenting cell co-culture as described in Section I.B.2, such as due to the ability to stimulate expression of T cell activation markers, including expression of activation markers on neo-antigen reactive TIL. In some cases, recombinant IL-21 can also be included in cultures to expand tumor-reactive T cells during the second expansion phase as described in Section I.D, such as to support proliferation and stabilization of memory phenotype.

In some embodiments, the recombinant IL-21 is added to the culture medium at a concentration between at or about 0.5 IU/mL and at or about 20 IU/mL, between at or about 0.5 IU/mL and at or about 15 IU/mL, between at or about 0.5 IU/mL and at or about 10 IU/mL, between at or about 0.5 IU/mL and at or about 5 IU/mL, between at or about 0.5 IU/mL and at or about 2.5 IU/mL, between at or about 0.5 IU/mL and at or about 1 IU/mL, between at or about 1 IU/mL and at or about 20 IU/mL, between at or about 1 IU/mL and at or about 15 IU/mL, between at or about 1 IU/mL and at or about 10 IU/mL, between at or about 1 IU/mL and at or about 5 IU/mL, between at or about 1 IU/mL and at or about 2.5 IU/mL, between at or about 2.5 IU/mL and at or about 20 IU/mL, between at or about 2.5 IU/mL and at or about 15 IU/mL, between at or about 2.5 IU/mL and at or about 10 IU/mL, between at or about 2.5 IU/mL and at or about 5 IU/mL, between at or about 5 IU/mL and at or about 20 IU/mL, between at or about 5 IU/mL and at or about 15 IU/mL, between at or about 5 IU/mL and at or about 10 IU/mL, between at or about 10 IU/mL and at or about 20 IU/mL, between at or about 10 IU/mL and at or about 15 IU/mL, or between at or about 15 IU/mL and at or about 20 IU/mL. In some embodiments, the IL-21 is added to the culture medium in an amount between at or about 0.5 IU/mL and at or about 2.5 IU/mL. In some embodiments, the IL-21 is added to the culture medium at or about 1 IU/mL.

In some embodiments, the incubation is carried out with a higher dose IL-21.

In some embodiments, the recombinant IL-21 is added to the culture medium at a concentration between at or about 500 IU/mL and at or about 5000 IU/mL, such as between at or about 500 IU/mL and at or about 4000 IU/mL, between at or about 500 IU/mL and at or about 2000 IU/mL, between at or about 500 IU/mL and at or about 1500 IU/mL, between at or about 500 IU/mL and at or about 1000 IU/mL, between at or about 500 IU/mL and at or about 750 IU/mL, between at or about 750 IU/mL and at or about 5000 IU/mL, between at or about 750 IU/mL and at or about 4000 IU/mL, between at or about 750 IU/mL and at or about 2000 IU/mL, between at or about 750 IU/mL and at or about 1500 IU/mL, between at or about 750 IU/mL and at or about 1000 IU/mL, between at or about 1000 IU/mL and at or about 5000 IU/mL, between at or about 1000 IU/mL and at or about 4000 IU/mL, between at or about 1000 IU/mL and at or about 2000 IU/mL, between at or about 1000 IU/mL and at or about 1500 IU/mL, between at or about 1500 IU/mL and at or about 5000 IU/mL, between at or about 1500 IU/mL and at or about 4000 IU/mL, between at or about 1500 IU/mL and at or about 2000 IU/mL, between at or about 2000 IU/mL and at or about 5000 IU/mL, such as between at or about 2000 IU/mL and at or about 4000 IU/mL, or between at or about 4000 IU/mL and at or about 5000 IU/mL. In some embodiments, the recombinant IL-21 is added to the cell culture media at a concentration of at or about 500 IU/mL, at or about 600 IU/mL, at or about 700 IU/mL, at or about 800 IU/mL, at or about 900 IU/mL, at or about 1000 IU/mL, at or about 1100 IU/mL, at or about 1200 IU/mL, at or about 1300 IU/mL, at or about 1400 IU/mL, at or about 1500 IU/mL, at or about 1600 IU/mL, at or about 1700 IU/mL, at or about 1800 IU/mL, at or about 1900 IU/mL or at or about 2000 IU/mL, or any concentration between any of the foregoing. In some embodiments, IL-21 is added to the culture medium at a concentration of at or about 1000 IU/mL.

In some embodiments, recombinant IL-21 and IL-2 are added to the culture medium. In some embodiments, recombinant IL-21 is added at a concentration of 500 IU/mL to 2000 IU/mL (e.g. at or about 1000 IU/mL) and recombinant IL-2 is added at a concentration of 200 IU/mL to 1000 IU/mL (e.g. at or about 300 IU/mL). In some embodiments, the initial expansion (e.g. first expansion) is carried out in the presence of recombinant IL-21 added at 1000 IU/mL and recombinant IL-2 added at 300 IU/mL. In some embodiments, at least one other recombinant modulatory cytokine from IL-7, IL-15, IL-23, IL-25, IL-27 or IL-35 is added to the culture meduium.

In some embodiments, recombinant IL-23 is present in the cell culture medium. IL-23 is a cytokine that signals through the IL-23 receptor, which is typically upregulated on activated memory T cells. IL-23 binding leads to activation of the JAK/STAT pathway, namely JAK2 and STAT3. The JAK signaling leads to activation of NF-kB p50/p65, which binds IL17 promoter and up-regulates its expression. STAT3 activation leads to direct binding of IL-17 promoters as well as RORyT. In some aspects, this dual mechanism leads to potent and sustained IL-17 production for the maintenance of Th17 cell subsets. IL-23 plays a role in inflammatory T cell responses and is a target for therapeutic intervention in numerous autoimmune diseases. In some aspects, the activity of IL-23 as a pro-inflammatory cytokine that is known to act on memory T cells could be used to activate and expand antigen-experienced T cells.

IL-23 contains two subunits linked by a disulfide bond, namely the P19 (IL23a) subunit and the P40 (IL12b) subunit. An exemplary sequence of human IL-23 is set forth as:
P19 (UniProt Q9NPF7 20-189; SEQ ID NO:1) 
RAVPGGSSPAWTQCQQLSQKLCTLAWSAHPLVGHMDLREEGDEETTNDVPHIQC
GDGCDPQGLRDNSQFCLQRIHQGLIFYEKLLGSDIFTGEPSLLPDSPVGQLHASLLG
LSQLLQPEGHHWETQQIPSLSPSQPWQRLLLRFKILRSLQAFVAVAARVFAHGAA
TLSP
P40 (UniProt P29460 23-328; SEQ ID NO:2)

IWELKKDVYVVELDWYPDAPGEMVVLTCDTPEEDGITWTLDQSSEVLGSGKTLTI
QVKEFGDAGQYTCHKGGEVLSHSLLLLHKKEDGIWSTDILKDQKEPKNKTFLRCE
AKNYSGRFTCWWLTTISTDLTFSVKSSRGSSDPQGVTCGAATLSAERVRGDNKEY
EYSVECQEDSACPAAEESLPIEVMVDAVHKLKYENYTSSFFIRDIIKPDPPKNLQLK
PLKNSRQVEVSWEYPDTWSTPHSYFSLTFCVQVQGKSKREKKDRVFTDKTSATVI
CRKNASISVRAQDRYYSSSWSEWASVPCS

In some embodiments, recombinant IL-23 is a heterodimer containing a sequence of amino acids that has at least at or about 85%, 86%, 87%, 88%, 89%, 90%, 91%, 92%, 93%, 94%, 95%, 96%, 97%, 98%, 99% or 100% sequence identity to the sequence set forth in SEQ ID NO:1 and at least at or about 85%, 86%, 87%, 88%, 89%, 90%, 91%, 92%, 93%, 94%, 95%, 96%, 97%, 98%, 99% or 100% sequence identity to the sequence set forth in SEQ ID NO:2, in which both subunits of the heterodimer are linked by a disulfide bond and the sequence exhibits activity of recombinant IL-23, such as ability to bind and mediate signaling via the IL-23 receptor . In some embodiments, recombinant IL-23 has the sequence set forth in SEQ ID NO:1 and SEQ ID NO:2 linked by a disulfide bond. The exemplification of the SEQ ID NOs is not to be construed as limiting. For example, the particular sequence, or individual subunits thereof, of recombinant IL-23 can be several amino acids longer or shorter at either or both of the N-terminus or C-terminus, such as 1-10, e.g., 1, 2, 3, 4, 5, 6 or 7 amino acids longer or shorter, than the sequence of amino acids set forth in the respective SEQ ID NO: 1 and/or 2. In some embodiments, recombinant IL-23 is a human sequence. In particular embodiments, the IL-23 is a GMP grade reagent

Recombinant IL-23 can be included in cell culture media during various stages of the provided process. In some cases, recombinant IL-23 can be included in the initial T cell expansion (first expansion), such as in solid tumor cultures, or other samples known or expected to contain tumor reactive T cells or TILs, to promote the preferential activation and recovery of antigen experienced T cells, leading to an increased frequency of neo-antigen reactive cells isolated from bulk T cells. In some cases, recombinant IL-23 can also be included in cultures to expand selected tumor-reactive T cells during the second expansion phase, such as described in Section I.D, which could boost their sustained activity and proliferation during the expansion process.

In some embodiments, the recombinant IL-23 is added to the culture medium at a concentration between at or about 1 nM and at or about 500 nM, such as between at or about 1 nM and at or about 400 nM, between at or about 1 nM and at or about 300 nM, between at or about 1 nM and at or about 200 nM, between at or about 1 nM and at or about 100 nM, between at or about 1 nM and at or about 50 nM, between at or about 1 nM and at or about 25 nM, between at or about 1 nM and at or about 10 nM, between at or about 1 nM and at or about 5 nM, between at or about 5 nM and at or about 500 nM, between at or about 5 nM and at or about 400 nM, between at or about 5 nM and at or about 300 nM, between at or about 5 nM and at or about 200 nM, between at or about 5 nM and at or about 100 nM, between at or about 5 nM and at or about 50 nM, between at or about 5 nM and at or about 25 nM, between at or about 5 nM and at or about 10 nM, between at or about 10 nM and at or about 500 nM, between at or about 10 nM and at or about 400 nM, between at or about 10 nM and at or about 300 nM, between at or about 10 nM and at or about 200 nM, between at or about 10 nM and at or about 100 nM, between at or about 10 nM and at or about 50 nM, between at or about 10 nM and at or about 25 nM, between at or about 25 nM and at or about 500 nM, between at or about 25 nM and at or about 400 nM, between at or about 25 nM and at or about 300 nM, between at or about 25 nM and at or about 200 nM, between at or about 25 nM and at or about 100 nM, between at or about 25 nM and at or about 50 nM, between at or about 50 nM and at or about 500 nM, between at or about 50 nM and at or about 400 nM, between at or about 50 nM and at or about 300 nM, between at or about 50 nM and at or about 200 nM, between at or about 50 nM and at or about 100 nM, between at or about 100 nM and at or about 500 nM, between at or about 100 nM and at or about 400 nM, between at or about 100 nM and at or about 300 nM, between at or about 100 nM and at or about 200 nM, between at or about 200 nM and at or about 500 nM, between at or about 200 nM and at or about 400 nM, between at or about 200 nM and at or about 300 nM, between at or about 300 nM and at or about 500 nM, between at or about 300 nM and at or about 400 nM, or between at or about 400 nM and at or about 500 nM. In some embodiments, the recombinant IL-23 is added to the culture medium at a concentration of at or about 5 nM, at or about 10 nM, at or about 20 nM, at or about 30 nM, at or about 40 nM, at or about 50 nM, at or about 60 nM, at or about 70 nM, at or about 80 nM, at or about 90 nM or at or about 100 nM, or any value between any of the foregoing.

In some embodiments, the recombinant IL-23 is added to the culture medium at a concentration of between at or about 0.1 ng/mL and at or about 2000 ng/mL. In some embodiments, the recombinant IL-23 is added to the culture medium at a concentration of between at or about 0.1 ng/mL and at or about 1000 ng/mL, such as between at or about 0.1 ng/mL and at or about 500 ng/mL, between at or about 0.1 ng/mL and at or about 250 ng/mL, between at or about 0.1 ng/mL and at or about 100 ng/mL, between at or about 0.1 ng/mL and at or about 50 ng/mL, between at or about 0.1 ng/mL and at or about 10 ng/mL, between at or about 0.1 ng/mL and at or about 1 ng/mL, between at or about 1 ng/mL and at or about 1000 ng/mL, between at or about 1 ng/mL and at or about 500 ng/mL, between at or about 1 ng/mL and at or about 250 ng/mL, between at or about 1 ng/mL and at or about 100 ng/mL, between at or about 1 ng/mL and at or about 50 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 250 ng/mL, between at or about 10 ng/mL and at or about 100 ng/mL, between at or about 10 ng/mL and at or about 50 ng/mL, between at or about 50 ng/mL and at or about 1000 ng/mL, between at or about 50 ng/mL and at or about 500 ng/mL, between at or about 50 ng/mL and at or about 250 ng/mL, between at or about 50 ng/mL and at or about 100 ng/mL, between at or about 100 ng/mL and at or about 1000 ng/mL, between at or about 100 ng/mL and at or about 500 ng/mL, between at or about 100 ng/mL and at or about 250 ng/mL, between at or about 250 ng/mL and at or about 1000 ng/mL, between at or about 250 ng/mL and at or about 500 ng/mL, or between at or about 500 ng/mL and at or about 1000 ng/mL.

In some embodiments, the recombinant IL-23 is added to the culture medium at a concentration of at or about 1 ng/mL, at or about 5 ng/mL, at or about 10 ng/mL, at or about 20 ng/mL, at or about 30 ng/mL, at or about 40 ng/mL, at or about 50 ng/mL, at or about 60 ng/mL, at or about 70 ng/mL, at or about 80 ng/mL, at or about 90 ng/mL or at or about 100 ng/mL or any value between any of the foregoing.

In some embodiments, the recombinant IL-23 is added to the culture medium at a concentration of at or about 200 ng/mL, at or about 300 ng/mL, at or about 400 ng/mL, at or about 500 ng/mL, at or about 600 ng/mL, at or about 700 ng/mL, at or about 800 ng/mL, at or about 900 ng/mL, at or about 1000 ng/mL, at or about 1200 ng/mL, at or about 1400 ng/mL or at or about 1600 ng/mL, at or about 1800 ng/mL or at or about 2000 ng/mL, or any value between any of the foregoing.

In some embodiments, recombinant IL-2 and recombinant IL-23 are added to the culture medium. In some embodiments, recombinant IL-2 is added at a concentration of 200 IU/mL to 1000 IU/mL (e.g. at or about 300 IU/mL) and recombinant IL-23 is added at a concentration of 100 ng/mL to 2000 ng/mL (e.g. between at or about 250 ng/mL and 1000 ng/mL, such as at or about 250 ng/mL, at or about 500 ng/mL or at or about 1000 ng/mL). In some embodiments, the intial expansion is carried out in the presence of recombinant IL-2 added at a concentration of 200 IU/mL to 1000 IU/mL (e.g. at or about 300 IU/mL) and recombinant IL-23 added at a concentration of between 100 ng/mL and 2000 ng/mL (e.g. between at or about 250 ng/mL and 1000 ng/mL, such as at or about 250 ng/mL, at or about 500 ng/mL or at or about 1000 ng/mL). In some embodiments, at least one other recombinant modulatory cytokine from IL-7, IL-21, IL-15, IL-25, IL-27 or IL-35 is added to the culture medium.

In some embodiments, recombinant IL-25 is present in the cell culture medium. IL-25 belongs to the IL-17 family and is also known as IL-17E. IL-25 binds a heterodimeric receptor that is composed of two subunits IL-17RA and IL-17RB. IL-25 is an inflammatory cytokine that typically supports Th2 cell development. IL-25 has been shown to reduce IFN_{γ} production and bias immune responses away from Th1/Th17 responses. IL-25 also has been shown to stimulate NFkB activity, which can broadly activate cells.

An exemplary sequence of human IL-25 is set forth as:

(UniProt Q9H293 33-177; SEQ ID NO:3)
YSHWPSCCPSKGQDTSEELLRWSTVPVPPLEPARPNRHPESCRASEDGPLNSRAISP
WRYELDRDLNRLPQDLYHARCLCPHCVSLQTGSHMDPRGNSELLYHNQTVFYRR
PCHGEKGTHKGYCLERRLYRVSLACVCVRPRVMG

In some embodiments, recombinant IL-25 has a sequence of amino acids that has at least at or about 85%, 86%, 87%, 88%, 89%, 90%, 91%, 92%, 93%, 94%, 95%, 96%, 97%, 98%, 99% or 100% sequence identity to the sequence set forth in SEQ ID NO:3, in which the sequence exhibits activity of recombinant IL-25, such as ability to bind to a subunit of its heterodimeric receptor and mediate signaling via the IL-25 (IL-17RA/IL-17RB) receptor . In some embodiments, recombinant IL-25 has the sequence set forth in SEQ ID NO:3. The exemplification of the SEQ ID NOs is not to be construed as limiting. For example, the particular sequence, or individual subunits thereof, of recombinant IL-25 can be several amino acids longer or shorter at either or both of the N-terminus or C-terminus, such as 1-10, e.g., 1, 2, 3, 4, 5, 6 or 7 amino acids longer or shorter, than the sequence of amino acids set forth in the respective SEQ ID NO: 3. In some embodiments, recombinant IL-25 is a human sequence. In particular embodiments, the IL-25 is a GMP grade reagent.

In some embodiments, the recombinant IL-25 is added to the culture medium at a concentration between at or about 0.001 nM and at or about 10 nM, such as at a concentration between at or about 0.001 nM and at or about 5 nM, between at or about 0.001 nM and at or about 2.5 nM, between at or about 0.001 nM and at or about 1 nM, between at or about 0.001 nM and at or about 0.5 nM, between at or about 0.001 nM and at or about 0.1 nM, between at or about 0.001 nM and at or about 0.05 nM, between at or about 0.001 nM and at or about 0.01 nM, between at or about 0.001 nM and at or about 0.005 nM, between at or about 0.005 nM and at or about 10 nM, between at or about 0.005 nM and at or about 5 nM, between at or about 0.005 nM and at or about 2.5 nM, between at or about 0.005 nM and at or about 1 nM, between at or about 0.005 nM and at or about 0.5 nM, between at or about 0.005 nM and at or about 0.1 nM, between at or about 0.005 nM and at or about 0.05 nM, between at or about 0.005 nM and at or about 0.01 nM, between at or about 0.01 nM and at or about 10 nM, between at or about 0.01 nM and at or about 5 nM, between at or about 0.01 nM and at or about 2.5 nM, between at or about 0.01 nM and at or about 1 nM, between at or about 0.01 nM and at or about 0.5 nM, between at or about 0.01 nM and at or about 0.1 nM, between at or about 0.01 nM and at or about 0.05 nM, between at or about 0.05 nM and at or about 10 nM, between at or about 0.05 nM and at or about 5 nM, between at or about 0.05 nM and at or about 2.5 nM, between at or about 0.05 nM and at or about 1 nM, between at or about 0.05 nM and at or about 0.5 nM, between at or about 0.05 nM and at or about 0.1 nM, between at or about 0.1 nM and at or about 10 nM, between at or about 0.1 nM and at or about 5 nM, between at or about 0.1 nM and at or about 2.5 nM, between at or about 0.1 nM and at or about 1 nM, between at or about 0.1 nM and at or about 0.5 nM, between at or about 0.5 nM and at or about 10 nM, between at or about 0.5 nM and at or about 5 nM, between at or about 0.5 nM and at or about 2.5 nM, between at or about 0.5 nM and at or about 1 nM, between at or about 1 nM and at or about 10 nM, between at or about 1 nM and at or about 5 nM, between at or about 1 nM and at or about 2.5 nM, between at or about 2.5 nM and at or about 10 nM, between at or about 2.5 nM and at or about 5 nM, or between at or about 5 nM and at or about 10 nM. In some embodiments, the recombinant IL-25 is added to the culture medium at a concentration of at or about 0.01 nM, 0.02 nM, 0.03 nM, 0.04 nM, 0.05 nM, 0.06 nM, 0.07 nM, 0.08 nM, 0.09 nM or 1 nM, 1.5 nM or 2 nM or any value between any of the foregoing.

In some embodiments, the recombinant IL-25 is added to the culture medium at a concentration between at or about 0.01 ng/mL and at or about 500 ng/mL, between at or about 0.01 ng/mL and at or about 250 ng/mL, between at or about 0.01 ng/mL and at or about 100 ng/mL, between at or about 0.01 ng/mL and at or about 50 ng/mL, between at or about 0.01 ng/mL and at or about 20 ng/mL, between at or about 0.01 ng/mL and at or about 10 ng/mL, between at or about 0.01 ng/mL and at or about 5 ng/mL, between at or about 0.01 ng/mL and at or about 1 ng/mL, between at or about 0.01 ng/mL and at or about 0.05 ng/mL, between at or about 0.05 ng/mL and at or about 500 ng/mL, between at or about 0.05 ng/mL and at or about 250 ng/mL, between at or about 0.05 ng/mL and at or about 100 ng/mL, between at or about 0.05 ng/mL and at or about 50 ng/mL, between at or about 0.05 ng/mL and at or about 20 ng/mL, between at or about 0.05 ng/mL and at or about 10 ng/mL, between at or about 0.05 ng/mL and at or about 5 ng/mL, between at or about 0.05 ng/mL and at or about 1 ng/mL, between at or about 1 ng/mL and at or about 500 ng/mL, between at or about 1 ng/mL and at or about 250 ng/mL, between at or about 1 ng/mL and at or about 100 ng/mL, between at or about 1 ng/mL and at or about 50 ng/mL, between at or about 1 ng/mL and at or about 20 ng/mL, between at or about 1 ng/mL and at or about 10 ng/mL, between at or about 1 ng/mL and at or about 5 ng/mL, between at or about 5 ng/mL and at or about 500 ng/mL, between at or about 5 ng/mL and at or about 250 ng/mL, between at or about 5 ng/mL and at or about 100 ng/mL, between at or about 5 ng/mL and at or about 50 ng/mL, between at or about 5 ng/mL and at or about 20 ng/mL, between at or about 5 ng/mL and at or about 10 ng/mL, between at or about 10 ng/mL and at or about 500 ng/mL, between at or about 10 ng/mL and at or about 250 ng/mL, between at or about 10 ng/mL and at or about 100 ng/mL, between at or about 10 ng/mL and at or about 50 ng/mL, between at or about 10 ng/mL and at or about 20 ng/mL, between at or about 20 ng/mL and at or about 500 ng/mL, between at or about 20 ng/mL and at or about 250 ng/mL, between at or about 20 ng/mL and at or about 100 ng/mL, between at or about 20 ng/mL and at or about 50 ng/mL, between at or about 50 ng/mL and at or about 500 ng/mL, between at or about 50 ng/mL and at or about 250 ng/mL, between at or about 50 ng/mL and at or about 100 ng/mL, between at or about 100 ng/mL and at or about 500 ng/mL, between at or about 100 ng/mL and at or about 250 ng/mL, or between at or about 250 ng/mL and at or about 500 ng/mL. In some embodiments, the recombinant IL-25 is added to the culture medium at a concentration between at or about 1 ng/mL, at or about 2 ng/mL, at or about 3 ng/mL, at or about 4 ng/mL, at or about 5 ng/mL, at or about 6 ng/mL, at or about 7 ng/mL, at or about 8 ng/mL, at or about 9 ng/mL, at or about 10 ng/mL, at or about 15 ng/mL or at or about 20 ng/mL, or any value between any of the foregoing.

Recombinant IL-25 can be included in cell culture media during various stages of the provided process. In some cases, recombinant IL-25 can be included in the initial T cell expansion (first expansion), such as during TIL isolation and expansion from solid tissue, to support preservation and expansion of Th2 CD4 T cells. In some cases, recombinant IL-25 can be included in cultures to expand selected tumor-reactive T cells during the second expansion phase, such as described in Section I.D. For example, IL-25 can be included in day 9-16 culture media during TIL expansion to promote CD4/CD8 balance and/or sustain T cell activation rates. The use of IL-25 may help to drive T cell proliferation as well as promote NFkB activity and bolster T cell expansion and activation.

In some embodiments, the recombinant IL-25 is added to the culture medium at a concentration of between at or about 0.1 ng/mL and at or about 2000 ng/mL, such as between at or about 0.1 ng/mL and at or about 1000 ng/mL, between at or about 0.1 ng/mL and at or about 500 ng/mL, between at or about 0.1 ng/mL and at or about 250 ng/mL, between at or about 0.1 ng/mL and at or about 100 ng/mL, between at or about 0.1 ng/mL and at or about 50 ng/mL, between at or about 0.1 ng/mL and at or about 10 ng/mL, between at or about 0.1 ng/mL and at or about 1 ng/mL, between at or about 1 ng/mL and at or about 1000 ng/mL, between at or about 1 ng/mL and at or about 500 ng/mL, between at or about 1 ng/mL and at or about 250 ng/mL, between at or about 1 ng/mL and at or about 100 ng/mL, between at or about 1 ng/mL and at or about 50 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 250 ng/mL, between at or about 10 ng/mL and at or about 100 ng/mL, between at or about 10 ng/mL and at or about 50 ng/mL, between at or about 50 ng/mL and at or about 1000 ng/mL, between at or about 50 ng/mL and at or about 500 ng/mL, between at or about 50 ng/mL and at or about 250 ng/mL, between at or about 50 ng/mL and at or about 100 ng/mL, between at or about 100 ng/mL and at or about 1000 ng/mL, between at or about 100 ng/mL and at or about 500 ng/mL, between at or about 100 ng/mL and at or about 250 ng/mL, between at or about 250 ng/mL and at or about 1000 ng/mL, between at or about 250 ng/mL and at or about 500 ng/mL, or between at or about 500 ng/mL and at or about 1000 ng/mL.

In some embodiments, the recombinant IL-25 is added to the culture medium at a concentration of at or about 1 ng/mL, at or about 5 ng/mL, at or about 10 ng/mL, at or about 20 ng/mL, at or about 30 ng/mL, at or about 40 ng/mL, at or about 50 ng/mL, at or about 60 ng/mL, at or about 70 ng/mL, at or about 80 ng/mL, at or about 90 ng/mL or at or about 100 ng/mL or any value between any of the foregoing.

In some embodiments, the recombinant IL-25 is added to the culture medium at a concentration of at or about 200 ng/mL, at or about 300 ng/mL, at or about 400 ng/mL, at or about 500 ng/mL, at or about 600 ng/mL, at or about 700 ng/mL, at or about 800 ng/mL, at or about 900 ng/mL, at or about 1000 ng/mL, at or about 1200 ng/mL, at or about 1400 ng/mL or at or about 1600 ng/mL, at or about 1800 ng/mL or at or about 2000 ng/mL, or any value between any of the foregoing.

In some embodiments, recombinant IL-2 and recombinant IL-25 are added to the culture medium. In some embodiments, recombinant IL-2 is added at a concentration of 200 IU/mL to 1000 IU/mL (e.g. at or about 300 IU/mL) and recombinant IL-25 is added at a concentration of 100 ng/mL to 2000 ng/mL (e.g. between at or about 250 ng/mL and 1000 ng/mL, such as at or about 250 ng/mL, at or about 500 ng/mL or at or about 1000 ng/mL). In some embodiments, the initial expansion is carried out in the presence of recombinant IL-2 added at a concentration of 200 IU/mL to 1000 IU/mL (e.g. at or about 300 IU/mL) and recombinant IL-25 added at a concentration of between 100 ng/mL and 2000 ng/mL (e.g. between at or about 250 ng/mL and 1000 ng/mL, such as at or about 250 ng/mL, at or about 500 ng/mL or at or about 1000 ng/mL). In some embodiments, at least one other recombinant modulatory cytokine from IL-7, IL-21, IL-15, IL-23, IL-27 or IL-35 is added to the culture meduium.

In some embodiments, recombinant IL-27 is present in the cell culture medium. IL-27 is a cytokine that signals through the IL-27 receptor, initiating activating of signaling pathways including JAK-STAT and p38 MAPK. In some cases, IL-27 can induce or suppress Tregs and in some cases other T cell subsets, such as TH1 cells. IL-27 can modulate Treg responses, and program effector T cells into a stem-like memory effector cells, which may enhance T-cell survival in the tumor microenvironment.

IL-27 is a heterodimer of 2 chains, IL27A (IL27p28) and IL27B (EBI3). An exemplary sequence of human IL-27 is set forth as:
P28: 
FPRPPGRPQL SLQELRREFT VSLHLARKLL SEVRGQAHRF AESHLPGVNL
YLLPLGEQLP DVSLTFQAWR RLSDPERLCF ISTTLQPFHA LLGGLGTQGR
WTNMERMQLW AMRLDLRDLQ RHLRFQVLAA GFNLPEEEEE EEEEEEEERK
GLLPGALGSA LQGPAQVSWP QLLSTYRLLH SLELVLSRAV RELLLLSKAG
HSVWPLGFPT LSPQP (SEQ ID NO:4)
EB13

RKGPP AALTLPRVQC RASRYPIAVD CSWTLPPAPN STSPVSFIAT
YRLGMAARGH SWPCLQQTPT STSCTITDVQ LFSMAPYVLN VTAVHPWGSS
SSFVPFITEH IIKPDPPEGV RLSPLAERQL QVQWEPPGSW PFPEIFSLKY
WIRYKRQGAA RFHRVGPIEA TSFILRAVRP RARYYVQVAA QDLTDYGELS
DWSLPATATM SLGK (SEQ ID NO:5)

In some embodiments, recombinant IL-27 is a heterodimer containing a sequence of amino acids that has at least at or about 85%, 86%, 87%, 88%, 89%, 90%, 91%, 92%, 93%, 94%, 95%, 96%, 97%, 98%, 99% or 100% sequence identity to the sequence set forth in SEQ ID NO:4 and at least at or about 85%, 86%, 87%, 88%, 89%, 90%, 91%, 92%, 93%, 94%, 95%, 96%, 97%, 98%, 99% or 100% sequence identity to the sequence set forth in SEQ ID NO:5, in which the heterodimer exhibits activity of recombinant IL-27, such as ability to bind and mediate signaling via the IL-27 receptor. In some embodiments, recombinant IL-27 has the sequence set forth in SEQ ID NO:4 and SEQ ID NO:5 linked as a heterodimer. The exemplification of the SEQ ID NOs is not to be construed as limiting. For example, the particular sequence, or individual subunits thereof, of recombinant IL-27 can be several amino acids longer or shorter at either or both of the N-terminus or C-terminus, such as 1-10, e.g., 1, 2, 3, 4, 5, 6 or 7 amino acids longer or shorter, than the sequence of amino acids set forth in the respective SEQ ID NO: 4 and/or 5. In some embodiments, recombinant IL-27 is a human sequence. In particular embodiments, the IL-27 is a GMP grade reagent.

Recombinant IL-27 can be included in cell culture media during various stages of the provided process. In some cases, recombinant IL-27 can be included in the initial T cell expansion (first expansion), such as in solid tumor cultures, or other samples known or expected to contain tumor reactive T cells or TILs, to promote the preferential activation and recovery of antigen experienced T cells, leading to an increased frequency of neo-antigen reactive cells isolated from bulk T cells. In some cases, recombinant IL-27 can also be included in cultures to expand selected tumor-reactive T cells during the final expansion (e.g. second expansion) phase, such as described in Section I.D, which could boost their sustained activity and proliferation during the expansion process.

In some embodiments, the recombinant IL-27 is added to the culture medium at a concentration of between at or about 0.1 ng/mL and at or about 2000 ng/mL, such as between at or about 0.1 ng/mL and at or about 1000 ng/mL, between at or about 0.1 ng/mL and at or about 500 ng/mL, between at or about 0.1 ng/mL and at or about 250 ng/mL, between at or about 0.1 ng/mL and at or about 100 ng/mL, between at or about 0.1 ng/mL and at or about 50 ng/mL, between at or about 0.1 ng/mL and at or about 10 ng/mL, between at or about 0.1 ng/mL and at or about 1 ng/mL, between at or about 1 ng/mL and at or about 1000 ng/mL, between at or about 1 ng/mL and at or about 500 ng/mL, between at or about 1 ng/mL and at or about 250 ng/mL, between at or about 1 ng/mL and at or about 100 ng/mL, between at or about 1 ng/mL and at or about 50 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 250 ng/mL, between at or about 10 ng/mL and at or about 100 ng/mL, between at or about 10 ng/mL and at or about 50 ng/mL, between at or about 50 ng/mL and at or about 1000 ng/mL, between at or about 50 ng/mL and at or about 500 ng/mL, between at or about 50 ng/mL and at or about 250 ng/mL, between at or about 50 ng/mL and at or about 100 ng/mL, between at or about 100 ng/mL and at or about 1000 ng/mL, between at or about 100 ng/mL and at or about 500 ng/mL, between at or about 100 ng/mL and at or about 250 ng/mL, between at or about 250 ng/mL and at or about 1000 ng/mL, between at or about 250 ng/mL and at or about 500 ng/mL, or between at or about 500 ng/mL and at or about 1000 ng/mL. In some embodiments, the concentration is between 400 ng/mL and 500 ng/mL.

In some embodiments, the recombinant IL-27 is added to the culture medium at a concentration of at or about 200 ng/mL, at or about 300 ng/mL, at or about 400 ng/mL, at or about 500 ng/mL, at or about 600 ng/mL, at or about 700 ng/mL, at or about 800 ng/mL, at or about 900 ng/mL, at or about 1000 ng/mL, at or about 1200 ng/mL, at or about 1400 ng/mL or at or about 1600 ng/mL, at or about 1800 ng/mL or at or about 2000 ng/mL, or any value between any of the foregoing.

In some embodiments, recombinant IL-2 and recombinant IL-27 are added to the culture medium. In some embodiments, recombinant IL-2 is added at a concentration of 200 IU/mL to 1000 IU/mL (e.g. at or about 300 IU/mL) and recombinant IL-27 is added at a concentration of 100 ng/mL to 2000 ng/mL (e.g. between at or about 250 ng/mL and 1000 ng/mL, such as at or about 250 ng/mL, at or about 500 ng/mL or at or about 1000 ng/mL). In some embodiments, the initial expansion is carried out in the presence of recombinant IL-2 added at a concentration of 200 IU/mL to 1000 IU/mL (e.g. at or about 300 IU/mL) and recombinant IL-27 added at a concentration of between 100 ng/mL and 2000 ng/mL (e.g. between at or about 250 ng/mL and 1000 ng/mL, such as at or about 250 ng/mL, at or about 500 ng/mL or at or about 1000 ng/mL). In some embodiments, at least one other recombinant modulatory cytokine from IL-7, IL-21, IL-15, IL-23, IL-25 or IL-35 is added to the culture meduium.

In some embodiments, recombinant IL-35 is present in the cell culture medium. IL-35 is a cytokine that can in some cases suppress inflammatory responses IL-35 also has selectve activities on different T cell subsets. In T cells, IL-35 binds gp130 and IL-12Rβ2 to signal through either gp130/IL-12Rβ2 heterodimers or homodimers of each subunit. Engagement of receptors by IL-35 elicts STAT activation and signaling, such as via JAK-STAT mediated pathways.

IL-35 is a heterodimeric protein containing the p35 subunit from IL-12 (IL-12α) and the β subunit from IL-27 (EBI3).
P35 
RNLPVATPDP GMFPCLHHSQ NLLRAVSNML QKARQTLEFY PCTSEEIDHE
DITKDKTSTV EACLPLELTK NESCLNSRET SFITNGSCLA SRKTSFMMAL
CLSSIYEDLK MYQVEFKTMN AKLLMDPKRQ IFLDQNMLAV IDELMQALNF
NSETVPQKSS LEEPDFYKTK IKLCILLHAF RIRAVTIDRV MSYLNAS (SEQ ID
NO:6)
EB13

RKGPP AALTLPRVQC RASRYPIAVD CSWTLPPAPN STSPVSFIAT
YRLGMAARGH SWPCLQQTPT STSCTITDVQ LFSMAPYVLN VTAVHPWGSS
SSFVPFITEH IIKPDPPEGV RLSPLAERQL QVQWEPPGSW PFPEIFSLKY
WIRYKRQGAA RFHRVGPIEA TSFILRAVRP RARYYVQVAA QDLTDYGELS
DWSLPATATM SLGK (SEQ ID NO:5)

In some embodiments, recombinant IL-35 is a heterodimer containing a sequence of amino acids that has at least at or about 85%, 86%, 87%, 88%, 89%, 90%, 91%, 92%, 93%, 94%, 95%, 96%, 97%, 98%, 99% or 100% sequence identity to the sequence set forth in SEQ ID NO:6 and at least at or about 85%, 86%, 87%, 88%, 89%, 90%, 91%, 92%, 93%, 94%, 95%, 96%, 97%, 98%, 99% or 100% sequence identity to the sequence set forth in SEQ ID NO:5, in which the heterodimer exhibits activity of recombinant IL-35, such as ability to bind and mediate signaling via the IL-35 receptor (e.g. gp130 and IL-12Rβ2 subunits). In some embodiments, recombinant IL-35 has the sequence set forth in SEQ ID NO:6 and SEQ ID NO:5 linked as a heterodimer. The exemplification of the SEQ ID NOs is not to be construed as limiting. For example, the particular sequence, or individual subunits thereof, of recombinant IL-35 can be several amino acids longer or shorter at either or both of the N-terminus or C-terminus, such as 1-10, e.g., 1, 2, 3, 4, 5, 6 or 7 amino acids longer or shorter, than the sequence of amino acids set forth in the respective SEQ ID NO: 4 and/or 5. In some embodiments, recombinant IL-35 is a human sequence. In particular embodiments, the IL-35 is a GMP grade reagent.

Recombinant IL-35 can be included in cell culture media during various stages of the provided process. In some cases, recombinant IL-35 can be included in the initial T cell expansion (first expansion), such as in solid tumor cultures, or other samples known or expected to contain tumor reactive T cells or TILs, to promote the preferential activation and recovery of antigen experienced T cells, leading to an increased frequency of neo-antigen reactive cells isolated from bulk T cells. In some cases, recombinant IL-35 can also be included in cultures to expand selected tumor-reactive T cells during the final expansion (e.g. second expansion) phase, such as described in Section I.D, which could boost their sustained activity and proliferation during the expansion process.

In some embodiments, the recombinant IL-35 is added to the culture medium at a concentration of between at or about 0.1 ng/mL and at or about 2000 ng/mL, such as between at or about 0.1 ng/mL and at or about 1000 ng/mL, between at or about 0.1 ng/mL and at or about 500 ng/mL, between at or about 0.1 ng/mL and at or about 250 ng/mL, between at or about 0.1 ng/mL and at or about 100 ng/mL, between at or about 0.1 ng/mL and at or about 50 ng/mL, between at or about 0.1 ng/mL and at or about 10 ng/mL, between at or about 0.1 ng/mL and at or about 1 ng/mL, between at or about 1 ng/mL and at or about 1000 ng/mL, between at or about 1 ng/mL and at or about 500 ng/mL, between at or about 1 ng/mL and at or about 250 ng/mL, between at or about 1 ng/mL and at or about 100 ng/mL, between at or about 1 ng/mL and at or about 50 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 250 ng/mL, between at or about 10 ng/mL and at or about 100 ng/mL, between at or about 10 ng/mL and at or about 50 ng/mL, between at or about 50 ng/mL and at or about 1000 ng/mL, between at or about 50 ng/mL and at or about 500 ng/mL, between at or about 50 ng/mL and at or about 250 ng/mL, between at or about 50 ng/mL and at or about 100 ng/mL, between at or about 100 ng/mL and at or about 1000 ng/mL, between at or about 100 ng/mL and at or about 500 ng/mL, between at or about 100 ng/mL and at or about 250 ng/mL, between at or about 250 ng/mL and at or about 1000 ng/mL, between at or about 250 ng/mL and at or about 500 ng/mL, or between at or about 500 ng/mL and at or about 1000 ng/mL. In some embodiments, the concentration is between 400 ng/mL and 500 ng/mL.

In some embodiments, the recombinant IL-35 is added to the culture medium at a concentration of at or about 200 ng/mL, at or about 300 ng/mL, at or about 400 ng/mL, at or about 500 ng/mL, at or about 600 ng/mL, at or about 700 ng/mL, at or about 800 ng/mL, at or about 900 ng/mL, at or about 1000 ng/mL, at or about 1200 ng/mL, at or about 1400 ng/mL or at or about 1600 ng/mL, at or about 1800 ng/mL or at or about 2000 ng/mL, or any value between any of the foregoing.

In some embodiments, recombinant IL-2 and recombinant IL-35 are added to the culture medium. In some embodiments, recombinant IL-2 is added at a concentration of 200 IU/mL to 1000 IU/mL (e.g. at or about 300 IU/mL) and recombinant IL-35 is added at a concentration of 100 ng/mL to 2000 ng/mL (e.g. between at or about 250 ng/mL and 1000 ng/mL, such as at or about 250 ng/mL, at or about 500 ng/mL or at or about 1000 ng/mL). In some embodiments, the initial expansion is carried out in the presence of recombinant IL-2 added at a concentration of 200 IU/mL to 1000 IU/mL (e.g. at or about 300 IU/mL) and recombinant IL-35 added at a concentration of between 100 ng/mL and 2000 ng/mL (e.g. between at or about 250 ng/mL and 1000 ng/mL, such as at or about 250 ng/mL, at or about 500 ng/mL or at or about 1000 ng/mL). In some embodiments, at least one other recombinant modulatory cytokine from IL-7, IL-21, IL-15, IL-23, IL-25 or IL-27 is added to the culture meduium.

In particular embodiments, T cell stimulatory agent(s) present during the incubation, such as for expansion of cells, contains recombinant IL-2. In some embodiments, one or more other stimulating agent can be included such as one or more other recombinant cytokine from IL-7, IL-15, IL-21, IL-25, and/or IL-23, or an anti-CD3 antibody (e.g. OKT-3). In some cases in which an anti-CD3 antibody (e.g. OKT-3) the T cell stimulating agent(s) also can include a costimulating agent, such as provided by antigen-presenting feeder cells, such as PBMCs, or a soluble anti-CD28 antibody.

In particular embodiments, T cell stimulatory agent(s) present during the incubation, such as for expansion of cells contains recombinant IL-2 and an anti-CD3 antibody.

In particular embodiments, T cell stimulatory agent(s) present during the incubation, such as for expansion of cells contains recombinant IL-2, an anti-CD3 antibody, e.g. OKT-3, and antigen-presenting feeder cells, such as PBMCs.

In particular embodiments, T cell stimulatory agent(s) present during the incubation, such as for expansion of cells contains recombinant IL-2, an anti-CD3 antibody, e.g. OKT-3, and an anti-CD28 antibody. In some embodiments, the anti-CD3 antibody and/or anti-CD28 antibody are soluble. In some embodiments, one or both of the anti-CD3 antibody and anti-CD28 antibody are bound to a solid surface, such as a bead (e.g., DYNABEADS^{®} M-450 CD3/CD28 T Cell Expander).

In particular embodiments, T cell stimulatory agent(s) present during the incubation, such as for expansion of cells contains recombinant IL-2, recombinant IL-15, recombinant IL-7, an anti-CD3 antibody, e.g. OKT-3, and antigen-presenting feeder cells, such as PBMCs.

In particular embodiments, T cell stimulatory agent(s) present during the incubation, such as for expansion of cells contains recombinant IL-2, recombinant IL-15, recombinant IL-7, an anti-CD3 antibody, e.g. OKT-3, and an anti-CD28 antibody. In some embodiments, the anti-CD3 antibody and/or anti-CD28 antibody are soluble. In some embodiments, one or both of the anti-CD3 antibody and anti-CD28 antibody are bound to a solid surface, such as a bead (e.g., DYNABEADS^{®} M-450 CD3/CD28 T Cell Expander).

In particular embodiments, T cell stimulatory agent(s) present during the incubation, such as for expansion of cells contains recombinant IL-15 and recombinant IL-7, an anti-CD3 antibody, e.g. OKT-3, and antigen-presenting feeder cells, such as PBMCs.

In particular embodiments, T cell stimulatory agent(s) present during the incubation, such as for expansion of cells contains recombinant IL-15 and recombinant IL-7, an anti-CD3 antibody, e.g. OKT-3, and an anti-CD28 antibody. In some embodiments, the anti-CD3 antibody and/or anti-CD28 antibody are soluble. In some embodiments, one or both of the anti-CD3 antibody and anti-CD28 antibody are bound to a solid surface, such as a bead (e.g., DYNABEADS^{®} M-450 CD3/CD28 T Cell Expander).

In some embodiments, the incubation with the T cell stimulatory agent(s) is carried out under conditions for initial expansion of T cells from the biological sample. In some embodiments, the cells are cultured at about 37 °C with about 5% CO₂. The culture media containing the T cell stimulatory agent(s) can be a serum-free media.

In some embodiments, the incubation with the T cell stimulatory agent(s) is carried out for at or about 1 day, such as generally at or about 2 days, 3 days, 4 days, 5 days, 6 days, 7 days, 8 days, 9 days, 10 days, 11 days, 12 days, or any range of time between any of the foregoing. In some embodiments, the incubation with the T cell stimulatory agent(s) is carried out for 7 to 21 days, such as 7 days, 8 days, 9 days, 10 days, 11 days, 12 days, 13 days, 14 days, 15 days, 16 days, 17 days, 18 days, 19 days, 20 days or 21 days, or any value between any of the foregoing. In some embodiments, the incubation is carried out for 7-14 days. In some embodiments, the incubation is carried out for 7-10 days. In some embodiments, the incubation is for at or about 7 days. In some embodiments, the incubation is for at or about 8 days. In some embodiments, the incubation is for at or about 9 days. In some embodiments, the incubation is for at or about 10 days.

In some embodiments, the incubation with the T cell stimulatory agent(s) is a minimal expansion such that it does not result in downregulation of the T cell activation marker (e.g. PD-1, CD39 and/or TIGIT). For instance, the incubation with the T cell stimulatory agent(s) in the initial expansion is a short culture so that the markers CD39, PD1 and/or TIGIT are still present during the sorting step and the cells have not downregulated those markers.

In some embodiments, the incubation with the T cell stimulatory agent(s), such as for the initial expansion of T cells in the input sample, is carried out for at or about 1 days. In some embodiments, the incubation with the T cell stimulatory agent(s), such as for the initial expansion of T cells in the input sample, is carried out for at or about 2 days. In some embodiments, the incubation with the T cell stimulatory agent(s), such as for the initial expansion of T cells in the input sample, is carried out for at or about 3 days. In some embodiments, the incubation with the T cell stimulatory agent(s), such as for the initial expansion of T cells in the input sample, is carried out for at or about 4 days. In some embodiments, the incubation with the T cell stimulatory agent(s), such as for the initial expansion of T cells in the input sample, is carried out for at or about 5 days. In some embodiments, the incubation with the T cell stimulatory agent(s), such as for the initial expansion of T cells in the input sample, is carried out for at or about 6 days. In some embodiments, the incubation with the T cell stimulatory agent(s), such as for the initial expansion of T cells in the input sample, is carried out for at or about 7 days.

The incubation, such as for initial expansion of T cells in the input sample, can be carried out under GMP conditions. In some embodiments, the incubation is in a closed system, which in some aspects may be a closed automated system. In some embodiments, the culture media containing the T cell stimulatory agent(s) can be a serum-free media. In some embodiments, the incubation is carried out in a closed automated system and with serum-free media.

In some embodiments, the initial expansion of cells under the one or more stimulatory conditions is in a culture vessel suitable for cell expansion. In some embodiments, the culture vessel is a gas permeable culture vessel, such as a G-Rex system (e.g. G-Rex 10, G-Rex 10M, G-Rex 100 M/100M-CS or G-Rex 500 M/500M-CS). In some embodiments the culture vessel is a microplate, flask, bar or other culture vessel suitable for expansion of cells in a closed system. In some embodiments, expansion can be carried out in a bioreactor. In some embodiments, the initial expansion can be carried out using a cell expansion system by transfer of the cells to gas permeable bags, such as in connection with a bioreactor (e.g. Xuri Cell Expansion System W25 (GE Healthcare)). In an embodiment, the cell expansion system includes a culture vessel, such as a bag, e.g. gas permeable cell bag, with a volume that is about 50 mL, about 100 mL, about 200 mL, about 300 mL, about 400 mL, about 500 mL, about 600 mL, about 700 mL, about 800 mL, about 900 mL, about 1 L, about 2 L, about 3 L, about 4 L, about 5 L, about 6 L, about 7 L, about 8 L, about 9 L, and about 10 L, or any value between any of the foregoing. In some embodiments, the process is automated or semi-automated. Examples of suitable bioreactors for the automated perfusion expansion include, but are not limited to, GE Xuri W25, GE Xuri W5, Sartorius BioSTAT RM 20 | 50, Finesse SmartRocker Bioreactor Systems, and Pall XRS Bioreactor Systems, or Miltenyi Prodigy. In some aspects, the expansion culture is carried out under static conditions. In some embodiments, the expansion culture is carried out under rocking conditions. The medium can be added in bolus or can be added on a perfusion schedule. In some embodiments, the bioreactor maintains the temperature at or near 37°C and CO2 levels at or near 5% with a steady air flow at, at about, or at least 0.01 L/min, 0.05 L/min, 0.1 L/min, 0.2 L/min, 0.3 L/min, 0.4 L/min, 0.5 L/min, 1.0 L/min, 1.5 L/min, or 2.0 L/min or greater than 2.0 L/min. In certain embodiments, at least a portion of the culturing is performed with perfusion, such as with a rate of 290 ml/day, 580 ml/day, and/or 1160 ml/day.

In some embodiments, the cells are seeded in an appropriate culture vessel (e.g. gas permeable bag) at a density of from 0.5 x 10⁶ cells/mL to 1.5 x 10⁶ cells/mL. In some embodiments, the density is at or about 0.5 x 10⁶ cells/mL, 0.75 x 10⁶ cells/mL, 1 x 10⁶ cells/mL, 1.25 x 10⁶ cells/mL or 1.5 x 10⁶ cells/mL, or any value between any of the foregoing.

In some aspects, cells are expanded in an automated closed expansion system that is perfusion enabled. Perfusions can continuously add media to the cells to ensure an optimal growth rate is achieved.

The expansion methods can be carried out under GMP conditions, including in a closed automated system and using serum free medium. In some embodiments, any one or more of the steps of the method can be carried out in a closed system or under GMP conditions. In certain embodiments, all process operations are performed in a GMP suite. In some embodiments, a closed system is used for carrying out one or more of the other processing steps of a method for manufacturing, generating or producing a cell therapy. In some embodiments, one or more or all of the processing steps, e.g., isolation, selection and/or enrichment, processing, culturing steps including incubation in connection with expansion of the cells, and formulation steps is carried out using a system, device, or apparatus in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some embodiments, immediately after the incubation, the stimulated cells can be collected for subsequent co-culture with APCs, such as in accord with methods described in Section I.B.2 below.

In some embodiments, the stimulated cells are collected and are cryofrozen. The provision of an intermediate hold step by cryopreservation after the initial expansion phase can be used to coordinate timing with the neoepitope identification and peptide generation, such as described in Section I.B.1 and/or the generation of APCs as described in Section I.B.2. In some embodiments, for cryopreservation, the stimulated cells are formulated as a composition with a cryoprotectant. In some embodiments, the cryoprotectant is or comprises DMSO and/or glycerol. In some embodiments, compositions formulated for cryopreservation can be stored at low temperatures, such as ultra low temperatures, for example, storage with temperature ranges from -40 °C to -150°C, such as or about 80 °C ± 6.0 ° C.

In some embodiments, the cryopreserved cells are prepared for subsequent steps by thawing. In some cases, the cells can be ready for subsequent culturing with APCs and peptides immediately after thawing following one or more wash steps.

### B. Neoantigen identification and Co-culture of T cells With APCs

In certain embodiments of the provided methods, the methods can include a step of co-culturing a population of tumor-reactive T cells with antigen presenting cells that present one or more neoantigen peptide that corresponds to nonsynonymous somatic mutations associated in the tumor of a subject. In such other aspect of the provided methods, the co-culturing step further enriches for tumor-reactive T cells as T cells that recognize neoantigen peptides derived from sequencing data from the patient's tumor can become activated. The methods for co-culturing can include methods for identification of neoepitopes and generation of peptides, followed by contacting the peptides with antigen presenting cells for presentation to a T cell population that is known or suspected of containing tumor reactive T ce1s1.

In some embodiments, after the co-culturing step, a selection can be carried out for upregulation of activation markers. In some cases, the selection can be for cells positive for any upregulation marker as described herein (e.g. PD-1, CD39 and/or TIGIT or other upregulation marker). Then, cells from the culture are expanded to create a therapeutic composition containing an expanded population of tumor specific reactive cells.

In other cases, the selection of cells after the co-culturing can be the second selection in a process for enriching and expanding tumor reactive T cells. For instance, in some embodimetns, cells digested directly from a tumor fragment from a subject, or after an initial (e.g. minimal expansion) of the cells therefrom, are enriched or selected (e.g. based on selection of cells positive for PD-1, CD39 and/or TIGIT), and the selected T cell population is co-cultured with the antigen presenting cells presenting the peptide epitopes. After the co-culture incubations, cells from the co-culture can be further enriched for tumor reactive T cells, such as by selection of any upregulation marker described herein or combinations thereof, e.g. 4-1BB (CD137) or OX40 (CD134) or 4-1BB (CD137) and OX40 (CD134) double positive cells. Then, cells from the culture are expanded to create a therapeutic composition containing an expanded population of tumor specific reactive cells.

### 1. Neoepitope identification and peptide generation

In some aspects, the provided methods include a step of generating or identifying *in silico* a plurality of peptides (also referred to as "P" or "n-mers") that contain at least one cancer-specific cancer neoepitope, and a further step of filtering *in silico* the peptides to so obtain a subset of neoepitope sequences. In some embodiments, at least one synthetic peptide is prepared using sequence information from the subset of neoepitope sequences, and the synthetic peptide is then employed in methods to enrich for tumor-reactive T cells in accord with the provided methods.

In some embodiments, the cancer-specific cancer neoepitope is determined by identifying or isolating a tumor-associated antigen or peptide sequence thereof from a cancer cell from a subject. The cancer cell may be obtained from any bodily sample derived from a patient which contains or is expected to contain tumor or cancer cells. The bodily sample may be any tissue sample such as blood, a tissue sample obtained from the primary tumor or from tumor metastases, a lymph node sample or any other sample containing tumor or cancer cells.

In some embodiments, the tumor is a hematological tumor. Non- limiting examples of hematological tumors include leukemia, including acute leukemias (such as 1 lq23- positive acute leukemia, acute lymphocytic leukemia, acute myeloid leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

In some embodiments, the tumor is a solid tumor. Non-limiting examples of solid tumors, such as sarcomas and carcinomas, include fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer (including basal breast carcinoma, ductal carcinoma and lobular breast carcinoma), lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, astrocytoma, medulloblastoma, craniopharyngioma, ependymoma, pinealoma, hemangioblastoma, acoustic neuroma, oligodendroglioma, meningioma, melanoma, neuroblastoma and retinoblastoma). In several examples, a tumor is melanoma, lung cancer, lymphoma breast cancer or colon cancer.

In some embodiments, the cancer is a gastrointestinal cancer involving a cancer of the gastrointestinal tract (GI tract), including cancers of the upper or lower digestive tract, or an accessory organ of digestion, such as esophagus, stomach, biliary system, pancreas, small intestine, large intestine, rectum or anus. In some embodiments, the cancer is an espohageal cancer, stomach (gastric) cancer, pancreatic cancer, liver cancer (hepatocellular carcinoma), gallbladder cancer, cancer of the mucosa-associated lymphoid tissue (MALT lymphoma), cancer of the biliary tree, colorectal cancer (including colon cancer, rectum cancer or both), anal cancer, or a gastrointestinal carcinoid tumor. In particular embodiments, the cancer is a colorectal cancer.

In some embodiments, the tumor is from a breast cancer, such as a ductal carcinoma or a lobular carcinoma. In some embodiments, the tumor is from a prostate cancer. In some embodiments, tumor is from a skin cancer, such as a basal cell carcinoma, a squamous cell carcinoma, a Kaposi's sarcoma, or a melanoma. In some embodiments, the tumor is from a lung cancer, such as an adenocarcinoma, a bronchiolaveolar carcinoma, a large cell carcinoma, or a small cell carcinoma. In some embodiments, the tumor is from a brain cancer, such as a glioblastoma or a meningioma. In some embodiments, the tumor is from a gastrointestinal cancer, such as any described above. In some embodiments, the tumor is from a colon cancer. In some embodiments, the tumor is from a liver cancer, such as a hepatocellular carcinoma. In some embodiments, the tumor is from a pancreatic cancer. In some embodiments, the tumor is from a kidney cancer, such as a renal cell carcinoma. In some embodiments, the tumor is from a testicular cancer.

In some embodiments, the cancer is not a melanoma. Melanoma is a cancer that generally has a high mutational rate. High tumor mutation burden has been thought to be a particularly desired prognostic marker for success related to treatment with an immunotherapy targeting tumor neoantigens (Simpson et al., Journal of Clinical Oncology 2017, 35:15_suppl, 9567-9567; McGranahan et al. Science 2016, 351:1463-1469) In some embodiments, the provided methods can be used in cancers that have a lower tumor mutation burden, since the methods are carried out to actively (as opposed to passively) enrich for tumor reactive T cells.

In some embodiments, the subject is a subject with a tumor mutational burden (TMB) of less than 8 mutations. TMB includes the number of non-synomymous mutations per tumor. In some embodiments, TMB can be calculated by counting the number of synonymous and non-synonymous mutations across a 0.8- to 1.2-megabase (Mb) region, and reporting the result as mutations/Mb. In some embodiments, TMB can be determined by next generation sequencing (NGS) on tumor tissue samples. In some cases, whole exome sequencing can be used or computational germline status filtering can be used (Chalmers et al. Genome Med 2017 9:34). In some embodiments, the subject has a TMB of less than at or about 60 mutations/Mb, such as less than at or about 55 mutations/Mb, less than at or about 50 mutations/Mb, less than at or about 45 mutations/Mb, less than at or about 40 mutations/Mb, less then at or about 30 mutations/Mb, less than at or about 25 mutations per Mb, or less than at or about 20 mutations/Mb, or any value between any of the foregoing. In some embodiments, the subject has a TMB of less than at or about 41 mutations/Mb, less than at or about 40 mutations/Mb, less than at or about 39 mutations/Mb, less than at or about 38 mutations/Mb, less than at or about 37 mutations/Mb or less.

In some embodiments, the peptide (P) is a tumor-associated antigen derived from premalignant conditions, such as variants of carcinoma *in situ,* or vulvar intraepithelial neoplasia, cervical intraepithelial neoplasia, or vaginal intraepithelial neoplasia.

In some aspects, nucleic acid from such cells of the tumor or cancer is obtained and sequenced. In embodiments, the protein-coding region of genes in a genome is obtained, such as by omics analysis, such as by analysis of whole genomic sequencing data, exome sequencing data, and/or transcriptome data. To identify tumor-specific sequences, sequencing data can be compared to a reference sequencing data, such as data obtained from a normal cell or noncancerous cell from the same subject. In some embodiments, next-generation sequencing (NGS) methods are used.

In some embodiments, the methods include a step of using matched normal omics data of a tumor. In such methods, the *in silico* analysis involves an omics analysis to identify mutations in the tumor relative to normal tissue of the same patient, such as non-diseased tissue of the same patient. It is generally contemplated that matched normal omics data are whole genomic sequencing data, exome sequencing data, and/or transcriptome data, and that the matched normal omics data are matched against normal before treatment of the patient. In a particular embodiment, whole exome sequencing is performed on healthy and diseased tissue to identify somatic mutations associated with the tumor.

In some embodiments, omics data are obtained from one or more patient biopsy samples following standard tissue processing protocol and sequencing protocols. In particular embodiments, the data are patient matched tumor data (e.g., tumor versus same patient normal). In some cases, non- matched or matched versus other reference (e.g., prior same patient normal or prior same patient tumor, or homo statisticus) are also deemed suitable for use herein. The omics data may be fresh omics data or omics data that were obtained from a prior procedure (or even different patient). For example, neoepitopes may be identified from a patient tumor in a first step by whole genome and/or exome analysis of a tumor biopsy (or lymph biopsy or biopsy of a metastatic site) and matched normal tissue (i.e., non-diseased tissue from the same patient such as peripheral blood). In some embodiments, genomic analysis can be processed via location-guided synchronous comparison of the so obtained omics information.

The genomic analysis can be performed by any number of analytic methods. In particular embodiments, the methods include WGS (whole genome sequencing) and exome sequencing of both tumor and matched normal sample using next generation sequencing such as massively parallel sequencing methods, ion torrent sequencing, pyrosequencing. Computational analysis of the sequence data may be performed in numerous manners. In some embodiments, the data format is in SAM, BAM, GAR, or VCF format. As an example, analysis can be performed *in silico* by location-guided synchronous alignment of tumor and normal samples as, for example, disclosed in US 2012/0059670A1 and US 2012/0066001 Al using BAM files and BAM servers. Alternative file formats for sequence analysis (e.g., SAM, GAR, FASTA, etc.) are also contemplated.

In some of any embodiments, peptides (P) comprising neoantigens arising from a missense mutation encompass the amino acid change encoded by 1 or more nucleotide polymorphisms. Peptides (P) comprising neoantigens that arise from frameshift mutations, splice site variants, insertions, inversions and deletions should encompass the novel peptide sequences and junctions of novel peptide sequences. Peptides (P) comprising neoantigens with novel post-translational modifications should encompass the amino acids bearing the post-translational modification(s), such as a phosphate or glycan.

Once these mutations are identified, neoepitopes are then identified. Neoepitopes are mutant peptides that are recognized by a patient's T cells. These neoepitopes must be presented by a tumor or antigen presenting cell by the MHC complex and then be recognized by a TCR on the T cell. In some embodiments, the provided methods include a step of calculation of one or more neoepitopes to define neoepitopes that are specific to the tumor and patient. Consequently, it should be recognized that patient and cancer specific neoepitopes can be identified from omics information in an exclusively *in silico* environment that ultimately predicts potential epitopes that are unique to the patient and tumor type. In particular aspects, the so identified cancer neoepitopes are unique to the patient and the particular cancer in the patient (e.g., having a frequency of less than 0.1% of all neoepitopes, and more typically less than 0.01% in a population of cancer patients diagnosed with the same cancer), but that the so identified cancer neoepitopes have a high likelihood of being presented in a tumor.

In some of any embodiments, the length of the peptide (P) depends on the specific application and is typically between about 5 to about 50 amino acids. In preferred embodiments, the peptide (P) is between about 7 to 35 amino acids, e.g., 7, 8, 9, 10, 11, 12, 13, 14, 15, 16, 17, 18, 19, 20, 21, 22, 23, 24, 25, 26, 27, 28, 29, 30, 31, 32, 33, 34 or 35 amino acids. In some aspects, the methods can be carried out with an individual peptide that includes a change(s) (e.g. mutations) in the amino acid sequences. In some aspects, the methods can be carried out with a pool of peptides, where peptides of the pool contain a change(s) (e.g. mutations) in the amino acid sequences. The pool of peptides can include tens to hundreds of individual peptides. In some cases, the pool of peptides includes 2, 3, 4, 5, 6, 7, 8, 9, 10, 11, 12, 13, 14, 15, 16, 17, 18, 19, 20, 30, 40, 50, 60, 70, 80, 90, 100 or more individual peptides, or any value between any of the foregoing. The pool of peptides can represent one neo-antigen or can represent several neo-antigens. In some cases, a pool of peptides can include multiple overlapping peptides of the same neo-antigen. Thus, for a tumor-associated antigen, the antigen may be divided into 7 to 35 amino acid, e.g., 25 amino acid, peptides (P) wherein each peptide (P) contains a unique composition of amino acids; or, the peptides (P) can be overlapping peptide pools wherein an antigen is divided into a set number of 7 to 35 amino acid, e.g., 25 amino acid, peptides (P) that have overlapping sequences. In some cases, each of the peptides of the overlapping pool of an antigen can be offset by a set number of amino acid residues, such as 5, 6, 7, 8, 9, 10, 11, 12, 13, 15 or 15 amino acids. In some embodiments, each of the peptides of the overlapping pool of an antigen is offset by 10 amino acids. In some embodiments, each of the peptides of the overlapping pool of an antigen is offset by 12 amino acids. For example, an overlapping peptide pool comprising a 100 amino acid antigen may be divided into eight 25 amino acid peptides (P) that are each offset by 12 amino acids (i.e., each subsequent 25 amino acid peptide comprising a 100 amino acid peptide sequence starts at the 13^{th} amino acid position from the prior peptide). Those skilled in the art understand that many permutations exist for generating a peptide pool from an antigen.

The neoepitope sequences as contemplated herein can be defined as sequence stretches with relatively short length (e.g., 5-30 mers, more typically 7- 11 mers, or 12-25 mers) wherein such stretches include the change(s) (e.g. mutations) in the amino acid sequences. Most typically, the change(s) is/are located centrally or near the center (e.g., less than 4, or less than 5, or less than 6 amino acids from center position). In particular aspects, neoepitope sequences contemplated herein will especially include those in which a single amino acid is exchanged relative to the matched normal sequence, and in which the position of the changed amino acid is centrally located, or near the center of the neoepitope sequence (e.g., in a 9-mer, the changed amino acid is at position 2, 3, 4, or 5, and more typically at position 3, 4, or 5, and most typically at position 4 or 5). It should be appreciated that a single amino acid change may be presented in numerous neoepitope sequences that include the changed amino acid, depending on the position of the changed amino acid.

In particular embodiments, neoepitopes will be calculated to have a length of between 2-50 amino acids, more typically between 5-30 amino acids, and most typically between 9-15 amino acids. For example, where the epitope is to be presented by the MHC-I complex, a typical epitope length will be about 8- 11 amino acids, while the typical epitope for presentation via MHC-II complex will have a length of about 13- 17 amino acids. As will be readily appreciated, since the position of the changed amino acid in the neoepitope may be other than central, the actual peptide sequence and with that the actual topology of the neoepitope may vary considerably. Moreover, where the neoepitope is presented to an immune competent (or other) cell as a synthetic peptide, it should be appreciated that the synthetic peptide may be significantly longer than the peptide portion that is ultimately bound by the MHC-I or MHC-II system to so allow for proteolytic processing in the cell. For example, contemplated synthetic peptides may therefore have between 8 and 15 amino acids upstream and downstream of the changed amino acid.

Various algorithms have been developed and can be used to map T cell epitopes (both MHC Class I and Class II-restricted) within protein molecules of various origins. In some embodiments, many programs utilize availability of the large-scale peptide-MHC binding affinity matrix from experimental measurements to train machine learning (ML)-based classifiers to distinguish MHC-binders from non-binders (see e.g., Zhao et al. (2018) PLoS Comput Biol 14(11): e1006457). Exemplary predictor methods for MHC class I (e.g. 9-mer) include *smm,* smmpmbec, ann (NetMHC3.4), NetMHC4, PickPocket, consensus, NetMHCpan2.8, NetMHCpan3, NetMHCpan4, NetMHCcons, mhcflurry, mhcflurry_pan, or MixMHCpred. Exemplary predictor methods for MHC class II (e.g. 15-mer ) include NetMHCIIpan, NetMHCII2.3, nn_align, smm_align, consensus, comblib, tepitope, or mhcflurry. Any of such methods can be used.

In embodiments where the synthetic peptide is used for direct MHC-I binding, the overall length will be between 8 and 10 amino acids. In embodiments, where the synthetic peptide is used for direct MHC-II binding, the overall length will be between 12 and 25 amino acids, such as between 14 and 20 amino acids. In some cases, where the synthetic peptide is processed in the cell (typically via proteasome processing) prior to MHC presentation, the overall length will typically be between 10 and 40 amino acids, with the changed amino acid at or near a central position in the synthetic peptide. In some embodiments, a peptide for MHC-I binding is a 9-mer. In some embodiments, a peptide for MHC-II binding is a 23-mer. In some embodiments, a peptide for MHC-II binding is a 25-mer.

As an example, a peptide (P) can include 0-25 amino acids on either side flanking the amino acid change or novel junction that arises due to a mutation. In one embodiment, the peptide (P) is a neoantigen sequence that comprises the 12 amino acids on either side flanking the amino acid change that arises from a single nucleotide polymorphism, for example, a 25 amino acid peptide, wherein the 13^{th} amino acid is the amino acid residue resulting from the single nucleotide polymorphism. In some embodiments, the peptide (P) is a neoantigen sequence that comprises the 12 amino acids on either side flanking an amino acid with a novel post-translational modification, for example, a 25 amino acid peptide, wherein the 13^{th} amino acid is the amino acid residue resulting from the novel post-translational modification site. In other embodiments, the peptide (P) is a neoantigen sequence that comprises 0-12 amino acids on either side flanking a novel junction created by an insertion, deletion or inversion. In some cases, the peptide (P) comprising neoantigens resulting from novel sequences can encompass the entire novel sequence, including 0-25 amino acids on either side of novel junctions that may also arise.

In some embodiments, further downstream analysis may be performed on the so identified sequence differences to identify those that lead to a new peptide sequence based on the cancer and patient specific mutation. Neoepitopes may therefore be identified by considering the type (e.g., deletion, insertion, transversion, transition, translocation) and impact of the mutation (e.g., non-sense, missense, frame shift, etc.), and may as such serve as a content filter through which silent and other non-relevant (e.g., non-expressed) mutations are eliminated.

In some embodiments, identified neoepitopes can be further filtered *in silico* against an identified patient HLA- type. Such HLA-matching is thought to ensure strong binding of the neoepitopes to the MHC-I complex of nucleated cells and the MHC-II complex of specific antigen presenting cells. Targeting both antigen presentation systems is particularly thought to produce a therapeutically effective and durable immune response involving both the cellular and the humoral branches of the immune system. It should also be appreciated that thusly identified HLA-matched neoepitopes can be biochemically validated *in vitro.*

HLA determination for both MHC-I and MHC-II can be done using various methods. In some embodiments, the HLA-type can be predicted from omics data *in silico* using a reference sequence containing most or all of the known and/or common HLA-types. For example, a patient's HLA-type is ascertained (using wet chemistry or *in silico* determination), and a structural solution for the HLA-type is calculated or obtained from a database, which is then used as a docking model *in silico* to determine binding affinity of the neoepitope to the HLA structural solution. Suitable systems for determination of binding affinities include the NetMHC platform (see e.g., Nucleic Acids Res. 2008 Jul 1; 36(Web Server issue): W509-W512.), HLAMatchmaker (http://www. epitopes.net/downloads.html), and IEDB Analysis Resource (http://tools.immuneepitope.org/ mhcii/). Neoepitopes with high affinity (e.g., less than 100 nM, less than 75 nM, less than 50 nM for MHC-I; less than 500 nM, less than 300 nM, less than 100 nM for MHC-II) against the previously determined HLA-type are then selected. In calculating the highest affinity, modifications to the neoepitopes may be implemented by adding N- and/or C-terminal modifications to the epitope to further increase binding of a synthetic neoepitope to the HLA-type of the patient. Thus, neoepitopes may be native as identified or further modified to better match a particular HLA-type. In some embodiments, neoepitopes can be scored/ranked based on allele frequency multiplied by the transcripts per million number to get a likelihood score. This score can then be further augmented using HLA information and calculated or actual binding affinity to the patient's HLA type.

Among provided embodiments are embodiments in which the neoepitopes are compared against a database that contains known human sequences to so avoid use of a human-identical sequence.

After the *in silico* identification of suitable neoepitope sequences, corresponding synthetic peptides are then prepared *in vitro* (e.g. , using solid phase synthesis). In particular embodiments, a library of synthetic peptides is prepared representing a plurality of different neoepitopes from the subject. The library can include 100, 1000, 10000 or more different peptides. To obtain a synthetic antibody against the identified neoepitope(s), it is contemplated that the epitope identified is prepared *in vitro* to yield a synthetic peptide.

Various methods can be used to prepare synthetic peptides. For example, peptides with cancer neoepitope sequences can be prepared on a solid phase (e.g., using Mernfied synthesis), via liquid phase synthesis, or from smaller peptide fragments. Peptide epitopes can be obtained by chemical synthesis using a commercially available automated peptide synthesizer. In some embodiments, the peptides can be synthesized, for example, by using the Fmoc-polyamide mode of solid-phase peptide synthesis which is disclosed by Lu et al (1981).J. Org. Chem. 46,3433 and the references therein. In some aspects, peptides can be produced by expression of a recombinant nucleic acid in a suitable host and with a suitable expression system. In some aspects, recombinant methods can be used where multiple neoepitopes are on a single peptide chain, such as with spacers between neoepitopes or cleavage sites).

The peptides can be purified by any one, or a combination of techniques such as recrystallization, size exclusion chromatography, ion-exchange chromatography, hydrophobic interaction chromatography, and reverse-phase high performance liquid chromatography using e.g. acetonitrile/water gradient separation. In some embodiments, peptides can be precipitated and further purified, for example by high performance liquid chromatography (HPLC). Analysis of peptides can be carried out using thin layer chromatography, electrophoresis, in particular capillary electrophoresis, solid phase extraction (CSPE), reverse-phase high performance liquid chromatography, amino-acid analysis after acid hydrolysis and by fast atom bombardment (FAB) mass spectrometric analysis, as well as MALDI and ESI-Q-TOF mass spectrometric analysis.

### 2. Co-culture with APCs

In embodiments of the provided methods, once the neoepitopes that encode for proteins are synthesized a plurality of the synthetic peptides are contacted with antigen presenting cells under conditions to present peptides in the context of an MHC molecule and incubated with T cells from a population of T cells for recognition of the peptides presented on the APCs. In some embodiments, the synthetic peptides are pulsed into autologous or allogeneic APCs that are then co-cultured with patient T cells. Antigen presenting cells are used to present these peptides. T cells that recognize these peptides on the surface of the APC can then be isolated, such as by methods described below. The incubated cells can be cultured under conditions that enrich for and expand tumor-reactive T cells, i.e. T cells containing endogenous TCR that are reactive to peptides present on the APCs, in the culture. In some embodiments, the methods include culturing the T cells under conditions for expansion until a threshold amount of T cells is obtained and/or until up to 20 days after initiation of incubation. In some embodiments, of the provided methods the method can include co-culturing the T cells with the APCs over the course of several hours to days and then separating antigen presenting cells from the population of T cells for the expansion of the T cells under conditions to enrich or expand tumor-reactive T cells. In some embodiments, the separating can include isolating or selecting reactive T cells from culture based on one or more T cell activation markers on T cells.

Various methods can be used for culturing cells for antigen-specificity, see e.g. US published application No. US2017/0224800.

In some embodiments, the tumor reactive T cells are co-cultured with APCs that have been contacted or exposed to present a peptide, e.g. containing a mutated amino acid sequence, such as neoepitope peptides as described above. The method may comprise inducing autologous antigen presenting cells (APCs) of the patient to present the mutated amino acid sequence. The APCs may include any cells which present peptide fragments of proteins in association with major histocompatibility complex (MHC) molecules on their cell surface. The MHC molecule can be any MHC molecule expressed by the patient including, but not limited to, MHC Class I, MHC Class II, HLA-A, HLA-B, HLA-C, HLA-DM, HLA-DO, HLA-DP, HLA-DQ, and HLA-DR molecules. The APCs may include, for example, any one or more of macrophages, DCs, Langerhans cells, B-lymphocytes, and T-cells. In particular embodiments, the APCs are DCs. In some particular embodiments, the APCs are B cells. In some embodiments, the APCs are artificial APCs.

In particular embodiments, the APCs include cells that are able to present Class I and Class II restricted molecules. For example, B cells and DCs both have the ability to present MHC class I and MHC class II restricted molecules. In some embodiments, the APC cell sample includes B cells and DCs. In some embodiments, the APC cell sample is enriched for B cells, such as by selection or isolation from a primary cell sample. In some embodiments, the APC cell sample is enriched for DCs, such as by selection or isolation from a primary cell sample.

In some embodiments, the APCs express MHC class I and/or MHC class II molecules with a matched HLA from which the source of T cells has been obtained. In particular embodiments, both the APCs and T cells have been isolated from the same subject, i.e. are autologous to the cancer patient. In some embodiments, the method may comprise inducing autologous antigen presenting cells (APCs) of the patient to present the mutated amino acid sequence. By using autologous APCs from the patient, the methods may identify T cells that have antigenic specificity for a mutated amino acid sequence encoded by a cancer-specific mutation that is presented in the context of an MHC molecule expressed by the patient.

In some embodiments, the APCs are cells from a blood or apheresis sample from a subject, such as the patient. In some embodiments, the APCs include cells present in a peripheral blood mononuclear cell (PBMC) sample. Typically, APCs function in a PBMC culture primarily involves monocytes and B cells. In some embodiments, a population of isolated PBMCs can be used as APCs in the provided methods. PBMCs can be obtained using standard methods such as Ficoll-Paque gradient separation. In some cases, the APCs are or include B cells that are isolated from the blood or apheresis sample or from a PBMC sample. In other cases, the APCs are or include monocytes isolated from the blood or apheresis sample or from a PBMC sample. In some aspects, the monocytes can be used as a source for preparing monocyte-derived DCs for use as APCs. In some embodiments, a source of monocyte-derived DCs (e.g. CD11c^{high}MHCII^{high}CD14^{low} cells) can be generated ex vivo from isolated monocytes, by culture with GM-CSF and IL-4 for 4 to 6 days to produce monocyte-derived dendritic cells. In particular embodiments, the monocytes are isolated from PBMCs such as by CD14 selection, and then are cultured with GM-CSF and IL-4 for 4 to 6 days.

In some embodiments, the APCs are primary cells (e.g. B cells or monocyte-derived DCs) that are replication competent, for example, the cells are not subjected to irradiation, heat treatment or other method that would result in their inactivation. In particular embodiments, the provided methods do not use irradiated APCs. In some embodiments, the APCs are freshly isolated primary cells obtained from the subject, or are derived from primary cells obtained from the subject. In some embodiments, the APCs have been cryopreserved and subsequently thawed prior to the co-culture with the stimulated T cells in accord with provided methods.

In some particular embodiments, B cells are used as a source of APCs and are generated from a patient apheresis, such as an apheresis autologous to the subject from which the tumor fragments and/or T cells were obtained. In other particular embodiments, monocyte-derived dendritic cells are used as a source of APCs and are generated from monocytes from a patient apheresis, such as an apheresis autologous to the subject from which the tumor fragment and/or T cells are obtained.

In some embodiments, the isolated or generated APCs are collected and are cryofrozen. The provision of an intermediate hold step by cryopreservation after the isolation or generation of APCs can be used to coordinate timing with the neoepitope identification and peptide generation such as described in Section I.B.1 and/or initial expansion of T cells, such as described in Section I.A.2. In some embodiments, for cryopreservation, the isolated or generated APCs are formulated as a composition with a cryoprotectant. In some embodiments, the cryoprotectant is or comprises DMSO and/or s glycerol. In some embodiments, compositions formulated for cryopreservation can be stored at low temperatures, such as ultra low temperatures, for example, storage with temperature ranges from -40 °C to -150 °C, such as or about 80 °C ± 6.0 ° C.

In some embodiments, the cryopreserved cells are prepared for subsequent steps by thawing. In some cases, the cells can be ready for subsequent culturing with T cells and peptides immediately after thawing following one or more wash steps.

In particular embodiments, the methods for enriching or selecting tumor reactive cells are initiated by contacting PBMCs with the mutated amino acid sequence, such as one or more, such as a plurality of, neoepitope peptides. The PBMCs/peptides can then be cultured with stimulated T cells. The PBMCs and T cells can be obtained from the same subject.

In particular embodiments, the methods for enriching or selecting tumor reactive cells are initiated by contacting B cells with the mutated amino acid sequence, such as one or more, such as a plurality of, neoepitope peptides. The B cell/peptides can then be cultured with stimulated T cells. The B cells and T cells can be obtained from the same subject.

In particular embodiments, the methods for enriching or selecting tumor reactive cells are initiated by contacting monocyte-derived DCs with the mutated amino acid sequence, such as one or more, such as a plurality of, neoepitope peptides. The monocyte-derived DCs/peptides can then be cultured with stimulated T cells. The monocyte-derived DCs and T cells can be obtained or derived from the same subject.

In some embodiments, the APC is an artificial antigen presenting cell (aAPC). Typically, aAPCs include features of natural APCs, including expression of an MHC molecule, stimulatory and costimulatory molecule(s), Fc receptor, adhesion molecule(s) and/or the ability to produce or secrete cytokines (e.g. IL-2). Normally, an aAPC is a cell line that lacks expression of one or more of the above, and is generated by introduction (e.g. by transfection or transduction) of one or more of the missing elements from among an MHC molecule, a low affinity Fc receptor (CD32), a high affinity Fc receptor (CD64), one or more of a co-stimulatory signal (e.g. CD7, B7-1 (CD80), B7-2 (CD86), PD-L1, PD-L2, 4-1BBL, OX40L, ICOS-L, ICAM, CD30L, CD40, CD70, CD83, HLA-G, MICA, MICB, HVEM, lymphotoxin beta receptor, ILT3, ILT4, 3/TR6 or a ligand of B7-H3; or an antibody that specifically binds to CD27, CD28, 4-1BB, OX40, CD30, CD40, PD-1, ICOS, LFA-1, CD2, CD7, LIGHT, NKG2C, B7-H3, Toll ligand receptor or a ligand of CD83), a cell adhesion molecule (e.g. ICAM-1 or LFA-3) and/or a cytokine (e.g. IL-2, IL-4, IL-6, IL-7, IL-10, IL-12, IL-15, IL-21, interferon-alpha (IFNα), interferon-beta (IFNβ), interferon-gamma (IFNγ), tumor necrosis factor-alpha (TNFα), tumor necrosis factor-beta (TNFβ), granulocyte macrophage colony stimulating factor (GM-CSF), and granulocyte colony stimulating factor (GCSF). In some cases, an aAPC does not normally express an MHC molecule, but can be engineered to express an MHC molecule or, in some cases, is or can be induced to express an MHC molecule, such as by stimulation with cytokines. In some cases, aAPCs also can be loaded with a stimulatory or co-stimulatory ligand, which can include, for example, an anti-CD3 antibody, an anti-CD28 antibody or an anti-CD2 antibody. Exemplary of a cell line that can be used as a backbone for generating an aAPC is a K562 cell line or fibroblast cell line. Various aAPCs are known in the art, see e.g., U.S. Patent No. 8,722,400, published application No. US2014/0212446; Butler and Hirano (2014) Immunol Rev., 257(1):10. 1111/imr.12129; Suhoshki et al. (2007) Mol. Ther., 15:981-988). In particular embodiments, the methods for enriching or selecting tumor reactive cells are initiated by contacting aAPCs with the mutated amino acid sequence, such as one or more, such as a plurality of, neoepitope peptides. The aAPC/peptides can then be cultured with stimulated T cells.

Inducing APCs (e.g. B cells or monocyte-derived DCs) to present the mutated amino acid sequence may be carried out using various suitable methods. In an embodiment, inducing APCs to present the mutated amino acid sequence (e.g. peptide neoepitope) comprises pulsing the APCs with synthetic peptides comprising the mutated amino acid sequence or a pool of peptides, each peptide in the pool comprising a different mutated amino acid sequence. In some cases, the APCs are pulsed with the peptides using electroporation into an antigen presenting cell. The synthetic peptides can then be presented by the antigen presenting cells to be recognized by CD8 cells (MHC class I) or CD4 cells (MHC class II). In certain particular embodiments, synthetic peptides are generated to be suitable for expression by MHC class I restricted molecules for recognition by CD8 cells. In other particular embodiments, synthetic peptides are generated to be suitable for expression by MHC class IIrestricted molecules for recognition by CD4 cells.

In some embodiments, the APCs (e.g. PBMCs, B cells or monocyte-derived DCs) are contacted with a single peptide or a pool of peptides. The pool of peptide can represent many different mutated amino acid sequences, such as 5, 10, 20, 25, 30, 40, 50, 60, 70, 80, 90, 100, 150, 200, 300, 400, 500, 600, 700, 800, 900 or 100 peptides, or any value between any of the foregoing.

The peptides or pool of peptides are loaded onto antigen presenting cells (e.g. dendritic cells), such as by peptide pulsing, at a concentrations suitable for their presentation on the surface of a major histocompatibility complex (MHC).

In some embodiments, the peptide concentration representing an individual or single peptide can range between at or about 0.00000 1 µg/mL and at or about 10 µg/mL. In some embodiments, the peptide concentration representing an individual or single peptide can range between at or about 0.00001 µg/mL and at or about 10 µg/mL, at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.00001 µg/mL and at or about 0.001 µg/mL, at or about 0.00001 µg/mL and at or about 0.0001 µg/mL, at or about 0.0001 µg/mL and 10 µg/mL, at or about 0.0001 µg/mL and at or about 1 µg/mL, at or about 0.0001 µg/mL and at or about 0.1 µg/mL, at or about 0.0001 µg/mL and at or about 0.01 µg/mL, at or about 0.0001 µg/mL and at or about 0.001 µg/mL, at or about 0.001 µg/mL and at or about 10 µg/mL, at or about 0.001 µg/mL and at or about 1 µg/mL, at or about 0.001 µg/mL and at or about 0.1 µg/mL, at or about 0.001 µg/mL and at or about 0.01 µg/mL, at or about 0.01 µg/mL and at or about 10 µg/mL, at or about 0.01 µg/mL and at or about 1 µg/mL, at or about 0.01 µg/mL and at or about 0.1 µg/mL, at or about 0.1 µg/mL and at or about 10 µg/mL, at or about 0.1 µg/mL and at or about 1 µg/mL, or at or about 1 µg/mL and at or about 10 µg/mL. In some embodiments, the concentration representing an individual or single peptide can be at or about 0.00000 1 µg/mL, at or about 0.00001 µg/mL, at or about 0.0001 µg/mL, at or about 0.001 µg/mL, at or about 0.01 µg/mL, at or about 0.1 µg/mL, at or about 1 µg/mL, or any value between any of the foregoing.

In some embodiments, the peptides are a pool of peptides representing many different mutated amino acid sequences and the concentration on average of individual or single peptides in the pool can range between at or about 0.00000 1 µg/mL and at or about 10 µg/mL. In some embodiments, the peptides are a pool of peptides representing many different mutated amino acid sequences and the concentration on average of individual or single peptides in the pool can range between at or about 0.00001 µg/mL and at or about 10 µg/mL, at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.00001 µg/mL and at or about 0.001 µg/mL, at or about 0.00001 µg/mL and at or about 0.0001 µg/mL, at or about 0.0001 µg/mL and 10 µg/mL, at or about 0.0001 µg/mL and at or about 1 µg/mL, at or about 0.0001 µg/mL and at or about 0.1 µg/mL, at or about 0.0001 µg/mL and at or about 0.01 µg/mL, at or about 0.0001 µg/mL and at or about 0.001 µg/mL, at or about 0.001 µg/mL and at or about 10 µg/mL, at or about 0.001 µg/mL and at or about 1 µg/mL, at or about 0.001 µg/mL and at or about 0.1 µg/mL, at or about 0.001 µg/mL and at or about 0.01 µg/mL, at or about 0.01 µg/mL and at or about 10 µg/mL, at or about 0.01 µg/mL and at or about 1 µg/mL, at or about 0.01 µg/mL and at or about 0.1 µg/mL, at or about 0.1 µg/mL and at or about 10 µg/mL, at or about 0.1 µg/mL and at or about 1 µg/mL, or at or about 1 µg/mL and at or about 10 µg/mL. In some embodiments, the concentration on average of individual or single peptides in the pool can be at or about 0.00000 1 µg/mL, at or about 0.00001 µg/mL, at or about 0.0001 µg/mL, at or about 0.001 µg/mL, at or about 0.01 µg/mL, at or about 0.1 µg/mL, at or about 1 µg/mL, or any value between any of the foregoing.

In some embodiments, the concentration of individual peptides of the one or more non-native peptide is, on average, less than 0.02 µg/mL. In some embodiments, the concentration of individual peptides of the one or more non-native peptides is, on average, from at or about 0.00001 µg/mL to at or about 0.01 µg/mL, such as at or about 0.00001 µg/mL to at or about 0.005 µg/mL, at or about 0.00001 µg/mL to at or about 0.002 µg/mL, at or about 0.00001 µg/mL to at or about 0.001 µg/mL, at or about 0.00001 µg/mL to at or about 0.0005 µg/mL, at or about 0.00001 µg/mL to at or about 0.0002 µg/mL, at or about 0.00001 µg/mL to at or about 0.0001 µg/mL, at or about 0.00001 µg/mL to at or about 0.00005 µg/mL, at or about 0.00001 µg/mL to at or about 0.00002 µg/mL, at or about 0.00002 µg/mL to at or about 0.005 µg/mL, at or about 0.00002 µg/mL to at or about 0.002 µg/mL, at or about 0.00002 µg/mL to at or about 0.001 µg/mL, at or about 0.00002 µg/mL to at or about 0.0005 µg/mL, at or about 0.00002 µg/mL to at or about 0.0002 µg/mL, at or about 0.00002 µg/mL to at or about 0.0001 µg/mL, at or about 0.00002 µg/mL to at or about 0.00005 µg/mL, at or about 0.00005 µg/mL to at or about 0.005 µg/mL, at or about 0.00005 µg/mL to at or about 0.002 µg/mL, at or about 0.00005 µg/mL to at or about 0.001 µg/mL, at or about 0.00005 µg/mL to at or about 0.0005 µg/mL, at or about 0.00005 µg/mL to at or about 0.0002 µg/mL, at or about 0.00005 µg/mL to at or about 0.0001 µg/mL, at or about 0.0001 µg/mL to at or about 0.005 µg/mL, at or about 0.0001 µg/mL to at or about 0.002 µg/mL, at or about 0.0001 µg/mL to at or about 0.001 µg/mL, at or about 0.0001 µg/mL to at or about 0.0005 µg/mL, at or about 0.0001 µg/mL to at or about 0.0002 µg/mL, at or about 0.0005 µg/mL to at or about 0.005 µg/mL, at or about 0.0005 µg/mL to at or about 0.002 µg/mL, at or about 0.0005 µg/mL to at or about 0.001 µg/mL, at or about 0.001 µg/mL to at or about 0.005 µg/mL, at or about 0.001 µg/mL to at or about 0.002 µg/mL, or at or about 0.002 µg/mL to at or about 0.005 µg/mL.

In some embodiments, the peptide concentration, representing the single peptide or pool of peptides, can range between at or about 0.0001 µg/mL and at or about 40 µg/mL. The peptide concentration, representing the single peptide or pool of peptides, can range between at or about 0.001 µg/mL and at or about 40 µg/mL, at or about 0.001 µg/mL and at or about 25 µg/mL, 0.001 µg/mL and at or about 10 µg/mL, 0.001 µg/mL and at or about 5 µg/mL, 0.001 µg/mL and at or about 1 µg/mL , 0.001 µg/mL and at or about 0.5 µg/mL, 0.001 µg/mL and at or about 0.1 µg/mL, 0.001 µg/mL and at or about 0.01 µg/mL, 0.01 µg/mL and at or about 40 µg/mL, such as at or about 0.01 µg/mL and at or about 25 µg/mL, at or about 0.01 µg/mL and at or about 10 µg/mL, at or about 0.01 µg/mL and at or about 5 µg/mL, at or about 0.01 µg/mL and at or about 1 µg/mL, at or about 0.01 µg/mL and at or about 0.5 µg/mL, at or about 0.01 µg/mL and at or about 0.1 µg/mL, at or about 0.01 µg/mL and at or about 0.05 µg/mL, 0.05 µg/mL and at or about 40 µg/mL, at or about 0.05 µg/mL and at or about 25 µg/mL, at or about 0.05 µg/mL and at or about 10 µg/mL, at or about 0.05 µg/mL and at or about 5 µg/mL, at or about 0.05 µg/mL and at or about 1 µg/mL, at or about 0.05 µg/mL and at or about 0.5 µg/mL, at or about 0.05 µg/mL and at or about 0.1 µg/mL, 0.1 µg/mL and at or about 40 µg/mL, such as at or about 0.1 µg/mL and at or about 25 µg/mL, at or about 0.1 µg/mL and at or about 10 µg/mL, at or about 0.1 µg/mL and at or about 5 µg/mL, at or about 0.1 µg/mL and at or about 1 µg/mL, at or about 0.1 µg/mL and at or about 0.5 µg/mL, 0.5 µg/mL and at or about 40 µg/mL, at or about 0.5 µg/mL and at or about 25 µg/mL, at or about 0.5 µg/mL and at or about 10 µg/mL, at or about 0.5 µg/mL and at or about 5 µg/mL, at or about 0.5 µg/mL and at or about 1 µg/mL, 1 µg/mL and at or about 40 µg/mL, at or about 1 µg/mL and at or about 25 µg/mL, at or about 1 µg/mL and at or about 10 µg/mL, at or about 1 µg/mL and at or about 5 µg/mL, 5 µg/mL and at or about 40 µg/mL, at or about 5 µg/mL and at or about 25 µg/mL, at or about 5 µg/mL and at or about 10 µg/mL, 10 µg/mL and at or about 40 µg/mL, at or about 10 µg/mL and at or about 25 µg/mL, or at or about 25 µg/mL and at or about 40 µg/mL. In some embodiments, the peptide concentration, representing the single peptide or pool of peptides can be at or about 0.0001 µg/mL, at or about 0.001 µg/mL, at or about 0.01 µg/mL, at or about 0.1 µg/mL, at or about 1 µg/mL, at or about 10 µg/mL, at or about 20 µg/mL, at or about 30 µg/mL or at or about 40 µg/mL or any value between any of the foregoing. In some embodiments, the peptide concentrations is the concentration of a pool of peptides. In some embodiments, the peptide concentration is a concentration of a single or individual peptide.

In an embodiment, inducing APCs (e.g. B cells or monocyte-derived DCs) to present the mutated amino acid sequence comprises introducing a nucleotide sequence encoding the mutated amino acid sequence into the APCs. The nucleotide sequence is introduced into the APCs so that the APCs express and display the mutated amino acid sequence, bound to an MHC molecule, on the cell membrane. The nucleotide sequence encoding the mutated amino acid may be RNA or DNA. Introducing a nucleotide sequence into APCs may be carried out in any of a variety of different ways. Non-limiting examples of techniques that are useful for introducing a nucleotide sequence into APCs include transformation, transduction, transfection, and electroporation. In some cases, peptides for binding MHC class II restricted molecules are presented as a gene encoding DNA of the mutation and electroporated into the antigen presenting cell. This DNA will then be in-vitro transcribed into RNA encoding peptides on the surface for recognition by CD4+ cells. In some cases, Tandem Mini Gene methods can be employed to do this for MHC class II restricted molecules, see e.g. published PCT Patent Application Number WO2016/053338 and Parkhurst et al. (2016) Clin Cancer Res., 23:2491-505. In an embodiment in which more than one gene is identified, the method may comprise preparing more than one nucleotide sequence, each encoding a mutated amino acid sequence encoded by a different gene, and introducing each nucleotide sequence into a different population of APCs. In this regard, multiple populations of APCs, each population expressing and displaying a different mutated amino acid sequence, may be obtained. For example, in the case where tandem minigenes are used, APCs (e.g. B cells or monocyte-derived DCs) are electroporated with a mixture of DNA (plurality of DNA) encoding a different mutated amino acid sequences, which will then be in-vitro transcribed into RNA encoding peptides for surface recognition by CD4+ T cells. In some embodiments, APCs (e.g. B cells or monocyte-derived DCs) are electroporated using the Lonza 4D Nucleofector continuous electroporation system.

The methods include adding T cells (e.g. from patient having a tumor) with the culture of APCs presenting the peptides and co-culturing the APCs and T cells for a period of time to allow presentation and recognition of the peptide on the surface of APCs by one or more T cells in the population. In provided embodiments, the T cells include a population of the stimulated T cells.

In some embodiments, for peptide pulsing APCs (e.g. B cells or monocyte-derived DCs) are incubated with peptides for between at or about 2 hours and at or about 48 hours, such as between at or about 2 hours and at or about 36 hours, between at or about 2 hours and at or about 24 hours, between at or about 2 hours and at or about 24 hours, between at or about 2 hours and at or about 18 hours, between at or about 2 hours and at or about 12 hours, between at or about 2 hours and at or about 6 hours, between at or about 6 hours and at or about 48 hours, between at or about 6 hours and at or about 36 hours, between at or about 6 hours and at or about 24 hours, between at or about 6 hours and at or about 24 hours, between at or about 6 hours and at or about 18 hours, between at or about 6 hours and at or about 12 hours, between at or about 12 hours and at or about 48 hours, between at or about 12 hours and at or about 36 hours, between at or about 12 hours and at or about 24 hours, between at or about 12 hours and at or about 18 hours, between at or about 18 hours and at or about 48 hours, between at or about 18 hours and at or about 36 hours, between at or about 18 hours and at or about 24 hours, between at or about 24 hours and at or about 48 hours, between at or about 24 hours and at or about 36 hours, or between at or about 36 hours and at or about 48 hours. In some embodiments, the APCs (e.g. B cells or monocyte-derived DCs) are incubated with peptides for at or about 4 hours, at or about 6 hours, at or about 7 hours, at or about 8 hours, at or about 9 hours, at or about 10 hours, at or about 12 hours, at or about 14 hours, at or about 16 hours, at or about 18 hours, at or about 20 hours, at or about 22 hours, at or about 24 hours, or any value between any of the foregoing. In particular embodiments, the APCs (e.g. PBMCs, B cells or monocyte-derived DCs) are incubated with peptides overnight, such as for between at or about 8 to 12 hours. In some embodiments, the co-culture incubation is for at or about 6 hours.

The T cells (e.g. stimulated T cells) and APCs (e.g. B cells or monocyte-derived DCs) can be present in a culture at a ratio of T cells to APC of 1:100 to 100:1, such as 1:50 to 50:1, 1:25 to 25:1, 1:10 to 10:1, or 1:5 to 5:1. In some embodiments, the ratio of T cells (e.g. stimulated T cells) to APC is at or about 1:100, at or about 1:50, at or about 1:25, at or about 1:10, at or about 1:5, at or about 1:2.5, at or about 1:1, at or about 2:5:1, at or about 5:1, at or about 10:1, at or about 25:1, at or about 50:1 or at or about 100:1, or any value between any of the foregoing. In some embodiments, the ratio of T cells (e.g. stimulated T cells) to APC is between 20:1 and 1:1, between 15:1 and 1:1, between 10:1 and 1:1, between 5:1 and 1:1, or between 2.5:1 and 1:1. In some embodiments, the ratio of T cells (e.g. stimulated T cells) to APC is between 1:20 and 1:1, between 1:15 and 1:1, between 1:10 and 1:1, between 1:5 and 1:1, or between 1:2.5 and 1:1. In particular embodiments, co-culture will be performed by mixing the T cells, e.g. population of stimulated T cells, and APC (e.g. B cells or monocyte-derived DC) at approximately a 3:1 ratio. In some embodiments, co-culture will be performed by mixing the T cells, e.g. population of stimulated T cells, and APC (e.g. B cells or monocyte-derived DC) at approximately a 1:1 ratio.

In some embodiments, one or more recombinant cytokine for sustaining T cells is added to the co-culture. In some embodiments, the recombinant cytokine can include one or more of IL-2, IL-7, IL-15 or IL-21. In some embodiments, the co-culturing is carried out in the presence of recombinant IL-2, IL-15 and IL-7. In some embodiments, the co-culturing is carried out in the presence of a IL-2. In some embodiments, the co-culturing is carried out in the presence of IL-15 and IL-17, which, in some aspects does not additionally include IL-2.

The recombinant cytokine generally is a recombinant human protein. In particular embodiments, the recombinant cytokine is present in the cell culture medium during the co-culture at a concentration of at least at or about or at or about 10 IU/mL, at least at or about or at or about 100 IU/mL, at least at or about or at or about 1000 IU/mL, at least at or about or at or about 1500 IU/mL, at least at or about or at or about 2000 IU/mL, at least at or about or at or about 2500 IU/mL, at least at or about or at or about 3000 IU/mL, at least at or about or at or about 3500 IU/mL, at least at or about or at or about 4000 IU/mL, at least at or about or at or about 4500 IU/mL, at least at or about or at or about 5000 IU/mL, at least at or about or at or about 5500 IU/mL, at least at or about or at or about 6000 IU/mL, at least at or about or at or about 6500 IU/mL, at least at or about or at or about 7000 IU/mL, at least at or about or at or about 7500 IU/mL, or at least at or about or at or about 8000 IU/mL. In an embodiment, the cell culture medium comprises between at or about 10 IU/mL and at or about 100 IU/mL, at or about 100 IU/mL and at or about 1000 IU/mL, at or about 1000 and at or about 2000 IU/mL, between at or about 2000 and at or about 3000 IU/mL, between at or about 3000 and 4000 at or about IU/mL, between at or about 4000 and at or about 5000 IU/mL, between at or about 5000 and at or about 6000 IU/mL, between at or about 6000 and at or about 7000 IU/mL, between at or about 7000 and at or about 8000 IU/mL, each inclusive.

In some embodiments, recombinant IL-2 is present in the cell culture medium. In some embodiments, recombinant IL-2 is added to the culture medium at a concentration between at or about 10 IU/mL and at or about 1000 IU/mL, such as between at or about 10 IU/mL and at or about 600 IU/mL, between at or about 10 IU/mL and at or about 400 IU/mL, between at or about 10 IU/mL and at or about 200 IU/mL, between at or about 10 IU/mL and at or about 100 IU/mL, between at or about 10 IU/mL and at or about 50 IU/mL, between at or about 50 IU/mL and at or about 1000 IU/mL, between at or about 50 IU/mL and at or about 600 IU/mL, between at or about 50 IU/mL and at or about 400 IU/mL, between at or about 50 IU/mL and at or about 200 IU/mL, between at or about 50 IU/mL and at or about 100 IU/mL, between at or about 100 IU/mL and at or about 1000 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 1000 IU/mL, between at or about 200 IU/mL and at or about 600 IU/mL, between at or about 200 IU/mL and at or about 400 IU/mL, between at or about 400 IU/mL and at or about 1000 IU/mL, between at or about 400 IU/mL and at or about 600 IU/mL or between at or about 600 IU/mL and at or about 1000 IU/mL. In some embodiments, recombinant IL-2 is present in an amount that is between 50 and 400 IU/mL.

In some embodiments, the incubation is carried out with a higher dose IL-2. In some aspects, IL-2 is the only recombinant cytokine added to the culture. In some embodiments, the recombinant IL-2 is added to the culture medium at a concentration between at or about 1000 IU/mL at or about 8000 IU/mL, such as between at or about 1000 IU/mL and at or about 7000 IU/mL, between at or about 1000 IU/mL and at or about 6000 IU/mL, between at or about 1000 IU/mL and at or about 5000 IU/mL, between at or about 1000 IU/mL and at or about 4000 IU/mL, between at or about 1000 IU/mL and at or about 2000 IU/mL, 2000 IU/mL at or about 8000 IU/mL, between at or about 2000 IU/mL and at or about 7000 IU/mL, between at or about 2000 IU/mL and at or about 6000 IU/mL, between at or about 2000 IU/mL and at or about 5000 IU/mL, between at or about 2000 IU/mL and at or about 4000 IU/mL, 4000 IU/mL at or about 8000 IU/mL, between at or about 4000 IU/mL and at or about 7000 IU/mL, between at or about 4000 IU/mL and at or about 6000 IU/mL, between at or about 4000 IU/mL and at or about 5000 IU/mL, between at or about 5000 IU/mL at or about 8000 IU/mL, between at or about 5000 IU/mL and at or about 7000 IU/mL, between at or about 5000 IU/mL and at or about 6000 IU/mL, between at or about 6000 IU/mL at or about 8000 IU/mL, between at or about 6000 IU/mL and at or about 7000 IU/mL or between at or about 7000 IU/mL and at or about 8000 IU/mL. In some embodiments, recombinant IL-2 is present in an amount that is or is about 6000 IU/mL.

In some embodiments, recombinant IL-15 is present in the cell culture medium. In some embodiments, the recombinant IL-15 is added to the culture medium at a concentration between at or about 10 IU/mL and 500 IU/mL, such as between at or about 10 IU/mL and at or about 400 IU/mL, between at or about 10 IU/mL and at or about 300 IU/mL, between at or about 10 IU/mL and at or about 200 IU/mL, between at or about 10 IU/mL and at or about 100 IU/mL, between at or about 10 IU/mL and at or about 70 IU/mL, between at or about 10 IU/mL and at or about 50 IU/mL, between at or about 10 IU/mL and at or about 30 IU /mL, between at or about 30 IU/mL and 500 IU/mL, between at or about 30 IU/mL and at or about 400 IU/mL, between at or about 30 IU/mL and at or about 300 IU/mL, between at or about 30 IU/mL and at or about 200 IU/mL, between at or about 30 IU/mL and at or about 100 IU/mL, between at or about 30 IU/mL and at or about 70 IU/mL, between at or about 30 IU/mL and at or about 50 IU/mL, between at or about 50 IU/mL and at or about 400 IU/mL, between at or about 50 IU/mL and at or about 500 IU/mL, between at or about 50 IU/mL and at or about 300 IU/mL, between at or about 50 IU/mL and at or about 200 IU/mL, between at or about 50 IU/mL and at or about 100 IU/mL, between at or about 50 IU/mL and at or about 70 IU/mL, between at or about 70 IU/mL and at or about 500 IU/mL, between at or about 70 IU/mL and at or about 400 IU/mL, between at or about 70 IU/mL and at or about 300 IU/mL, between at or about 70 IU/mL and at or about 200 IU/mL, between at or about 70 IU/mL and at or about 100 IU/mL, between at or about 100 IU/mL and at or about 500 IU/mL, between at or about 100 IU/mL and at or about 400 IU/mL, between at or about 100 IU/mL and at or about 300 IU/mL, between at or about 100 IU/mL and at or about 200 IU/mL, between at or about 200 IU/mL and at or about 500 IU/mL, between at or about 200 IU/mL and at or about 400 IU/mL, between at or about 200 IU/mL and at or about 300 IU/mL, between at or about 300 IU/mL and at or about 500 IU/mL, between at or about 200 IU/mL and at or about 400 IU/mL, or between at or about 400 IU/mL and at or about 500 IU/mL. In some embodiments, the IL-15 is added to the culture medium in an amount between at or about 100 IU/mL and at or about 200 IU/mL. In some embodiments, the IL-15 is added to the culture medium at or about 180 IU/mL.

In some embodiments, recombinant IL-7 is added to the culture medium. In some embodiments, the recombinant IL-7 is added to the culture medium at a concentration between at or about 100 IU/mL and at or about 2000 IU/mL, between at or about 100 IU/mL and at or about 1500 IU/mL, between at or about 100 IU/mL and at or about 1000 IU/mL, between at or about 100 IU/mL and at or about 800 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 2000 IU/mL, between at or about 200 IU/mL and at or about 1500 IU/mL, between at or about 200 IU/mL and at or about 1000 IU/mL, between at or about 200 IU/mL and at or about 800 IU/mL, between at or about 200 IU/mL and at or about 600 IU/mL, between at or about 200 IU/mL and at or about 400 IU/mL, between at or about 400 IU/mL and at or about 2000 IU/mL, between at or about 400 IU/mL and at or about 1500 IU/mL, between at or about 400 IU/mL and at or about 1000 IU/mL, between at or about 400 IU/mL and at or about 800 IU/mL, between at or about 400 IU/mL and at or about 600 IU/mL, between at or about 600 IU/mL and at or about 2000 IU/mL, between at or about 600 IU/mL and at or about 1500 IU/mL, between at or about 600 IU/mL and at or about 1000 IU/mL, between at or about 600 IU/mL and at or about 800 IU/mL, between at or about 800 IU/mL and at or about 2000 IU/mL, between at or about 800 IU/mL and at or about 1500 IU/mL, between at or about 800 IU/mL and at or about 1000 IU/mL, between at or about 1000 IU/mL and at or about 2000 IU/mL, between at or about 1000 IU/mL and at or about 1500 IU/mL, between at or about 1500 IU/mL and at or about 2000 IU/mL. In some embodiments, the IL-7 is added to the culture medium in an amount between at or about 1000 IU/mL and at or about 2000 IU/mL. In some embodiments, the IL-7 is added to the culture medium at or about 600 IU/mL.

In some embodiments, recombinant IL-21 is added to the culture medium. In some embodiments, the recombinant IL-21 is added to the culture medium at a concentration between at or about 0.5 IU/mL and at or about 20 IU/mL, between at or about 0.5 IU/mL and at or about 15 IU/mL, between at or about 0.5 IU/mL and at or about 10 IU/mL, between at or about 0.5 IU/mL and at or about 5 IU/mL, between at or about 0.5 IU/mL and at or about 2.5 IU/mL, between at or about 0.5 IU/mL and at or about 1 IU/mL, between at or about 1 IU/mL and at or about 20 IU/mL, between at or about 1 IU/mL and at or about 15 IU/mL, between at or about 1 IU/mL and at or about 10 IU/mL, between at or about 1 IU/mL and at or about 5 IU/mL, between at or about 1 IU/mL and at or about 2.5 IU/mL, between at or about 2.5 IU/mL and at or about 20 IU/mL, between at or about 2.5 IU/mL and at or about 15 IU/mL, between at or about 2.5 IU/mL and at or about 10 IU/mL, between at or about 2.5 IU/mL and at or about 5 IU/mL, between at or about 5 IU/mL and at or about 20 IU/mL, between at or about 5 IU/mL and at or about 15 IU/mL, between at or about 5 IU/mL and at or about 10 IU/mL, between at or about 10 IU/mL and at or about 20 IU/mL, between at or about 10 IU/mL and at or about 15 IU/mL, or between at or about 15 IU/mL and at or about 20 IU/mL. In some embodiments, the IL-21 is added to the culture medium in an amount between at or about 0.5 IU/mL and at or about 2.5 IU/mL. In some embodiments, the IL-21 is added to the culture medium at or about 1 IU/mL.

The co-culture of APCs and T cells can be incubated at a temperature suitable for the presentation of peptides on MHC and the activation of T cells in the culture, for example, at least about 25 degrees Celsius, generally at least about 30 degrees, and generally at or about 37 degrees Celsius. In some embodiments, the incubation is carried out for up to 96 hours. The incubation can be carried out for 24 hours to 96 hours, such as at or about 24 hours, at or about 36 hours, at or about 48 hours, at or about 60 hours, at or about 72 hours, at or about 84 hours or at or about 96 hours, or for a time between any of the foregoing. In particular embodiments, the co-culture is incubated for 24 to 48 hours.

In some embodiments, at the end of the co-culturing tumor reactive T cells are separated from APCs present in the co-culture. In some embodiments, the separation can include methods that select away or remove the APCs. In some embodiments, the separation can include methods that positively select or retain the T cells present in the co-culture. In some embodiments, total T cells in the co-culture can be selected. In particular embodiments, tumor reactive T cells or T cells that express one or more upregulation marker, e.g. activation markers, associated with tumor-reactive T cells can be selected.

### C. Selection of Cells

In embodiments of the provided methods, the methods involve enrichment or selection of tumor reactive T cells or T cells that are likely or suspected of being tumor reactive T cells by selecting or isolating T cells that are positive for CXCL13 and/or an exhaustion marker such as one or more of PD-1, CD39 and/or TIGIT.

In some embodiments, T cells that are positive for CXCL13 and/or an exhaustion marker such as one or more of PD-1, CD39 and/or TIGIT are selected or enriched from a population of T cells that have been isolated or selected from a biological sample, such as described in Section I.A.1. In some embodiments, T cells that are positive for CXCL13 are selected or enriched from a population of T cells that have been isolated or selected from a biological sample, such as described in Section I.A.1. In some embodiments, T cells that are positive for one or more of PD-1, CD39 and TIGIT are selected or enriched from a population of T cells that have been isolated or selected from a biological sample, such as described in Section I.A.1. In some embodiments, T cells that are positive for PD-1 and CD39 are selected or enriched from the population of T cells. In some embodiments, T cells that are positive of PD-1 and TIGIT are selected or enriched from the population of T cells. In some embodiments, T cells that are positive for CD39 and TIGIT are selected or enriched from the population of T cells. In aspects of any such embodiments, the biological sample is a tumor fragment containing a population of T cells and the cells positive for any of the above markers can be selected directly from cells of tumor fragments. In other aspects of any such embodiments, tumor fragments are enzymatically digested into a single cell suspension containing a population of T cells and the cells positive for any of the above markers can be selected directly from cells of the digested cell suspension.

In some embodiments, T cells that are positive for CXCL13 and/or an exhaustion marker such as one or more of PD-1, CD39 and/or TIGIT is further selected or enriched from a population of T cells after stimulation or expansion, such as described in Section I. A.2. In some embodiments, T cells that are positive for CXCL13 are selected or enriched from a population of T cells that have been initially expanded (e.g. first population of expanded T cells), such as described in Section I.A.2. In some embodiments, T cells that are positive for one or more of PD-1, CD39 and TIGIT are selected or enriched from a population of T cells initially expanded T cells (e.g. first expanded population), such as described in Section I.A.2. In some embodiments, T cells that are positive for PD-1 and CD39 are selected or enriched from the population of initially expanded T cells (e.g. first expanded population). In some embodiments, T cells that are positive of PD-1 and TIGIT are selected or enriched from the initially expanded population of T cells (e.g. first expanded population). In some embodiments, T cells that are positive for CD39 and TIGIT are selected or enriched from the initially expanded population of T cells (e.g. first expanded population).

In some embodiments, T cells that are positive for CXCL13 and/or an exhaustion marker such as one or more of PD-1, CD39 and/or TIGIT is further selected or enriched from a population of T cells after their co-culture with APCs, such as described in Section I.B. In some embodiments, T cells that are positive for CXCL13 are selected or enriched from a coculture containing a reactive T cell population, such as described in Section I.B. In some embodiments, T cells that are positive for one or more of PD-1, CD39 and TIGIT are selected or enriched from a co-culture containing a reactive T cell population, such as described in Section I.B. In some embodiments, T cells that are positive for PD-1 and CD39 are selected or enriched from a co-culture containing a reactive T cell population. In some embodiments, T cells that are positive of PD-1 and TIGIT are selected or enriched from from a co-culture containing a reactive T cell population. In some embodiments, T cells that are positive for CD39 and TIGIT are selected or enriched from a co-culture containing a reactive T cell population.

In some embodiments, the methods can include a combination of any of the above selections for obtaining or enriching tumor reactive T cells or T cells that are likely or suspected of being tumor reactive T cells. In some embodiments, the enriched population of cells is used in subsequent processing steps, such as subsequent processing steps involving incubation, stimulation or activation, and/or expansion in accord with one or more steps of any of the provided methods.

In aspects of any of the provided embodiments, selection of cells positive for CXCL13 and/or an exhaustion marker such as one or more of PD-1, CD39 and/or TIGIT can be carried out in accord with any of the provided methods. In some embodiments, enriching for a T cell that is surface positive for one or more cell surface marker includes any method for separation based on such markers. In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner. Methods of selection of cells include, but are not limited to, bead selection (e.g. serial bead passage for positive/negative selection of cells), immunoaffinity chromatography (e.g. serial elution for positive/negative selection, and flow cytometry sorting. For use in accord with the provided methods, the selection method meets GMP standards.

In particular embodiments, selection of cells is carried out by flow cytometry-based cell sorting. Compared to other methods, flow cytometry-based cell sorting has the advantage that cells can be isolated in a single step on the basis of multiple parameters for each cell, thereby achieving a higher yield of cells and a higher purity that may not be possible with bead-based (e.g. magnetic bead-based) separations. Using flow cytometry sorting, a single process can remove and isolate specific populations based on a complex cell surface phenotype. Cell selection sorting equipment can be used that has a sufficiently high-throughput to handle large volumes and cell numbers. Non-limiting cell sorting equipment includes, for example, Sony FX500 or the Tyto cell sorting systems (Miltenyi). For use in provided methods, the flow cytometer instrument is GMP compliant. Method of cell sorting to achieve multiparameter sorting for two or more cell surface markers (e.g. CD39, PD-1 and TIGIT) can be carried out using multicolor flurophore reagents that are compatible. It is within the level of a skilled artisan to choose appropriate fluorphores and reagents, such as by choosing a bright fluorophore and choosing fluorophores that have minimal to no spectral overlap.

In some embodiments, cells are selected directly from a single cell suspension input sample prepared by enzymatic or mechanical digestion of tumor fragments, in which the selection is carried out using a CXCL13 capture or CD39/PD1/TIGIT positive selection. In some embodiments, the selected cells are then stimulated for expansion using methods as described, such as by incubation or culture in the presence of one or more of IL-2, IL-7, IL-15 or IL-21. In some embodiments, the stimulation would not include culture with an anti-CD3 antibody (OKT3) or other costimulatory molecules. In some embodiments, the stimulation may include culture with an anti-CD3 antibody (OKT3) or other costimulatory molecules.

In some embodiments, cells are selected after co-culture with APC presenting neo-peptide (mutated peptides) and selected based on CXCL13 secretion or CD39/PD1/TIGIT upregulation using a CXCL13 capture or CD39/PD1/TIGIT positive selection. In aspects of the method, the co-culture would further include on or more recombinant cytokine, such as one or more of IL-2, IL-7, IL-15 or IL-21. In some embodiments, following enrichment by selection of cells from the co-culture, the enriched cells are then stimulated for expansion using methods as described, such as by incubation or culture in the presence of one or more of IL-2, IL-7, IL-15 or IL-21. In some embodiments, the stimulation would not include culture with an anti-CD3 antibody (OKT3) or other costimulatory molecules. In some embodiments, the stimulation may include culture with an anti-CD3 antibody (OKT3) or other costimulatory molecules.

In some aspects, prior to or concurrently with the selection or enrichment, T cells can be enriched or selected from a biological sample, such as based on T cell markers CD3, CD4 or CD8. In some embodiments, such enrichment includes selecting or isolating T cells that are surface positive for CD3, CD4 and/or CD8. For example, such CD3+ T cells, or CD4⁺ and/or CD8⁺ populations, can be further sorted into sub-populations by positive or negative selection for markers expressed or expressed to a relatively higher degree on tumor-reactive T cells or on T cells having expression of CXCL13 and/or an exhaustion marker such as one or more of PD-1, CD39 and/or TIGIT or of a T cell activation marker (e.g. CD134 and/or CD137) associated with tumor-reactive T cells. In some embodiments, such cells include or are enriched for tumor-reactive T cells or T cells associated with tumor-reactive T cells. In some embodiments, the selection results in a population of T cells enriched for CD3+ T cells or CD4+ cells and CD8+ cells, that are further positive for one of more of CXCL13 and/or an exhaustion marker such as one or more of PD-1, CD39 and/or TIGIT.

In some embodiments, any antibody reagent used to select cells in accord with the provided methods is a GMP antibody reagent or is an ASR reagent.

In some embodiments, cells are selected that are surface positive for CD39, PD-1 and/or TIGIT. In some cases, staining methods also can include selecting CD4 and/or CD8 T cells from a sample. Methods and antibody reagetns to select for cells positive for these markers are known and commercially available. Table 1 lists exemplary antibodies for use in staining and selection or sorting of cells as described herein.

| **Table 1 :Exemplary Staining Antibodies** | | | | |
|---|---|---|---|---|
| Target | Name | Product No. | Manufacturer | Clone |
| TIGIT | | | | |
| TIGIT | PE anti-human TIGIT (VSTM3) | 372704 | BioLegend | A15153G |
| TIGIT | Human TIGIT APC-conjugated Antibody | FAB7898A | R&D Systems | 741182 |
| TIGIT | TIGIT Antibody, anti-human | 130-096-382 | Miltenyi | 4E1.2 |
| TIGIT | PE Mouse Anti-Mouse TIGIT | 565168 | BD | 1G9 |

| CD39 | | | | |
|---|---|---|---|---|
| CD39 | Anti-CD39 Mouse Monoclonal Antibody (APC (Allophycocyanin)) | 328210 | BioLegend | A1 |
| CD39 | PE Mouse anti-Human CD39 | 555464 | BD | TU66 |
| CD39 | CD39 Antibody, anti-human | 130-093-504 | Miltenyi | MZ18-23C8 |
| CD39 | CD39-PC5.5, BA54 | B55385 | Beckman Coulter | BA54 |

| PD-1 | | | | |
|---|---|---|---|---|
| PD-1 | Anti-CD279 (PD-1) Mouse Monoclonal Antibody (Brilliant Violet^{®} 421) | 329920 | BioLegend | EH12.2H7 |
| PD-1 | BB515 Mouse Anti-Human CD279 (PD-1) | 564494 | BD | EH12.1 |
| PD-1 | CD279 (PD1) Antibody, anti-human | 130-117-384 | Miltenyi | PD1.3.1.3 |
| PD-1 | CD279 (PD1) Antibody, anti-human, REAfinity^{™} | 130-120-388 | Miltenyi | REA1165 |
| PD-1 | CD279-PC5.5, PD1.3 | B32613 | Beckman Coulter | PD1.3 |

| CD4 | | | | |
|---|---|---|---|---|
| CD4 | Anti-CD4 Mouse Monoclonal Antibody (Brilliant Violet^{®} 510) | 317444 | BioLegend | OKT4 |

| CD8 | | | | |
|---|---|---|---|---|
| CD8a | PE/Cy7 anti-human CD8a | 301012 | BioLegend | RPA-T8 |

In some embodiments, cells are selected that secrete CXCL13. Methods to select for secreted molecules from cells are known. In some embodiments, a cytokine-specific catch system can be used in which a cytokine-specific "catch" antibody is conjugated to an antibody specific to a cell surface receptor on a T cells (e.g. CD45). For example, a catch reagent containing an anti-CXCL13 antibody conjugated with a CD45-specific monoclonal antibody can be incubated with the population of cells. The cells can be incubated for 1-2 hours, such as 30-45 minutes, to allow cytokine secretion and binding of the cytokine to the cytokine-specific catch reagent on the secreting cells. In some cases, cells can be detected by labeling one or both of the cytokine-specific antibody or the T cell-specific antibody with a fluorophore. In other examples, a secondary antibody specific to the cytokine-specific antibody can be labeled with a fluorophore and added to the incubation as a detection antibody. Cells that are positive for the fluorophore can be identified by flow cytometry. In further embodiments, the caught cytokine may be further magnetically labeled with a specific antibody conjugated to super-paramagnetic particles for enrichment by magnetic cell sorting (MACs).

In some aspects, positive selection is carried out for surface expression of CXCR5. Methods for selection of surface receptors on cells can be by any of a number of techniques, such as generally involving antibody binding with an antibody specific reagent and subsequent enrichment by magnetic separation or fluorescence-activated cell sorting (FACS).

In some embodiments, cells can be selected for positive surface expression of one or more additional activation marker in accord with any of the provided methods. In some embodiments, the additional selection can be carried out on the same sample population together with selections for CD39, PD-1 and/or TIGIT, or CXCL13 as provided herein. In other embodiments, the additional selection is carried out in the same process but as an additional step on a different sample population from the selections for CD39, PD-1 and/or TIGIT, or CXCL13 as provided herein. For instance, in some embodiments, a selection for one or more of CD39, PD-1 and/or TIGIT can be carried out directly on a digested tumor sample, or a sample therefrom that has been initially expanded as described, and a further selection step for another activation marker (e.g. CD137 and/or CD134) can be carried out on a population of cells produced in a subsequent step of the method, e.g. after the co-culture.

In some aspects, positive selection is carried out for surface expression of one or more activation marker or exhaustion marker. These markers can then be used to select reactive cells. In particular, among T cell markers for selection are those that are upregulated and/or whose expression is specifically detected following antigen stimulation of T cells, such that antigen specific effectors can be identified as a surrogate of an antigen that is activating or stimulating the cells. For example, following antigen-induced stimulation, human T-cells undergo dynamic functional and phenotypic changes, including upregulated surface expression of receptor molecules. The upregulation of surface molecules provides the opportunity to identify and isolate antigen-specific T-cells, such as tumor-reactive T cells, through antibody binding of the upregulated determinant and subsequent enrichment by flow cytometry, including by methods involving magnetic separation and fluorescence-activated cell sorting (FACS).

In some embodiments, the marker is one or more of CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT. In some embodiments, tumor-reactive T cells or T cells associated with tumor-reactive T cells are selected, enriched or isolated based on positive surface expression of at least two or more of such markers, such as at least 3, 4, 5 or 6 T cell markers. In some embodiments, the tumor-reactive T cells or T cells associated with tumor-reactive T cells are selected, enriched or isolated based on positive surface expression of two or more of CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT. Methods for selection of surface receptors on cells can be by any of a number of techniques, such as generally involving antibody binding with an antibody specific reagent and subsequent enrichment by magnetic separation or fluorescence-activated cell sorting (FACS).

In some embodiments, the one or more marker is PD-1, CD39 and/or TIGIT. In some embodiments, the one or more marker is PD-1, CD39 and TIGIT (PD-1/CD39/TIGIT).

In some embodiments, the selection is for surface expression of CD137 (41BB). In some embodiments, the selection is for surface expression of CD134 (OX40). In some embodiments, the selection is for surface expression of CD137 and CD134.

In some embodiments, tumor-reactive T cells are selected using an MHC tetramer bound to a mutation-associated or tumor-associated peptide. In some embodiments, the tetramers are prepared using MHC class I or MHC class II algorithms. In some embodiments, the tetramer is detectably labeled, such as fluorescently labeled. In some embodiments, the tetramer is HLA-matched to the subject from which the source of biological cells is obtained. In some embodiments, selection of cells using an MHC tetramer is directly from a cell source, e.g. peripheral blood, for a sample from a subject. In some embodiments, selection of cells using an MHC tetramer is after selecting or enriching T cells that are surface positive for a T cell activation marker.

In some embodiments, the T cells for use in connection with the provided methods can be enriched or sorted a variety of ways including, but not limited to, magnetic bead separation, fluorescent cell sorting, and disposable closed cartridge based cell sorters. In particular aspects, one or more reagents specific to T cells or a subset thereof, such as reagents specific to T cell activation markers for selecting reactive cells, can be used including, but not limited to, florescent antibodies, nanoparticles or beads on cell selection equipment, but not limited to, the CliniMACS, Sony FX500 or the Tyto cell sorting systems (Miltenyi).

In some aspects, T cells can be selected from a biological sample, such as based on T cell markers CD3, CD4 or CD8. In some embodiments, selecting for a T cell that is surface positive for one or more cell surface marker includes any method for separation based on such markers.

In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner. In some embodiments, the immunoaffinity-based selections include contacting a sample containing cells, such as a sample containing a bulk population of T cells, e.g. primary human T cells, containing CD3+ T cells or CD4+ and CD8+ cells, with an antibody or binding partner that specifically binds to the cell surface marker or markers. In some embodiments, the antibody or binding partner is bound to a solid support or matrix, such as a sphere or bead, for example a nanoparticle, microbeads, nanobeads, including agarose, magnetic bead or paramagnetic beads, to allow for separation of cells for positive and/or negative selection. In some embodiments, the spheres or beads can be packed into a column to effect immunoaffinity chromatography, in which a sample containing cells, such as primary human T cells containing CD3+ T cells or CD4+ and CD8+ cells, is contacted with the matrix of the column and subsequently eluted or released therefrom. In other embodiments, the antibody or binding partner is detectably labeled.

In some aspects, the sample or composition of cells to be separated is incubated with small, magnetizable or magnetically responsive material, such as magnetically responsive particles or microparticles, such as nanoparticles or paramagnetic beads. The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select. Such beads are known and are commercially available from a variety of sources including, in some aspects, Dynabeads^{®} (Life Technologies, Carlsbad, CA), MACS^{®} beads (Miltenyi Biotec, San Diego, CA) or Streptamer^{®} bead reagents (IBA, Germany). In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained.

In certain embodiments, the sample is contacted with a binding agent, e.g., a detectably labeled binding agent, that specifically binds to a cell surface marker. In certain embodiments, the detectably labeled binding agent(s) are fluorescently labeled. In certain embodiments, T cells labeled with binding agents specific to a cell surface marker are identified by flow cytometry. In certain embodiments, the method further includes separating any resultant T cells labeled with the binding agent(s) from other components of the sample to produce a composition enriched for T cells surface positive for the one or more cell surface marker. Cell selection sorting equipment can be used that has a sufficiently high-throughput to handle large volumes and cell numbers. Non-limiting cell sorting equipment includes, for example, Sony FX500 or the Tyto cell sorting systems (Miltenyi).

The incubation generally is carried out under conditions whereby the antibodies or binding partners, or molecules, such as secondary antibodies or other reagents, which specifically bind to such antibodies or binding partners, which are attached to the magnetic particle or bead and/or are detectably labeled, specifically bind to cell surface molecules if present on cells within the sample. In some aspects, cells bound to the antibodies can be recovered or separated from non-bound cells in the sample.

In some aspects, a combination of positive and negative selection is performed during the same selection step, where the positive and negative fractions are retained and further processed or subject to further separation steps. Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100 % enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells. For example, in a selection of one of CD4+ or CD8+ cells, the selection would enriches for said population, either the CD4+ or CD8+ population, but also can contain some residual or small percentage of other non-selected cells, which can, in some cases, include the other of the CD4 or CD8 population still being present in the enriched population.

In some embodiments, isolation is carried out by enrichment for a particular cell population by positive selection, or depletion of a particular cell population, by negative selection. In some embodiments, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

In particular embodiments, a T cell population is one that includes both CD4+ and CD8+ T cells. Many cancers, including solid tumors, such as many common epithelial indications (e.g. GI), express class I and class II restricted mutations. In order for a T cell product to target such indications, e.g. common epithelial indications, it is contemplated that both CD8+ T cells to recognize class I MHC-restricted molecules and CD4+ T cells to recognize Class II MHC-restricted molecules are necessary.

In some embodiments, the methods further include isolation, selection and/or enrichment of CD3+ cells. In some embodiments, the methods include isolation, selection and/or enrichment of CD4+ and CD8+ cells. In some aspects, a CD4⁺ or CD8⁺ selection step, such as positive selection for CD4 and positive selection for CD8, is used to separate CD4⁺ helper and CD8⁺ cytotoxic T cells. Such selections in some aspects are carried out simultaneously and in other aspects are carried out sequentially, in either order. In some embodiments, the methods include enriching for CD4+ and CD8+ T cells by selecting for T cells surface positive for CD3 or by sequential or simultaneous selection for T cells surface positive for CD4 and T cells surface positive for CD8. Such CD3+ T cells, or CD4⁺ and/or CD8⁺ populations, can be further sorted into sub-populations by positive or negative selection for markers expressed or expressed to a relatively higher degree on tumor-reactive T cells or on T cells having expression of T cell markers associated with tumor-reactive T cells, e.g. as described above.

In some embodiments, the selections produces an enriched population of cells, such as a population of cells enriched for CD3+ T cells or CD4+ cells and CD8+ cells, that are further positive for one of more of such T cell marker, e.g. CXCL13 and/or PD-1/TIGIT/CD39. In some embodiments, such cells include or are enriched for tumor-reactive T cells or T cells associated with tumor-reactive T cells. In some embodiments, the enriched population of cells is used in subsequent processing steps, such as subsequent processing steps involving incubation, stimulation or activation, and/or expansion in accord with one or more steps of any of the provided methods.

In some embodiments, the enriched population of cells are enriched cells from a starting sample as describe above, in which the percentage of cells of a particular phenotype, e.g. tumor-reactive CD3+ T cells or CD3+ T cells surface positive for one or more T cell activation marker as described herein, in the enriched population of cells in increased by at least 10%, 20%, 30%, 40%, 50%, 60%, 70%, 80%, 90%, 100%, 500%, 1000%, 5000% or more greater than the percentage of such cells in the starting sample. In some embodiments, the purity of tumor-reactive CD3+ T cells or CD3+ T cells surface positive for one or more T cell activation marker in the enriched composition, i.e. the percentage of cells positive for the selected cell surface marker versus total cells in the population of enriched cells, is at least 90%, 91%, 92%, 93%, 94%, and is generally at least 95%, 96%, 97%, 98%, 99% or greater.

In some embodiments, the enriched population of cells are enriched cells from a starting sample as describe above, in which the percentage of cells of a particular phenotype, e.g. tumor-reactive CD3+ T cells or CD3+ T cells surface positive for one or more T cell marker, e.g. CXCL13 and/or PD-1/CD39/TIGIT, in the enriched population of cells in increased by at least 10%, 20%, 30%, 40%, 50%, 60%, 70%, 80%, 90%, 100%, 500%, 1000%, 5000% or more greater than the percentage of such cells in the starting sample. In some embodiments, the purity of tumor-reactive CD3+ T cells or CD3+ T cells surface positive for one or more T cell marker in the enriched composition, i.e. the percentage of cells positive for the selected cell surface marker (e.g. CXCL13 and/or PD-1/CD39/TIGIT) versus total cells in the population of enriched cells, is at least 90%, 91%, 92%, 93%, 94%, and is generally at least 95%, 96%, 97%, 98%, 99% or greater.

### D. Incubation for Expansion and Harvesting

In some embodiments, the provided methods include performing an incubation for expansion of the selected cells with one or more T-cell stimulating agent of lymphocytes under conditions to produce a population of expanded T cells. In some embodiments, in which a co-culture with APCs is carried out, the T cells from the co-culture, or selected T cells therefrom, are further incubated under conditions to expand the cells *ex vivo* following the co-culture. The incubation is carried out in the presence of one or more T cell stimulatory agent(s) under conditions for stimulating the T cells, such as to expand the T cells. The T cell stimulatory agent(s) can include any as described in Section A.2 above. In some aspects of provided methods, the incubation for expansion expands the selected or enriched cells, such as selected for cells positive for an activation marker (e.g. CXCL13 or PD-1 and/or TIGIT).

In provided embodiments, a T cell population from from the immediate subsequent step (e.g. following co-culture of cells as described in Section I.B, or following selection of cells as described in Section I.C. with or without a co-culture and with or without an initial expansion) are incubated in the presence of one or more T cell stimulatory agent(s) under conditions for stimulating the T cells. The output of the expansion containing the population of expanded T cells are harvested or collected as a therapeutic cell composition. The harvested cells can be formulated with a pharmaceutically acceptable expient suitable for administering the cells to a subject (e.g. a human patient, such as via autologous adoptive transfer). In some embodiments, the harvested cells are formulated with a cryoprotectant and cryopreserved, in which case the therapeutic composition of cells is thawed prior to administration to a subject.

In some aspects, the incubation for expansion is the only expansion carried out during the process for producing a population of expanded T cells in accord with the provided methods. In some embodiments, the expanded population of T cells is harvested or collected as a therapeutic cell composition.

In other aspects of provided methods, the incubation for expansion is a second expansion and is carried out in a method in which an initial expansion, e.g. as described in Section 1.A.2 had been perfomed. For example, after the initial expansion step, a selection or sort of the the initially expanded cells for cells positive for an activation marker (e.g. CXCL13 or PD-1 and/or TIGIT) is carried out, such as described in Section I.C. Then, a second expansion step to expand the population of selected cells is performed. In some embodiments, the second population of expanded T cells is harvested or collected as a therapeutic cell composition.

In further aspects of the provided methods, an initial expansion is not performed prior to selection of cells from a sample containing T cells (e.g. single cell suspension digest), but the incubation for expansion at the end of the process is split into at least two expansions, e.g. a first expansion and a second expansion. For instance, in some embodiments, a first expansion of a population of T cells (e.g. a population of T cells following co-culture of cells as described in Section I.B, or following selection of cells as described in Section I.C. with or without a co-culture) is carried out in the presence of one or more first T cell stimulatory agent(s) under conditions for expanding the cells to a first expanded T cell population, and then the first expanded T cell population is cultured with one or more second T-cell stimulating agent under conditions to produce a second expanded population of T cells, wherein the second population of expanded T cells is for use as a therapeutic cell composition. In such embodiments, the one or more first T cell stimulatory agent(s) and the one or more second T cell stimulatory agent(s) are the same.

In some embodiments, the expansion culture with a T cell stimulatory agent(s) does not include incubation with an agent or agents that engage CD3 and a costimulatory molecule, such as CD28. In some embodiments, the expansion culture with a T cell stimulatory agent(s) does not include incubation with an anti-CD3 antibody, such as OKT3. In some embodiments, the expansion culture with a T cell stimulatory agent(s) does not include incubation with an anti-CD3 (e.g. OKT3)/anti-CD28 antibody, presented by APC's, immobilized on a solid surface (e.g. bead), or as a soluble antibody. In some embodiment, the expansion culture with a T cell stimulatory agent(s) does not include incubation with soluble anti-CD3, such as OKT3. In some embodiment, the expansion culture with a T cell stimulatory agent(s) does not include incubation with an anti-CD3/anti-CD28, including such reagents immobilized on beads, e.g. as provided by Dynabeads. In some embodiments, the expansion culture with a T cell stimulatory agent(s) does not include incubation with APCs, such as irradiated APCs. In some embodiments, the expansion culture with a T cell stimulatory agent(s) does not include incubation with non-dividing PBMCs, such as irradiated PBMCs.

In some of any of the provided embodiments, the T cell stimulatory agent(s) is selected from an agent that initiates TCR/CD3 intracellular signaling and/or an agent that initiates signaling via a costimulatory receptor. In some of any of the provided embodiments, the agent that initiates TCR/CD3 intracellular signaling is an anti-CD3 antibody, such as OKT3. In some of any of the provided embodiments, the agent that initiates signaling via a costimulatory receptor comprises peripheral blood mononuclear cells (PBMCs), optionally non-dividing or irradiated PBMCs. In some of any of the provided embodiments, the agent that initiates signaling via a costimulatory receptor is an anti-CD28 antibody. In some of any of the provided embodiments, the T cell stimulatory agent(s) is an anti-CD3 antibody and an anti-CD28 antibody that each are soluble.

In embodiments of the provided methods, the stimulating conditions include one or more agent, e.g., ligand, which turns on or initiates TCR/CD3 intracellular signaling cascade in a T cell and/or a costimulatory signal in a T cell. Such agents can include antibodies, such as those specific for a TCR component, e.g., anti-CD3, and/or costimulatory receptor, e.g. anti-CD28 or anti-4-1BB. In some embodiments, such agents are added to the culture medium as soluble antibodies. In other embodiments, such agents are bound to solid support such as a bead. In some embodiments, the T cell stimulatory agent(s) includes anti-CD3/CD28 conjugated magnetic beads (e.g., DYNABEADS^{®} M-450 CD3/CD28 T Cell Expander).

An anti-CD3 antibody can include any antibody directed against or that can specifically bind the CD3 receptor on the surface of T cells, typically human CD3 on human T cells. Anti-CD3 antibodies include OKT3, also known as muromonab. Anti-CD3 antibodies also include the UHCTI clone, also known as T3 and CD3E. Other anti-CD3 antibodies include, for example, otelixizumab, teplizumab, and visilizumab. The anti-CD3 antibody can be added as a soluble reagent or bound to a bead. In particular embodiments, the anti-CD3 antibody is soluble.

In particular embodiments, the T cell stimulatory agent(s) include an anti-CD3 antibody, which is added to the cell culture medium during the incubation. In some embodiments, the anti-CD3 antibody is added at a concentration ranging between at or about 0.1 ng/mL and 50 ng/mL, such between at or about 0.5 ng/mL and at or about 50 ng/mL, between at or about 0.5 ng/mL and at or about 30 ng/mL, between at or about 0.5 ng/mL and at or about 15 ng/mL, between at or about 0.5 ng/mL and at or about 5 ng/mL, between at or about 0.5 ng/mL and at or about 1 ng/mL, between at or about 1 ng/mL and at or about 50 ng/mL, between at or about 1 ng/mL and at or about 30 ng/mL, between at or about 1 ng/mL and at or about 15 ng/mL, between at or about 1 ng/mL and at or about 5 ng/mL, between at or about 5 ng/mL and at or about 50 ng/mL, between at or about 5 ng/mL and at or about 30 ng/mL, between at or about 5 ng/mL and at or about 15 ng/mL, between at or about 15 ng/mL and at or 50 ng/mL, between at or about 15 ng/mL and at or about 30 ng/mL or between at or about 30 ng/mL and at or about 50 ng/mL, each inclusive.

In particular embodiments, the anti-CD3 antibody is OKT3. In an embodiment, the cell culture medium comprises about 0.1 ng/mL, about 0.5 ng/mL, about 1 ng/mL, about 2.5 ng/mL, about 5 ng/mL, about 7.5 ng/mL, about 10 ng/mL, about 15 ng/mL, about 20 ng/mL, about 25 ng/mL, about 30 ng/mL, about 35 ng/mL, about 40 ng/mL, about 50 ng/mL, about 60 ng/mL, about 70 ng/mL, about 80 ng/mL, about 90 ng/mL, about 100 ng/mL, about 200 ng/mL, about 500 ng/mL, and about 1 µg/mL of OKT3 antibody. In an embodiment, the cell culture medium comprises between 0.1 ng/mL and 1 ng/mL, between 1 ng/mL and 5 ng/mL, between 5 ng/mL and 10 ng/mL, between 10 ng/mL and 20 ng/mL, between 20 ng/mL and 30 ng/mL, between 30 ng/mL and 40 ng/mL, between 40 ng/mL and 50 ng/mL, and between 50 ng/mL and 100 ng/mL of OKT3 antibody.

In some embodiments, the T cell stimulatory agent(s) includes incubation with an anti-CD3 antibody and incubation with a further agent that specifically binds to CD28 or stimulates or induces a CD28-mediated signal in cells. In some embodiments, the CD28-mediated signal can be initiated or provided by anti-CD28 antibody or antigen-binding fragment thereof. In some embodiments, the CD28-mediated signal can be provided by antigen-presenting feeder cells (APCs), such as peripheral blood mononuclear cells (PBMC).

In some embodiments, the T cell stimulatory agent(s) can include adding to the population of T cells feeder cells, such as non-dividing peripheral blood mononuclear cells (PBMC). In some aspects, the non-dividing feeder cells can comprise gamma- irradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells. In some embodiments, the resulting population of cells contains at least about 5, 10, 20, or 40 or more PBMC feeder cells for each T lymphocyte in the initial population to be expanded. In some embodiments, the ratio of T cells to PBMCs and/or antigen-presenting cells is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 375, about 1 to 400, or about 1 to 500.

In some embodiments, the T cell stimulatory agent(s) can include adding to the population of cells an anti-CD28 antibody or antigen-binding fragment thereof. An anti-CD28 antibody can include any antibody directed against or that can specifically bind the CD28 receptor on the surface of T cells. Non-limiting examples of anti-CD28 antibodies include NA/LE (e.g. BD Pharmingen), IM1376 (e.g. Beckman Coulter), or 15E8 (e.g. Miltenyi Biotec). The anti-CD28 antibody can be added as a soluble reagent or bound to a bead. In particular embodiments, the anti-CD3 antibody is soluble. In some embodiments, the anti-CD28 antibody is added at a concentration ranging between at or about 1 ng/mL and 1000 ng/mL, between at or about 1 ng/mL and 500 ng/mL, between at or about 1 ng/mL and at or about 100 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 100 ng/mL, between at or about 100 ng/mL and at or about 1000 ng/mL, between at or about 100 ng/mL and at or about 500 ng/mL or between at or about 500 ng/mL and at or about 1000 ng/mL.

In general, the culturing and incubations can occur in the presence of recombinant cytokines. In some embodiments, the cytokine is added or is exogenous to the culture media. In some embodiments, the culturing is carried out in the presence of a recombinant T cell stimulating cytokine, such as IL-2, IL-7, IL-15, IL-21, IL-25, IL-23, IL-27 and/or IL-35. In some embodiments, the T cell stimulatory agent(s) include a recombinant T cell stimulating cytokine, such as IL-2, IL-7, IL-15, IL-21, IL-25 and/or IL-23. In some of any of the provided embodiments, the culturing is carried out in the presence of a recombinant cytokine selected from the group consisting of IL-2, IL-15, IL-7, IL-21, Il-25 and IL-23. In some embodiments, the culturing and incubation is carried out in the presence of recombinant IL-2, IL-15 and IL-7. In some embodiments, the culturing is carried out in the presence of a IL-2. In some embodiments, the culturing is carried out in the presence of IL-15 and IL-17, which, in some aspects does not additionally include IL-2. In some embodiments, the T cell stimulating cytokine includes IL-2, alone or in combination with another cytokine from IL-25, IL-23, IL-27 and/or IL-35.

In some embodiments, the choice of cytokine or combination of cytokines is within the level of a skilled artisan, so long as the cytokine or cytokines provide activity to stimulate the T cells to expand or proliferate. The activity to stimulate tumor reactive T cells can be direct or indirect. In some embodiments, the one or more cytokines directly stimulate tumor reactive T cells to proliferate. In some embodiments, the one or more cytokines suppress Tregulatory T cells, thereby indirectly stimulating or enhancing proliferation of desired tumor reactive T cells.

The recombinant cytokine generally is a recombinant human protein. In particular embodiments, the recombinant cytokine is present in the cell culture medium during the incubation at a concentration of at least or at least about 0.5 IU/mL, at least or at least about 1.0 IU/mL, at least or at least about 5 IU/mL, at least at or about or at or about 10 IU/mL, at least at or about or at or about 100 IU/mL, at least at or about or at or about 1000 IU/mL, at least at or about or at or about 1500 IU/mL, at least at or about or at or about 2000 IU/mL, at least at or about or at or about 2500 IU/mL, at least at or about or at or about 3000 IU/mL, at least at or about or at or about 3500 IU/mL, at least at or about or at or about 4000 IU/mL, at least at or about or at or about 4500 IU/mL, at least at or about or at or about 5000 IU/mL, at least at or about or at or about 5500 IU/mL, at least at or about or at or about 6000 IU/mL, at least at or about or at or about 6500 IU/mL, at least at or about or at or about 7000 IU/mL, at least at or about or at or about 7500 IU/mL, or at least at or about or at or about 8000 IU/mL. In an embodiment, the cell culture medium comprises between at or about 10 IU/mL and at or about 100 IU/mL, at or about 100 IU/mL and at or about 1000 IU/mL, at or about 1000 and at or about 2000 IU/mL, between at or about 2000 and at or about 3000 IU/mL, between at or about 3000 and 4000 at or about IU/mL, between at or about 4000 and at or about 5000 IU/mL, between at or about 5000 and at or about 6000 IU/mL, between at or about 6000 and at or about 7000 IU/mL, between at or about 7000 and at or about 8000 IU/mL, each inclusive.

In some embodiments, recombinant IL-2 is present in the cell culture medium. In some embodiments, recombinant IL-2 is added to the culture medium at a concentration between at or about 10 IU/mL and at or about 1000 IU/mL, such as between at or about 10 IU/mL and at or about 600 IU/mL, between at or about 10 IU/mL and at or about 400 IU/mL, between at or about 10 IU/mL and at or about 200 IU/mL, between at or about 10 IU/mL and at or about 100 IU/mL, between at or about 10 IU/mL and at or about 50 IU/mL, between at or about 50 IU/mL and at or about 1000 IU/mL, between at or about 50 IU/mL and at or about 600 IU/mL, between at or about 50 IU/mL and at or about 400 IU/mL, between at or about 50 IU/mL and at or about 200 IU/mL, between at or about 50 IU/mL and at or about 100 IU/mL, between at or about 100 IU/mL and at or about 1000 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 1000 IU/mL, between at or about 200 IU/mL and at or about 600 IU/mL, between at or about 200 IU/mL and at or about 400 IU/mL, between at or about 400 IU/mL and at or about 1000 IU/mL, between at or about 400 IU/mL and at or about 600 IU/mL or between at or about 600 IU/mL and at or about 1000 IU/mL. In some embodiments, recombinant IL-2 is present in an amount that is between 50 and 400 IU/mL.

In some embodiments, the incubation is carried out with a higher dose IL-2. In some aspects, IL-2 is the only recombinant cytokine added to the culture. In some embodiments, the recombinant IL-2 is added to the culture medium at a concentration between at or about 1000 IU/mL at or about 8000 IU/mL, such as between at or about 1000 IU/mL and at or about 7000 IU/mL, between at or about 1000 IU/mL and at or about 6000 IU/mL, between at or about 1000 IU/mL and at or about 5000 IU/mL, between at or about 1000 IU/mL and at or about 4000 IU/mL, between at or about 1000 IU/mL and at or about 2000 IU/mL, 2000 IU/mL at or about 8000 IU/mL, between at or about 2000 IU/mL and at or about 7000 IU/mL, between at or about 2000 IU/mL and at or about 6000 IU/mL, between at or about 2000 IU/mL and at or about 5000 IU/mL, between at or about 2000 IU/mL and at or about 4000 IU/mL, 4000 IU/mL at or about 8000 IU/mL, between at or about 4000 IU/mL and at or about 7000 IU/mL, between at or about 4000 IU/mL and at or about 6000 IU/mL, between at or about 4000 IU/mL and at or about 5000 IU/mL, between at or about 5000 IU/mL at or about 8000 IU/mL, between at or about 5000 IU/mL and at or about 7000 IU/mL, between at or about 5000 IU/mL and at or about 6000 IU/mL, between at or about 6000 IU/mL at or about 8000 IU/mL, between at or about 6000 IU/mL and at or about 7000 IU/mL or between at or about 7000 IU/mL and at or about 8000 IU/mL. In some embodiments, recombinant IL-2 is present in an amount that is or is about 6000 IU/mL.

In some embodiments, recombinant IL-15 is present in the cell culture medium. In some embodiments, the recombinant IL-15 is added to the culture medium at a concentration between at or about 10 IU/mL and 500 IU/mL, such as between at or about 10 IU/mL and at or about 400 IU/mL, between at or about 10 IU/mL and at or about 300 IU/mL, between at or about 10 IU/mL and at or about 200 IU/mL, between at or about 10 IU/mL and at or about 100 IU/mL, between at or about 10 IU/mL and at or about 70 IU/mL, between at or about 10 IU/mL and at or about 50 IU/mL, between at or about 10 IU/mL and at or about 30 IU /mL, between at or about 30 IU/mL and 500 IU/mL, between at or about 30 IU/mL and at or about 400 IU/mL, between at or about 30 IU/mL and at or about 300 IU/mL, between at or about 30 IU/mL and at or about 200 IU/mL, between at or about 30 IU/mL and at or about 100 IU/mL, between at or about 30 IU/mL and at or about 70 IU/mL, between at or about 30 IU/mL and at or about 50 IU/mL, between at or about 50 IU/mL and at or about 400 IU/mL, between at or about 50 IU/mL and at or about 500 IU/mL, between at or about 50 IU/mL and at or about 300 IU/mL, between at or about 50 IU/mL and at or about 200 IU/mL, between at or about 50 IU/mL and at or about 100 IU/mL, between at or about 50 IU/mL and at or about 70 IU/mL, between at or about 70 IU/mL and at or about 500 IU/mL, between at or about 70 IU/mL and at or about 400 IU/mL, between at or about 70 IU/mL and at or about 300 IU/mL, between at or about 70 IU/mL and at or about 200 IU/mL, between at or about 70 IU/mL and at or about 100 IU/mL, between at or about 100 IU/mL and at or about 500 IU/mL, between at or about 100 IU/mL and at or about 400 IU/mL, between at or about 100 IU/mL and at or about 300 IU/mL, between at or about 100 IU/mL and at or about 200 IU/mL, between at or about 200 IU/mL and at or about 500 IU/mL, between at or about 200 IU/mL and at or about 400 IU/mL, between at or about 200 IU/mL and at or about 300 IU/mL, between at or about 300 IU/mL and at or about 500 IU/mL, between at or about 200 IU/mL and at or about 400 IU/mL, or between at or about 400 IU/mL and at or about 500 IU/mL. In some embodiments, the IL-15 is added to the culture medium in an amount between at or about 100 IU/mL and at or about 200 IU/mL. In some embodiments, the IL-15 is added to the culture medium at or about 180 IU/mL.

In some embodiments, the incubation is carried out with a higher dose IL-15.

In some embodiments, the recombinant IL-15 is added to the culture medium at a concentration between at or about 500 IU/mL and at or about 5000 IU/mL, such as between at or about 500 IU/mL and at or about 4000 IU/mL, between at or about 500 IU/mL and at or about 2000 IU/mL, between at or about 500 IU/mL and at or about 1500 IU/mL, between at or about 500 IU/mL and at or about 1000 IU/mL, between at or about 500 IU/mL and at or about 750 IU/mL, between at or about 750 IU/mL and at or about 5000 IU/mL, between at or about 750 IU/mL and at or about 4000 IU/mL, between at or about 750 IU/mL and at or about 2000 IU/mL, between at or about 750 IU/mL and at or about 1500 IU/mL, between at or about 750 IU/mL and at or about 1000 IU/mL, between at or about 1000 IU/mL and at or about 5000 IU/mL, between at or about 1000 IU/mL and at or about 4000 IU/mL, between at or about 1000 IU/mL and at or about 2000 IU/mL, between at or about 1000 IU/mL and at or about 1500 IU/mL, between at or about 1500 IU/mL and at or about 5000 IU/mL, between at or about 1500 IU/mL and at or about 4000 IU/mL, between at or about 1500 IU/mL and at or about 2000 IU/mL, between at or about 2000 IU/mL and at or about 5000 IU/mL, such as between at or about 2000 IU/mL and at or about 4000 IU/mL, or between at or about 4000 IU/mL and at or about 5000 IU/mL. In some embodiments, the recombinant IL-15 is added to the cell culture media at a concentration of at or about 500 IU/mL, at or about 600 IU/mL, at or about 700 IU/mL, at or about 800 IU/mL, at or about 900 IU/mL, at or about 1000 IU/mL, at or about 1100 IU/mL, at or about 1200 IU/mL, at or about 1300 IU/mL, at or about 1400 IU/mL, at or about 1500 IU/mL, at or about 1600 IU/mL, at or about 1700 IU/mL, at or about 1800 IU/mL, at or about 1900 IU/mL or at or about 2000 IU/mL, or any concentration between any of the foregoing. In some embodiments, IL-15 is added to the culture medium at a concentration of at or about 1000 IU/mL.

In some embodiments, the expansion is carried out in the presence of recombinant IL-15 added at a concentration of 500 IU/mL to 2000 IU/mL (e.g. at or about 1000 IU/mL). In some embodiments, the expansion is carried out in the presence of recombinant IL-15 added at a concentration of at or about 1000 IU/mL. In some embodiments, at least one other recombinant modulatory cytokine from IL-23, IL-25, IL-27 or IL-35 is added to the culture meduium.

In some embodiments, recombinant IL-15 and IL-2 are added to the culture medium. In some embodiments, recombinant IL-15 is added at a concentration of 500 IU/mL to 2000 IU/mL (e.g. at or about 1000 IU/mL) and recombinant IL-2 is added at a concentration of 200 IU/mL to 1000 IU/mL (e.g. at or about 300 IU/mL). In some embodiments, the expansion is carried out in the presence of recombinant IL-15 added at 1000 IU/mL and recombinant IL-2 added at 300 IU/mL. In some embodiments, at least one other recombinant modulatory cytokine from IL-7, IL-21, IL-23, IL-25, IL-27 or IL-35 is added to the culture meduium.

In some embodiments, recombinant IL-7 is added to the culture medium. In some embodiments, the recombinant IL-7 is added to the culture medium at a concentration between at or about 100 IU/mL and at or about 2000 IU/mL, between at or about 100 IU/mL and at or about 1500 IU/mL, between at or about 100 IU/mL and at or about 1000 IU/mL, between at or about 100 IU/mL and at or about 800 IU/mL, between at or about 100 IU/mL and at or about 600 IU/mL, between at or about 100 IU/mL and at or about 400 IU/mL, between at or about 100 IU/mL and at or about 200 IU/mL, between at or about 200 IU/mL and at or about 2000 IU/mL, between at or about 200 IU/mL and at or about 1500 IU/mL, between at or about 200 IU/mL and at or about 1000 IU/mL, between at or about 200 IU/mL and at or about 800 IU/mL, between at or about 200 IU/mL and at or about 600 IU/mL, between at or about 200 IU/mL and at or about 400 IU/mL, between at or about 400 IU/mL and at or about 2000 IU/mL, between at or about 400 IU/mL and at or about 1500 IU/mL, between at or about 400 IU/mL and at or about 1000 IU/mL, between at or about 400 IU/mL and at or about 800 IU/mL, between at or about 400 IU/mL and at or about 600 IU/mL, between at or about 600 IU/mL and at or about 2000 IU/mL, between at or about 600 IU/mL and at or about 1500 IU/mL, between at or about 600 IU/mL and at or about 1000 IU/mL, between at or about 600 IU/mL and at or about 800 IU/mL, between at or about 800 IU/mL and at or about 2000 IU/mL, between at or about 800 IU/mL and at or about 1500 IU/mL, between at or about 800 IU/mL and at or about 1000 IU/mL, between at or about 1000 IU/mL and at or about 2000 IU/mL, between at or about 1000 IU/mL and at or about 1500 IU/mL, between at or about 1500 IU/mL and at or about 2000 IU/mL. In some embodiments, the IL-7 is added to the culture medium in an amount between at or about 1000 IU/mL and at or about 2000 IU/mL. In some embodiments, the IL-7 is added to the culture medium at or about 600 IU/mL.

In some embodiments, recombinant IL-7 and IL-2 are added to the culture medium. In some embodiments, recombinant IL-7 is added at a concentration of 400 IU/mL to 2000 IU/mL (e.g. at or about 600 IU/mL or 1000 IU/mL) and recombinant IL-2 is added at a concentration of 200 IU/mL to 1000 IU/mL (e.g. at or about 300 IU/mL). In some embodiments, the expansion is carried out in the presence of recombinant IL-7 added at 1000 IU/mL and recombinant IL-2 added at 300 IU/mL. In some embodiments, the expansion is carried out in the presence of recombinant IL-7 added at 600 IU/mL and recombinant IL-2 added at 300 IU/mL. In some embodiments, at least one other recombinant modulatory cytokine from IL-15, IL-21, IL-23, IL-25, IL-27 or IL-35 is added to the culture meduium.

In some embodiments, recombinant IL-15 and IL-7 are added to the culture medium. In some embodiments, recombinant IL-15 is added at a concentration of 500 IU/mL to 2000 IU/mL (e.g. at or about 1000 IU/mL) and recombinant IL-7 is added at a concentration of 400 IU/mL to 2000 IU/mL (e.g. at or about 600 IU/mL or 1000 IU/mL). In some embodiments, the expansion is carried out in the presence of recombinant IL-15 added at 1000 IU/mL and recombinant IL-7 added at 1000 IU/mL. In some embodiments, the expansion is carried out in the presence of recombinant IL-15 added at 1000 IU/mL and recombinant IL-7 added at 600 IU/mL. In some embodiments, at least one other recombinant modulatory cytokine from IL-2, IL-21, IL-23, IL-25, IL-27 or IL-35 is added to the culture meduium.

In some embodiments, recombinant IL-21 is added to the culture medium. In some embodiments, the recombinant IL-21 is added to the culture medium at a concentration between at or about 0.5 IU/mL and at or about 20 IU/mL, between at or about 0.5 IU/mL and at or about 15 IU/mL, between at or about 0.5 IU/mL and at or about 10 IU/mL, between at or about 0.5 IU/mL and at or about 5 IU/mL, between at or about 0.5 IU/mL and at or about 2.5 IU/mL, between at or about 0.5 IU/mL and at or about 1 IU/mL, between at or about 1 IU/mL and at or about 20 IU/mL, between at or about 1 IU/mL and at or about 15 IU/mL, between at or about 1 IU/mL and at or about 10 IU/mL, between at or about 1 IU/mL and at or about 5 IU/mL, between at or about 1 IU/mL and at or about 2.5 IU/mL, between at or about 2.5 IU/mL and at or about 20 IU/mL, between at or about 2.5 IU/mL and at or about 15 IU/mL, between at or about 2.5 IU/mL and at or about 10 IU/mL, between at or about 2.5 IU/mL and at or about 5 IU/mL, between at or about 5 IU/mL and at or about 20 IU/mL, between at or about 5 IU/mL and at or about 15 IU/mL, between at or about 5 IU/mL and at or about 10 IU/mL, between at or about 10 IU/mL and at or about 20 IU/mL, between at or about 10 IU/mL and at or about 15 IU/mL, or between at or about 15 IU/mL and at or about 20 IU/mL. In some embodiments, the IL-21 is added to the culture medium in an amount between at or about 0.5 IU/mL and at or about 2.5 IU/mL. In some embodiments, the IL-21 is added to the culture medium at or about 1 IU/mL.

In some embodiments, the incubation is carried out with a higher dose IL-21.

In some embodiments, the recombinant IL-21 is added to the culture medium at a concentration between at or about 500 IU/mL and at or about 5000 IU/mL, such as between at or about 500 IU/mL and at or about 4000 IU/mL, between at or about 500 IU/mL and at or about 2000 IU/mL, between at or about 500 IU/mL and at or about 1500 IU/mL, between at or about 500 IU/mL and at or about 1000 IU/mL, between at or about 500 IU/mL and at or about 750 IU/mL, between at or about 750 IU/mL and at or about 5000 IU/mL, between at or about 750 IU/mL and at or about 4000 IU/mL, between at or about 750 IU/mL and at or about 2000 IU/mL, between at or about 750 IU/mL and at or about 1500 IU/mL, between at or about 750 IU/mL and at or about 1000 IU/mL, between at or about 1000 IU/mL and at or about 5000 IU/mL, between at or about 1000 IU/mL and at or about 4000 IU/mL, between at or about 1000 IU/mL and at or about 2000 IU/mL, between at or about 1000 IU/mL and at or about 1500 IU/mL, between at or about 1500 IU/mL and at or about 5000 IU/mL, between at or about 1500 IU/mL and at or about 4000 IU/mL, between at or about 1500 IU/mL and at or about 2000 IU/mL, between at or about 2000 IU/mL and at or about 5000 IU/mL, such as between at or about 2000 IU/mL and at or about 4000 IU/mL, or between at or about 4000 IU/mL and at or about 5000 IU/mL. In some embodiments, the recombinant IL-21 is added to the cell culture media at a concentration of at or about 500 IU/mL, at or about 600 IU/mL, at or about 700 IU/mL, at or about 800 IU/mL, at or about 900 IU/mL, at or about 1000 IU/mL, at or about 1100 IU/mL, at or about 1200 IU/mL, at or about 1300 IU/mL, at or about 1400 IU/mL, at or about 1500 IU/mL, at or about 1600 IU/mL, at or about 1700 IU/mL, at or about 1800 IU/mL, at or about 1900 IU/mL or at or about 2000 IU/mL, or any concentration between any of the foregoing. In some embodiments, IL-21 is added to the culture medium at a concentration of at or about 1000 IU/mL.

In some embodiments, recombinant IL-21 and IL-2 are added to the culture medium. In some embodiments, recombinant IL-21 is added at a concentration of 500 IU/mL to 2000 IU/mL (e.g. at or about 1000 IU/mL) and recombinant IL-2 is added at a concentration of 200 IU/mL to 1000 IU/mL (e.g. at or about 300 IU/mL). In some embodiments, the expansion is carried out in the presence of recombinant IL-21 added at 1000 IU/mL and recombinant IL-2 added at 300 IU/mL. In some embodiments, at least one other recombinant modulatory cytokine from IL-7, IL-15, IL-23, IL-25, IL-27 or IL-35 is added to the culture medium.

In some embodiments, recombinant IL-23 is added to the culture medium. In some embodiments, recombinant IL-23 is added to the culture medium at a concentration between at or about 1 nM and at or about 500 nM, such as between at or about 1 nM and at or about 400 nM, between at or about 1 nM and at or about 300 nM, between at or about 1 nM and at or about 200 nM, between at or about 1 nM and at or about 100 nM, between at or about 1 nM and at or about 50 nM, between at or about 1 nM and at or about 25 nM, between at or about 1 nM and at or about 10 nM, between at or about 1 nM and at or about 5 nM, between at or about 5 nM and at or about 500 nM, between at or about 5 nM and at or about 400 nM, between at or about 5 nM and at or about 300 nM, between at or about 5 nM and at or about 200 nM, between at or about 5 nM and at or about 100 nM, between at or about 5 nM and at or about 50 nM, between at or about 5 nM and at or about 25 nM, between at or about 5 nM and at or about 10 nM, between at or about 10 nM and at or about 500 nM, between at or about 10 nM and at or about 400 nM, between at or about 10 nM and at or about 300 nM, between at or about 10 nM and at or about 200 nM, between at or about 10 nM and at or about 100 nM, between at or about 10 nM and at or about 50 nM, between at or about 10 nM and at or about 25 nM, between at or about 25 nM and at or about 500 nM, between at or about 25 nM and at or about 400 nM, between at or about 25 nM and at or about 300 nM, between at or about 25 nM and at or about 200 nM, between at or about 25 nM and at or about 100 nM, between at or about 25 nM and at or about 50 nM, between at or about 50 nM and at or about 500 nM, between at or about 50 nM and at or about 400 nM, between at or about 50 nM and at or about 300 nM, between at or about 50 nM and at or about 200 nM, between at or about 50 nM and at or about 100 nM, between at or about 100 nM and at or about 500 nM, between at or about 100 nM and at or about 400 nM, between at or about 100 nM and at or about 300 nM, between at or about 100 nM and at or about 200 nM, between at or about 200 nM and at or about 500 nM, between at or about 200 nM and at or about 400 nM, between at or about 200 nM and at or about 300 nM, between at or about 300 nM and at or about 500 nM, between at or about 300 nM and at or about 400 nM, or between at or about 400 nM and at or about 500 nM. In some embodiments, the recombinant IL-23 is added to the culture medium at a concentration of at or about 5 nM, at or about 10 nM, at or about 20 nM, at or about 30 nM, at or about 40 nM, at or about 50 nM, at or about 60 nM, at or about 70 nM, at or about 80 nM, at or about 90 nM or at or about 100 nM, or any value between any of the foregoing.

In some embodiments, recombinant IL-23 is added to the culture medium at a concentration of between at or about 0.1 ng/mL and at or about 1000 ng/mL, such as between at or about 0.1 ng/mL and at or about 500 ng/mL, between at or about 0.1 ng/mL and at or about 250 ng/mL, between at or about 0.1 ng/mL and at or about 100 ng/mL, between at or about 0.1 ng/mL and at or about 50 ng/mL, between at or about 0.1 ng/mL and at or about 10 ng/mL, between at or about 0.1 ng/mL and at or about 1 ng/mL, between at or about 1 ng/mL and at or about 1000 ng/mL, between at or about 1 ng/mL and at or about 500 ng/mL, between at or about 1 ng/mL and at or about 250 ng/mL, between at or about 1 ng/mL and at or about 100 ng/mL, between at or about 1 ng/mL and at or about 50 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 250 ng/mL, between at or about 10 ng/mL and at or about 100 ng/mL, between at or about 10 ng/mL and at or about 50 ng/mL, between at or about 50 ng/mL and at or about 1000 ng/mL, between at or about 50 ng/mL and at or about 500 ng/mL, between at or about 50 ng/mL and at or about 250 ng/mL, between at or about 50 ng/mL and at or about 100 ng/mL, between at or about 100 ng/mL and at or about 1000 ng/mL, between at or about 100 ng/mL and at or about 500 ng/mL, between at or about 100 ng/mL and at or about 250 ng/mL, between at or about 250 ng/mL and at or about 1000 ng/mL, between at or about 250 ng/mL and at or about 500 ng/mL, or between at or about 500 ng/mL and at or about 1000 ng/mL. In some embodiments, the recombinant IL-23 is added to the culture medium at a concentration of at or about 1 ng/mL, at or about 5 ng/mL, at or about 10 ng/mL, at or about 20 ng/mL, at or about 30 ng/mL, at or about 40 ng/mL, at or about 50 ng/mL, at or about 60 ng/mL, at or about 70 ng/mL, at or about 80 ng/mL, at or about 90 ng/mL or at or about 100 ng/mL or any value between any of the foregoing.

In some embodiments, the recombinant IL-23 is added to the culture medium at a concentration of at or about 1 ng/mL, at or about 5 ng/mL, at or about 10 ng/mL, at or about 20 ng/mL, at or about 30 ng/mL, at or about 40 ng/mL, at or about 50 ng/mL, at or about 60 ng/mL, at or about 70 ng/mL, at or about 80 ng/mL, at or about 90 ng/mL or at or about 100 ng/mL or any value between any of the foregoing.

In some embodiments, the recombinant IL-23 is added to the culture medium at a concentration of at or about 200 ng/mL, at or about 300 ng/mL, at or about 400 ng/mL, at or about 500 ng/mL, at or about 600 ng/mL, at or about 700 ng/mL, at or about 800 ng/mL, at or about 900 ng/mL, at or about 1000 ng/mL, at or about 1200 ng/mL, at or about 1400 ng/mL or at or about 1600 ng/mL, at or about 1800 ng/mL or at or about 2000 ng/mL, or any value between any of the foregoing.

In some embodiments, recombinant IL-2 and recombinant IL-23 are added to the culture medium. In some embodiments, recombinant IL-2 is added at a concentration of 200 IU/mL to 1000 IU/mL (e.g. at or about 300 IU/mL) and recombinant IL-23 is added at a concentration of 100 ng/mL to 2000 ng/mL (e.g. between at or about 250 ng/mL and 1000 ng/mL, such as at or about 250 ng/mL, at or about 500 ng/mL or at or about 1000 ng/mL). In some embodiments, the expansion (e.g. second expansion) is carried out in the presence of recombinant IL-2 added at a concentration of 200 IU/mL to 1000 IU/mL (e.g. at or about 300 IU/mL) and recombinant IL-23 added at a concentration of between 100 ng/mL and 2000 ng/mL (e.g. between at or about 250 ng/mL and 1000 ng/mL, such as at or about 250 ng/mL, at or about 500 ng/mL or at or about 1000 ng/mL). In some embodiments, at least one other recombinant modulatory cytokine from IL-7, IL-21, IL-15, IL-25, IL-27 or IL-35 is added to the culture medium.

In some embodiments, recombinant IL-25 is added to the culture medium. In some embodiments, the recombinant IL-25 is added to the culture medium at a concentration between at or about 0.001 nM and at or about 10 nM, such as at a concentration between at or about 0.001 nM and at or about 5 nM, between at or about 0.001 nM and at or about 2.5 nM, between at or about 0.001 nM and at or about 1 nM, between at or about 0.001 nM and at or about 0.5 nM, between at or about 0.001 nM and at or about 0.1 nM, between at or about 0.001 nM and at or about 0.05 nM, between at or about 0.001 nM and at or about 0.01 nM, between at or about 0.001 nM and at or about 0.005 nM, between at or about 0.005 nM and at or about 10 nM, between at or about 0.005 nM and at or about 5 nM, between at or about 0.005 nM and at or about 2.5 nM, between at or about 0.005 nM and at or about 1 nM, between at or about 0.005 nM and at or about 0.5 nM, between at or about 0.005 nM and at or about 0.1 nM, between at or about 0.005 nM and at or about 0.05 nM, between at or about 0.005 nM and at or about 0.01 nM, between at or about 0.01 nM and at or about 10 nM, between at or about 0.01 nM and at or about 5 nM, between at or about 0.01 nM and at or about 2.5 nM, between at or about 0.01 nM and at or about 1 nM, between at or about 0.01 nM and at or about 0.5 nM, between at or about 0.01 nM and at or about 0.1 nM, between at or about 0.01 nM and at or about 0.05 nM, between at or about 0.05 nM and at or about 10 nM, between at or about 0.05 nM and at or about 5 nM, between at or about 0.05 nM and at or about 2.5 nM, between at or about 0.05 nM and at or about 1 nM, between at or about 0.05 nM and at or about 0.5 nM, between at or about 0.05 nM and at or about 0.1 nM, between at or about 0.1 nM and at or about 10 nM, between at or about 0.1 nM and at or about 5 nM, between at or about 0.1 nM and at or about 2.5 nM, between at or about 0.1 nM and at or about 1 nM, between at or about 0.1 nM and at or about 0.5 nM, between at or about 0.5 nM and at or about 10 nM, between at or about 0.5 nM and at or about 5 nM, between at or about 0.5 nM and at or about 2.5 nM, between at or about 0.5 nM and at or about 1 nM, between at or about 1 nM and at or about 10 nM, between at or about 1 nM and at or about 5 nM, between at or about 1 nM and at or about 2.5 nM, between at or about 2.5 nM and at or about 10 nM, between at or about 2.5 nM and at or about 5 nM, or between at or about 5 nM and at or about 10 nM. In some embodiments, the recombinant IL-25 is added to the culture medium at a concentration of at or about 0.01 nM, 0.02 nM, 0.03 nM, 0.04 nM, 0.05 nM, 0.06 nM, 0.07 nM, 0.08 nM, 0.09 nM or 1 nM, 1.5 nM or 2 nM or any value between any of the foregoing.

In some embodiments, the recombinant IL-25 is added to the culture medium at a concentration between at or about 0.01 ng/mL and at or about 500 ng/mL, between at or about 0.01 ng/mL and at or about 250 ng/mL, between at or about 0.01 ng/mL and at or about 100 ng/mL, between at or about 0.01 ng/mL and at or about 50 ng/mL, between at or about 0.01 ng/mL and at or about 20 ng/mL, between at or about 0.01 ng/mL and at or about 10 ng/mL, between at or about 0.01 ng/mL and at or about 5 ng/mL, between at or about 0.01 ng/mL and at or about 1 ng/mL, between at or about 0.01 ng/mL and at or about 0.05 ng/mL, between at or about 0.05 ng/mL and at or about 500 ng/mL, between at or about 0.05 ng/mL and at or about 250 ng/mL, between at or about 0.05 ng/mL and at or about 100 ng/mL, between at or about 0.05 ng/mL and at or about 50 ng/mL, between at or about 0.05 ng/mL and at or about 20 ng/mL, between at or about 0.05 ng/mL and at or about 10 ng/mL, between at or about 0.05 ng/mL and at or about 5 ng/mL, between at or about 0.05 ng/mL and at or about 1 ng/mL, between at or about 1 ng/mL and at or about 500 ng/mL, between at or about 1 ng/mL and at or about 250 ng/mL, between at or about 1 ng/mL and at or about 100 ng/mL, between at or about 1 ng/mL and at or about 50 ng/mL, between at or about 1 ng/mL and at or about 20 ng/mL, between at or about 1 ng/mL and at or about 10 ng/mL, between at or about 1 ng/mL and at or about 5 ng/mL, between at or about 5 ng/mL and at or about 500 ng/mL, between at or about 5 ng/mL and at or about 250 ng/mL, between at or about 5 ng/mL and at or about 100 ng/mL, between at or about 5 ng/mL and at or about 50 ng/mL, between at or about 5 ng/mL and at or about 20 ng/mL, between at or about 5 ng/mL and at or about 10 ng/mL, between at or about 10 ng/mL and at or about 500 ng/mL, between at or about 10 ng/mL and at or about 250 ng/mL, between at or about 10 ng/mL and at or about 100 ng/mL, between at or about 10 ng/mL and at or about 50 ng/mL, between at or about 10 ng/mL and at or about 20 ng/mL, between at or about 20 ng/mL and at or about 500 ng/mL, between at or about 20 ng/mL and at or about 250 ng/mL, between at or about 20 ng/mL and at or about 100 ng/mL, between at or about 20 ng/mL and at or about 50 ng/mL, between at or about 50 ng/mL and at or about 500 ng/mL, between at or about 50 ng/mL and at or about 250 ng/mL, between at or about 50 ng/mL and at or about 100 ng/mL, between at or about 100 ng/mL and at or about 500 ng/mL, between at or about 100 ng/mL and at or about 250 ng/mL, or between at or about 250 ng/mL and at or about 500 ng/mL. In some embodiments, the recombinant IL-25 is added to the culture medium at a concentration between at or about 1 ng/mL, at or about 2 ng/mL, at or about 3 ng/mL, at or about 4 ng/mL, at or about 5 ng/mL, at or about 6 ng/mL, at or about 7 ng/mL, at or about 8 ng/mL, at or about 9 ng/mL, at or about 10 ng/mL, at or about 15 ng/mL or at or about 20 ng/mL, or any value between any of the foregoing.

n some embodiments, the recombinant IL-25 is added to the culture medium at a concentration of between at or about 0.1 ng/mL and at or about 2000 ng/mL, such as between at or about 0.1 ng/mL and at or about 1000 ng/mL, between at or about 0.1 ng/mL and at or about 500 ng/mL, between at or about 0.1 ng/mL and at or about 250 ng/mL, between at or about 0.1 ng/mL and at or about 100 ng/mL, between at or about 0.1 ng/mL and at or about 50 ng/mL, between at or about 0.1 ng/mL and at or about 10 ng/mL, between at or about 0.1 ng/mL and at or about 1 ng/mL, between at or about 1 ng/mL and at or about 1000 ng/mL, between at or about 1 ng/mL and at or about 500 ng/mL, between at or about 1 ng/mL and at or about 250 ng/mL, between at or about 1 ng/mL and at or about 100 ng/mL, between at or about 1 ng/mL and at or about 50 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 250 ng/mL, between at or about 10 ng/mL and at or about 100 ng/mL, between at or about 10 ng/mL and at or about 50 ng/mL, between at or about 50 ng/mL and at or about 1000 ng/mL, between at or about 50 ng/mL and at or about 500 ng/mL, between at or about 50 ng/mL and at or about 250 ng/mL, between at or about 50 ng/mL and at or about 100 ng/mL, between at or about 100 ng/mL and at or about 1000 ng/mL, between at or about 100 ng/mL and at or about 500 ng/mL, between at or about 100 ng/mL and at or about 250 ng/mL, between at or about 250 ng/mL and at or about 1000 ng/mL, between at or about 250 ng/mL and at or about 500 ng/mL, or between at or about 500 ng/mL and at or about 1000 ng/mL.

In some embodiments, the recombinant IL-25 is added to the culture medium at a concentration of at or about 1 ng/mL, at or about 5 ng/mL, at or about 10 ng/mL, at or about 20 ng/mL, at or about 30 ng/mL, at or about 40 ng/mL, at or about 50 ng/mL, at or about 60 ng/mL, at or about 70 ng/mL, at or about 80 ng/mL, at or about 90 ng/mL or at or about 100 ng/mL or any value between any of the foregoing.

In some embodiments, the recombinant IL-25 is added to the culture medium at a concentration of at or about 200 ng/mL, at or about 300 ng/mL, at or about 400 ng/mL, at or about 500 ng/mL, at or about 600 ng/mL, at or about 700 ng/mL, at or about 800 ng/mL, at or about 900 ng/mL, at or about 1000 ng/mL, at or about 1200 ng/mL, at or about 1400 ng/mL or at or about 1600 ng/mL, at or about 1800 ng/mL or at or about 2000 ng/mL, or any value between any of the foregoing.

In some embodiments, recombinant IL-2 and recombinant IL-25 are added to the culture medium. In some embodiments, recombinant IL-2 is added at a concentration of 200 IU/mL to 1000 IU/mL (e.g. at or about 300 IU/mL) and recombinant IL-25 is added at a concentration of 100 ng/mL to 2000 ng/mL (e.g. between at or about 250 ng/mL and 1000 ng/mL, such as at or about 250 ng/mL, at or about 500 ng/mL or at or about 1000 ng/mL). In some embodiments, the expansion (e.g. second expansion) is carried out in the presence of recombinant IL-2 added at a concentration of 200 IU/mL to 1000 IU/mL (e.g. at or about 300 IU/mL) and recombinant IL-25 added at a concentration of between 100 ng/mL and 2000 ng/mL (e.g. between at or about 250 ng/mL and 1000 ng/mL, such as at or about 250 ng/mL, at or about 500 ng/mL or at or about 1000 ng/mL). In some embodiments, at least one other recombinant modulatory cytokine from IL-7, IL-21, IL-15, IL-23, IL-27 or IL-35 is added to the culture meduium.

In some embodiments, the recombinant IL-27 is added to the culture medium at a concentration of between at or about 0.1 ng/mL and at or about 2000 ng/mL, such as between at or about 0.1 ng/mL and at or about 1000 ng/mL, between at or about 0.1 ng/mL and at or about 500 ng/mL, between at or about 0.1 ng/mL and at or about 250 ng/mL, between at or about 0.1 ng/mL and at or about 100 ng/mL, between at or about 0.1 ng/mL and at or about 50 ng/mL, between at or about 0.1 ng/mL and at or about 10 ng/mL, between at or about 0.1 ng/mL and at or about 1 ng/mL, between at or about 1 ng/mL and at or about 1000 ng/mL, between at or about 1 ng/mL and at or about 500 ng/mL, between at or about 1 ng/mL and at or about 250 ng/mL, between at or about 1 ng/mL and at or about 100 ng/mL, between at or about 1 ng/mL and at or about 50 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 250 ng/mL, between at or about 10 ng/mL and at or about 100 ng/mL, between at or about 10 ng/mL and at or about 50 ng/mL, between at or about 50 ng/mL and at or about 1000 ng/mL, between at or about 50 ng/mL and at or about 500 ng/mL, between at or about 50 ng/mL and at or about 250 ng/mL, between at or about 50 ng/mL and at or about 100 ng/mL, between at or about 100 ng/mL and at or about 1000 ng/mL, between at or about 100 ng/mL and at or about 500 ng/mL, between at or about 100 ng/mL and at or about 250 ng/mL, between at or about 250 ng/mL and at or about 1000 ng/mL, between at or about 250 ng/mL and at or about 500 ng/mL, or between at or about 500 ng/mL and at or about 1000 ng/mL. In some embodiments, the concentration is between 400 ng/mL and 500 ng/mL.

In some embodiments, the recombinant IL-27 is added to the culture medium at a concentration of at or about 200 ng/mL, at or about 300 ng/mL, at or about 400 ng/mL, at or about 500 ng/mL, at or about 600 ng/mL, at or about 700 ng/mL, at or about 800 ng/mL, at or about 900 ng/mL, at or about 1000 ng/mL, at or about 1200 ng/mL, at or about 1400 ng/mL or at or about 1600 ng/mL, at or about 1800 ng/mL or at or about 2000 ng/mL, or any value between any of the foregoing.

In some embodiments, recombinant IL-2 and recombinant IL-27 are added to the culture medium. In some embodiments, recombinant IL-2 is added at a concentration of 200 IU/mL to 1000 IU/mL (e.g. at or about 300 IU/mL) and recombinant IL-27 is added at a concentration of 100 ng/mL to 2000 ng/mL (e.g. between at or about 250 ng/mL and 1000 ng/mL, such as at or about 250 ng/mL, at or about 500 ng/mL or at or about 1000 ng/mL). In some embodiments, the expansion (e.g. second expansion) is carried out in the presence of recombinant IL-2 added at a concentration of 200 IU/mL to 1000 IU/mL (e.g. at or about 300 IU/mL) and recombinant IL-27 added at a concentration of between 100 ng/mL and 2000 ng/mL (e.g. between at or about 250 ng/mL and 1000 ng/mL, such as at or about 250 ng/mL, at or about 500 ng/mL or at or about 1000 ng/mL). In some embodiments, at least one other recombinant modulatory cytokine from IL-7, IL-21, IL-15, IL-23, IL-25 or IL-35 is added to the culture meduium.

In some embodiments, the recombinant IL-35 is added to the culture medium at a concentration of between at or about 0.1 ng/mL and at or about 2000 ng/mL, such as between at or about 0.1 ng/mL and at or about 1000 ng/mL, between at or about 0.1 ng/mL and at or about 500 ng/mL, between at or about 0.1 ng/mL and at or about 250 ng/mL, between at or about 0.1 ng/mL and at or about 100 ng/mL, between at or about 0.1 ng/mL and at or about 50 ng/mL, between at or about 0.1 ng/mL and at or about 10 ng/mL, between at or about 0.1 ng/mL and at or about 1 ng/mL, between at or about 1 ng/mL and at or about 1000 ng/mL, between at or about 1 ng/mL and at or about 500 ng/mL, between at or about 1 ng/mL and at or about 250 ng/mL, between at or about 1 ng/mL and at or about 100 ng/mL, between at or about 1 ng/mL and at or about 50 ng/mL, between at or about 1 ng/mL and at or about 10 ng/mL, between at or about 10 ng/mL and at or about 1000 ng/mL, between at or about 10 ng/mL and at or about 500 ng/mL, between at or about 10 ng/mL and at or about 250 ng/mL, between at or about 10 ng/mL and at or about 100 ng/mL, between at or about 10 ng/mL and at or about 50 ng/mL, between at or about 50 ng/mL and at or about 1000 ng/mL, between at or about 50 ng/mL and at or about 500 ng/mL, between at or about 50 ng/mL and at or about 250 ng/mL, between at or about 50 ng/mL and at or about 100 ng/mL, between at or about 100 ng/mL and at or about 1000 ng/mL, between at or about 100 ng/mL and at or about 500 ng/mL, between at or about 100 ng/mL and at or about 250 ng/mL, between at or about 250 ng/mL and at or about 1000 ng/mL, between at or about 250 ng/mL and at or about 500 ng/mL, or between at or about 500 ng/mL and at or about 1000 ng/mL. In some embodiments, the concentration is between 400 ng/mL and 500 ng/mL.

In some embodiments, the recombinant IL-35 is added to the culture medium at a concentration of at or about 200 ng/mL, at or about 300 ng/mL, at or about 400 ng/mL, at or about 500 ng/mL, at or about 600 ng/mL, at or about 700 ng/mL, at or about 800 ng/mL, at or about 900 ng/mL, at or about 1000 ng/mL, at or about 1200 ng/mL, at or about 1400 ng/mL or at or about 1600 ng/mL, at or about 1800 ng/mL or at or about 2000 ng/mL, or any value between any of the foregoing.

In some embodiments, recombinant IL-2 and recombinant IL-35 are added to the culture medium. In some embodiments, recombinant IL-2 is added at a concentration of 200 IU/mL to 1000 IU/mL (e.g. at or about 300 IU/mL) and recombinant IL-35 is added at a concentration of 100 ng/mL to 2000 ng/mL (e.g. between at or about 250 ng/mL and 1000 ng/mL, such as at or about 250 ng/mL, at or about 500 ng/mL or at or about 1000 ng/mL). In some embodiments, the expansion (e.g. second expansion) is carried out in the presence of recombinant IL-2 added at a concentration of 200 IU/mL to 1000 IU/mL (e.g. at or about 300 IU/mL) and recombinant IL-35 added at a concentration of between 100 ng/mL and 2000 ng/mL (e.g. between at or about 250 ng/mL and 1000 ng/mL, such as at or about 250 ng/mL, at or about 500 ng/mL or at or about 1000 ng/mL). In some embodiments, at least one other recombinant modulatory cytokine from IL-7, IL-21, IL-15, IL-23, IL-25 or IL-27 is added to the culture meduium.

In provided embodiments, this expansion (e.g. second expansion) can occur in the presence of a T cell adjuvant, such as any described in Section II. In some aspects, the T cell adjuvant is a costimulatory agonist, such as a Tumor Necrosis Factor Super Family Receptor (TNFSR) agonists including but not limited to agonists of OX40 and 41BB. In some embodiments, the T cell adjuvant is an apoptosis inhibitor including but not limited to caspase inhibitors or inhibitors of the Fas/Fas ligand axis. These soluble agonists and apoptosis inhibitors can be present in the culture for up to the maximum culture time of the expansion step or a minimum of 24 hours.

The sorted or selected T cells can be expanded under the one or more stimulatory conditions in a culture vessel suitable for cell expansion. In some embodiments, the culture vessel is a gas permeable culture vessel, such as a G-Rex system (e.g. G-Rex 10, G-Rex 10M, G-Rex 100 M/100M-CS or G-Rex 500 M/500M-CS). In some embodiments the culture vessel is a microplate, flask, bar or other culture vessel suitable for expansion of cells in a closed system. In some embodiments, expansion can be carried out in a bioreactor. In some embodiments the composition of expanded T cells is removed from a closed system and placed in and/or connected to a bioreactor for expansion. The sorted or selected T cells can be expanded using a cell expansion system by transfer to the cell to gas permeable bags, such as in connection with a bioreactor (e.g. Xuri Cell Expansion System W25 (GE Healthcare)). In an embodiment, the cell expansion system includes a culture vessel, such as a bag, e.g. gas permeable cell bag, with a volume that is about 50 mL, about 100 mL, about 200 mL, about 300 mL, about 400 mL, about 500 mL, about 600 mL, about 700 mL, about 800 mL, about 900 mL, about 1 L, about 2 L, about 3 L, about 4 L, about 5 L, about 6 L, about 7 L, about 8 L, about 9 L, and about 10 L, or any value between any of the foregoing. In some embodiments, the process is automated or semi-automated. Examples of suitable bioreactors for the automated perfusion expansion include, but are not limited to, GE Xuri W25, GE Xuri W5, Sartorius BioSTAT RM 20 | 50, Finesse SmartRocker Bioreactor Systems, and Pall XRS Bioreactor Systems, or Miltenyi Prodigy. In some aspects, the expansion culture is carried out under static conditions. In some embodiments, the expansion culture is carried out under rocking conditions. The medium can be added in bolus or can be added on a perfusion schedule. In some embodiments, the bioreactor maintains the temperature at or near 37°C and CO2 levels at or near 5% with a steady air flow at, at about, or at least 0.01 L/min, 0.05 L/min, 0.1 L/min, 0.2 L/min, 0.3 L/min, 0.4 L/min, 0.5 L/min, 1.0 L/min, 1.5 L/min, or 2.0 L/min or greater than 2.0 L/min. In certain embodiments, at least a portion of the culturing is performed with perfusion, such as with a rate of 290 ml/day, 580 ml/day, and/or 1160 ml/day.

In some embodiments, the cells are seeded in an appropriate culture vessel (e.g. gas permeable bag) at a density of from 0.5 x 10⁶ cells/mL to 1.5 x 10⁶ cells/mL. In some embodiments, the density is at or about 0.5 x 10⁶ cells/mL, 0.75 x 10⁶ cells/mL, 1 x 10⁶ cells/mL, 1.25 x 10⁶ cells/mL or 1.5 x 10⁶ cells/mL, or any value between any of the foregoing.

In some aspects, cells are expanded in an automated closed expansion system that is perfusion enabled. Perfusions can continuously add media to the cells to ensure an optimal growth rate is achieved.

In some embodiments, expansion is carried out using a Xuri cell expansion system bioreactor. The cells can be seeded at 0.5-1.5 million cells per mL. The cells can be cultured under static or rocking conditions. The medium can be added in bolus or on a perfusion schedule. In embodiments, the bioreactor maintains the temperature at or near 37°C and CO2 levels at or near 5%. The volume of the culture can be maintained at approximately 0.5 L to 1.0 L. In some embodiments, the expansion is carried out for 7-14 days such as 7-10 days. In some aspects, expansion results in a 100 million to 50 billion cells after the expansion and/or in a fold-expansion of 10 to 1000-fold expansion.

In some embodiments, expansion is carried out using a Miltenyi Prodigy bioreactor. The cells can be seeded at 0.5-1.5 million cells per mL. The cells can be cultured under static or shaking conditions. The medium can be added in bolus or on a perfusion schedule. In embodiments, the bioreactor maintains the temperature at or near 37°C and CO2 levels at or near 5%. The volume of the culture can be maintained at approximately 70 mL to 400 mL. In some embodiments, the expansion is carried out for 7-14 days such as 7-10 days. In some aspects, expansion results in a 100 million to 3 billion cells after the expansion and/or in a 10 to 1000-fold expansion.

In some embodiments, expansion is carried out using a gas-permeable bag. The cells can be seeded at 0.5-1.5 million cells per mL. The cells can be cultured under static conditions. In embodiments, the bioreactor maintains the temperature at or near 37°C and CO2 levels at or near 5%. In such aspects, when the cell concentration exceeds 2.0 million cells per mL, medium can be added to bring the cell concentration to between 0.5 and 1.0 million cells per mL. If the volume reaches the maximum volume of the bag the cells would be added to a larger bag or multiple bags for culture under the same conditions. In some embodiments, the expansion is carried out for 7-14 days such as 7-10 days.

The expansion methods can be carried out under GMP conditions, including in a closed automated system and using serum free medium. In some embodiments, any one or more of the steps of the method can be carried out in a closed system or under GMP conditions. In certain embodiments, all process operations are performed in a GMP suite. In some embodiments, a closed system is used for carrying out one or more of the other processing steps of a method for manufacturing, generating or producing a cell therapy. In some embodiments, one or more or all of the processing steps, e.g., isolation, selection and/or enrichment, processing, culturing steps including incubation in connection with expansion of the cells, and formulation steps is carried out using a system, device, or apparatus in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some embodiments, the incubation with the T cell stimulatory agent(s) for expansion of tumor-reactive cells is carried out for at or about 1 day to 35 days, such as 1 day to 28 day. In some embodiments, the incubation is carried out until reaching a fold expansion of tumor reactive T cells or T cells from the input sample that is at least 10-fold, at least 20-fold, at least 50-fold, at least 100-fold, at least 200-fold, at least 400-fold, at least 500-fold, at least 600-fold, at least 700-fold, at least 800-fold, at least 900-fold, at least 1000-fold, at least 1250-fold, at least 1500-fold, at least 2000-fold, at least 2500-fold, at least 3000-fold, at least 3500-fold, at least 4000-fold, at least 4500-fold, at least 5000-fold or more.

In some embodiments, the incubation for expansion with the T cell stimulatory agent is for at or about 1 day, such as generally at or about 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, or 30 days, or any range of time between any of the foregoing. In some embodimetns, the incubation for expansion is for at or about 7 days to 28 days. In some embodiments, the incubation for expansion is for about 7 days to 21 days. In some embodiments, the insbuation for expansion is for about 7 days to 14 days.

In some embodiments, the incubation with the T cell stimulatory agent(s) for expansion of tumor-reactive cells is carried out for at or about 1 day, such as generally at or about 2 days, 3 days, 4 days, 5 days, 6 days, 7 days, 8 days, 9 days, 10 days, 11 days, 12 days, or any range of time between any of the foregoing. In some embodiments, the incubation with the T cell stimulatory agent(s) for expansion of tumor-reactive cells is carried out for 7-21 days, such as 7 days, 8 days, 9 days, 10 days, 11 days, 12 days, 13 days, 14, days, 15 days, 16 days, 17 days, 18 days, 19 days, 20 days or 21 days, or any value between any of the foregoing. In some embodiments, the incubation is carried out for 7-14 days. In some embodiments, the incubation is carried out for 7-10 days. In some embodiments, the incubation is for at or about 7 days. In some embodiments, the incubation is for at or about 8 days. In some embodiments, the incubation is for at or about 9 days. In some embodiments, the incubation is for at or about 10 days.

In some embodiments, the incubation with the T cell stimulatory agent(s) for expansion (e.g. after the selection of cells for the activation markers, such as PD-1, CD39 and/or TIGIT) is split into two expansion phases, e.g. a first expansion and a second expansion. In some embodiments, the total duration of the expansions can be any duration as described above. In some embodiments, a first expansion includes incubation with the T cell stimulatory agent(s) for 1-21 days, such as 1-14 days. In some embodiments, a first expansion includes incubation with the T cell stimulatory agent(s) for 7-21 days, such as 7 days, 8 days, 9 days, 10 days, 11 days, 12 days, 13 days, 14, days, 15 days, 16 days, 17 days, 18 days, 19 days, 20 days or 21 days, or any value between any of the foregoing. In some embodiments, the incubation for the first expansion is carried out for 7-14 days. In some embodiments, the incubation for the first expansion is carried out for 7-10 days. In some embodiments, the incubation is for at or about 7 days. In some embodiments, the incubation for the first expansion is for at or about 8 days. In some embodiments, the incubation for the first expansion is for at or about 9 days. In some embodiments, the incubation for the first expansion for at or about 10 days. In some embodiments, a second expansion includes incubation with the T cell stimulatory agent(s) for 1-21 days, such as 1-14 days. In some embodiments, a second expansion includes incubation with the T cell stimulatory agent(s) for 7-21 days, such as 7 days, 8 days, 9 days, 10 days, 11 days, 12 days, 13 days, 14, days, 15 days, 16 days, 17 days, 18 days, 19 days, 20 days or 21 days, or any value between any of the foregoing. In some embodiments, the incubation the second expansion is carried out for 7-14 days. In some embodiments, the incubation for the second expansion is carried out for 7-10 days. In some embodiments, the incubation for the second expansion is for at or about 7 days. In some embodiments, the incubation for the second expansion is for at or about 8 days. In some embodiments, the incubation for the second expansion is for at or about 9 days. In some embodiments, the incubation for the second expansion for at or about 10 days.

In some cases, media can be exchanged daily, every other day, every third day, every 5^{th} day or once a week during the time of the culture or incubation. In some embodiments, the stimulating agents (e.g. cytokines, anti-CD3) are replenished at each media exchange.

In some embodiments, the methods of culturing for expanding cells in accord with any of the provided methods is carried out until a threshold amount of cells, such as tumor-reactive cells or cells positive for one or more T cell activation marker, is obtained. In some embodiments, the method of culturing for expanding cells in accord with any of the provided method is carried out until up to 30 days from the time of enriching the lymphocytes. In some embodiments, the method of culturing for expanding cells in accord with any of the provided method is carried out until up to 20 days after the initiation of the first expansion. In some embodiments, the method of culturing for expanding cells in accord with any of the provided methods is carried out until up to 20 days after initiation of the co-culturing. In some of any of the provided embodiments, harvesting is carried out within 20 days after initiation of the culturing and/or the enriching of T cells comprising tumor-reactive cells. In some of any of the provided embodiments, the cells are harvested 7 to 20 days, 7 to 14 days, 7 to 10 days, 10 to 20 days, 10 to 14 days or 14 to 20 days after the initiation of the culturing. It is understood that reference to the number of days is with reference to days in which the cells are present in a culture and do not include time in which the cells from any one or more of the steps may be stored under conditions for cryopreservation.In some of any of the provided embodiments, the culturing is carried out until a threshold amount of cells is achieved that is between at or about 0.5 x 10⁸ and at or about 50 x 10⁹ total cells or total viable cells, between at or about 0.5 x 10⁸ and at or about 30 x 10⁹ total cells or total viable cells, between 0.5 x 10⁸ and at or about 12 x 10⁹ total cells or total viable cells, between at or about 0.5 x 10⁸ and at or about 60 x 10⁸ total cells or total viable cells, between at or about 0.5 x 10⁸ and at or about 15 x 10⁸ total cells or total viable cells, between at or about 0.5 x 10⁸ and at or about 8 x 10⁸ total cells or total viable cells, between at or about 0.5 x 10⁸ and at or about 3.5x 10⁸ total cells or total viable cells, between at or about 0.5 x 10⁸ and at or about 1 x 10⁸ total cells or total viable cells, between 1 x 10⁸ and at or about 50 x 10⁹ total cells or total viable cells, between at or about 1 x 10⁸ and at or about 30 x 10⁹ total cells or total viable cells, between 1 x 10⁸ and at or about 12 x 10⁹ total cells or total viable cells, between at or about 1 x 10⁸ and at or about 60 x 10⁸ total cells or total viable cells, between at or about 1 x 10⁸ and at or about 15 x 10⁸ total cells or total viable cells, between at or about 1 x 10⁸ and at or about 8 x 10⁸ total cells or total viable cells, between at or about 1 x 10⁸ and at or about 3.5x 10⁸ total cells or total viable cells, between at or about 3.5 x 10⁸ and at or about 50 x 10⁹ total cells or total viable cells, between at or about 3.5 x 10⁸ and at or about 30 x 10⁹ total cells or total viable cells, between at or about 3.5 x 10⁸ and at or about 12 x 10⁹ total cells or total viable cells, between at or about 3.5 x 10⁸ and at or about 60 x 10⁸ total cells or total viable cells, between at or about 3.5 x 10⁸ and at or about 15 x 10⁸ total cells or total viable cells, between at or about 3.5 x 10⁸ and at or about 8 x 10⁸ total cells or total viable cells, between at or about 8 x 10⁸ and at or about 50 x 10⁹ total cells or total viable cells, between at or about 8 x 10⁸ and at or about 30 x 10⁹ total cells or total viable cells, between at or about 8 x 10⁸ and at or about 12 x 10⁹ total cells or total viable cells, between at or about 8 x 10⁸ and at or about 60 x 10⁸ total cells or total viable cells, between at or about 8 x 10⁸ and at or about 15 x 10⁸ total cells or total viable cells, between at or about 15 x 10⁸ and at or about 50 x 10⁹ total cells or total viable cells, between at or about 15 x 10⁸ and at or about 30 x 10⁹ total cells or total viable cells, between at or about 15 x 10⁸ and at or about 12 x 10⁹ total cells or total viable cells, between at or about 15 x 10⁸ and at or about 60 x 10⁸ total cells or total viable cells, between at or about 60 x 10⁸ and at or about 50 x 10⁹ total cells or total viable cells, between at or about 60 x 10⁸ and at or about 30 x 10⁹ total cells or total viable cells, between at or about 60 x 10⁸ and at or about 12 x 10⁹ total cells or total viable cells, between at or about 12 x 10⁹ and at or about 50 x 10⁹ total cells or total viable cells, between at or about 12 x 10⁹ and at or about 30 x 10⁹ total cells or total viable cells, or between at or about 30 x 10⁹ and at or about 60 x 10⁹ total cells or total viable cells, each inclusive.

In some of any of the provided embodiments, the culturing is carried out until a threshold amount of cells is achieved of at least or greater than at or about 500 x 10⁶ tumor-reactive T cells or T cells, or viable cells thereof.

In some of any of the provided embodiments, the method results in a fold-expansion of T cells or in a fold-expansion of tumor reactive T cells that is at least at or about 2-fold, at least at or about 5-fold, at least at or about 10-fold, at least at or about 25-fold, at least at or about 50-fold, at least at or about 100-fold, at least at or about 250-fold, at least at or about 500-fold, at least at or about 1000-fold, or more. In some cases, the fold-expansion is at least at or about 2000-fold, 3000-fold, 4000-fold, 5000-fold or more.

Upon reaching a therapeutic dose after expansion the product can be concentrated and frozen in cryopreservation medium. Also provided herein are populations of T cells produced by methods described herein and pharmaceutical compositions thereof.

In some of any of the provided embodiments, the method further includes formulating the harvested cells with a cryoprotectant. In some embodiments, the cryoprotectant is selected from glycerol, propylene glycol, dimethyl sulfoxide (DMSO), or a combination thereof. In some embodiments, the cryoprotectant includes DMSO. In some embodiments, the cryoprotectant is DMSO.

In some embodiments, the cells are formulated with a cyropreservative solution that contains 1.0% to 30% DMSO solution, such as a 5% to 20% DMSO solution or a 5% to 10% DMSO solution. In some embodiments, the cryopreservation solution is or contains, for example, PBS containing 20% DMSO and 8% human serum albumin (HSA), or other suitable cell freezing media. In some embodiments, the cryopreservative solution is or contains, for example, at least or about 7.5% DMSO. In some embodiments, the processing steps can involve washing the harvested cells to replace the cells in a cryopreservative solution. In some embodiments, the cells are frozen, e.g., cryopreserved or cryoprotected, in media and/or solution with a final concentration of or of about 12.5%, 12.0%, 11.5%, 11.0%, 10.5%, 10.0%, 9.5%, 9. 0%, 8.5%, 8.0%, 7.5%, 7.0%, 6.5%, 6.0%, 5.5%, or 5.0% DMSO, or between 1% and 15%, between 6% and 12%, between 5% and 10%, or between 6% and 8% DMSO. In particular embodiments, the cells are frozen, e.g., cryopreserved or cryoprotected, in media and/or solution with a final concentration of or of about 5.0%, 4.5%, 4.0%, 3.5%, 3.0%, 2.5%, 2.0%, 1.5%, 1.25%, 1.0%, 0.75%, 0.5%, or 0.25% HSA, or between 0.1% and - 5%, between 0.25% and 4%, between 0.5% and 2%, or between 1% and 2% HSA.

### II. T CELL MODULATORY AGENTS OR ADJUVANTS

In some embodiments, the one or more of the steps of provided methods that involve culturing a population of T cells containing tumor reactive T cells ex vivo, including one or more steps of the methods as described in Section I above, can include culture or incubation of cells with additional modulatory agents, such as apoptosis inhibitors or T cell adjuvants, including pharmaceutical agonists. The addition of one or more modulatory agents or T cell adjuvants to the manufacturing of T cells can increase the functionality of the T cells ex vivo and for use in in-vivo methods of treatment. In particular embodiments, such methods can enrich for expansion of reactive T cells compared to non-reactive and promote their survival and growth in culture ex vivo. It is contemplated that the provided methods can increase expansion to a therapeutic dose to a much greater extent than existing methods and/or increase functionality of the T cell therapy for therapeutic effect.

In some embodiments, the methods for incubation or culturing in one or more steps of the method can additionally include (1) the use of one or more modulatory agents, for example additional T cell adjuvants, such as prior to or concurrently with standard T cell stimulatory agent(s) such as anti-CD3 and/or recombinant cytokines, e.g. IL-2.

In particular embodiments, the modulatory agent or T cell adjuvant, such as a costimulatory agonist or an apoptosis inhibitor, is a soluble protein, such as a protein that is not bound or attached to a solid surface (e.g. a bead or other solid support). The modulatory agent or T cell adjuvants can include small molecules, peptides or proteins. Among such T cell adjuvants are soluble ligands, antibody or antigen-binding fragments or other binding agents. In some embodiments, a costimulatory agonist can include a molecule that specifically binds to a costimulatory molecule, such as 4-1BB or OX40, to induce or stimulate a costimulatory signal in the cells. In some embodiments, an apoptosis inhibitor can include a molecule that specifically binds to a receptor that mediates or is involved in inducing apoptosis in a cell. In some embodiments, these molecules can be easily removed during the manufacturing process, such as by washing the cells in connection with cell manufacturing or prior to final formulation of the cells for administration.

In aspects of the provided methods, the one or more modulatory agent or T cell adjuvant can be included during one or more or all of the steps of the provided methods. In some embodiments, the one or more modulatory agent or T cell adjuvant is included during the first or initial expansion of T cells from the input sample. In some embodiments, the one or more modulatory agent or T cell adjuvant is including during the second or final expansion of T cells, such as which may be carried out after enrichment of tumor-reactive T cells from the co-culture. In some embodiments, the one or more modulatory agent or T cell adjuvant is included during both the first expansion and the second expansion. In some cases, the one or more modulatory agent or T cell adjuvant is including during the co-culture of T cells with the APCs/peptide neoepitopes.

In embodiments of any of the provided methods, the incubation with each of the at least one modulatory agent or T cell adjuvant, such as one or more costimulatory agonist and/or apoptosis inhibitor, is independently continued during the entire course of the culturing or during a portion thereof. In some embodiments, the incubation with each of the at least one modulatory agent or T cell adjuvant is for no more than 14 days, no more than 12 days, no more than 10 days, no more than 7 days, no more than 5 days, no more than 3 days or no more than 2 days. In some embodiments, the incubation with each of the at least one modulatory agent or T cell adjuvant is independently for 12 hours to 96 hours, such as 24 hours to 48 hours, and generally is at or about 48 hours.
γ mediumwhich themediumselectiveelicits

### A. Immunosuppressive Blocking Agents

In provided embodiments, the methods include *ex vivo* incubation or culture of cells containing a population of T cells with one or more blocking agents that is able to reduce or decrease activity of an immunosuppressive factor, e.g. a cytokine, growth factor or enzyme, such as one or more of IL-27, IL-35, TGFbeta or IDO, under conditions to modulate activity of T cells.

In some embodiments, a population of T cells is incubated or cultured, such as during the first or second expansion, in the presence of an agent that blocks or reduces the activity of IL-27. In some embodiments, the culturing or incubation, such as during the first and/or second expansion, is carried out in the presence of a blocking agent that blocks or reduces the activity of IL-35. In some embodiments, the culturing or incubation, such as during the first and/or second expansion, is carried out in the presence of a blocking agent that blocks or reduces the activity of TGFbeta. In some embodiments, the culturing or incubation, such as during the first and/or second expansion, is carried out in the presence of a blocking agent that blocks or reduces the activity of IDO. In some embodiments, a combination of any of the above approaches can be used.

The immunosuppressive blocking agent or antagonist can be any molecule that binds to the cytokine or growth factor and inhibits or reduces its ability to bind to its receptor and/or mediate signaling via its receptor.

In some embodiments, the immunosuppressive blocking agent can be a soluble form of the natural receptor of the cytokine or growth factor. In some cases, an extracellular ligand binding portion of a cognate receptor can be generated as a fusion protein by linkage with an immunoglobulin Fc to generate a soluble antagonist reagent. In some embodiments, the Fc is an IgG1 Fc or is a variant thereof with reduced Fc effector function, such as reduced ability to bind FcγR, C1q and/or mediate antibody-dependent cell cytotoxicity (ADCC). Exemplary mutations in an immunoglobulin IgG1 Fc to reduce effector function include, but are not limited to, L235E, G236A, N297A, L234A/L235A, E233P/L234V/L235A, C220S/C226S/C229S/P238S, C226S/C229S/E233P/L234V/L235A, M252Y/S254T/T256E, K326W.

In some embodiments, the immunosuppressive blocking agent or antagonist can be an antibody or antigen-binding fragment that binds to the cytokine or growth factor. For example, binding to the cytokine or growth factor can inhibit or reduce the ability of the respective cytokine or growth factor to binds to its respective cognate receptor.

In some embodiments, the immunosuppressive blocking agents reduces or decreases binding of the cytokine or growth factor to its cognate receptor or a subunit thereof. In some embodiments, binding is reduced by greater than or greater than about 50%, 60%, 70%, 80%, 90% or more. In some embodiments, the immunosuppressive blocking agent reduces or decreases signaling by the cognate receptor of an immunosuppressive cytokine or growth factor, such as reduces or decreases signaling y greater than or greater than about 50%, 60%, 70%, 80%, 90% or more.

In some embodiments, an immunosuppressive blocking agent that reduces, decreases or inhibits activity of IL-27 is present in the cell culture medium. IL-27 is a heterodimer containing the p28 subunit and the Epstein-Barr virus-induced gene 3 (EBI3; also known as IL-27beta). IL-27 is a cytokine that binds to the IL-27 receptor (IL-27R), which is composed of two subunits, IL-27Ralpha and gp130 (also known as IL-27beta). Binding of IL-27 to the IL-27 receptor induces JAK-STAT and p38 MAPK signaling. IL-27 has both regulatory and pro-inflammatory functions. IL-27 has been shown to upregulate PD-L1 and IDO in tumor cells, which, in some cases, leads to a strongly immunosuppressive environment. This activity could lead to enhanced suppression and exhaustion of TILs when still in the presence of solid tumor.

In some embodiments, the immunosuppressive blocking agent is a soluble form of a subunit of the IL-27 receptor. In some embodiments, the immunosuppressive blocking agent is a soluble form of the IL-27Ralpha. For example, a blocking agent can be a IL-27Ra Fc fusion protein. In some embodiments, a IL-27 blocking agent can include residues Gly34-Lys516 of the human IL-27R alpha (e.g. UniProt Accession No. Q6UWB1) linked to an Fc of human IgG1 (e.g. residues Pro100-Lys330 of IgG1). IL-27Ra Fc fusion protein blocking agents for use in the provided methods are known and/or are commercially available, see e.g. Catalog No. 1479-TC-050 from R&D Systems. In some embodiments, the blocking agent is a natural soluble form of IL-27Rα (sIL-27Rα), see e.g. Dietrich et al. J Immunol. 192:5382-5389. In some embodiments, the immunosuppressive blocking agent is a soluble form of gp130. For example, a blocking agent can be a gp130 Fc fusion protein. In some embodiments, a IL-27 blocking agent can include residues Glu23-Ile618 of the human gp130 (e.g. UniProt Accession No. P40189) linked to an Fc of human IgG1 (e.g. residues Pro100-Lys330 of IgG1). Gp130 Fc fusion protein blocking agents for use in the provided methods are known and/or are commercially available, see e.g. Catalog No. 671-GP-100 from R&D Systems.

In some embodiments, the immunosuppressive blocking agent is a monoclonal antibody against IL-27 that blocks the ability of IL-27 to bind to the IL-27R or a subunit thereof. Various monoclonal antibodies are known and available. In some embodiments, that antibody is directed against the IL-27beta (IL-27b) chain of the cytokine, which may also act to block the activity of IL-35 due to the shared subunit of the respective cytokines. Various monoclonal antibody against IL-27b are known. Exemplary antibodies include, but are not limited to, antibody MAB6456 (R&D Systems) or clone V1.4H6.25.

In some embodiments, the immunosuppressive blocking agent is a monoclonal antibody against the IL-27R or a subunit thereof.

A IL-27 blocking agent can be included in cell culture media during various stages of the provided process. In some cases, an IL-27 blocking agent can be included in the initial T cell expansion (first expansion), such as during TIL isolation and expansion from solid tumor, which can prevent the creation of an immunosuppressive environment and/or prevent the induction of regulatory T cells. In some cases, an IL-27 blocking agent can be included in cultures to expand selected tumor-reactive T cells during an incubation for expansion (e.g. second expansion), such as described in Section I.D. For example, IL-27 blockade in culture after expansion of TIL and the isolation of neo-antigen reactive T cells could provide benefits to tumor reactive T cells or TIL. IL-27 signaling could promote a suppressive, regulatory phenotype that would prevent potent cytolytic activity of neo-antigen specific TIL. The use of IL-27 blocking agents in the provided processes could avoid any immunosuppressive stimulation while promoting activity of tumor reactive T cells or TIL.

In some embodiments, an immunosuppressive blocking agent that reduces, decreases or inhibits activity of IL-35 is present in the cell culture medium. IL-35 is a heterodimer that is composed of the Epstein-Barr virus induced gene 3 (EBI3, also called IL-17beta) and the p35 subunit (shared with IL-12). IL-35 binds to the IL-35 receptor which is composed of the IL-12Rβ2 and gp130 (also known as IL-27beta) chains. IL-35 is an immunosuppressive cytokine in which binding to its receptor signals through STAT1/STAT4 to induce TGFβ and IL-35 production. IL-35 suppresses anti-tumor T cells and promotes regulatory T cell responses and proliferation of regulatory T cells. Increased IL-35 levels have been positively correlated with tumor size and negatively correlated with progression-free survival. Blockade of IL-35 production and/or signaling have shown beneficial results in cancer as they reduce numbers of regulatory T cells and limit tumor growth. Blockade of IL-35 has also prevented the exhaustion of tumor-specific T cell subsets.

In some embodiments, the immunosuppressive blocking agent is a monoclonal antibody against IL-35 that blocks the ability of IL-35 to bind to the IL-35R or a subunit thereof. Various monoclonal antibodies are known and available. In certain embodiments, the antibody or antigen-binding fragment does not bind to or recognize the p35 subunit of IL-35, since this is shared with IL-12. In particular embodiments, the antibody is directed against the IL-27beta (EBI3) subunit. Various monoclonal antibody against IL-27b are known. An exemplary antibody is anti-EBI3 antibody/IL-35 clone V1.4H6.25 or MAB6456.

In some embodiments, the immunosuppressive blocking agent is a monoclonal antibody against the IL-35R or a subunit thereof.

An IL-35 blocking agent can be included in cell culture media during various stages of the provided process. In some cases, an IL-35 blocking agent can be included in the initial T cell expansion (first expansion), such as during TIL isolation and expansion from solid tumor, which can prevent immunosuppressive signaling in the tumor microenvironment, thereby leading to increased TIL recovery and proliferation. In such examples, the blocking agent, e.g. antibody, can also prevent the outgrowth of regulatory T cells and diminish their presence in the isolated TIL cultures. In some cases, an IL-35 blocking agent can be included in cultures to expand selected tumor-reactive T cells during an incubation for expansion (e.g. second expansion), such as described in Section I.D.

In some embodiments, an immunosuppressive blocking agent that reduces, decreases or inhibits activity of TGFbeta (TGFβ) is present in the cell culture medium. TGFβ is produced by regulatory T cells and is a potent inhibitor of effector T cell function. TGFβ is also produced by epithelial or endothelial cells and contribute to the strong immunosuppressive tumor microenvironment. In the context of fully developed tumors, the upregulation of TGFβ can lead to the downregulation of cytotoxic function and increase exhaustion of TIL. Overall, high levels of TGFβ have been shown to inhibit anti-tumor T cell immunity and promote tumor survival.

In some embodiments, the immunosuppressive blocking agent is a monoclonal antibody against TGFβ that blocks the ability of TGFβ to bind to its receptor. In some embodiments, the antibody is fresolimumab (GC1008) or an antigen-binding fragment thereof. Fresolimumab is an antibody that binds to and inhibits all isoforms of TGF-β. Other immunosuppressive blocking agents include, but are not limited to, small molecule compounds that block transcription of the *TGFβ1* gene, such as pyrrole-imidazole polyamide drugs; antisense RNAs that target *TGFβ1* or *TGFβ2* mRNAs for degradation (e.g. ISTH0036 or ISTH0047); antibodies against TGFβ ligands (e.g. fresolimumab described above; also XPA681, XPA089, LY238770) or receptors (e.g. LY3022859); or small molecule ATP-mimetic TβRI kinase inhibitors (e.g. galunisertib or TEW-7197), see e.g. Akhurst Cold Spring Harb Perspect Biol 2017,9:a022301.

A TGFβ blocking agent can be included in cell culture media during various stages of the provided process. In some cases, a TGFβ blocking agent can be included in the initial T cell expansion (first expansion), such as during TIL isolation and expansion from solid tumor, which can reduce immunosuppressive signaling. For example, as solid tumors from patients with high tumor burden will have high levels of TGFβ, the potential immunosuppressive signaling could prevent TIL recovery and expansion. This could also create a positive feedback loop to increase the outgrowth of regulatory T cells and boost additional TGFβ production. Blockade of this signaling with blocking agents, such as anti-TGFβ antibodies, can enhance recovery of activated TIL (i.e. not exhausted), promote TIL expansion, and prevent increases of regulatory T cells. In some cases, a TGFβ blocking agent can be included in cultures to expand selected tumor-reactive T cells during an incubation for expansion (e.g. second expansion), such as described in Section I.D.

In some embodiments, an immunosuppressive blocking agent that reduces, decreases or inhibits activity of Indoleamine-pyrrole 2,3-dioxygenase (IDO) is present in the cell culture medium. IDO is a heme-containing enzyme that in humans is encoded by the IDOl gene. It is one of three enzymes that catalyze the first and rate-limiting step in the kynurenine pathway, the O2-dependent oxidation of L-tryptophan to N-formylkynurenine, the others being IDO2 and tryptophan 2,3-dioxygenase (TDO). IDO has been implicated in immune modulation through its ability to limit T-cell function and engage mechanisms of immune tolerance. Emerging evidence suggests that IDO becomes activated during tumor development, helping malignant cells escape eradication by the immune system. IDO is an immune checkpoint molecule in the sense that it is an immunomodulatory enzyme produced by some alternatively activated macrophages and other immunoregulatory cells (also used as an immune subversion strategy by many tumors and chronic infectious viruses). IDO is known to suppress T and NK cells, generate and activate Tregs and myeloid-derived suppressor cells, and promote the growth of new blood cells to feed the tumor (angiogenesis).

Various inhibitors of IDO are known. IDO inhibitors are chemical inhibitors of IDOl enzyme activity, thus preventing tryptophan depletion and restoring the proliferative capacity of T cells. An example of an inhibitor is PF-06840003 (available from MedKoo Biosciences, Inc.). Other IDO inhibitors include, but are not limited to, Epacadostat (INCB24360), INCB23843, navoximod (GDC-0919), BMS-986205, imatinib, or 1-methyl-tryptophan.

An IDO inhibitor can be used as a blocking agent in cell culture media during various stages of the provided process. In some cases, an IDO inhibitor can be included in the initial T cell expansion (first expansion), such as during TIL isolation and outgrowth or expansion from solid tumor, which can prevent immunoregulatory cell function and regulatory T cell outgrowth. For example, as antigen presenting cells and endothelial cells present in the tumor microenvironment produce IDO as a mechanism of immunosuppression, the use of inhibitors could counteract this effect and lead to enhanced neo-antigen reactive TIL activation and proliferation in initial TIL expansion experiments. In some cases, an IL-35 blocking agent can be included in cultures to expand selected tumor-reactive T cells during an incubation for expansion (e.g. second expansion), such as described in Section I.D.

In embodiments of any of the provided methods, the one or more immunosuppressive blocking agents is added to the cell culture medium during the incubation. In some embodiments, a immunosuppressive blocking agent is added at a concentration ranging between at or about 0.1 µg/mL to at or about 100 µg/mL, at or about 0.1 µg/mL and at or about 50 µg/mL, at or about 0.1 µg/mL and at or about 25 µg/mL, at or about 0.1 µg/mL and at or about 10 µg/mL, at or about 0.1 µg/mL and at or about 5 µg/mL, at or about 0.1 µg/mL and at or about 1 µg/mL, at or about 0.1 µg/mL and at or about 0.5 µg/mL, 0.5 µg/mL to at or about 100 µg/mL, at or about 0.5 µg/mL and at or about 50 µg/mL, at or about 0.5 µg/mL and at or about 25 µg/mL, at or about 0.5 µg/mL and at or about 10 µg/mL, at or about 0.5 µg/mL and at or about 5 µg/mL, at or about 0.5 µg/mL and at or about 1 µg/mL, 1 µg/mL to at or about 100 µg/mL, at or about 1 µg/mL and at or about 50 µg/mL, at or about 1 µg/mL and at or about 25 µg/mL, at or about 1 µg/mL and at or about 10 µg/mL, at or about 1 µg/mL and at or about 5 µg/mL, at or about 5 µg/mL to at or about 100 µg/mL, at or about 5 µg/mL and at or about 50 µg/mL, at or about 5 µg/mL and at or about 25 µg/mL, at or about 5 µg/mL and at or about 10 µg/mL, at or about 10 µg/mL to at or about 100 µg/mL, at or about 10 µg/mL and at or about 50 µg/mL, at or about 10 µg/mL and at or about 25 µg/mL, at or about 25 µg/mL to at or about 100 µg/mL, at or about 25 µg/mL and at or about 50 µg/mL or at or about 50 µg/mL and at or about 100 µg/mL, each inclusive.

In embodiments of any of the provided methods, the one or more immunosuppressive blocking agents is added to the cell culture medium during the incubation. In some embodiments, a immunosuppressive blocking agent is added at a concentration ranging between at or about 0.001 µM and at or about 10 µM, at or about 0.001 µM and at or about 5 µM, between at or about 0.001 µM and at or about 1 µM, between at or about 0.001 µM and at or about 0.5 µM, between at or about 0.001 µM and at or about 0.1 µM, between at or about 0.001 µM and at or about 0.05 µM, between at or about 0.001 µM and at or about 0.01 µM, between at or about 0.001 µM and at or about 0.005 µM, between at or about 0.005 µM and at or about 10 µM, at or about 0.005 µM and at or about 5 µM, between at or about 0.005 µM and at or about 1 µM, between at or about 0.005 µM and at or about 0.5 µM, between at or about 0.005 µM and at or about 0.1 µM, between at or about 0.005 µM and at or about 0.05 µM, between at or about 0.005 µM and at or about 0.01 µM, between at or about 0.01 µM and at or about 10 µM, at or about 0.01 µM and at or about 5 µM, between at or about 0.01 µM and at or about 1 µM, between at or about 0.01 µM and at or about 0.5 µM, between at or about 0.01 µM and at or about 0.1 µM, between at or about 0.01 µM and at or about 0.05 µM, between at or about 0.05 µM and at or about 10 µM, at or about 0.05 µM and at or about 5 µM, between at or about 0.05 µM and at or about 1 µM, between at or about 0.05 µM and at or about 0.5 µM, between at or about 0.05 µM and at or about 0.1 µM, between at or about 0.1 µM and at or about 10 µM, at or about 0.1 µM and at or about 5 µM, between at or about 0.1 µM and at or about 1 µM, between at or about 0.1 µM and at or about 0.5 µM, between at or about 0.5 µM and at or about 10 µM, at or about 0.5 µM and at or about 5 µM, between at or about 0.5 µM and at or about 1 µM, between at or about 1 µM and at or about 10 µM, at or about 1 µM and at or about 5 µM, or between at or about 5 µM and at or about 10 µM. In some embodiments, a immunosuppressive blocking agent is added at a concentration that is at or about 0.001 µM, at or about 0.005 µM, at or about 0.01 µM, at or about 0.05 µM, at or about 0.1 µM, at or about 0.5 µM, at or about 1 µM, at or about 2 µM, at or about 3 µM, at or about 4 µM, at or about 5 µM, at or about 6 µM, at or about 7 µM, at or about 8 µM, at or about 9 µM or at or about 10 µM, or any value between any of the foregoing.

In some embodiments, subsequent to or concurrently with incubation with the one or more immunosuppressive blocking agent, the population of T cells also is contacted with a T cell stimulatory agent(s), such as an anti-CD3 or anti-CD28 stimulatory agent and/or a recombinant T cell stimulatory cytokine, such as IL-2, IL-7, IL-21 and/or IL-15, under conditions to induce or mediate proliferation of T cells in the population. In some embodiments, the T cell stimulatory agent(s) includes a T cell stimulatory cytokine from IL-2, IL-7, IL-21 and/or IL-15. In particular embodiments, the T cell stimulatory agent(s) at least includes recombinant IL-2. In some such aspects, the inclusion of an immunosuppressive blocking agent improves ex vivo recovery and/or expansion of potential tumor reactive T cells of interest, such as tumor infiltrating lymphocytes (TILs), such as following their isolation and stimulation from a sample from a subject and/or during enrichment and expansion of the tumor reactive T cells during culture.

### B. T cell Stimulatory Agonists

In provided embodiments, one or more steps of the provided methods include *ex vivo* incubation of cells with a costimulatory agonist under conditions to stimulate or activate a costimulatory receptor expressed by one or more of the T cells in the sample. In particular embodiments, the costimulatory agonist is a 4-1BB agonist. In other particular embodiments, the costimulatory agonist is an OX40 agonist. In some embodiments, subsequent to or concurrently with incubation with the costimulatory agent, the population of T cells also is contacted with a T cell stimulatory agent(s), such as an anti-CD3 and/or more recombinant cytokines from recombinant IL-2, IL-7, IL-15 and/or IL-21, under conditions to induce or mediate proliferation of T cells in the population. In some such aspects, the costimulatory agonist, such as a 4-1BB agonist or an OX40 agonist, provides an initial stimulation to enhance or boost the proliferative capacity and/or functional activity of T cells in the population.

In aspects of any of the provided methods, a population of T cells is incubated in the presence of one or more costimulatory agonist. In particular embodiments, the costimulatory agonist is a molecule that specifically binds to a costimulatory molecule on the surface of T cells to stimulate one or more intracellular signal in the cell and/or to stimulate one or more functional or biological activity of the T cell. In some embodiments, the agonist promotes the survival and activity of the T cell. In some embodiments, the costimulatory molecule is a member of the tumor necrosis factor superfamily of receptors (TNFSR). Exemplary costimulatory molecules include, but are not limited to, 4-1BB, OX40, GITR and CD27. In some embodiments, the costimulatory agonist is a 4-1BB agonist, an OX40 agonist, a GITR agonist or a CD27 agonist.

In some embodiments, the costimulatory agonist is or comprises an antibody or antigen-binding fragment that specifically binds to the costimulatory receptor.

In some embodiments, the costimulatory agonist is or comprises an extracellular binding domain, or a specific binding portion thereof, of a ligand of the costimulatory receptor. In some cases, the extracellular binding domain, or a specific binding fragment thereof, is provided as fusion protein with another polypeptide, such as to increase binding avidity of the agonist. For example, in some cases the polypeptide is a multimerization domain that can promote dimerization, trimerization, tetramerization or pentamerization of the molecule. In particular embodiments, the fusion protein is a dimer. In some embodiments, the multimerization domain includes any sequence of amino acids that can interact with a complementary multimerization domain to form a stable protein-protein interaction to produce a multimer of the polypeptide molecule with another polypeptide molecule. For example, a multimerization domain can be a molecule that is able to form disulfide bonds with a complementary molecule. Exemplary multimerization domains include immunoglobulin sequences or portions thereof, leucine zippers, hydrophobic regions, hydrophilic regions, and compatible protein-protein interaction domains. The multimerization domain, for example, can be an immunoglobulin constant region or domain, such as, for example, the Fc domain or portions thereof from IgG, including IgG1, IgG2, IgG3 or IgG4 subtypes, IgA, IgE, IgD and IgM and modified forms thereof. In some embodiments, the costimulatory agonist is an Fc fusion protein.

In some embodiments, the costimulatory agonist is an OX40 (CD134) agonist. OX40, a cell surface glycoprotein and member of the tumor necrosis factor receptor family (TNFRSF), is expressed on T-lymphocytes and provides a co-stimulatory signal for the proliferation and survival of activated T-cells. OX40 generally is not constitutively expressed on resting naive T cells, unlike CD28. OX40 is a secondary co-stimulatory immune checkpoint molecule, which, in some aspects, is expressed after 24 to 72 hours following activation; its ligand, OX40L, is also not expressed on resting antigen presenting cells, but is following their activation. Expression of OX40 is dependent on full activation of the T cell; in some cases, such as without CD28 stimulation, expression of OX40 is delayed and its expression is lower. OX40 can be expressed on T cells in the body (co-culture with tumor), after activation (e.g. with an anti-CD3, such asOKT3/anti-CD28) or after an ex vivo co-culture of APC induced to present a tumor antigen target. Binding of OX40 by OX40L triggers an activation of the OX40 pathway. In some embodiments, activation of this pathway leads to upregulation of other pathways leading to increased activation, survival, memory response, and reduction of immune suppressive activity.

In some embodiments, the OX40 agonist may be an antibody or antigen-binding fragment or a fusion protein capable of binding to human or mammalian OX40. In some embodiments, an OX40 agonist binds to human OX40, such as human OX40 expressed on the surface of a T cell. In some embodiments the OX40 agonist specifically binds to OX40 and abrogates antibody-dependent cellular toxicity (ADCC), for example NK cell cytotoxicity. In some embodiments, the OX40 agonist abrogates antibody-dependent cell phagocytosis (ADCP). In some embodiments, the OX40 agonist abrogates complement-dependent cytotoxicity (CDC). In some aspects, when an OX40 agonist binds to the OX40 protein receptor, it triggers a co-stimulatory signal that is associated with increased production of T cells and inflammatory cytokines. OX40 agonists for use in the provided methods include any known to a skilled artisan.

In some embodiments, an OX40 agonist is a fusion protein. OX40 fusion proteins include those comprising an Fc domain fused to a portion of OX40L, see e.g. Sadun et al., (2009) J. Immunother., 182:1481-89. In some embodiments, a multimeric OX40 agonist, such as a trimeric or hexameric OX40 agonist (with three of six ligand binding domains) may be used. Trimeric (trivalent) or hexameric (or hexavalent) or greater fusion proteins containing three TNFRSF binding domains and as a fusion with an Fc are known and can be used, see e.g. Gieffers et al. (2013) Cancer Therapeutics, 12:2735-47.

In some embodiments, the OX40 agonist is a fusion protein in which one or more domains of OX40L is covalently linked to one or more additional protein domains. Exemplary OX40L fusion proteins that can be used as OX40 agonists are described in U.S. Patent Nos. 6,312,700; 7,622,444, International Patent Application Publication Nos. WO2011109789; and WO2010105068. In some embodiments, the OX40 agonist includes an OX40L fusion polypeptide that self- assembles into a multimeric (e.g., trimeric or hexameric) OX40L fusion protein. Such fusion proteins are described, e.g., in Morris et al. (2007) Mol Immunol. 44(12): 3112-3121, U.S. Patent No. 7,959,925. A specific fusion protein that can be used according to some embodiments provided herein is MEDI6383 (produced by AZY/Medlmmune), a human OX40 ligand fusion protein, see e.g. U.S. Patent No. 6,312,700.

In some embodiments, an OX40 agonist is an antibody or antigen-binding fragment that specifically binds OX40. Exemplary OX40 agonists for use in the provided methods include, but are not limited to, tavolixizumab (also known as MEDI0562 or MEDI-0562), 11D4 (see U.S. Patent Nos. 7,960,515; 8,236,930; 9,028,824), 18D8 (see e.g. U.S. Patent Nos. 7,960,515; 8,236,930; 9,028,824); Hu119-122 (see e.g. U.S. Patent Nos. 9,006,399 and 9,163,085, and International Patent Publication No. WO2012/027328); Hu106-222 (see e.g. U.S. Patent Nos. 9,006,399 and 9,163,085, and International Patent Publication No. WO2012/027328); MEDI6469 (also known as 9B12; see e.g. Weinberg et al. (2006) J. Immunother., 29:575-585); pogalizumab (also known as MOXR0916 and RG7888; Genentech, Inc.); GSK3174998 (GlaxoSmithKline), or PF-04518600 (PF-8600; Hamid et al. (2016) Journal of Clinical Immunology, 34:3079); BMS 986178; or an antigen-binding fragment of any of the foregoing. An OX40 agonist also includes any binding molecule, such as any antibody or antigen-binding fragment, that contains six CDRs as contained in tavolizizumab, 11D4, 18D8, Hu119-122 , Hu106-22, MED16469, pogalizumab, GSK3174998, PF-04518600 or BMS 986178.

In some embodiments, the OX40 agonist is an OX40 agonist set forth in any of International Patent Application Publication Nos. WO 95/12673, WO 95/21925, WO 2006/121810, WO 2012/027328, WO 2013/028231, WO 2013/038191, and WO 2014/148895; European Patent Application EP 0672141; U.S. Patent Application Publication Nos. US 2010/136030, US 2014/377284, US 2015/190506, and US 2015/132288 (including clones 20E5 and 12H3); and U.S. Patent Nos. 7,504,101, 7,550,140, 7,622,444, 7,696,175, 7,960,515, 7,961,515, 8,133,983, 9,006,399, and 9,163,085. An OX40 agonist also may include commercially available antibodies such as L106 BD (Pharmingen Product #340420); ACT35 (Santa Cruz Biotechnology, Catalog #20073); or anti-mCD134/mOX40 (clone OX86), commercially available from InVivoMAb, BioXcell Inc, West Lebanon, NH.

Other OX40 agonists that can be used according to any of the provided embodiments include nucleotides, expression vectors and peptides, such as disclosed for example in Linch et al. (2015) Front Oncol. 5: 34, U.S. Patent No. 6,312,700 and U.S. Application Publication No. 20140271677.

In some embodiments, the costimulatory agonist is a 4-1BB (CD137) agonist. In some embodiments, the 4-1BB agonist may be an antibody or antigen-binding fragment or a fusion protein capable of binding to human or mammalian 4-1BB. In some embodiments, a 4-1BB agonist binds to human 4-1BB, such as human 4-1BB expressed on the surface of a T cell. 4-1BB (CD137, tumor necrosis factor receptor superfamily 9) is an inducible costimulatory receptor expressed on activated T and natural killer (NK) cells. 4-1BB ligation on T cells triggers a signaling cascade that results in upregulation of anti-apoptotic molecules, cytokine secretion, and enhanced effector function. In dysfunctional T cells that have a decreased cytotoxic capacity, 4-1BB ligation demonstrates a potent ability to restore effector functions. On NK cells, 4-1BB signaling can increase antibody-dependent cell-mediated cytotoxicity. Agonistic monoclonal antibodies targeting 4-1BB have been developed to harness 4-1BB signaling for cancer immunotherapy. Preclinical results in a variety of induced and spontaneous tumor models suggest that targeting 4-1BB with agonist antibodies can lead to tumor clearance and durable antitumor immunity.

In some embodiments the 4-1BB agonist binds specifically to 4-1BB in a manner sufficient to reduce toxicity. In some embodiments, the 4-1BB agonist is an agonistic 4-lBB monoclonal antibody or fusion protein that abrogates antibody-dependent cellular toxicity (ADCC), for example NK cell cytotoxicity. In some embodiments, the 4-lBB agonist is an agonistic 4-1BB monoclonal antibody or fusion protein that abrogates antibody-dependent cell phagocytosis (ADCP). In some embodiments, the 4-lBB agonist is an agonistic 4-lBB monoclonal antibody or fusion protein that abrogates complement-dependent cytotoxicity (CDC).

In some embodiments, a 4-1BB agonist is a fusion protein. 4-1BB fusion proteins include those comprising an Fc domain fused to 4-1BBL. In some embodiments, the fusion protein is a dimeric (divalent), trimeric (trivalent) or hexameric (hexavalent) or greater fusion comprising two or more, such as three, four or more, domains of 4-1BBL for binding 4-1BB fused to an Fc.

In an embodiment, the 4- 1BB agonist is a 4-1BB agonistic fusion protein described in International Patent Application Publication Nos. WO 2008/025516 Al, WO 2009/007120 Al, WO 2010/003766 Al, WO 2010/010051 Al, and WO 2010/078966 Al; U.S. Patent Application Publication Nos. US 2011/0027218 Al, US 2015/0126709 Al, US 2011/0111494 Al, US 2015/0110734 Al, and US 2015/0126710 Al; and U.S. Patent Nos. 9,359,420, 9,340,599, 8,921,519, and 8,450,460.

In some embodiments, a 4-1BB agonist is an antibody or antigen-binding fragment that specifically binds 4-1BB. In some embodiments, the 4-1BB agonist is EU-101 (Eutilex Co. Ltd.) utomilumab, or urelumab, or an antigen-binding fragment thereof. In some embodiments, the 4-1BB agonist is utomilumab (also known as PF-05082566, PF-2566, or MOR-7480). The preparation and properties of utomilumab and its variants and fragments are described in U.S. Patent Nos. 8,821,867; 8,337,850; and 9,468,678, and International Patent Application Publication No. WO 2012/032433 Al. In some embodiments, the 4-1BB agonist is urelumab (also known as BMS-663513 or 20H4,9.h4a. The preparation and properties of urelumab and its variants and fragments are described in U.S. Patent Nos. 7,288,638 and 8,962,804. An OX40 agonist also includes any binding molecule, such as any antibody or antigen-binding fragment, that contains six CDRs as contained in utomilumab or urelumab.

In an embodiment, the 4- 1BB agonist is selected from the group consisting of 1D8, 3Elor, 4B4 (BioLegend 309809), H4-1BB-Ml27 (BD Pharmingen 552532), BBK2 (Thermo Fisher MS621PABX), 145501 (Leinco Technologies B591), the antibody produced by cell line deposited as ATCC No. HB-11248 and disclosed in U.S. Patent No. 6,974,863, 5F4 (BioLegend 31 1503), C65-485 (BD Pharmingen 559446), antibodies disclosed in U.S. Patent Application Publication No. US 2005/0095244, antibodies disclosed in U.S. Patent No. 7,288,638 (such as 20H4.9-IgGl (BMS-663031)), antibodies disclosed in U.S. Patent No. 6,887,673 (such as 4E9 or BMS-554271), antibodies disclosed in U.S. Patent No. 7,214,493, antibodies disclosed in U.S. Patent No. 6,303,121, antibodies disclosed in U.S. Patent No. 6,569,997, antibodies disclosed in U.S. Patent No. 6,905,685 (such as 4E9 or BMS-554271), antibodies disclosed in U.S. Patent No. 6,362,325 (such as 1D8 or BMS-469492; 3H3 or BMS-469497; or 3El), antibodies disclosed in U.S. Patent No. 6,974,863 (such as 53A2); antibodies disclosed in U.S. Patent No. 6,210,669 (such as 1D8, 3B8, or 3El), antibodies described in U.S. Patent No. 5,928,893, antibodies disclosed in U.S. Patent No. 6,303,121, antibodies disclosed in U.S. Patent No. 6,569,997, antibodies disclosed in International Patent Application Publication Nos. WO 2012/177788, WO 2015/119923, and WO 2010/042433, and fragments, derivatives, conjugates, variants, or biosimilars thereof.

In some embodiments, the costimulatory agonist is a CD27 agonist. In some embodiments the CD27 agonist binds specifically to CD27 in a manner sufficient to reduce toxicity. In some embodiments, the CD27 agonist is an agonistic CD27 monoclonal antibody or fusion protein that abrogates antibody-dependent cellular toxicity (ADCC), for example NK cell cytotoxicity. In some embodiments, the CD27 agonist is an agonistic CD27 monoclonal antibody or fusion protein that abrogates antibody-dependent cell phagocytosis (ADCP). In some embodiments, the CD27 agonist is an agonistic CD27 monoclonal antibody or fusion protein that abrogates complement-dependent cytotoxicity (CDC).

In some embodiments, a CD27 agonist is an antibody or antigen-binding fragment that specifically binds CD27. In a particular embodiment, the CD27 agonist is the monoclonal antibody varlilumab (also known as CDX-1127 or IFS), is an antigen-binding fragment thereof. The preparation and properties of varlilumab are described in International Patent Application Publication No. WO 2016/145085 A2 and U.S. Patent Application Publication Nos. US 2011/0274685 Al and US 2012/0213771 Al.

In some embodiments, a CD27 agonist is a fusion protein. CD27 fusion proteins include those comprising an Fc domain fused to a ligand of CD27 (CD70). In some embodiments, the fusion protein is a dimeric (divalent), trimeric (trivalent) or hexameric (hexavalent) or greater fusion comprising two or more, such as three, four or more CD70 domains for binding CD27 fused to an Fc.

In an embodiment, the CD27 agonist is a CD27 agonist described in U.S. Patent Application Publication No. US 2014/0112942 Al, US 2011/0274685 Al, or US 2012/0213771 Al, or International Patent Application Publication No. WO 2012/004367 Al.

In some embodiments, the costimulatory agonist is a GITR agonist. In some embodiments the GITR agonist binds specifically to GITR in a manner sufficient to reduce toxicity. In some embodiments, the GITR agonist is an agonistic GITR monoclonal antibody or fusion protein that abrogates antibody-dependent cellular toxicity (ADCC), for example NK cell cytotoxicity. In some embodiments, the GITR agonist is an agonistic GITR monoclonal antibody or fusion protein that abrogates antibody-dependent cell phagocytosis (ADCP). In some embodiments, the GITR agonist is an agonistic GITR monoclonal antibody or fusion protein that abrogates complement-dependent cytotoxicity (CDC).

In some embodiments, a GITR agonist is an antibody or antigen-binding fragment that specifically binds GITR. In an embodiment, the GITR agonist is the agonistic, anti-GITR monoclonal antibody TRX518 (TolerRx, Inc.), also known as 6C8 and Ch-6C8-Agly. The preparation, properties, and uses of 6C8 and 2F8 antibodies, and their variants, are described in U.S. Patent Nos. 7,812,135; 8,388,967; and 9,028,823.

In some embodiments, the GITR agonist is the monoclonal antibody 1D7, or an antigen-binding fragment thereof. The preparation and properties of 1D7 are described in U.S. Patent Application Publication No. US 2015/0064204 Al. In an embodiment, the GITR agonist is the monoclonal antibody 33C9, or an antigen-binding fragment thereof. The preparation and properties of 33C9 are described in U.S. Patent Application Publication No. US 2015/0064204 Al. In an embodiment, the GITR agonist is the monoclonal antibody 33F6, or is an antigen-binding fragment thereof. The preparation and properties of 33F6 are described in U.S. Patent Application Publication No. US 2015/0064204 Al. In an embodiment, the GITR agonist is the monoclonal antibody 34G4, or is an antigen-binding fragment thereof. The preparation and properties of 34G4 are described in U.S. Patent Application Publication No. US 2015/0064204 Al. In an embodiment, the GITR agonist is the monoclonal antibody 35B 10, or is an antigen-binding fragment thereof. The preparation and properties of 35B1O are described in U.S. Patent Application Publication No. US 2015/0064204 Al. In an embodiment, the GITR agonist is the monoclonal antibody 41E11, or is an antigen-binding fragment thereof. The preparation and properties of 41E11 are described in U.S. Patent Application Publication No. US 2015/0064204 Al. In an embodiment, the GITR agonist is the monoclonal antibody 41GS, or is an antigen-binding fragment thereof. The preparation and properties of 41G5 are described in U.S. Patent Application Publication No. US 2015/0064204 Al. In an embodiment, the GITR agonist is the monoclonal antibody 42A11, or is an antigen-binding fragment thereof. The preparation and properties of 42A11 are described in U.S. Patent Application Publication No. US 2015/0064204 Al. In an embodiment, the GITR agonist is the monoclonal antibody 44C1, or is an antigen-binding fragment thereof. The preparation and properties of 44C1 are described in U.S. Patent Application Publication No. US 2015/0064204 Al. In an embodiment, the GITR agonist is the monoclonal antibody 45A8, or is an antigen-binding fragment thereof. The preparation and properties of 45A8 are described in U.S. Patent Application Publication No. US 2015/0064204 Al. In an embodiment, the GITR agonist is the monoclonal antibody 46E11, or is an antigen-binding fragment thereof. The preparation and properties of 46E11 are described in U.S. Patent Application Publication No. US 2015/0064204 Al. In an embodiment, the GITR agonist is the monoclonal antibody 48H12, or is an antigen-binding fragment thereof. The preparation and properties of 48H12 are described in U.S. Patent Application Publication No. US 2015/0064204 Al. In an embodiment, the GITR agonist is the monoclonal antibody 48H7, or is an antigen-binding fragment thereof. The preparation and properties of 48H7 are described in U.S. Patent Application Publication No. US 2015/0064204 Al. In an embodiment, the GITR agonist is the monoclonal antibody 49D9, or is an antigen-binding fragment thereof. The preparation and properties of 49D9 are described in U.S. Patent Application Publication No. US 2015/0064204 Al. In an embodiment, the GITR agonist is the monoclonal antibody 49E2, or is an antigen-binding fragment thereof. The preparation and properties of 49E2 are described in U.S. Patent Application Publication No. US 2015/0064204 Al. In an embodiment, the GITR agonist is the monoclonal antibody 48A9, or is an antigen-binding fragment thereof. The preparation and properties of 48A9 are described in U.S. Patent Application Publication No. US 2015/0064204 Al. In an embodiment, the GITR agonist is the monoclonal antibody 5H7, or is an antigen-binding fragment thereof. The preparation and properties of 5H7 are described in U.S. Patent Application Publication No. US 2015/0064204 Al. In an embodiment, the GITR agonist is the monoclonal antibody 7Al0, or is an antigen-binding fragment thereof. The preparation and properties of 7A10 are described in U.S. Patent Application Publication No. US 2015/0064204 Al. In an embodiment, the GITR agonist is the monoclonal antibody 9H6, or is an antigen-binding fragment thereof. The preparation and properties of 9H6 are described in U.S. Patent Application Publication No. US 2015/0064204 Al.

In an embodiment, the GITR agonist is an agonistic anti-GITR monoclonal antibody with described in U.S. Patent No. 8,709,424; U.S. Patent Application Publication Nos. US 2012/0189639 Al and US 2014/0348841 Al, and International Patent Application Publication No. WO 2011/028683 Al (Merck Sharp & Dohme Corp.). In an embodiment, the GITR agonist is an agonistic, anti-GITR monoclonal antibody selected from the group consisting of 36E5, 3D6, 61 G6, 6H6, 61F6, 1D8, 17F10, 35D8, 49Al, 9E5, and 31H6, and antigen-binding fragments thereof. The structure, properties, and preparation of these antibodies are described in U.S. Patent No. 8,709,424; U.S. Patent Application Publication Nos. US 2012/0189639 Al and US 2014/0348841 Al.

In an embodiment, the GITR agonist is a GITR agonist described in International Patent Application Publication Nos. WO 2013/039954 Al and WO 2011/028683 Al; U.S. Patent Application Publication Nos. US 2013/0108641 Al, US 2012/0189639 Al, and US 2014/0348841 Al; and U.S. Patent Nos. 7,812,135; 8,388,967; and 9,028,823.

In embodiments of any of the provided methods, the ratio of T cells (e.g. tumor-reactive T cells) to costimulatory agonists (cells to moles) in the expansion method is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 500, about 1 to 1000, or about 1 to 10000.

In embodiments of any of the provided methods, the one or more co-stimulatory agonist is added to the cell culture medium during the incubation. In some embodiments, each of the one or more costimulatory agonist is indepedently added at a concentration ranging between at or about 0.1 µg/mL to at or about 100 µg/mL, at or about 0.1 µg/mL and at or about 50 µg/mL, at or about 0.1 µg/mL and at or about 25 µg/mL, at or about 0.1 µg/mL and at or about 10 µg/mL, at or about 0.1 µg/mL and at or about 5 µg/mL, at or about 0.1 µg/mL and at or about 1 µg/mL, at or about 0.1 µg/mL and at or about 0.5 µg/mL, 0.5 µg/mL to at or about 100 µg/mL, at or about 0.5 µg/mL and at or about 50 µg/mL, at or about 0.5 µg/mL and at or about 25 µg/mL, at or about 0.5 µg/mL and at or about 10 µg/mL, at or about 0.5 µg/mL and at or about 5 µg/mL, at or about 0.5 µg/mL and at or about 1 µg/mL, 1 µg/mL to at or about 100 µg/mL, at or about 1 µg/mL and at or about 50 µg/mL, at or about 1 µg/mL and at or about 25 µg/mL, at or about 1 µg/mL and at or about 10 µg/mL, at or about 1 µg/mL and at or about 5 µg/mL, at or about 5 µg/mL to at or about 100 µg/mL, at or about 5 µg/mL and at or about 50 µg/mL, at or about 5 µg/mL and at or about 25 µg/mL, at or about 5 µg/mL and at or about 10 µg/mL, at or about 10 µg/mL to at or about 100 µg/mL, at or about 10 µg/mL and at or about 50 µg/mL, at or about 10 µg/mL and at or about 25 µg/mL, at or about 25 µg/mL to at or about 100 µg/mL, at or about 25 µg/mL and at or about 50 µg/mL or at or about 50 µg/mL and at or about 100 µg/mL, each inclusive. In some embodiments, the costimulatory agonist is added at a concentration of at or about 1 µg/mL, at or about 5 µg/mL, at or about 10 µg/mL, at or about 20 µg/mL, at or about 30 µg/mL, at or about 40 µg/mL, at or about 50 µg/mL, or any value between any of the foregoing.

In some embodiments, the costimulatory agonist is added with recombinant IL-2 to the culture medium. In some embodiments, recombinant IL-2 is added at a concentration of 200 IU/mL to 1000 IU/mL (e.g. at or about 300 IU/mL) and the costimulatory agonist is added at a concentration of 0.1 µg/mL to at or about 100 µg/mL (e.g. 1 µg/mL to 50 µg/mL, such as at or about 12.5 µg/mL or 50 µg/mL). In some embodiments, an initial expansion (first expansion) (e.g. described in Section I.A.2) is carried out in the presence of recombinant IL-2 added at a concentration of 200 IU/mL to 1000 IU/mL (e.g. at or about 300 IU/mL) and the costimulatory agonist is added at a concentration of 0.1 µg/mL to at or about 100 µg/mL (e.g. 1 µg/mL to 50 µg/mL, such as at or about 12.5 µg/mL or 50 µg/mL). In some embodiments, the co-culture (e.g. described in Section I.B.2) is carried out in the presence of recombinant IL-2 added at a concentration of 200 IU/mL to 1000 IU/mL (e.g. at or about 300 IU/mL) and the costimulatory agonist is added at a concentration of 0.1 µg/mL to at or about 100 µg/mL (e.g. 1 µg/mL to 50 µg/mL, such as at or about 12.5 µg/mL or 50 µg/mL). In some embodiments, an incubation for expansion (e.g. second expansion) (e.g. Section I.D) is carried out in the presence of recombinant IL-2 added at a concentration of 200 IU/mL to 1000 IU/mL (e.g. at or about 300 IU/mL) and the costimulatory agonist is added at a concentration of 0.1 µg/mL to at or about 100 µg/mL (e.g. 1 µg/mL to 50 µg/mL, such as at or about 12.5 µg/mL or 50 µg/mL).

In some embodiments, a costimulatory agonist is administered to a subject prior to isolation or selection of T cells for carrying out the culture methods for expansion. In such embodiments, it is contemplated that tumor reactive T cells or T cells surface positive for one or more activation marker as described is rejuvenated in vivo prior to ex vivo isolation, selection and/or enrichment for culturing cells in accord with the provided methods. In some such embodiments, the a costimulatory agonist is administered to a subject by infusing a dose selected from the group consisting of about 5 mg, about 8 mg, about 10 mg, about 20 mg, about 25 mg, about 50 mg, about 75 mg, 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg, about 1400 mg, about 1500 mg, about 1600 mg, about 1700 mg, about 1800 mg, about 1900 mg, and about 2000 mg, or a value between any of the foregoing. In some embodiments, an effective dosage of a costimulatory agonist disclosed herein is in the range of about 1 mg to about 500 mg, about 10 mg to about 300 mg, about 20 mg to about 250 mg, about 25 mg to about 200 mg, about 1 mg to about 50 mg, about 5 mg to about 45 mg, about 10 mg to about 40 mg, about 15 mg to about 35 mg, about 20 mg to about 30 mg, about 23 mg to about 28 mg, about 50 mg to about 150 mg, about 60 mg to about 140 mg, about 70 mg to about 130 mg, about 80 mg to about 120 mg, about 90 mg to about 110 mg, or about 95 mg to about 105 mg, about 98 mg to about 102 mg, about 150 mg to about 250 mg, about 160 mg to about 240 mg, about 170 mg to about 230 mg, about 180 mg to about 220 mg, about 190 mg to about 210 mg, about 195 mg to about 205 mg, or about 198 to about 207 mg.

In an embodiment, a costimulatory agonist is administered weekly. In an embodiment, a costimulatory agonist is administered every two weeks. In an embodiment, a costimulatory agonist is administered every three weeks. In an embodiment, a costimulatory agonist is administered monthly. In an embodiment, a costimulatory agonist is administered intravenously in a dose of 8 mg given every three weeks for 4 doses over a 12-week period. In an embodiment, a costimulatory agonist is administered at a lower initial dose, which is escalated when administered at subsequent intervals administered monthly. For example, the first infusion can deliver 300 mg of a costimulatory agonist, and subsequent weekly doses could deliver 2,000 mg of a costimulatory agonist for eight weeks, followed by monthly doses of 2,000 mg of a costimulatory agonist.

### C. Immune Checkpoint Inhibitors

In some embodiments, the T cell modulatory agent is an immune checkpoint inhibitor that inhibits an immune checkpoint pathway. The immune system has multiple inhibitory pathways that are involved in maintaining self-tolerance and for modulating immune responses. It is known that tumors can use certain immune-checkpoint pathways as a major mechanism of immune resistance, particularly against T cells that are specific for tumor antigens *(*Pardoll, 2012, Nature Reviews Cancer 12:252-264*).* Because many such immune checkpoints are initiated by ligand-receptor interactions, they can be readily blocked by antibodies against the ligands and/or their receptors.

Therefore, therapy with antagonistic molecules blocking an immune checkpoint pathway, such as small molecules, nucleic acid inhibitors (e.g., RNAi) or antibody molecules, are becoming promising avenues of immunotherapy for cancer and other diseases.

As used herein, the term "immune checkpoint inhibitor" refers to molecules that totally or partially reduce, inhibit, interfere with or modulate one or more checkpoint proteins. Checkpoint proteins regulate T cell activation or function. These proteins are responsible for co-stimulatory or inhibitory interactions of T cell responses. Immune checkpoint proteins regulate and maintain self-tolerance and the duration and amplitude of physiological immune responses.

Immune checkpoint inhibitors include any agent that blocks or inhibits in a statistically significant manner, the inhibitory pathways of the immune system. Such inhibitors may include small molecule inhibitors or may include antibodies, or antigen binding fragments thereof, that bind to and block or inhibit immune checkpoint receptor ligands. Illustrative immune checkpoint molecules that may be targeted for blocking or inhibition include, but are not limited to, PD1 (CD279), PDL1 (CD274, B7-H1), PDL2 (CD273, B7-DC), CTLA-4, LAG3 (CD223), TIM3, 4-1BB (CD137), 4-1BBL (CD137L), GITR (TNFRSF18, AITR), CD40, OX40 (CD134, TNFRSF4), CXCR2, tumor associated antigens (TAA), B7-H3, B7-H4, BTLA, HVEM, GAL9, B7H3, B7H4, VISTA, KIR, 2B4 (belongs to the CD2 family of molecules and is expressed on all NK, γδ, and memory CD8+ (αβ) T cells), CD160 (also referred to as BY55) and CGEN-15049. In some embodiments, the immune checkpoint inhibitor is an antibody. Immune checkpoint inhibitors include antibodies, or antigen binding fragments thereof, or other binding proteins, that bind to and block or inhibit the activity of one or more of PD1, PDL1, PDL2, CTLA-4, LAG3, TIM3, 4-1BB, 4-1BBL, GITR, CD40, OX40, CXCR2, TAA, B7-H3, B7-H4, BTLA, HVEM, GAL9, B7H3, B7H4, VISTA, KIR, 2B4, CD160, and CGEN-15049. Illustrative immune checkpoint inhibitors include Ipilimumab (anti-CTLA4), Pembrolizumab (anti-PD1), Tremelimumab (CTLA-4 blocking antibody), anti-OX40L (e.g. oxelumbab), and PD-L1 monoclonal antibody (Anti-B7-H1; MEDI4736).

In some embodiments, the checkpoint inhibitor inhibits the activity of PD1. Programmed cell death 1 (PD1) is an immune checkpoint protein that is expressed in B cells, NK cells, and T cells (Shinohara et al., 1995, Genomics 23:704-6*;* Blank et al., 2007, Cancer Immunol Immunother 56:739-45*;* Finger et al., 1997, Gene 197:177-87*;* Pardoll, 2012, Nature Reviews Cancer 12: 252-264). The major role of PD1 is to limit the activity of T cells in peripheral tissues during inflammation in response to infection, as well as to limit autoimmunity *(*Pardoll, 2012, Nature Reviews Cancer 12:252-264*).* PD1 expression is induced in activated T cells and binding of PD1 to one of its endogenous ligands acts to inhibit T-cell activation by inhibiting stimulatory kinases and also acting to inhibit the TCR "stop signal" *(*Pardoll, 2012, Nature Reviews Cancer 12:252-264*).* PD1 is highly expressed on regulatory T cells and may increase their proliferation in the presence of ligand *(*Pardoll, 2012, Nature Reviews Cancer 12:252-264*).* Anti-PD 1 antibodies have been used for treatment of melanoma, non-small-cell lung cancer, bladder cancer, prostate cancer, colorectal cancer, head and neck cancer, triple-negative breast cancer, leukemia, lymphoma and renal cell cancer *(*Topalian et al., 2012, N Engl J Med 366:2443-54*;* Lipson et al., 2013, Clin Cancer Res 19:462-8*;* Berger et al., 2008, Clin Cancer Res 14:3044-51*;* Gildener-Leapman et al., 2013, Oral Oncol 49:1089-96*;* Menzies & Long, 2013, Ther Adv Med Oncol 5:278-85). Exemplary anti-PD1 antibodies include nivolumab (Opdivo^{©} by BMS), pembrolizumab (Keytruda^{©} by Merck), pidilizumab (CT-011 by Cure Tech), lambrolizumab (MK-3475 by Merck), and AMP-224 (Merck).

In some embodiments, the checkpoint inhibitor inhibits the activity of PD-L1. PD-L1 (also known as CD274 and B7-H1) and PD-L2 (also known as CD273 and B7-DC) are ligands for PD1, found on activated T cells, B cells, myeloid cells, macrophages, and some types of tumor cells. The complex of PD1 and PD-L1 inhibits proliferation of CD8+ T cells and reduces the immune response *(*Topalian et al., 2012, N Engl J Med 366:2443-54*;* Brahmer et al., 2012, N Eng J Med 366:2455-65*).* Anti-PD-L1 antibodies have been used for treatment of non-small cell lung cancer, melanoma, colorectal cancer, renal-cell cancer, pancreatic cancer, gastric cancer, ovarian cancer, breast cancer, and hematologic malignancies *(*Brahmer et al., N Eng J Med 366:2455-65*;* Ott et al., 2013, Clin Cancer Res 19:5300-9*;* Radvanyi et al., 2013, Clin Cancer Res 19:5541*;* Menzies & Long, 2013, Ther Adv Med Oncol 5:278-85*;* Berger et al., 2008, Clin Cancer Res 14:13044-51). Exemplary anti-PD-L1 antibodies include MDX-1105 (Medarex), MEDI4736 (Medimmune) MPDL3280A (Genentech), BMS-935559 (Bristol-Myers Squibb) and MSB0010718C.

In some embodiments, the checkpoint inhibitor inhibits the activity of CTLA-4. Cytotoxic T-lymphocyte-associated antigen (CTLA-4), also known as CD152, is a co-inhibitory molecule that functions to regulate T-cell activation. CTLA-4 is a member of the immunoglobulin superfamily that is expressed exclusively on T-cells. CTLA-4 acts to inhibit T-cell activation and is reported to inhibit helper T-cell activity and enhance regulatory T-cell immunosuppressive activity *(*Pardoll, 2012, Nature Reviews Cancer 12:252-264*).* Anti-CTLA-4 antibodies have been used in clinical trials for the treatment of melanoma, prostate cancer, small cell lung cancer, non-small cell lung cancer (Robert & Ghiringhelli, 2009, Oncologist 14:848-61*;* Ott et al., 2013, Clin Cancer Res 19:5300*;* Weber, 2007, Oncologist 12:864-72*;* Wada et al., 2013, J Transl Med 11:89). Exemplary anti-CTLA-4 antibodies include ipilimumab (Bristol-Myers Squibb) and tremelimumab (Pfizer). Ipilimumab has received FDA approval for treatment of metastatic melanoma (Wada et al., 2013, J Transl Med 11:89).

In some embodiments, the checkpoint inhibitor inhibits the activity of LAG-3. Lymphocyte activation gene-3 (LAG-3), also known as CD223, is another immune checkpoint protein. LAG-3 has been associated with the inhibition of lymphocyte activity and in some cases the induction of lymphocyte anergy. LAG-3 is expressed on various cells in the immune system including B cells, NK cells, and dendritic cells. LAG-3 is a natural ligand for the MHC class II receptor, which is substantially expressed on melanoma-infiltrating T cells including those endowed with potent immune-suppressive activity (Goldberg et al., Curr Top Microbiol Immunol (344) 269-278, 2011*).* Exemplary anti-LAG-3 antibodies include BMS-986016, also known as relatlimab. IMP321 is a soluble version of the immune checkpoint molecule LAG-3, which activates dendritic cells, increasing antigen presentation.

In some embodiments, the checkpoint inhibitor inhibits the activity of TIM-3. T cell immunoglobulin domain and mucin domain-3 (TIM-3), also known as CD366, was initially identified on activated Th1 cells and has been shown to be a negative regulator of the immune response. Blockade of TIM-3 promotes T cell mediated anti-tumor immunity and has anti-tumor activity in a range of mouse tumor models. Combinations of TIM-3 blockade with other immunotherapeutic agents such as anti-PDL1 antibodies and others, can be additive or synergistic in increasing anti-tumor effects. TIM-3 expression has been associated with a number of different tumor types including melanoma, NSCLC and renal cancer, and additionally, expression of intratumoral TIM-3 has been shown to correlate with poor prognosis across a range of tumor types including NSCLC, cervical, and gastric cancers. Exemplary anti-TIM3 antibodies include TSR-022 and LY3321367.

In embodiments of any of the provided methods, the one or more immune checkpoint inhibitor is added to the cell culture medium during the incubation. In some embodiments, each of the one or more immune checkpoint inhibitor is independently added at a concentration ranging between at or about 0.1 µg/mL to at or about 100 µg/mL, such as at or about 0.1 µg/mL and at or about 50 µg/mL, at or about 0.1 µg/mL and at or about 25 µg/mL, at or about 0.1 µg/mL and at or about 10 µg/mL, at or about 0.1 µg/mL and at or about 5 µg/mL, at or about 0.1 µg/mL and at or about 1 µg/mL, at or about 0.1 µg/mL and at or about 0.5 µg/mL, 0.5 µg/mL to at or about 100 µg/mL, at or about 0.5 µg/mL and at or about 50 µg/mL, at or about 0.5 µg/mL and at or about 25 µg/mL, at or about 0.5 µg/mL and at or about 10 µg/mL, at or about 0.5 µg/mL and at or about 5 µg/mL, at or about 0.5 µg/mL and at or about 1 µg/mL, 1 µg/mL to at or about 100 µg/mL, at or about 1 µg/mL and at or about 50 µg/mL, at or about 1 µg/mL and at or about 25 µg/mL, at or about 1 µg/mL and at or about 10 µg/mL, at or about 1 µg/mL and at or about 5 µg/mL, at or about 5 µg/mL to at or about 100 µg/mL, at or about 5 µg/mL and at or about 50 µg/mL, at or about 5 µg/mL and at or about 25 µg/mL, at or about 5 µg/mL and at or about 10 µg/mL, at or about 10 µg/mL to at or about 100 µg/mL, at or about 10 µg/mL and at or about 50 µg/mL, at or about 10 µg/mL and at or about 25 µg/mL, at or about 25 µg/mL to at or about 100 µg/mL, at or about 25 µg/mL and at or about 50 µg/mL or at or about 50 µg/mL and at or about 100 µg/mL, each inclusive. In some embodiments, the immune checkpoint inhibitor is added at a concentration of at or about 1 µg/mL, at or about 5 µg/mL, at or about 10 µg/mL, at or about 20 µg/mL, at or about 30 µg/mL, at or about 40 µg/mL, at or about 50 µg/mL, or any value between any of the foregoing.

In some embodiments, subsequent to or concurrently with incubation with the costimulatory agent, the population of T cells also is contacted with a T cell stimulatory agent(s), such as a T cell stimulatory cytokine and/or an anti-CD3/anti-CD28 stimulatory agent, e.g. as anti-CD3/anti-CD28 beads, under conditions to induce or mediate proliferation of T cells in the population. In some embodiments, the T cell stimulatory cytokine includes one or more recombinant cytokines from recombinant IL-2, IL-7, IL-15 and/or IL-21, which can be included during the incubation to initially expand T cells in a population of cells from a subject.

In some embodiments, the immune checkpoint inhibitor is added with recombinant IL-2 to the culture medium. In some embodiments, recombinant IL-2 is added at a concentration of 200 IU/mL to 1000 IU/mL (e.g. at or about 300 IU/mL) and the immune checkpoint inhibitor is added at a concentration of 0.1 µg/mL to at or about 100 µg/mL (e.g. 1 µg/mL to 50 µg/mL, such as at or about 12.5 µg/mL or 50 µg/mL). In some embodiments, an initial expansion (first expansion) (e.g. described in Section I.A.2) is carried out in the presence of recombinant IL-2 added at a concentration of 200 IU/mL to 1000 IU/mL (e.g. at or about 300 IU/mL) and the immune checkpoint inhibitor is added at a concentration of 0.1 µg/mL to at or about 100 µg/mL (e.g. 1 µg/mL to 50 µg/mL, such as at or about 12.5 µg/mL or 50 µg/mL). In some embodiments, the co-culture (e.g. described in Section I.B.2) is carried out in the presence of recombinant IL-2 added at a concentration of 200 IU/mL to 1000 IU/mL (e.g. at or about 300 IU/mL) and the immune checkpoint inhibitor is added at a concentration of 0.1 µg/mL to at or about 100 µg/mL (e.g. 1 µg/mL to 50 µg/mL, such as at or about 12.5 µg/mL or 50 µg/mL). In some embodiments, an incubation for expansion (e.g. second expansion) (e.g. Section I.D) is carried out in the presence of recombinant IL-2 added at a concentration of 200 IU/mL to 1000 IU/mL (e.g. at or about 300 IU/mL) and the immune checkpoint inhibitor is added at a concentration of 0.1 µg/mL to at or about 100 µg/mL (e.g. 1 µg/mL to 50 µg/mL, such as at or about 12.5 µg/mL or 50 µg/mL).

In some embodiments, the immune checkpoint inhibitor is added with recombinant IL-15 to the culture medium. In some embodiments, recombinant IL-15 is added at a concentration of 10 IU/mL to 500 IU/mL (e.g. at or about 180 IU/mL) and the immune checkpoint inhibitor is added at a concentration of 0.1 µg/mL to at or about 100 µg/mL (e.g. 1 µg/mL to 50 µg/mL, such as at or about 12.5 µg/mL or 50 µg/mL). In some embodiments, an initiaal expansion (e.g. described in Section I.A.2) is carried out in the presence of recombinant IL-15 added at a concentration of 10 lU/mL to 500 IU/mL (e.g. at or about 180 IU/mL) and the immune checkpoint inhibitor is added at a concentration of 0.1 µg/mL to at or about 100 µg/mL (e.g. 1 µg/mL to 50 µg/mL, such as at or about 12.5 µg/mL or 50 µg/mL). In some embodiments, the co-culture (e.g. described in Section I.B.2) is carried out in the presence of recombinant IL-15 added at a concentration of 10 IU/mL to 500 IU/mL (e.g. at or about 180 IU/mL) and the immune checkpoint inhibitor is added at a concentration of 0.1 µg/mL to at or about 100 µg/mL (e.g. 1 µg/mL to 50 µg/mL, such as at or about 12.5 µg/mL or 50 µg/mL). In some embodiments, an incubation for expansion (e.g. second expansion) (e.g. Section I.D) is carried out in the presence of recombinant IL-15 added at a concentration of 10 IU/mL to 500 IU/mL (e.g. at or about 300 IU/mL) and the immune checkpoint inhibitor is added at a concentration of 0.1 µg/mL to at or about 100 µg/mL (e.g. 1 µg/mL to 50 µg/mL, such as at or about 12.5 µg/mL or 50 µg/mL).

### D. Apoptosis Inhibitor

In aspects of any of the provided methods, a population of T cells is incubated in the presence of one or more inhibitors of apoptosis or of an apoptotic signaling pathway in a cell (hereinafter "apoptosis inhibitor"). In provided embodiments, the methods include incubation of cells with an apoptosis inhibitor under conditions to reduce or prevent apoptosis of T cells in the sample. In particular embodiments, the apoptosis inhibitor is an inhibitor of the Fas/Fas ligand axis or is an inhibitor of caspase, both of which are involved in inducing apoptosis particularly of activated T cell. In some embodiment, the inclusion of an apoptosis inhibitor during the ex vivo manufacturing process of a T cell therapy results in an improved yield of T cells of interest during the expansion process. In particular aspects, such methods are used in connection with ex vivo manufacturing of tumor-reactive T cells, which represent a rare and infrequent endogenous population of cells. Even when such cells are enriched ex vivo by described co-culture methods they still may be susceptible to apoptosis during the process of expanding the cells. The provided methods rejuvenate such cells by increasing proliferation and supporting their activation and expansion while preventing or reducing apoptosis.

In some embodiments, subsequent to or concurrently with incubation with the apoptosis inhibitor, the population of T cells also is contacted with a T cell stimulatory agent(s), such as an anti-CD3 and/or one or more recombinant cytokines such as from recombinant IL-2, IL-7, IL-15 and/or IL-21, under conditions to induce or mediate proliferation of T cells in the population. In some such aspects, the apoptosis inhibitor protects the T cells from apoptosis thereby rejuvenating their potential of T cells in the population to proliferate and expand.

In some aspects, one or more phenotypes indicative of absence of apoptosis is decreased in cells produced by the provided methods. Apoptosis is a process of programmed cell death that includes a series of stereotyped morphological and biochemical events that lead to characteristic cell changes and death. These changes include blebbing, cell shrinkage, nuclear fragmentation, chromatin condensation, chromosomal DNA fragmentation, and global mRNA decay. Apoptosis is a well characterized process, and specific molecules associated with various stages are well known in the art. In the early stages of apoptosis, changes in the cellular and mitochondrial membrane become apparent. Biochemical changes are also apparent in the cytoplasm and nucleus of the cell. For example, the early stages of apoptosis can be indicated by activation of certain caspases, e.g., 2, 8, 9, and 10. The middle to late stages of apoptosis are characterized by further loss of membrane integrity, chromatin condensation and DNA fragmentation, include biochemical events such as activation of caspases 3, 6, and 7.

In certain embodiments, cells produced by the provided methods, or therapeutic T cell compositions provided herein, have a reduced percentage or frequency of cells positive for a marker of apoptosis. Various apoptosis markers are known to those of ordinary skill in the art and include, but are not limited to, an increase in activity of one or more caspases i.e. an activated caspase, an increase in PARP cleavage, activation and/or translocation of Bcl-2 family proteins, members of the cell death pathway, e.g., Fas and FADD, presence of nuclear shrinkage (e.g., monitored by microscope) and presence of chromosome DNA fragmentation (e.g., presence of chromosome DNA ladder) or with apoptosis assays that include TUNEL staining, and Annexin V staining. Annexin V is a protein that preferentially binds with high affinity phosphatidylserine (PS), which is a lipid that translocates from the inner to the outer leaflet of the plasma membrane during apoptosis. In some embodiments, cells produced by the provided methods, or therapeutic T cell compositions provided herein, have a reduced percentage or frequency of cells positive for expression of one or more factors associated with apoptosis, including pro-apoptotic factors known to initiate apoptosis, e.g., members of the death receptor pathway, activated members of the mitochondrial (intrinsic) pathway, such as Bcl-2 family members, e.g., Bax, Bad, and Bid, and caspases. In certain embodiments, cells produced by the provided methods, or therapeutic T cell compositions provided herein, have a reduced percentage or frequency of cells positive for staining with an indicator, e.g. Annexin V molecule, that will preferentially bind to cells undergoing apoptosis when incubated with or contacted to a cell composition. In any of such embodiments, the reduced frequency or percentage of such cells is reduced compared to a therapeutic T cell composition produced by a similar process but in which such process does not include incubation with the apoptosis inhibitor. In some embodiments, apoptosis is reduced by greater than at or about 1.5-fold, greater than at or about 2-fold, greater than at or about 3-fold, greater than at or about 5-fold, greater than at or about10-fold or more.

In particular embodiments, the apoptosis inhibitor is an inhibitor of the Fas/Fas ligand axis or is an inhibitor of caspase, both of which are involved in inducing apoptosis particularly of activated T cells. In aspects of the provide methods the apoptosis inhibitor can reduce or disrupt signaling mediated by the Fas/Fas-ligand axis and/or mediated by caspases.

In particular embodiments, the apoptosis inhibitor is an inhibitor of the Fas/Fas ligand axis or is an inhibitor of caspase, both of which are involved in inducing apoptosis particularly of activated T cells. In some embodiments, subsequent to or concurrently with incubation with the apoptosis inhibitor, the population of T cells also is contacted with a T cell stimulatory agent(s), such as a T cell stimulatory cytokine (e.g. IL-2) and/or an anti-CD3/anti-CD28 stimulatory agent, e.g. as anti-CD3/anti-CD28 beads, under conditions to induce or mediate proliferation of T cells in the population. In some such aspects, the apoptosis inhibitor protects the T cells from apoptosis thereby rejuvenating their potential of T cells in the population to proliferate and expand.

In some embodiments, the apoptosis inhibitor inhibits apoptosis by disrupting the Fas/Fas-ligand axis (CD95/CD95L axis). In some aspects, the apoptosis inhibitor inhibits apoptosis induced or mediated by CD95. Fas ligand (FasL or CD95L) is a type-II transmembrane protein that belongs to the tumor necrosis factor (TNF) family. Its binding with its receptor induces apoptosis. Fas ligand/receptor interactions play an important role in the regulation of the immune system and the progression of cancer. The activation of T-cells leads to their expression of Fas ligand. T cells are initially resistant to Fas-mediated apoptosis during clonal expansion, but become progressively more sensitive the longer they are activated, ultimately resulting in activation-induced cell death (AICD). In some aspects, this process is necessary in vivo to prevent an excessive immune response and eliminate autoreactive T-cells. Humans and mice with deleterious mutations of Fas or Fas ligand develop an accumulation of aberrant T-cells, leading to lymphadenopathy, splenomegaly, and lupus erythematosus.

In aspects of the provided methods, a population of T cells, such as a population containing tumor-reactive T cells, is incubated or contacted with an apoptosis inhibitor that disrupts or blocks the interaction between Fas and Fas ligand, in which such incubation is carried out concurrently or subsequently to activation of the T cells by antigen and/or by one or more T cell stimulatory agent(s) that activates or stimulates T cells in the population. In some embodiments, activation of T cells can upregulate expression of Fas ligand where it can interact with Fas also expressed on the cell surface, thereby engaging Fas and causing apoptosis. In some embodiments, an apoptosis inhibitors that blocks this interaction can be a binding molecule that specifically binds to Fas or Fas ligand to block their interactions, thereby reducing or blocking at least partially the Fas signaling pathway and/or apoptosis in the cell. Methods for determining and/or assessing Fas signal pathway activity are known to the person skilled in the art and are, for example, described by Lavrik et. al. (2012) Cell Death Differ., 19(1):36-41.

An inhibitor according to the disclosure may act on the protein level and/or the nucleic acid level. Inhibitors acting on the protein level may be selected from antibodies, proteins and/or small molecules. Inhibitors acting on the nucleic acid level are for example antisense molecules, RNAi molecules and/or ribozymes. The inhibitor binds to Fas (CD95) and/or the Fas ligand (CD95L). In a further embodiment, the Fas/Fas ligand interaction may be inhibited.

In some embodiments, the inhibitor is an antibody or a functional fragment thereof. In some aspects, the inhibitor being an antibody may bind to Fas (CD95). In some embodiments, the inhibitor being an antibody may bind to CD95L. An example of an antibody binding CD95L is Nok-1 or an antigen-binding fragment thereof, see e.g. Catalog No. 16-9919-81, ThermoFisher Scientific, Waltham MA).

In some embodiments, the apoptosis inhibitor is a soluble protein that can specifically bind to Fas ligand. In some embodiments, the apoptosis inhibitor is a soluble CD95 receptor molecule containing an extracellular portion of CD95 but without a transmembrane domain. Soluble CD95 receptor molecules are described in EP-A-0595659 or EP-A-0965637. In some embodiments, the apoptosis inhibitor is or includes CD95 receptor peptides, such as described in WO99/65935.

In some embodiments, the apoptosis inhibitor is a fusion protein that binds to Fas ligand. In a particular embodiment, the apoptosis inhibitor contains the extracellular domain of Fas (CD95) or a specific binding protein thereof that binds to Fas ligand, in which the extracellular domain or a specific binding portion is fused to a heterologous polypeptide, such as an Fc immunoglobulin molecule. In some embodiments, the soluble Fas molecule is any as described in WO99/144330 or WO99/50413. In some embodiments, the soluble Fas molecule is the molecule known as FLINT or DCR3 or a fragment thereof.

In particular embodiments the apoptosis inhibitors binds to Fas ligand (CD95 ligand) and is a fusion protein containing the extracellular Fas (CD95) domain and an Fc domain, in particular a human Fc domain. In an embodiment, an apoptosis inhibitor includes an extracellular domain of Fas, such including all or a contiguous of the extracellular domain of mature CD95 set forth as amino acids 26-173 of the CD95 (see e.g. UniProt Ascension No. P25445; U.S. Pat. No. 5,891,434). In some embodiments, the CD95 is a human CD95 and contains an extracellular domain with the following sequence (amino acids 26-173 of human CD95):

QVTDINSKGLELRKTVTTVETQNLEGLHHDGQFCHKPCPPGERKARDCTVNGD
EPDCVPCQEGKEYTDKAHFSSKCRRCRLCDEGHGLEVEINCTRTQNTKCRCKP
NFFCNSTVCEHCDPCTKCEHGIIKECTLTSNTKCKEEGSRSN (SEQ ID NO. 7)

In some embodiments, the Fas (CD95)-Fc fusion protein includes any as described in WO2014/013039 or WO2014/013037. In some embodiments, the extracellular Fas (CD95) domain of the fusion protein comprises the amino acid sequence up to amino acid 170, 171, 172 or 173 of human CD95. In particular embodiments, the fusion protein contains amino acids 26-172 of human CD95. In some embodiments, the Fc domain or functional fragment thereof comprises the CH2 and/or CH3 domain of an immunoglobulin, and optionally at least a part of the hinge region domain or a modified immunoglobulin domain derived therefrom. The immunoglobulin domain may be an IgG, IgM, IgD, or IgE immunoglobulin domain or a modified immunoglobulin domain derived, therefrom. In some embodiments, the Fc domain is an Fc of an IgG that contains at least a portion of a constant IgG immunoglobulin domain. The IgG immunoglobulin domain may be selected from IgG1, IgG2, IgG3 or IgG4 domains or from modified domains therefrom. In some embodiments, the Fc is a human Fc domain, such as a IgG Fc domain, e.g. a human IgG1 Fc domain. In particular embodiments, the extracellular domain of Fas or a specific binding fragment thereof is fused to an Fc immunoglobulin molecule including the hinge region e.g. from the human IgG1 molecule. A fusion protein comprising an extracellular CD95 domain and a human Fc domain is described in WO 95/27735 or WO2004/085478.

In some embodiments, the Fas (CD95)-Fc fusion protein is APG101 (asunercept) or is a functional fragment thereof.

In some embodiments, the Fas (CD95)-Fc fusion protein is CAN008 or is a functional fragment thereof.

In some embodiments, the apoptosis inhibitor inhibits apoptosis induced or mediated by caspase. Caspases are a family of related enzymes that play an important role as modulators of cellular functions, including functions that result in apoptosis and inflammation. Caspase activation and regulation is tightly controlled through a number of mechanisms. All caspases are expressed as enzymatically inactive forms known as pro-caspases, which can be activated following a variety of cellular insults or stimuli. Seven caspases, described above, are specifically involved in the process of apoptosis.

Apoptosis via caspase activation can be initiated in a number of overlapping ways, including via the mitochondrial pathway, via the death receptor pathway (I.e., Fas/FasL, TNF/TNF receptor), via the endoplasmic reticulum stress pathway, and via the apoptosis-inducing protease granzyme B.

In particular embodiments, an apoptosis inhibitor that is an inhibitor of caspase results in reduced activation of caspase in cells of the population. In certain embodiments, caspase activation can be detected by methods known to the person of ordinary skill. In some embodiments, an antibody that binds specifically to an activated caspase (i.e., binds specifically to the cleaved polypeptide) can be used to detect caspase activation. In another example, a fluorochrome inhibitor of caspase activity (FLICA) assay can be utilized to detect caspase-3 activation by detecting hydrolysis of acetyl Asp-Glu-Val-Asp 7-amido-4-methylcoumarin (Ac-DEVD-AMC) by caspase-3 (i.e., detecting release of the fluorescent 7-amino-4-methylcoumarin (AMC)). FLICA assays can be used to determine caspase activation by a detecting the product of a substrate processed by multiple caspases (e.g., FAM-VAD-FMK FLICA). Other techniques include The CASPASE-GLO^{®} caspase assays (PROMEGA) that use luminogenic caspase-8 tetrapeptide substrate (Z-LETD-aminoluciferin), the caspase-9 tetrapeptide substrate (Z-LEHD-aminoluciferin), the caspase-3/7 substrate (Z-DEVD-aminoluciferin), the caspase-6 substrate (Z-VEID-aminoluciferin), or the caspase-2 substrate (Z-VDVAD-aminoluciferin).

Examples for apoptosis inhibitors include both pan- and caspase specific inhibitors. Examples for apoptosis inhibitors include caspase inhibitors such as Emricasan (IDN-6556, PF-03491390), NAIP (neuronal apoptosis inhibitory protein; BIRC1), cIAP1 and cIAP2 (cellular inhibitor of apoptosis 1 and 2; BIRC2 and BIRC3, respectively), XIAP (X-chromosome binding IAP; BIRC4), survivin (BIRC5), BRUCE (Apollon; BIRC6), livin (BIRC7) and Ts-IAP (testis-specific IAP; BIRC8, Wedelolactone, NS3694, NSCI and Z-fluoromethyl ketone Z-VAD-FMK and any flouromethyl ketone variant therein (I.e., Z-FA-FMK, Z-VAD(OH)-FMK, Z-DEVD-FMK, Z-VAD(OM2)-FMK, Z-VDVAD-FMK, etc.) In some embodiments, the caspase inhibitor is a caspase-specific inhibitor. In some embodiments, the apoptosis inhibitor is a pan-caspase inhibitor.

In particular embodiments, the caspase inhibitor is XIAP. In some aspects, XIAP is able to stop apoptotic cell death that is induced by overproduction of caspases, such as via its ability to bind to and inhibit caspase 3, 7 and 9. The BIR2 domain of XIAP inhibits caspase 3 and 7, while BIR3 binds to and inhibits caspase 9.

In particular embodiments, the caspase inhibitor is Z-VAD-FMK (carbobenzoxy-valyl-alanyl-aspartyl-[O-methyl]- fluoromethylketone). In some aspects, Z-VAD-FMK is able to stop apoptotic cell death that is induced by caspases, such as via its ability to bind the active site of several caspase proteases.

In embodiments of any of the provided methods, the ratio of T cells (e.g. tumor-reactive T cells) to apoptosis inhibitor (cells to moles) in the expansion method is about 1 to 25, about 1 to 50, about 1 to 100, about 1 to 125, about 1 to 150, about 1 to 175, about 1 to 200, about 1 to 225, about 1 to 250, about 1 to 275, about 1 to 300, about 1 to 325, about 1 to 350, about 1 to 500, about 1 to 1000, or about 1 to 10000.

In embodiments of any of the provided methods, the one or more apoptosis inhibitor is added to the cell culture medium during the incubation. In some embodiments, each of the one or more apoptosis inhibitor is independently added at a concentration ranging between at or about 0.1 µg/mL to at or about 100 µg/mL, at or about 0.1 µg/mL and at or about 50 µg/mL, at or about 0.1 µg/mL and at or about 25 µg/mL, at or about 0.1 µg/mL and at or about 10 µg/mL, at or about 0.1 µg/mL and at or about 5 µg/mL, at or about 0.1 µg/mL and at or about 1 µg/mL, at or about 0.1 µg/mL and at or about 0.5 µg/mL, 0.5 µg/mL to at or about 100 µg/mL, at or about 0.5 µg/mL and at or about 50 µg/mL, at or about 0.5 µg/mL and at or about 25 µg/mL, at or about 0.5 µg/mL and at or about 10 µg/mL, at or about 0.5 µg/mL and at or about 5 µg/mL, at or about 0.5 µg/mL and at or about 1 µg/mL, 1 µg/mL to at or about 100 µg/mL, at or about 1 µg/mL and at or about 50 µg/mL, at or about 1 µg/mL and at or about 25 µg/mL, at or about 1 µg/mL and at or about 10 µg/mL, at or about 1 µg/mL and at or about 5 µg/mL, at or about 5 µg/mL to at or about 100 µg/mL, at or about 5 µg/mL and at or about 50 µg/mL, at or about 5 µg/mL and at or about 25 µg/mL, at or about 5 µg/mL and at or about 10 µg/mL, at or about 10 µg/mL to at or about 100 µg/mL, at or about 10 µg/mL and at or about 50 µg/mL, at or about 10 µg/mL and at or about 25 µg/mL, at or about 25 µg/mL to at or about 100 µg/mL, at or about 25 µg/mL and at or about 50 µg/mL or at or about 50 µg/mL and at or about 100 µg/mL, each inclusive.

In some embodiments, each of the one or more apoptosis inhibitor is independently added at a concentration ranging between 0.5 µM and 100 µM, such as a concentration between at and about 0.5 µM and at or about 50 µM, between at or about 0.5 µM and at or about 25 µM, between at or about 0.5 µM and at or about 10 µM, between at or about 0.5 µM and at or about 5 µM, between at or about 0.5 µM and at or about 1 µM, between at or about 1 µM and at or about 100 µM, between at or about 1 µM and at or about 50 µM, between at or about 1 µM and at or about 25 µM, between at or about 1 µM and at or about 10 µM, between at or about 1 µM and at or about 5 µM, between at or about 5 µM and at or about 100 µM, between at or about 5 µM and at or about 50 µM, between at or about 5 µM and at or about 25 µM, between at or about 5 µM and at or about 10 µM, between at or about 10 µM and at or about 100 µM, between at or about 10 µM and at or about 50 µM, between at or about 10 µM and at or about 25 µM, between at or about 25 µM and at or about 100 µM, between at or about 25 µM and at or about 50 µM, or between at or about 50 µM and at or about 100 µM, each inclusive.

In some embodiments, the apoptosis inhibitor is added with recombinant IL-2 to the culture medium. In some embodiments, recombinant IL-2 is added at a concentration of 200 IU/mL to 1000 IU/mL (e.g. at or about 300 IU/mL) and the apoptosis inhibitor is added at a concentration of 0.5 µM to 100 µM (e.g. 1 µM to 50 µM, such as at or about 12.5 µM or 50 µM). In some embodiments, an initial expansion (first expansion) (e.g. described in Section I.A.2) is carried out in the presence of recombinant IL-2 added at a concentration of 200 IU/mL to 1000 IU/mL (e.g. at or about 300 IU/mL) and the apoptosis inhibitor is added at a concentration of 0.5 µM to 100 µM (e.g. 1 µM to 50 µM, such as at or about 12.5 µM or 50 µM). In some embodiments, the co-culture (e.g. described in Section I.B.2) is carried out in the presence of recombinant IL-2 added at a concentration of 200 IU/mL to 1000 IU/mL (e.g. at or about 300 IU/mL) and the apoptosis inhibitor is added at a concentration of 0.5 µM to 100 µM (e.g. 1 µM to 50 µM, such as at or about 12.5 µM or 50 µM). In some embodiments, an incubation for expansion (e.g. second expansion) (e.g. Section I.D) is carried out in the presence of recombinant IL-2 added at a concentration of 200 IU/mL to 1000 IU/mL (e.g. at or about 300 IU/mL) and the apoptosis inhibitor is added at a concentration of 0.5 µM to 100 µM (e.g. 1 µM to 50 µM, such as at or about 12.5 µM or 50 µM).

In some embodiments, the apoptosis inhibitor is Z-VAD-FMK. In some embodiments, Z-VAD-FMK is added with recombinant IL-2 to the culture medium. In some embodiments, recombinant IL-2 is added at a concentration of 200 IU/mL to 1000 IU/mL (e.g. at or about 300 IU/mL) and Z-VAD-FMK is added at a concentration of 0.5 µM to 100 µM (e.g. 1 µM to 50 µM, such as at or about 12.5 µM or 50 µM). In some embodiments, an initial expansion (first expansion) (e.g. described in Section I.A.2) is carried out in the presence of recombinant IL-2 added at a concentration of 200 IU/mL to 1000 IU/mL (e.g. at or about 300 IU/mL) and Z-VAD-FMK is added at a concentration of 0.5 µM to 100 µM (e.g. 1 µM to 50 µM, such as at or about 12.5 µM or 50 µM). In some embodiments, the co-culture (e.g. described in Section I.B.2) is carried out in the presence of recombinant IL-2 added at a concentration of 200 IU/mL to 1000 IU/mL (e.g. at or about 300 IU/mL) and Z-VAD-FMK is added at a concentration of 0.5 µM to 100 µM (e.g. 1 µM to 50 µM, such as at or about 12.5 µM or 50 µM). In some embodiments, an incubation for expansion (second expansion) (e.g. Section I.D) is carried out in the presence of recombinant IL-2 added at a concentration of 200 IU/mL to 1000 IU/mL (e.g. at or about 300 IU/mL) and Z-VAD-FMK is added at a concentration of 0.5 µM to 100 µM (e.g. 1 µM to 50 µM, such as at or about 12.5 µM or 50 µM).

In some embodiments, the apoptosis inhibitor is added with recombinant IL-15 to the culture medium. In some embodiments, recombinant IL-15 is added at a concentration of 10 IU/mL to 500 IU/mL (e.g. at or about 180 IU/mL) and the apoptosis inhibitor is added at a concentration of 0.5 µM to 100 µM (e.g. 1 µM to 50 µM, such as at or about 12.5 µM or 50 µM). In some embodiments, an initial expansion (first expansion) (e.g. described in Section I.A.2) is carried out in the presence of recombinant IL-15 is added at a concentration of 10 IU/mL to 500 IU/mL (e.g. at or about 180 IU/mL) and the apoptosis inhibitor is added at a concentration of 0.5 µM to 100 µM (e.g. 1 µM to 50 µM, such as at or about 12.5 µM or 50 µM). In some embodiments, the co-culture (e.g. described in Section I.B.2) is carried out in the presence of recombinant IL-15 is added at a concentration of 10 IU/mL to 500 IU/mL (e.g. at or about 180 IU/mL) and the apoptosis inhibitor is added at a concentration of 0.5 µM to 100 µM (e.g. 1 µM to 50 µM, such as at or about 12.5 µM or 50 µM). In some embodiments, an incubation for expansion (e.g. second expansion) (e.g. Section I.D) is carried out in the presence of recombinant IL-15 is added at a concentration of 10 IU/mL to 500 IU/mL (e.g. at or about 180 IU/mL) and the apoptosis inhibitor is added at a concentration of 0.5 µM to 100 µM (e.g. 1 µM to 50 µM, such as at or about 12.5 µM or 50 µM).

In some embodiments, the apoptosis inhibitor is Z-VAD-FMK. In some embodiments, Z-VAD-FMK is added with recombinant IL-15 to the culture medium. In some embodiments, recombinant IL-15 is added at a concentration of 10 IU/mL to 500 IU/mL (e.g. at or about 180 IU/mL) and Z-VAD-FMK is added at a concentration of 0.5 µM to 100 µM (e.g. 1 µM to 50 µM, such as at or about 12.5 µM or 50 µM). In some embodiments, an initial expansion (first expansion) (e.g. described in Section I.A.2) is carried out in the presence of recombinant IL-15 is added at a concentration of 10 IU/mL to 500 IU/mL (e.g. at or about 180 IU/mL) and Z-VAD-FMK is added at a concentration of 0.5 µM to 100 µM (e.g. 1 µM to 50 µM, such as at or about 12.5 µM or 50 µM). In some embodiments, the co-culture (e.g. described in Section I.B.2) is carried out in the presence of recombinant IL-15 is added at a concentration of 10 IU/mL to 500 IU/mL (e.g. at or about 180 IU/mL) and Z-VAD-FMK is added at a concentration of 0.5 µM to 100 µM (e.g. 1 µM to 50 µM, such as at or about 12.5 µM or 50 µM). In some embodiments, an incubation for expansion (e.g. second expansion) (e.g. Section I.D) is carried out in the presence of recombinant IL-15 is added at a concentration of 10 IU/mL to 500 IU/mL (e.g. at or about 180 IU/mL) and Z-VAD-FMK is added at a concentration of 0.5 µM to 100 µM (e.g. 1 µM to 50 µM, such as at or about 12.5 µM or 50 µM).

In particular embodiments, a T cell modulatory agent is an inhibitor of heat shock proteins. Heat shock proteins (Hsps) are a diverse group of proteins which include molecular chaperones that can be produced by cells in response to stress. Stressors can include but are not limited heat, oxidative stress, infection, ischemia, exposure to heavy metals, and nutrient deficiency. Some Hsps have demonstrable anti-apoptotic effects, for example Hsp70 is described as attenuating apoptosis via inhibition of the mitochondrial translocation of Bax protein. Other Hsps are involved in signaling cascades which can promote the apoptotic response, like Hsp10 which is implicated in the activation of pro-caspase 3 (Ikwegbue et al., Pharmaceuticals 11(1): 2, 2018).

In certain embodiments, the Hsp inhibitor is an inhibitor of Hsp90. Hsp90 is an ATP-dependent protein chaperone that negatively inhibits Hsp70, despite Hsp90 and Hsp70 cooperating to prevent the dangerous aggregation of protein via heat-shock factor 1 in response to stress. Overexpression of Hsp90 has been observed to result in protein stabilization, cell proliferation, angiogenesis, and increased survival of cancer cells. Hsp90 has also been demonstrated to stabilize several receptors involved in oncogenic signaling pathways, including EGFR (Chatterjee et al., Int J Mol Sci (18)9, 2017). For these reasons and others, Hsp90 inhibitors have been evaluated in preclinical models of cancer as well as multiple phase I and II studies as both a single agent and in combination with other agents (Spreafico et al., Brit J of Cancer (112) 650-659, 2015).

Examples for hsp inhibitors include but are not limited to MKT-077, Dihydropyrimidines (I.e., SW02, MAL2-IIB, MAL3-101, NSC630668 etc.), flavonoids (I.e., epigallocatechin, myricetin etc.), 15-DSG, Apoptozole, VER-155008, Aptamer A17, Aptamer A8, cmHSP70.1. Examples for hsp90 inhibitors include but are not limited to 17-AAg, 17-DMAG, IPI-504, NVP-AUY922, AT13387, Ganetespib, KW-2478, CNF-2024 (BIIB021), Debio 0932, PU-H71, MPC-310, SNX-5422, Ds-2248, XL-888, TAS-116, and NVP-HSP990.

In particular embodiments, the hsp inhibitor is NVP-HSP990.

In some embodiments, each of the one or more hsp inhibitor is independently added at a concentration ranging between 1 nM and at or about 500 nM, such as a concentration between at or about 1 nM and at or about 250 nM, between at or about 1 nM and at or about 100 nM, between at or about 1 nM and at or about 50 nM, between at or about 1 nM and at or about 25 nM, between at or about 1 nM and at or about 10 nM, between at or about 1 nM and at or about 5 nM, between at or about 5 nM and at or about 500 nM, 5 nM and at or about 250 nM, between at or about 5 nM and at or about 100 nM, between at or about 5 nM and at or about 50 nM, between at or about 5 nM and at or about 25 nM, between at or about 5 nM and at or about 10 nM, between at or about 10 nM and at or about 500 nM, 10 nM and at or about 250 nM, between at or about 10 nM and at or about 100 nM, between at or about 10 nM and at or about 50 nM, between at or about 10 nM and at or about 25 nM, between at or about 25 nM and at or about 500 nM, 25 nM and at or about 250 nM, between at or about 25 nM and at or about 100 nM, between at or about 25 nM and at or about 50 nM, between at or about 50 nM and at or about 500 nM, 50 nM and at or about 250 nM, between at or about 50 nM and at or about 100 nM, between at or about 100 nM and at or about 500 nM, 100 nM and at or about 250 nM, or between at or about 250 nM and at or about 500 nM, each inclusive.

In some embodiments, the hsp inhibitor is independently added at a concentration ranging between 500 nM and at or about 1000 nM. In some embodiments, the hsp inhibitor is added at a concentration of at or about 500 nM, at or about 600 nM, at or about 700 nM, at or about 800 nM, at or about 900 nM, or at or about 1000 nM.

In some embodiments, the hsp inhibitor is added with recombinant IL-2 to the culture medium. In some embodiments, recombinant IL-2 is added at a concentration of 200 IU/mL to 1000 IU/mL (e.g. at or about 300 IU/mL) and the hsp inhibitor is added at a concentration of 1 nM to 1000 nM (e.g. at or about 1000 nM). In some embodiments, an initial expansion (first expansion) (e.g. described in Section I.A.2) is carried out in the presence of recombinant IL-2 added at a concentration of 200 IU/mL to 1000 IU/mL (e.g. at or about 300 IU/mL) and the hsp inhibitor is added at a concentration of 1 nM to 1000 nM (e.g. at or about 1000 nM). In some embodiments, the co-culture (e.g. described in Section I.B.2) is carried out in the presence of recombinant IL-2 added at a concentration of 200 IU/mL to 1000 IU/mL (e.g. at or about 300 IU/mL) and the hsp inhibitor is added at a concentration of 1 nM to 1000 nM (e.g. at or about 1000 nM). In some embodiments, an incubation for expansion (e.g. second expansion) (e.g. Section I.D) is carried out in the presence of recombinant IL-2 added at a concentration of 200 IU/mL to 1000 IU/mL (e.g. at or about 300 IU/mL) and the hsp inhibitor is added at a concentration of 1 nM to 1000 nM (e.g. at or about 1000 nM).

In some embodiments, the hsp inhibitor is added with recombinant IL-15 to the culture medium. In some embodiments, recombinant IL-15 is added at a concentration of 10 IU/mL to 500 IU/mL (e.g. at or about 1800 IU/mL) and the hsp inhibitor is added at a concentration of 1 nM to 1000 nM (e.g. at or about 1000 nM). In some embodiments, an initial expansion (first expansion) (e.g. described in Section I.A.2) is carried out in the presence of recombinant IL-15 is added at a concentration of 10 lU/mL to 500 IU/mL (e.g. at or about 1800 IU/mL) and the hsp inhibitor is added at a concentration of 1 nM to 1000 nM (e.g. at or about 1000 nM). In some embodiments, the co-culture (e.g. described in Section I.B.2) is carried out in the presence of recombinant IL-15 is added at a concentration of 10 IU/mL to 500 IU/mL (e.g. at or about 1800 IU/mL) and the hsp inhibitor is added at a concentration of 1 nM to 1000 nM (e.g. at or about 1000 nM). In some embodiments, an incubation for expansion (e.g. second expansion) (e.g. Section I.D) is carried out in the presence of recombinant IL-15 is added at a concentration of 10 lU/mL to 500 IU/mL (e.g. at or about 1800 IU/mL) and the hsp inhibitor is added at a concentration of 1 nM to 1000 nM (e.g. at or about 1000 nM).

### III. COMPOSITIONS AND PHARMACEUTICAL FORMULATIONS

Provided herein are compositions containing expanded T cells such as produced by any of the provided methods. In some embodiments, the compositions contain tumor reactive T cells or T cells containing an endogenous TCR specific to a tumor-associated antigen, e.g. neoantigen. In particular, among the provided compositions are compositions of cells that are enriched for tumor reactive T cells or T cells containing an endogenous TCR specific to a tumor-associated antigen, e.g. neoantigen.

In some embodiments, the composition comprises about 5-99% tumor-reactive T cells or T cells positive for the one or more selection marker (e.g. any as described in Section I.C), or any percentage of such cells between 5 and 99% inclusive. In some embodiments, the composition can include an increased or greater percentages of tumor-reactive CD3+ T cells or of CD3+ T cells positive for the one or more selection marker (e.g. any as described in Section I.D) relative to total CD3+ T cells or total cells in the composition compared to the percentage of such tumor-reactive CD3+ T cells or of CD3+ T cells positive for the one or more selection marker relative to total CD3+ T cells or total cells naturally present in the subject or biological sample from which the cells were isolated. In some embodiments, the percentage is increased at least or at least about 2-fold, 3-fold, 4-fold, 5-fold, 10-fold, 20-fold, 30-fold, 40-fold, 50-fold, 60-fold, 70-fold, 80-fold, 90-fold, 100-fold, 150-fold, 200-fold or more. In such embodiments, the one or more selection marker can be any as described, such as CXCL13 or PD-1/CD39/TIGIT.

In some embodiments, the composition can include at least at or about 20%, at least at or about 30%, at least at or about 40%, at least at or about 50%, at least at or about 60%, at least at or about 65%, at least at or about 70%, at least at or about 75%, at least at or about 80%, at least at or about 81%, at least at or about 82%, at least at or about 83%, at least at or about 84%, at least at or about 85%, at least at or about 86%, at least at or about 87%, at least at or about 88%, at least at or about 89%, at least at or about 90%, at least at or about 91%, at least at or about 92%, at least at or about 93%, at least at or about 94%, at least at or about 95%, at least at or about 96%, at least at or about 97%, at least at or about 98%, at least at or about 99%, or substantially 100% tumor-reactive CD3+ T cells or CD3+ T cells positive for one or more selection marker, e.g. CXCL13 or PD-1/CD39/TIGIT. In some embodiments, the composition comprises more than 30% tumor reactive CD3+ T cells or CD3+ T cells positive for one or more selection marker, e.g. e.g. CXCL13 or PD-1/CD39/TIGIT. In some embodiments, the composition comprises more than 40% tumor reactive CD3+ T cells or CD3+ T cells positive for one or more selection marker, e.g. e.g. CXCL13 or PD-1/CD39/TIGIT. In some embodiments, the composition comprises more than 50% tumor reactive CD3+ T cells or CD3+ T cells positive for one or more selection marker, e.g. e.g. CXCL13 or PD-1/CD39/TIGIT. In some embodiments, the composition comprises more than 60% tumor reactive CD3+ T cells or CD3+ T cells positive for one or more selection marker, e.g. e.g. CXCL13 or PD-1/CD39/TIGIT. In some embodiments, the composition comprises more than 70% tumor reactive CD3+ T cells or CD3+ T cells positive for one or more selection marker, e.g. e.g. CXCL13 or PD-1/CD39/TIGIT. In some embodiments, the composition comprises more than 80% tumor reactive CD3+ T cells or CD3+ T cells positive for one or more selection marker, e.g. e.g. CXCL13 or PD-1/CD39/TIGIT. In some embodiments, the composition comprises more than 90% tumor reactive CD3+ T cells or CD3+ T cells positive for one or more selection marker, e.g. e.g. CXCL13 or PD-1/CD39/TIGIT. In such embodiments, the one or more selection marker can be any as described in Section I.D, such as any one or more of e.g. CXCL13 or PD-1/CD39/TIGIT. In such embodiments, the selection marker includes cells positive for CXCL13. In some embodiments, the selection marker includes cells positive for PD-1/CD39/TIGIT.

In some embodiments, the tumor reactive can be present in the composition in a therapeutically effective amount. An effective amount of cells can vary depending on the patient, as well as the type, severity and extent of disease. Thus, a physician can determine what an effective amount is after considering the health of the subject, the extent and severity of disease, and other variables.

In certain embodiments, the number of such cells in the composition is a therapeutically effective amount. In some embodiments, the amount is an amount that reduces the severity, the duration and/or the symptoms associated with cancer, viral infection, microbial infection, or septic shock in an animal. In some embodiments, a therapeutically effective amount is a dose of cells that results in a reduction of the growth or spread of cancer by at least 2.5%, at least 5%, at least 10%, at least 15%, at least 25%, at least 35%, at least 45%, at least 50%, at least 75%, at least 85%, by at least 90%, at least 95%, or at least 99% in a patient or an animal administered a composition described herein relative to the growth or spread of cancer in a patient (or an animal) or a group of patients (or animals) not administered the composition. In some embodiments, a therapeutically effective amount is an amount to result in cytotoxic activity resulting in activity to inhibit or reduce the growth of cancer, viral and microbial cells.

In some embodiments, the composition has from at or about 10⁵ and at or about 10¹² tumor reactive CD3+ T cells or CD3+ T cells positive for one or more selection marker (e.g. CXCL13 or PD-1/CD39/TIGIT), or from at or about 10⁵ to at or about 10⁸ tumor reactive CD3+ T cells or CD3+ T cells positive for one or more selection marker (e.g. CXCL13 or PD-1/CD39/TIGIT), or from at or about 10⁶ and at or about 10¹² tumor reactive CD3+ T cells or CD3+ T cells positive for one or more selection marker (e.g. CXCL13 or PD-1/CD39/TIGIT), or from at or about 10⁸ and at or about 10¹¹ tumor reactive CD3+ T cells or CD3+ T cells positive for one or more selection marker (e.g. CXCL13 or PD-1/CD39/TIGIT), or from at or about 10⁹ and at or about 10¹⁰ tumor reactive CD3+ T cells or CD3+ T cells positive for one or more selection marker (e.g. CXCL13 or PD-1/CD39/TIGIT). In some embodiments, the composition comprises greater than or greater than at or about 10⁵ tumor reactive CD3+ T cells or CD3+ T cells positive for one or more selection marker (e.g. CXCL13 or PD-1/CD39/TIGIT), at or about 10⁶ tumor reactive CD3+ T cells or CD3+ T cells positive for one or more selection marker (e.g. CXCL13 or PD-1/CD39/TIGIT), at or about 10⁷ tumor reactive CD3+ T cells or CD3+ T cells positive for one or more selection marker (e.g. CXCL13 or PD-1/CD39/TIGIT), at or about 10⁸ tumor reactive CD3+ T cells or CD3+ T cells positive for one or more selection marker (e.g. CXCL13 or PD-1/CD39/TIGIT), at or about 10⁹ tumor reactive CD3+ T cells or CD3+ T cells positive for one or more selection marker (e.g. CXCL13 or PD-1/CD39/TIGIT), at or about 10¹⁰ tumor reactive CD3+ T cells or CD3+ T cells positive for one or more selection marker (e.g. CXCL13 or PD-1/CD39/TIGIT), at or about 10¹¹ tumor reactive CD3+ T cells or CD3+ T cells positive for one or more selection marker (e.g. CXCL13 or PD-1/CD39/TIGIT), or at or about 10¹² tumor reactive CD3+ T cells or CD3+ T cells positive for one or more selection marker (e.g. CXCL13 or PD-1/CD39/TIGIT). In some embodiments, such an amount can be administered to a subject having a disease or condition, such as to a cancer patient. In such embodiments, the one or more selection marker can be any as described in Section I.D, such as any one or more of e.g. CXCL13 or PD-1/CD39/TIGIT. In such embodiments, the selection marker includes cells positive for CXCL13. In some embodiments, the selection marker includes cells positive for PD-1/CD39/TIGIT.

In some embodiments, the composition comprises about 5-99% tumor-reactive T cells surface positive for the one or more T cell activation marker, such as CD137 (4-1BB) or CD134 (OX40), or any percentage of such cells between 5 and 99% inclusive. In some embodiments, the composition can include an increased or greater percentages of CD3+ T cells surface positive for the one or more T cell activation marker relative to total CD3+ T cells or total cells in the composition compared to the percentage of such tumor-reactive CD3+ T cells or of CD3+ T cells surface positive for the one or more T cell activation marker relative to total CD3+ T cells or total cells naturally present in the subject or biological sample from which the cells were isolated. In some embodiments, the percentage is increased at least or at least about 2-fold, 3-fold, 4-fold, 5-fold, 10-fold, 20-fold, 30-fold, 40-fold, 50-fold, 60-fold, 70-fold, 80-fold, 90-fold, 100-fold, 150-fold, 200-fold or more.

In some embodiments, the composition can include at least at or about 20%, at least at or about 30%, at least at or about 40%, at least at or about 50%, at least at or about 60%, at least at or about 65%, at least at or about 70%, at least at or about 75%, at least at or about 80%, at least at or about 81%, at least at or about 82%, at least at or about 83%, at least at or about 84%, at least at or about 85%, at least at or about 86%, at least at or about 87%, at least at or about 88%, at least at or about 89%, at least at or about 90%, at least at or about 91%, at least at or about 92%, at least at or about 93%, at least at or about 94%, at least at or about 95%, at least at or about 96%, at least at or about 97%, at least at or about 98%, at least at or about 99%, or substantially 100% tumor-reactive CD3+ T cells surface positive for one or more activation marker (e.g. CD134 and/or CD137). In some embodiments, the composition comprises more than 30% tumor reactive CD3+ T cells or CD3+ T cells surface positive for one or more activation marker (e.g. CD134 and/or CD137). In some embodiments, the composition comprises more than 40% tumor reactive CD3+ T cells or CD3+ T cells surface positive for one or more activation marker (e.g. CD134 and/or CD137). In some embodiments, the composition comprises more than 50% tumor reactive CD3+ T cells or CD3+ T cells surface positive for one or more activation marker (e.g. CD134 and/or CD137). In some embodiments, the composition comprises more than 60% tumor reactive CD3+ T cells or CD3+ T cells surface positive for one or more activation marker (e.g. CD134 and/or CD137). In some embodiments, the composition comprises more than 70% tumor reactive CD3+ T cells or CD3+ T cells surface positive for one or more activation marker (e.g. CD134 and/or CD137). In some embodiments, the composition comprises more than 80% tumor reactive CD3+ T cells or CD3+ T cells surface positive for one or more activation marker (e.g. CD134 and/or CD137). In some embodiments, the composition comprises more than 90% tumor reactive CD3+ T cells or CD3+ T cells surface positive for one or more activation marker (e.g. CD134 and/or CD137).

In some embodiments, the composition comprises CD3+ T cells as a percentage of total cells in the population that is greater than or greater than about 60%, greater than or greater than about 70%, greater than or greater than about 80%, greater than or greater than about 90% or greater than or greater than about 95%. In some embodiments, the composition contains CD4+ T cells and CD8+ T cells as a percentage of total cells in the population that is greater than or greater than about 60%, greater than or greater than about 70%, greater than or greater than about 80%, greater than or greater than about 90% or greater than or greater than about 95%. In particular embodiments, the composition contains a ratio of CD8+ T cells to CD4+ T cells that is between at or about 1:100 and at or about 100:1, between at or about 1:50 and at or about 50:1, between at or about 1:25 and at or about 25:1, between at or about 1:10 and at or about 10:1, between at or about 1:5 and at or about 5:1, or between at or about 1:2.5 and at or about 2.5:1.

In some embodiments, the volume of the composition is at least or at least about 10 mL, 50 mL, 100 mL, 200 mL, 300 mL, 400 mL or 500 mL, such as is from or from about 10 mL to 500 mL, 10 mL to 200 mL, 10 mL to 100 mL, 10 mL to 50 mL, 50 mL to 500 mL, 50 mL to 200 mL, 50 mL to 100 mL, 100 mL to 500 mL, 100 mL to 200 mL or 200 mL to 500 mL, each inclusive. In some embodiments, the composition has a cell density of at least or at least about 1 × 10⁵ cells/mL, 5 × 10⁵ cells/mL, 1 × 10⁶ cells/mL, 5 × 10⁶ cells/mL, 1 × 10⁷ cells/mL, 5 × 10⁷ cells/mL or 1 × 10⁸ cells/ mL. In some embodiment, the cell density of the composition is between or between about 1 × 10⁵ cells/mL to 1 × 10⁸ cells/mL, 1 × 10⁵ cells/mL to 1 × 10⁷ cells/mL, 1 × 10⁵ cells/mL to 1 × 10⁶ cells/mL, 1 × 10⁶ cells/mL to 1 × 10⁷ cells/mL, 1 × 10⁶ cells/mL to 1 × 10⁸ cells/mL, 1 × 10⁶ cells/mL to 1 × 10⁷ cells/mL or 1 × 10⁷ cells/mL to 1 × 10⁸ cells/mL, each inclusive.

Among the compositions are pharmaceutical compositions and formulations for administration, such as for adoptive cell therapy. In some embodiments, the engineered cells are formulated with a pharmaceutically acceptable carrier.

A pharmaceutically acceptable carrier can include all solvents, dispersion media, coatings, antibacterial and antifungal agents, isotonic and absorption delaying agents, and the like, compatible with pharmaceutical administration (Gennaro, 2000, Remington: The science and practice of pharmacy, Lippincott, Williams & Wilkins, Philadelphia, PA). Examples of such carriers or diluents include, but are not limited to, water, saline, Ringer's solutions, dextrose solution, and 5% human serum albumin. Liposomes and non-aqueous vehicles such as fixed oils may also be used. Supplementary active compounds can also be incorporated into the compositions. The pharmaceutical carrier should be one that is suitable for NK cells, such as a saline solution, a dextrose solution or a solution comprising human serum albumin.

In some embodiments, the pharmaceutically acceptable carrier or vehicle for such compositions is any non-toxic aqueous solution in which the cells can be maintained, or remain viable, for a time sufficient to allow administration of live cells. For example, the pharmaceutically acceptable carrier or vehicle can be a saline solution or buffered saline solution. The pharmaceutically acceptable carrier or vehicle can also include various bio materials that may increase the efficiency of cells. Cell vehicles and carriers can, for example, include polysaccharides such as methylcellulose (M. C. Tate, D. A. Shear, S. W. Hoffman, D. G. Stein, M. C. LaPlaca, Biomaterials 22, 1113, 2001, which is incorporated herein by reference in its entirety), chitosan (Suh J K F, Matthew H W T. Biomaterials, 21, 2589, 2000; Lahiji A, Sohrabi A, Hungerford D S, et al., J Biomed Mater Res, 51, 586, 2000, each of which is incorporated herein by reference in its entirety), N-isopropylacrylamide copolymer P(NIPAM-co-AA) (Y. H. Bae, B. Vernon, C. K. Han, S. W. Kim, J. Control. Release 53, 249, 1998; H. Gappa, M. Baudys, J. J. Koh, S. W. Kim, Y. H. Bae, Tissue Eng. 7, 35, 2001, each of which is incorporated herein by reference in its entirety), as well as Poly(oxyethylene)/poly(D,L-lactic acid-co-glycolic acid) (B. Jeong, K. M. Lee, A. Gutowska, Y. H. An, Biomacromolecules 3, 865, 2002, which is incorporated herein by reference in its entirety), P(PF-co-EG) (Suggs L J, Mikos A G. Cell Trans, 8, 345, 1999, which is incorporated herein by reference in its entirety), PEO/PEG (Mann B K, Gobin A S, Tsai A T, Schmedlen R H, West J L., Biomaterials, 22, 3045, 2001; Bryant S J, Anseth K S. Biomaterials, 22, 619, 2001, each of which is incorporated herein by reference in its entirety), PVA (Chih-Ta Lee, Po-Han Kung and Yu-Der Lee, Carbohydrate Polymers, 61, 348, 2005, which is incorporated herein by reference in its entirety), collagen (Lee C R, Grodzinsky A J, Spector M., Biomaterials 22, 3145, 2001, which is incorporated herein by reference in its entirety), alginate (Bouhadir K H, Lee K Y, Alsberg E, Damm K L, Anderson K W, Mooney D J. Biotech Prog 17, 945, 2001; Smidsrd O, Skjak-Braek G., Trends Biotech, 8, 71, 1990, each of which is incorporated herein by reference in its entirety).

In some embodiments, the composition, including pharmaceutical composition, is sterile. In some embodiments, isolation or enrichment of the cells is carried out in a closed or sterile environment, for example, to minimize error, user handling and/or contamination. In some embodiments, sterility may be readily accomplished, e.g., by filtration through sterile filtration membranes.

Also provided herein are compositions that are suitable for cryopreserving the provided T cells, including tumor-reactive T cells. In some embodiments, the composition comprises a cryoprotectant. In some embodiments, the cryoprotectant is or comprises DMSO and/or s glycerol. In some embodiments, compositions formulated for cryopreservation can be stored at low temperatures, such as ultra low temperatures, for example, storage with temperature ranges from -40 °C to -150°C, such as or about 80 °C ± 6.0 ° C.

In some embodiments, the cryopreserved cells are prepared for administration by thawing. In some cases, the cells can be administered to a subject immediately after thawing. In such an embodiment, the composition is ready-to-use without any further processing. In other cases, the cells are further processed after thawing, such as by resuspension with a pharmaceutically acceptable carrier, incubation with an activating or stimulating agent, or are activated washed and resuspended in a pharmaceutically acceptable buffer prior to administration to a subject.

### IV. METHODS OF TREATMENT AND THERAPEUTIC APPLICATIONS

Provided herein are compositions and methods relating to the provided therapeutic cell compositions described herein for use in treating diseases or conditions in a subject such as a cancer. Such methods and uses include therapeutic methods and uses, for example, involving administration of the therapeutic cells, or compositions containing the same, to a subject having a disease, condition, or disorder. In some cases, the disease or disorder is a tumor or cancer. In some embodiments, the cells or pharmaceutical composition thereof is administered in an effective amount to effect treatment of the disease or disorder. Uses include uses of the cells or pharmaceutical compositions thereof in such methods and treatments, and in the preparation of a medicament in order to carry out such therapeutic methods. In some embodiments, the methods thereby treat the disease or condition or disorder in the subject.

In some embodiments, the methods of treatment comprise administering an effective amount of a composition containing tumor reactive CD3+ T cells or CD3+ T cells positive for one or more marker as described herein (e.g. CXCL13 and/or PD-1/CD39/TIGIT). Such compositions can include any as described herein, including compositions produced by the provided methods.

In some embodiment, a subject (e.g. autologous) is administered from at or about 10⁵ to at or about 10¹² CD3+ T cells produced by any of the provided methods, or from at or about 10⁵ to at or about 10⁸ CD3+ T cells produced by any of the provided methods, or from at or about 10⁶ and at or about 10¹² CD3+ T cells produced by any of the provided methods, or from at or about 10⁸ and at or about 10¹¹ CD3+ T cells produced by any of the provided methods, or from at or about 10⁹ and at or about 10¹⁰ CD3+ T cells produced by any of the provided methods. In some embodiments, the therapeutically effective amount for administration comprises greater than or greater than at or about 10⁵ CD3+ T cells produced by any of the provided methods, at or about 10⁶ CD3+ T cells produced by any of the provided methods, at or about 10⁷ CD3+ T cells produced by any of the provided methods, at or about 10⁸ CD3+ T cells produced by any of the provided methods, at or about 10⁹ CD3+ T cells produced by any of the provided methods, at or about 10¹⁰ CD3+ T cells produced by any of the provided methods, at or about 10¹¹ CD3+ T cells produced by any of the provided methods, or at or about 10¹² CD3+ T cells produced by any of the provided methods. In some embodiments, such an amount can be administered to a subject having a disease or condition, such as to a cancer patient. In some embodiments, the number of T cells are administered are viable T cells.

In some embodiments, the methods of treatment comprise administering an effective amount of a composition containing tumor reactive CD3+ T cells or CD3+ T cells positive for one or more selection marker as described herein (e.g. CXCL13 and/or PD-1/CD39/TIGIT). Such compositions can include any as described herein, including compositions produced by the provided methods. In some embodiment from at or about 10⁵ to at or about 10¹² tumor reactive CD3+ T cells or CD3+ T cells positive for one or more selection marker, such as any as described, or from at or about 10⁵ to at or about 10⁸ tumor reactive CD3+ T cells or CD3+ T cells positive for one or more selection marker, or from at or about 10⁶ and at or about 10¹² tumor reactive CD3+ T cells or CD3+ T cells positive for one or more selection marker, or from at or about 10⁸ and at or about 10¹¹ tumor reactive CD3+ T cells or CD3+ T cells positive for one or more selection marker, or from at or about 10⁹ and at or about 10¹⁰ tumor reactive CD3+ T cells or CD3+ T cells positive for one or more selection marker are administered to the individual. In some embodiments, the therapeutically effective amount for administration comprises greater than or greater than at or about 10⁵ tumor reactive CD3+ T cells or CD3+ T cells positive for one or more selection marker, at or about 10⁶ tumor reactive CD3+ T cells or CD3+ T cells positive for one or more selection marker, at or about 10⁷ tumor reactive CD3+ T cells or CD3+ T cells positive for one or more selection marker, at or about 10⁸ tumor reactive CD3+ T cells or CD3+ T cells positive for one or more selection marker, at or about 10⁹ tumor reactive CD3+ T cells or CD3+ T cells positive for one or more selection marker, at or about 10¹⁰ tumor reactive CD3+ T cells or CD3+ T cells positive for one or more selection marker, at or about 10¹¹ tumor reactive CD3+ T cells or CD3+ T cells positive for one or more selection marker, or at or about 10¹² tumor reactive CD3+ T cells or CD3+ T cells positive for one or more selection marker. In some embodiments, such an amount can be administered to a subject having a disease or condition, such as to a cancer patient. In some embodiments, the number of T cells are administered are viable T cells. In such embodiments, the one or more selection marker can be any as described in Section I.D, such as any one or more of e.g. CXCL13 or PD-1/CD39/TIGIT. In such embodiments, the selection marker includes cells positive for CXCL13. In some embodiments, the selection marker includes cells positive for PD-1/CD39/TIGIT.

In some embodiments, the amount is administered as a flat dose. In other embodiments, the amount is administered per kilogram body weight of the subject.

In some embodiments, the composition, such as produced by any of the provided methods or containing tumor-reactive T cells or T cells positive for a selection marker (e.g. CXCL13 and/or PD-1/CD39/TIGIT), are administered to an individual soon after expansion according to the provided methods. In other embodiments, the expanded T cells, such as expanded tumor-reactive T cells or T cells positive for a selection marker, are cryopreserved prior to administration, such as by methods described above. For example, the T cells, such as tumor-reactive T cells or T cells positive for a selection marker, can be stored for greater than 6, 12, 18, or 24 months prior to administration to the individual. Such cryopreserved cells can be thawed prior to the administration.

In some embodiments, the provided compositions, such as provided by any of the provided methods or containing tumor-reactive T cells or T cells positive for a T cell selection marker, can be administered to a subject by any convenient route including parenteral routes such as subcutaneous, intramuscular, intravenous, and/or epidural routes of administration.

In some embodiments, the compositions, such as provided by any of the provided methods or containing tumor-reactive T cells or T cells positive for a selection marker may be administered in a single dose. Such administration may be by injection, e.g., intravenous injection. In some embodiments, tumor-reactive T cells or T cells positive for a selection marker may be administered in multiple doses. Dosing may be once, twice, three times, four times, five times, six times, or more than six times per year. Dosing may be once a month, once every two weeks, once a week, or once every other day. Administration of such compositions and cells may continue as long as necessary.

In some embodiments, the subject is administered a lymphodepleting therapy prior to the administration of the dose of cells from a provided compositions, such as produced by any of the provided methods or containing tumor-reactive T cells or T cells positive for a selection marker. The lymphodepleting therapy can include administration of Fludarabine and/or cyclophosphamide (the active form being referred to as mafosfamide) and combinations thereof. Such methods are described in Gassner et al. (Cancer Immunol Immunother . 201 1 , 60(1):75-85, Muranski, et al, Nat Clin Pract Oncol, 2006 3(12):668-681, Dudley, et al., J Clin Oncol 2008, 26:5233-5239, and Dudley, et al., J Clin Oncol. 2005, 23(10):2346-2357, all of which are incorporated by reference herein in their entireties. In some embodiments, the fludarabine is administered at a dosage of 10 mg/kg/day, 15 mg/kg/day, 20 mg/kg/day, 25 mg/kg/day, 30 mg/kg/day, 35 mg/kg/day, 40 mg/kg/day, or 45 mg/kg/day, or a dosage amount between a range of any of the foregoing. In some embodiments, the fludarabine is for 2-7 days, such as for 3-5 days, such as at or about 3 days, at or about 4 days or at or about 5 days. In some embodiments, the cyclophosphamide is administered at a dosage of 100 mg/m2/day, 150 mg/m2/day, 175 mg/m2/day, 200 mg/m2/day, 225 mg/m2/day, 250 mg/m2/day, 275 mg/m2/day, or 300 mg/m2/day. In some embodiments, the cyclophosphamide is administered intravenously (i.e., i.v.). In some embodiments, the cyclophosphamide treatment is for 2-7 days, such as 3-5 days, at or about 3 days, at or about 4 days or at or about 5 days. The lymphodepleting therapy is administered prior to the provided cell compositions. In some embodiments, the lymphodepleting therapy is carried out within a week of the administration of the provided cell compositions, such as 5-7 days prior to the administration of the dose of cells.

The compositions described herein can be used in a method for treating hyperproliferative disorders. In a preferred embodiment, they are for use in treating cancers. In some aspects, the cancer can be a melanoma, ovarian cancer, cervical cancer, lung cancer, bladder cancer, breast cancer, head and neck cancer, renal cell carcinoma, acute myeloid leukemia, colorectal cancer, and sarcoma. In some embodiments, the cancer is a cancer with a high mutational burden. In some embodiments, the cancer is melanoma, lung squamous, lung adenocarcinoma, bladder cancer, lung small cell cancer, esophageal cancer, colorectal cancer, cervical cancer, head and neck cancer, stomach cancer or uterine cancer.

In some embodiments, the cancer is an epithelial cancer. In some embodiments, the cancer is selected from non-small cell lung cancer (NSCLC), CRC, ovarian cancer, breast cancer, esophageal cancer, gastric cancer, pancreatic cancer, cholangiocarcinoma cancer, endometrial cancer. In some embodiments, the breast cancer is HR+/Her2- breast cancer. In some embodiments, the breast cancer is a triple negative breast cancer (TNBC). In some embodiments, the breast cancer is a HER2+ breast cancer.

In some embodiments, the subject has a cancer that is a hematological tumor. Non- limiting examples of hematological tumors include leukemia, including acute leukemias (such as 1 lq23- positive acute leukemia, acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

In some embodiments, the subject has a solid tumor cancer. Non-limiting examples of solid tumors, such as sarcomas and carcinomas, include fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer (including basal breast carcinoma, ductal carcinoma and lobular breast carcinoma), lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, astrocytoma, medulloblastoma, craniopharyngioma, ependymoma, pinealoma, hemangioblastoma, acoustic neuroma, oligodendroglioma, meningioma, melanoma, neuroblastoma and retinoblastoma). In several examples, a tumor is melanoma, lung cancer, lymphoma breast cancer or colon cancer.

In some embodiments, the cancer is a skin cancer. In particular embodiments, the cancer is a melanoma, such as a cutaneous melanoma. In some embodiments, the cancer is a merkel cell or metastatic cutaneous squamous cell carcinoma (CSCC).

In some embodiments, the tumor is a carcinoma, which is a cancer that develops from epithelial cells or is a cancer of epithelial origin. In some embodiments, the cancer arises from epithelial cells which include, but are not limited to, breast cancer, basal cell carcinoma, adenocarcinoma, gastrointestinal cancer, lip cancer, mouth cancer, esophageal cancer, small bowel cancer and stomach cancer, colon cancer, liver cancer, bladder cancer, pancreas cancer, ovary cancer, cervical cancer, lung cancer, breast cancer and skin cancer, such as squamous cell and basal cell cancers, prostate cancer, renal cell carcinoma, and other known cancers that effect epithelial cells throughout the body.

In some embodiments, the subject has a cancer that is a gastrointestinal cancer involving a cancer of the gastrointestinal tract (GI tract), including cancers or the upper or lower digestive tract, or an accessory organ of digestion, such as esophagus, stomach, biliary system, pancreas, small intestine, large intestine, rectum or anus. In some embodiments, the cancer is an esophageal cancer, stomach (gastric) cancer, pancreatic cancer, liver cancer (hepatocellular carcinoma), gallbladder cancer, cancer of the mucosa-associated lymphoid tissue (MALT lymphoma), cancer of the biliary tree, colorectal cancer (including colon cancer, rectum cancer or both), anal cancer, or a gastrointestinal carcinoid tumor. In particular embodiments, the cancer is a colorectal cancer.

In some embodiments, the cancer is a colorectal cancer. Colorectal cancer (CRC) is a common tumor of increasing incidence, which, in many cases, does not response to checkpoint inhibition or other immunotherapy. This is the case even though such cancers have properties that are associated with response, e.g. a reasonably high mutation rate and well established association of prognosis with level of T cell infiltration.

In some embodiments, the cancer is an ovarian cancer. In some embodiments, the cancer is a triple-negative breast cancer (TNBC).

In some embodiments, the cancer is lung cancer. In some embodiments, the cancer is a breast cancer. In some embodiments, the cancer is a colorectal cancer. In some embodiments, the cancer is pancreatic cancer. In some embodiments, the cancer is a merkel cell cancer. In some embodiments, the cancer is a metastatic cutaneous squamous cell carcinoma (CSCC). In some embodiments, the cancer is a melanoma.

In some embodiments, the subject is one whose cancer is refractory to, and or who has relapsed following treatment with, a checkpoint blockade, such as an anti-PD1 or anti-PD-L1 therapy.

In some embodiments, the subject is the same subject from with the biological sample was obtained for producing the therapeutic cell composition. In some such embodiments, the provided methods of treatment is an adoptive cell therapy with a therapeutic composition containing T cells autologous to the subject.

In some embodiments, the cell compositions provided herein are autologous to the subject to be treated. In such embodiments, the starting cells for expansion are isolated directly from a biological sample from the subject as described herein, in some cases including with enrichment for T cells positive for one or more selection marker as described, and cultured under conditions for expansion as provided herein. In some aspects, the biological sample from the subject is or includes a tumor or lymph node sample and such sample tumor and an amount of such tissue is obtained, such as by resection or biopsy (e.g. core needle biopsy or fine-needle aspiration). In some embodiments, following the culturing under conditions for expansion in accord with the provided methods the cells are formulated and optionally cryopreserved for subsequent administration to the same subject for treating the cancer.

In some embodiments, the cell compositions provided herein are allogenic to the subject to be treated. In some aspects, the subject from which the cells are derived or isolated is a healthy subject or is not known to have a disease or conditions, such as a cancer. In such embodiments, the starting cells for expansion are isolated directly from a biological sample from such a subject as described herein, in some cases including with enrichment for T cells positive for one or more selection marker as described, and cultured under conditions for expansion as provided herein. In some aspects, the biological sample from the subject is or includes a tumor or lymph node sample and such sample tumor and an amount of such tissue is obtained, such as by resection or biopsy (e.g. core needle biopsy or fine-needle aspiration). In some embodiments, following the culturing under conditions for expansion the cells are formulated and optionally cryopreserved for subsequent administration to the a different subject for treating a cancer in such different subject.

In some embodiments, the provided methods can be carried out with one or more other immunotherapies. In some embodiments, the immunotherapy is an immune modulating agent that is an immune checkpoint inhibitor. In some embodiments, the immune checkpoint inhibitor specifically binds a molecule selected from among CD25, PD-1, PD-L1, PD-L2, CTLA-4, LAG-3, TIM-3, 4-1BB, GITR, CD40, CD40L, OX40, OX40L, CXCR2, B7-H3, B7-H4, BTLA, HVEM, CD28, TIGIT and VISTA. In some embodiments, the immune checkpoint inhibitor is and antibody or antigen-binding fragment, a small molecule or a polypeptide. In some embodiments, the immune checkpoint inhibitor is selected from among nivolumab, pembrolizumab, pidilizumab, MK-3475, BMS-936559, MPDL3280A, ipilimumab, tremelimumab, IMP31, BMS-986016, urelumab, TRX518, dacetuzumab, lucatumumab, SEQ-CD40, CP-870, CP-893, MED16469, MEDI4736, MOXR0916, AMP-224, and MSB001078C, or is an antigen-binding fragment thereof.

In some embodiments, the provided methods include combination therapy of a cell therapy as described and PD-1 or PD-L1 inhibitors. A PD-1 or PD-L1 inhibitor can include binding antibodies, antagonists, or inhibitors (i.e., blockers).

In an embodiment, the PD-I inhibitor is nivolumab (commercially available as OPDIVO from Bristol-Myers Squibb Co.), or biosimilars, antigen-binding fragments, conjugates, or variants thereof. Nivolumab is a fully human IgG4 antibody blocking the PD-I receptor. In an embodiment, the anti-PD-I antibody is an immunoglobulin G4 kappa, anti-(human CD274) antibody. Nivolumab is assigned Chemical Abstracts Service (CAS) registry number 946414-94-4 and is also known as 5C4, BMS-936558, l\tIDX-1106, and ONO-4538. The preparation and properties of nivolumab are described in U.S. Patent No. 8,008,449 and International Patent Publication No. WO 2006/121168.

In another embodiment, the PD-1 inhibitor comprises pembrolizumab ( commercially available as KEYTRUDA from Merck & Co., Inc., Kenilworth, NJ, USA), or antigen-binding fragments, conjugates, or variants thereof. Pembrolizumab is assigned CAS registry number 1374853-91-4 and is also known as lambrolizumab, MK-3475, and SCH-900475. The properties, uses, and preparation of pembrolizumab are described in International Patent Publication No. WO 2008/156712 Al, U.S. Patent No. 8,354,509 and U.S. Patent Application Publication Nos. US 2010/0266617 Al, US 2013/0108651 Al, and US 2013/0109843 A2.

In an embodiment, the PD-LI inhibitor is durvalumab, also known as MEDI4736 (which is commercially available from Medimmune, LLC, Gaithersburg, JVIaryland, a subsidiary of AstraZeneca plc.), or antigen-binding fragments, conjugates, or variants thereof. In an embodiment, the PD-LI inhibitor is an antibody disclosed in U.S. Patent No. 8,779,108 or U.S. Patent Application Publication No. 2013/0034559.

In an embodiment, the PD-LI inhibitor is avelumab, also known as MSB0010718C (commercially available from Merck KGaA/EMD Serono), or antigen-binding fragments, conjugates, or variants thereof. The preparation and properties of avelumab are described in U.S. Patent Application Publication No. US 2014/0341917 Al.

In an embodiment, the PD-LI inhibitor is atezolizumab, also known as MPDL3280A or RG7446 (commercially available as TECENTRIQ from Genentech, Inc., a subsidiary of Roche Holding AG, Basel, Switzerland), or antigen-binding fragments, conjugates, or variants thereof. In an embodiment, the PD-LI inhibitor is an antibody disclosed in U.S. Patent No. 8,217,149, the disclosure of which is specifically incorporated by reference herein. In an embodiment, the PD-LI inhibitor is an antibody disclosed in U.S. Patent Application Publication Nos. 2010/0203056 Al, 2013/0045200 Al, 2013/0045201 Al, 2013/0045202 Al, or 2014/0065135 Al. The preparation and properties of atezolizumab are described in U.S. Patent No. 8,217,149.

### V. KITS AND ARTICLES OF MANUFACTURE

Provided herein are articles of manufacture and kits comprising the provided compositions, such as compositions containing T cells produced by any of the provided methods or containing or enriched for tumor-reactive T cells or T cells positive for a selection marker as described (e.g. CXCL13 and/or PD-1/CD39/TIGIT). In some embodiments, the compositions are produced by any of the provided methods.

Kits can optionally include one or more components such as instructions for use, devices and additional reagents (e.g., sterilized water or saline solutions for dilution of the compositions and/or reconstitution of lyophilized protein), and components, such as tubes, containers and syringes for practice of the methods. In some embodiments, the kits can further contain reagents for collection of samples, preparation and processing of samples, and/or reagents for quantitating the amount of one or more surface markers in a sample, such as, but not limited to, detection reagents, such as antibodies, buffers, substrates for enzymatic staining, chromagens or other materials, such as slides, containers, microtiter plates, and optionally, instructions for performing the methods. Those of skill in the art will recognize many other possible containers and plates and reagents that can be used in accord with the provided methods.

In some embodiments, the kits can be provided as articles of manufacture that include packing materials for the packaging of the cells, antibodies or reagents, or compositions thereof, or one or more other components. For example, the kits can contain containers, bottles, tubes, vial and any packaging material suitable for separating or organizing the components of the kit. The one or more containers may be formed from a variety of materials such as glass or plastic. In some embodiments, the one or more containers hold a composition comprising cells or an antibody or other reagents for use in the methods. The article of manufacture or kit herein may comprise the cells, antibodies or reagents in separate containers or in the same container.

In some embodiments, the one or more containers holding the composition may be a single-use vial or a multi-use vial, which, in some cases, may allow for repeat use of the composition. In some embodiments, the article of manufacture or kit may further comprise a second container comprising a suitable diluent. The article of manufacture or kit may further include other materials desirable from a commercial, therapeutic, and user standpoint, including other buffers, diluents, filters, needles, syringes, therapeutic agents and/or package inserts with instructions for use.

In some embodiments, the kit can, optionally, include instructions. Instructions typically include a tangible expression describing the cell composition, optionally, other components included in the kit, and methods for using such. In some embodiments, the instructions indicate methods for using the cell compositions for administration to a subject for treating a disease or condition, such as in accord with any of the provided embodiments. In some embodiments, the instructions are provided as a label or a package insert, which is on or associated with the container. In some embodiments, the instructions may indicate directions for reconstitution and/or use of the composition.

### VI. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known to the skilled person in this technical field. Reference to "about" a value or parameter herein includes (and describes) embodiments that are directed to that value or parameter *per se.* For example, description referring to "about X" includes description of "X".

The term "epitope" means a short peptide derived from a protein antigen, wherein the peptide binds to a major histocompatibility complex (MHC) molecule and is recognized in the MHC-bound context by a T cell. The epitope may bind an MHC class I molecule (e.g., HLA-A1 HLA-A2 or HLA-A3) or an MHC class II molecule.

The term "allogeneic" as used herein means a cell or tissue that is removed from one organism and then infused or adoptively transferred into a genetically dissimilar organism of the same species.

The term "autologous" as used herein means a cell or tissue that is removed from the same organism to which it is later infused or adoptively transferred.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, single chain antibody fragments, including single chain variable fragments (scFv), and single domain antibodies (e.g., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD. Among the provided antibodies are antibody fragments.

An "antibody fragment" or "antigen-binding fragment" refers to a molecule other than a conventional or intact antibody that comprises a portion of an conventional or intact antibody containing at least a variable region that binds an antigen. Examples of antibody fragments include but are not limited to Fv, single chain Fvs (sdFvs), Fab, Fab', Fab'-SH, F(ab')₂; diabodies; linear antibodies; an single-domain antibodies comprising only the V_{H} region (VHH).

As used herein, "bind," "bound" or grammatical variations thereof refers to the participation of a molecule in any attractive interaction with another molecule, resulting in a stable association in which the two molecules are in close proximity to one another. Binding includes, but is not limited to, non-covalent bonds, covalent bonds (such as reversible and irreversible covalent bonds), and includes interactions between molecules such as, but not limited to, proteins, nucleic acids, carbohydrates, lipids, and small molecules, such as chemical compounds including drugs.

The term "biological sample" means a quantity of a substance from a living thing or formerly living thing. Such substances include, but are not limited to, blood, (for example, whole blood), plasma, serum, urine, amniotic fluid, synovial fluid, endothelial cells, leukocytes, monocytes, other cells, organs, tissues, bone marrow, lymph nodes and spleen.

As used herein, "enriching" when referring to one or more particular cell type or cell population, refers to increasing the number or percentage of the cell type or population, e.g., compared to the total number of cells in or volume of the composition, or relative to other cell types, such as by positive selection based on markers expressed by the population or cell, or by negative selection based on a marker not present on the cell population or cell to be depleted. The term does not require complete removal of other cells, cell type, or populations from the composition and does not require that the cells so enriched be present at or even near 100 % in the enriched composition.

The term "concurrently" is used herein to refer to a procedure, such as an incubation, selection, enrichment or administration, involving two or more agents, where at least part of the particular procedure with one agent overlaps in time with at least a second agent.

The term "intermittently" is used herein to refer to a procedure, such as an incubation, selection, enrichment or administration, involving two or more agents, where the particular procedure involving each agent do not occur at regular intervals or are not continuous or stop and start repeatedly with periods in between.

The term "sequentially" is used herein to refer to a procedure, such as an incubation, selection, enrichment or administration, involving two or more agents, where the particular procedure involving each agent do not overlap in time.

As used herein, "isolated" or "purified with reference to a peptide, protein or polypeptide refers to a molecule which is substantially free of all other polypeptides, contaminants, starting reagents or other materials, or substantially free from chemical precursors or other chemicals when chemically synthesized. Preparations can be determined to be substantially free if they appear free of readily detectable impurities as determined by standard methods of analysis, such as high-performance liquid chromatography (HPLC), thin-layer chromatography (TLC) or capillary electrophoresis (CE), used by those of skill in the art to assess such purity, or sufficiently pure such that further purification would not detectably alter the physical and chemical properties of the substance.

As used herein, the term "recombinant" refers to a cell, microorganism, nucleic acid molecule, or vector that has been modified by introduction of an exogenous, such as heterologous, nucleic acid molecule, or refers to a cell or microorganism that has been altered such that expression of an endogenous nucleic acid molecule or gene is controlled, deregulated or constitutive, where such alterations or modifications may be introduced by genetic engineering. Genetic alterations may include, for example, modifications introducing nucleic acid molecules (which may include an expression control element, such as a promoter) encoding one or more proteins or enzymes, or other nucleic acid molecule additions, deletions, substitutions, or other functional disruption of or addition to a cell's genetic material. Exemplary modifications include those in coding regions or functional fragments thereof of heterologous or homologous polypeptides from a reference or parent molecule. The term "recombinant" also can refer to a protein product expressed from such a nucleic acid molecule or vector or from such cell or microorganism to which is introduced or modified with an exogenous nucleic acid.

As used herein, a composition refers to any mixture of two or more products, substances, or compounds, including cells. It may be a solution, a suspension, liquid, powder, a paste, aqueous, non-aqueous or any combination thereof.

As used herein, "optional" or "optionally" means that the subsequently described event or circumstance does or does not occur, and that the description includes instances where said event or circumstance occurs and instances where it does not. For example, an optionally substituted group means that the group is unsubstituted or is substituted.

The term "pharmaceutical composition" refers to a composition suitable for pharmaceutical use in a mammalian subject, often a human. A pharmaceutical composition typically comprises an effective amount of an active agent (e.g., cells, such as expanded in accord with the provided methods) and a carrier, excipient, or diluent. The carrier, excipient, or diluent is typically a pharmaceutically acceptable carrier, excipient or diluent, respectively.

A "pharmaceutically acceptable carrier" refers to a non-toxic solid, semisolid, or liquid filler, diluent, encapsulating material, formulation auxiliary, or carrier conventional in the art for use with a therapeutic agent that together comprise a "pharmaceutical composition" for administration to a subject. A pharmaceutically acceptable carrier is non-toxic to recipients at the dosages and concentrations employed and are compatible with other ingredients of the formulation. The pharmaceutically acceptable carrier is appropriate for the formulation employed.

Reference to "population of cells" herein is meant to refer to a number of cells that share a common trait. A population of cells generally contains a plurality of cells, such as greater than at or about 100 cells, at or about 1000 cells, and typically range from 1 × 10⁴ to 1 × 10¹⁰ in number.

The term "soluble" as used herein in reference to proteins, means that the protein is not bound, immobilized or attached to a particle, such as a cell or solid support, e.g. a bead. For example, a soluble protein includes a protein that is not bound as a transmembrane protein to the cell membrane of a cell. In some cases, solubility of a protein can be improved by linkage or attachment, directly or indirectly via a linker, to another molecule such as an Fc domain, which, in some cases, also can improve the stability and/or half-life of the protein. In some aspects, a soluble protein is an Fc fusion protein.

The term "specifically binds" as used herein means the ability of a protein, under specific binding conditions, to bind to a target protein such that its affinity or avidity is at least 10 times as great, but optionally 50, 100, 250 or 500 times as great, or even at least 1000 times as great as the average affinity or avidity of the same protein to a collection of random peptides or polypeptides of sufficient statistical size. A specifically binding protein need not bind exclusively to a single target molecule but may specifically bind to more than one target molecule. In some cases, a specifically binding protein may bind to a protein that has similarity in structural conformation with the target protein (e.g., paralogs or orthologs). Those of skill will recognize that specific binding to a molecule having the same function in a different species of animal (i.e., ortholog) or to a molecule having a substantially similar epitope as the target molecule (e.g., paralog) is possible and does not detract from the specificity of binding which is determined relative to a statistically valid collection of unique non-targets (e.g., random polypeptides). Solid-phase ELISA immunoassays, ForteBio Octet or Biacore measurements can be used to determine specific binding between two proteins. Generally, interactions between two binding proteins have dissociation constants (Kd) less than about 1×10⁻⁵ M, and often as low as about 1 × 10⁻¹² M. In certain aspects of the present disclosure, interactions between two binding proteins have dissociation constants of less than about 1×10⁻⁶ M, 1×10⁻⁷ M, 1×10⁻⁸ M, 1×10⁻⁹ M, 1×10⁻¹⁰ M, or 1×10⁻¹¹ M or less.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

As used herein, a "subject" is a mammal, such as a human or other animal, and typically is human. The subject can be male or female and can be any suitable age, including infant, juvenile, adolescent, adult, and geriatric subjects.

The terms "effective amount" or "therapeutically effective amount" refer to a quantity and/or concentration of a therapeutic composition, such as containing cells, e.g. expanded in accord with the provide methods, that when administered to a patient yields any manner in which the symptoms of a condition, disorder or disease or other indication, are ameliorated or otherwise beneficially altered. An effective amount for treating a disease or disorder may be an amount that relieves, lessens, or alleviates at least one symptom or biological response or effect associated with the disease or disorder, prevents progression of the disease or disorder, or improves physical functioning of the patient. In particular aspects, there is a statistically significant inhibition of disease progression as, for example, by ameliorating or eliminating symptoms and/or the cause of the disease. In the case of cell therapy, the effective amount is an effective dose or number of cells administered to a patient. In some embodiments the patient is a human patient.

As used herein, "disease," disorder" or "condition" refers to a pathological condition in an organism resulting from cause or condition including, but not limited to, infections, acquired conditions, genetic conditions, and characterized by identifiable symptoms. In particular, it is a condition where treatment is needed and/or desired.

The terms "treating," "treatment," or "therapy" of a disease or disorder as used herein mean slowing, stopping or reversing the disease or disorders progression, as evidenced by decreasing, cessation or elimination of either clinical or diagnostic symptoms, by administration of an immunomodulatory protein or engineered cells of the present invention either alone or in combination with another compound as described herein. "Treating," "treatment," or "therapy" also means a decrease in the severity of symptoms in an acute or chronic disease or disorder or a decrease in the relapse rate as for example in the case of a relapsing or remitting autoimmune disease course or a decrease in inflammation in the case of an inflammatory aspect of an autoimmune disease. "Preventing," "prophylaxis," or "prevention" of a disease or disorder as used in the context of this invention refers to the administration of an immunomodulatory protein or engineered cells expressing an immunomodulatory protein of the present invention, either alone or in combination with another compound, to prevent the occurrence or onset of a disease or disorder or some or all of the symptoms of a disease or disorder or to lessen the likelihood of the onset of a disease or disorder. For example, in the context of cancer, the terms "treatment" or, "inhibit," "inhibiting" or "inhibition" of cancer refers to at least one of: a statistically significant decrease in the rate of tumor growth, a cessation of tumor growth, or a reduction in the size, mass, metabolic activity, or volume of the tumor, as measured by standard criteria such as, but not limited to, the Response Evaluation Criteria for Solid Tumors (RECIST), or a statistically significant increase in progression free survival (PFS) or overall survival (OS).

The term "antigen" refers to a molecule that can induce an immune response. Typically, an antigen is a molecule that is capable of being bound by a recognition site on an immune molecule, such as an antibody or T cell receptor if presented by major histocompatibility complex (MHC) molecules. An antigen can have one or more epitopes in which each epitope that is part of the antigen can be bound by a recognition site of an antibody or TCR/MHC complex. In some embodiments, an antigen is capable of inducing a humoral immune response or a cellular immune response leading to the activation of B lymphocytes and/or T lymphocytes.

As used herein, a "tumor-associated antigen" or "tumor-specific antigen" refers to a protein or other molecule that is found only on cancer cells and not on normal cells.

As used herein, "neoantigen" refers to an antigen to which the immune system has not previously been exposed, such as one that arises by alteration of host antigens by viral infection, neoplastic transformation, drug metabolism or other manner. In particular aspects, a neoantigen is an antigen encoded by tumor-specific mutated genes or that is a new antigen that develops in a tumor cell.

The term "in vivo" refers to an event that takes plane in a mammalian subject's body.

The term "ex vivo" refers to an event that takes place on or in a tissue or cells from a mammalian subject but outside of the mammalian subject's body. Typically the event is carried out in an external environment. In particular aspects, an ex vivo procedure includes any in which an organ, cell or tissue is taken from a subject, typically a living body, for a treatment or procedure and then returned to the subject.

The term "in vitro" refers to an event that takes place in a test system, such as in a laboratory.

As used herein, a kit is a packaged combination that optionally includes other elements, such as additional reagents and instructions for use of the combination or elements thereof.

The term "package insert" is used to refer to instructions customarily included in commercial packages of therapeutic products, that contain information about the indications, usage, dosage, administration, combination therapy, contraindications and/or warnings concerning the use of such therapeutic products.

As used herein, an "article of manufacture" is a product that is made and, in some cases, that can be sold. In some embodiments, the term can refer to compositions contained in articles of packaging, such as in a container.

It is understood that aspects and embodiments of the invention described herein include "comprising," "consisting," and "consisting essentially of" aspects and embodiments.

### VII. EXEMPLARY EMBODIMENTS

Among the provided embodiments are:
1. A method for manufacturing tumor-reactive T cells, the method comprising: (a) selecting cells secreting chemokine (C-X-C motif) ligand 13 (CXCL13) and/or surface positive for C-X-C chemokine receptor type 5 (CXCR5), from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells.
2. The method of embodiment 1 wherein the method comprises selecting cells secreting CXCL13.
3. The method of embodiment 1 or 2, wherein the method comprises selecting cells surface positive for CXCR5.
4. The method of any of embodiments 1-3 wherein the selecting further comprises selecting cell surface positive for one or more further marker selected from , CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT, wherein the selecting cells secreting CXCL13 and/or surface positive for CXCR5 and the selecting cells surface positive for the one or more further marker is carried out simultaneously or sequentially in any order to obtain the selected cells.
5. The method of embodiment 4, wherein the one or more further marker is PD-1, CD39 and/or TIGIT.
6. The method of any of embodiments 4 or 5, wherein the one or more further marker is PD-1, CD39 and TIGIT.
7. The method of any of embodiments 1-6, , further comprising selecting, optionally by positive selection or negative selection, T cells surface positive for a T cell marker selected from CD3, CD4 or CD8, wherein the selecting cells surface positive for the T cell marker and the selecting cells secreting CXCL13 and/or surface positive for CXCR5 is carried out simultaneously or sequentially in any order to obtain the selected cells.
8. A method for manufacturing tumor-reactive T cells, the method comprising: (a) selecting cells surface positive for PD-1, CD39 and TIGIT from an input sample comprising T cells from a subject that has a tumor to obtain selected cells from the sample; and (b) performing an expansion by culture of the selected cells with one or more T-cell stimulating agent of lymphocytes under conditions to produce a population of expanded T cells.
9. The method of embodiment 8, wherein the selecting further comprises selecting cell surface positive for one or more further marker selected from CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT, wherein the selecting for cells surface positive for PD-1/CD39/TIGIT and the selecting cells surface positive for the further marker is carried out simultaneously or sequentially in any order to obtain the selected cells
10. The method of any of embodiments 8 or 9, wherein the selecting further comprises selecting cells secreting chemokine (C-X-C motif) ligand 13 (CXCL13), wherein the selecting for cells surface positive for PD-1/CD39/TIGIT and the selecting cells secreting CXCL13 is carried out simultaneously or sequentially in any order to obtain the selected cells.
11. The method of any of embodiments 8-10, further comprising selecting, optionally by positive selection or negative selection, T cells surface positive for a T cell marker selected from CD3, CD4 or CD8, wherein the selecting cells surface positive for the T cell marker and the selecting cells surface positive for PD-1/CD39/TIGIT is carried out simultaneously or sequentially in any order to obtain the selected cells.
12. The method of any of embodiments 1-11, wherein the input sample comprising T cells is from the peripheral blood or from a tumor.
13. The method of any of embodiments 1-12, wherein the input sample comprises tumor infiltrating lymphocytes.
14. The method of any of embodiments 1-13, wherein the input sample comprising T cells is derived from a resected tumor.
15. The method of any of embodiment 14, wherein the input sample comprising T cells is a single cell suspension processed by homogenization and/or enzymatic digestion of one or more tumor fragments from the resected tumor.
16. The method of any of embodiments 14 or 15, wherein the input sample comprising T cells is a single cell suspension processed by homogenization and enzymatic digestion of one or more tumor fragments from the resected tumor.
17. The method of any of embodiments 15 or 16, wherein the enzymatic digestion is by incubation with a collagenase, optionally collagenase IV or collagenase I/II.
18. The method of any of embodiments 1-13, wherein the one or more T-cell stimulating agent of lymphocytes is an anti-CD3 agent and/or a recombinant cytokine selected from one or more of IL-2, IL-7, IL-15, IL-21, IL-25, and IL-23.
19. The method of any of embodiments 1-18, wherein at least one of the one or more T-cell stimulating agent is recombinant IL-2.
20. The method of any of embodiments 1-19, wherein culture with the one or more T-cell stimulating agent further comprises an apoptosis inhibitor.
21. The method of any of embodiments 1-20, wherein the one or more T-cell stimulating agent is one or more first T-cell stimulating agent and the performing the expansion is a first expansion, wherein the method further comprises performing a second expansion by culture of the first expanded T cell population with one or more second T-cell stimulating agent under conditions to produce a second expanded population of T cells, wherein the second population of expanded T cells is for use as a therapeutic cell composition.
22. The method of any of embodiments 1-21, wherein the one or more T-cell stimulating agent is one or more first T-cell stimulating agent and the performing the expansion is a first expansion, wherein the method further comprises: (c) co-culturing the first expanded T cell population in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor; and (d) performing a second expansion by culture of the reactive T cell population with one or more second T-cell stimulating agent under conditions to produce a second population of expanded T cells, wherein the second population of expanded T cells is for use as a therapeutic cell composition.
23. The method of embodiments 1-21, wherein the one or more T-cell stimulating agent is one or more first T-cell stimulating agent and the performing the expansion is a first expansion, wherein the method further comprises: (c) co-culturing the first expanded T cell population in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor; (d) selecting, from the reactive T cell population, cells positive for one or more marker associated with reactive T cells comprising native T cell receptors reactive to mutation encoding peptides of the tumor, to produce an enriched population of the reactive T cells; and (e) performing a second expansion by culture of the enriched population of the reactive T cells with one or more second T-cell stimulating agent under conditions to produce a second population of expanded T cells, wherein the second population of expanded T cells is for use as a therapeutic cell composition.
24. The method of embodiment 23, wherein the one or more marker is a marker of a T cell exhaustion marker.
25. The method of embodiment 23 or 24, wherein the one or more marker is cell surface CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT.
26. The method of any of embodiments 23-25, wherein the one or marker is PD-1, CD39 and TIGIT.
27. The method of embodiment 23, wherein the one or more marker is secreted CXCL13.
28. The method of any of embodiments 23-27, further comprising selecting, optionally by positive selection or negative selection, T cells surface positive for a T cell marker selected from CD3, CD4 or CD8, wherein the selecting cells surface positive for the T cell marker and the selecting cells positive for a marker associated with reactive T cells is carried out simultaneously or sequentially in any order to obtain the enriched population of reactive T cells.
29. A method for manufacturing tumor-reactive T cells, the method comprising: (a) processing a biological sample containing T cells obtained from a donor subject that has a tumor to produce an input sample comprising T cells; (b) performing a first expansion by culture of the input sample comprising T cells with one or more first T-cell stimulating agent of lymphocytes under conditions to produce a first population of expanded T cells; (c) selecting cells secreting chemokine (C-X-C motif) ligand 13 (CXCL13) and/or surface positive for C-X-C chemokine receptor type 5 (CXCR5)from the first population of expanded cells to produce a selected population; and (d) performing a second expansion by culture of the selected population with one or more second T-cell stimulating agent under conditions to produce a second expanded population of T cells, wherein the second population of expanded T cells is for use as a therapeutic cell composition.
30. A method for manufacturing tumor-reactive T cells, the method comprising: (a) processing a biological sample containing T cells obtained from a donor subject that has a tumor to produce an input sample comprising T cells; (b) performing a first expansion by culture of the input sample comprising T cells with one or more first T-cell stimulating agent of lymphocytes under conditions to produce a first population of expanded T cells; (c) selecting cells secreting chemokine (C-X-C motif) ligand 13 (CXCL13) and/or surface positive for C-X-C chemokine receptor type 5 (CXCR5), from the first population of expanded cells to produce a selected population; (d) co-culturing the selected population in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor; and (e) performing a second expansion by culture of the reactive T cell population with one or more second T-cell stimulating agent under conditions to produce a second expanded population of T cells, wherein the second population of expanded T cells is for use as a therapeutic cell composition.
31. A method for manufacturing tumor-reactive T cells, the method comprising: (a) processing a biological sample containing T cells obtained from a donor subject that has a tumor to produce an input sample comprising T cells; (b) performing a first expansion by culture of the input sample comprising T cells with one or more first T-cell stimulating agent of lymphocytes under conditions to produce a first population of expanded T cells; (c) co-culturing the first population of expanded cells in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor; (d) selecting cells secreting chemokine (C-X-C motif) ligand 13 (CXCL13) and/or surface positive for C-X-C chemokine receptor type 5 (CXCR5), from the reactive T cell population to produce a selected population; and (e) performing a second expansion by culture of the selected population with one or more second T-cell stimulating agent under conditions to produce a second expanded population of T cells, wherein the second population of expanded T cells is for use as a therapeutic cell composition.
32. The method of any of embodiments 29-31, wherein the method comprises selecting cells secreting CXCL13.
33. The method of any of embodiments 29-32, wherein the method comprises selecting cells surface positive for CXCR5.
34. The method of any of embodiments 29-33, wherein the selecting further comprises selecting cell surface positive for one or more further marker selected from CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT, wherein the selecting cells secreting CXCL13 and/or surface positive for CXCR5 and the selecting cells surface positive for the one or more further marker is carried out simultaneously or sequentially in any order to produce the selected population.
35. The method of embodiments 34, wherein the one or more further marker is PD-1, CD39 and/or TIGIT.
36. The method of any of embodiments 34 or 35, wherein the one or more further marker is PD-1, CD39 and TIGIT.
37. The method of any of embodiments 29-36, further comprising selecting, optionally by positive selection or negative selection, T cells surface positive for a T cell marker selected from CD3, CD4 or CD8, wherein the selecting cells surface positive for the T cell marker and the selecting cells secreting CXCL13 and/or surface positive for CXCR5 is carried out simultaneously or sequentially in any order to produce the selected cell population.
38. A method for manufacturing tumor-reactive T cells, the method comprising: (a) processing a biological sample containing T cells obtained from a donor subject that has a tumor to produce an input sample comprising T cells; (b) performing a first expansion by culture of the sample comprising T cells with one or more first T-cell stimulating agent of lymphocytes under conditions to produce a first population of expanded T cells; (c) selecting cells surface positive for PD-1, CD39 and TIGIT from the first population of expanded cells to produce a selected population; and (d) performing a second expansion by culture of the selected population with one or more second T-cell stimulating agent under conditions to produce a second expanded population of T cells, wherein the second population of expanded T cells is for use as a therapeutic cell composition.
39. A method for manufacturing tumor-reactive T cells, the method comprising: (a) processing a biological sample containing T cells obtained from a donor subject that has a tumor to produce an input sample comprising T cells; (b) performing a first expansion by culture of the sample comprising T cells with one or more first T-cell stimulating agent of lymphocytes under conditions to produce a first population of expanded T cells; (c) selecting cells surface positive for PD-1, CD39 and TIGIT from the first population of expanded cells to produce a selected cell population; (d) co-culturing the selected cell population in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor; and (e) performing a second expansion by culture of the reactive T cell population with one or more second T-cell stimulating agent under conditions to produce a second expanded population of T cells, wherein the second population of expanded T cells is for use as a therapeutic cell composition.
40. A method for manufacturing tumor-reactive T cells, the method comprising: (a) processing a biological sample containing T cells obtained from a donor subject that has a tumor to produce an input sample comprising T cells; (b) performing a first expansion by culture of the sample comprising T cells with one or more first T-cell stimulating agent of lymphocytes under conditions to produce a first population of expanded T cells; (c) co-culturing the first population of expanded cells in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor; (d) selecting cells surface positive for PD-1, CD39 and TIGIT from the reactive T cell population to produce a selected cell population; and (e) performing a second expansion by culture of the selected cell population with one or more second T-cell stimulating agent under conditions to produce a second expanded population of T cells, wherein the second population of expanded T cells is for use as a therapeutic cell composition.
41. The method of any of embodiments 38-40, wherein the selecting further comprises selecting cell surface positive for one or more further marker selected from CD107a, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), TIM-3, or LAG-3, wherein the selecting for cells surface positive for PD-1/CD39/TIGIT and the selecting cells surface positive for the further marker is carried out simultaneously or sequentially in any order to produce the selected cell population
42. The method of any of embodiments 38-41, wherein the selecting further comprises selecting cells secreting chemokine (C-X-C motif) ligand 13 (CXCL13), wherein the selecting for cells surface positive for PD-1/CD39/TIGIT and the selecting cells surface positive for the further marker is carried out simultaneously or sequentially in any order to produce the selected cell population.
43. The method of any of embodiments 38-42, further comprising selecting, optionally by positive selection or negative selection, T cells surface positive for a T cell marker selected from CD3, CD4 or CD8, wherein the selecting cells surface positive for the T cell marker and the selecting cells surface positive for PD-1/CD39/TIGIT is carried out simultaneously or sequentially in any order to produce the selected cell population.
44. The method of any of embodiments 29-43, wherein the biological sample is a peripheral blood sample or a tumor sample.
45. The method of any of embodiments 29-44, wherein the input sample comprising T cells comprises tumor infiltrating lymphocytes (TILs).
46. The method of any of embodiments 29-45, wherein the input sample comprising T cells is derived from a resected tumor.
47. The method of any of embodiments 29-46, wherein the biological sample is a resected tumor from the subject and the input sample comprising T cells is one or more tumor fragments from the resected tumor.
48. The method of embodiment 47, wherein the one or more tumor fragments are 1-8 mm in diameter.
49. The method of embodiment 47 or 48, wherein the one or more tumor fragments are seeded for the first expansion at about 1 tumor fragment per 2 cm² .
50. The method of embodiment 46, wherein the input sample comprising T cells is a single cell suspension processed by homogenization and/or enzymatic digestion of one or more tumor fragments from the resected tumor.
51. The method of embodiment 46 or 50, wherein the input sample comprising T cells is a single cell suspension processed by homogenization and enzymatic digestion of one or more tumor fragments from a resected tumor.
52. The method of embodiments 50-51, wherein the enzymatic digestion is by incubation with a collagenase, optionally collagenase IV or collagenase I/II.
53. The method of any of embodiments 15-17 and 50-52, wherein the input sample comprising T cells is seeded for expansion at about 5 × 10⁵ to at or about 2 × 10⁶ total cells per 2 cm².
54. The method of any of embodiments 21-53, wherein the one or more first T-cell stimulating agent of lymphocytes is an anti-CD3 agent and/or a recombinant cytokine selected from one or more of IL-2, IL-7, IL-15, IL-21, IL-25, and IL-23.
55. The method of any of embodiments 21-54, wherein at least one of the one or more first T-cell stimulating agent is recombinant IL-2.
56. The method of any of embodiments 21-55, wherein the one or more first T cell stimulating agent comprises an anti-CD3 antibody, optionally OKT3, optionally wherein the concentration of the anti-CD3 antibody is at or about 50 ng/mL.
57. The method of any of embodiments 21-56, wherein the one or more first T-cell stimulating agent further comprises an apoptosis inhibitor.
58. The method of any of embodiments 21-57, wherein the one or more second T-cell stimulating agent of lymphocytes is an anti-CD3 agent and/or a recombinant cytokine selected from one or more of IL-2, IL-7, IL-15, IL-21, IL-25, and IL-23.
59. The method of any of embodiments 21-58, wherein at least one of the one or more second T-cell stimulating agent is recombinant IL-2.
60. The method of any of embodiments 18, 19, 54, 55, 58-59, wherein the concentration of recombinant IL-2 is from 100 IU/mL to 6000 IU/mL.
61. The method of any of embodiments 18, 19, 54, 55 and 58-60, wherein the concentration of recombinant IL-2 is from 300 IU/mL to 1000 IU/mL, optionally wherein the concentration of recombinant IL-2 is at or about 300 IU/mL.
62. The method of any of embodiments 21-61, wherein the one or more second T cell stimulating agent comprises an anti-CD3 antibody, optionally OKT3, optionally wherein the concentration of the anti-CD3 antibody is at or about 50 ng/mL.
63. The method of any of embodiments 21-62, wherein the one or more second T-cell stimulating agent further comprises an apoptosis inhibitor.
64. The method of any of embodiments 20, 57, or 63, wherein the apoptosis inhibitor reduces apoptosis induced by CD95 (Fas), optionally wherein the apoptosis inhibitor specifically binds CD95 (Fas) or CD95 ligand (Fas ligand).
65. The method of embodiment 64, wherein the apoptosis inhibitor is an antibody or antigen-binding fragment, optionally wherein the apoptosis inhibitor is an anti-Fas antibody or an anti-Fas ligand antibody.
66. The method of embodiment 64, wherein the apoptosis inhibitor is a fusion protein comprising the extracellular domain of CD95 (Fas) or a specific binding fragment thereof that binds to CD95 ligand (Fas ligand) fused to an Fc immunoglobulin domain, optionally wherein the apoptosis inhibitor is APG101 or CAN008.
67. The method of embodiments 20, 57, or 63, wherein the apoptosis inhibitor inhibits caspase activation or activity, optionally wherein the caspase is a caspase 2, a caspase 8, a caspase 9, a caspase 10, a caspase 3, a caspase 6 or a caspase 7, optionally wherein the caspase is a caspase 3.
68. The method of any of embodiments 20, 57, 63, or 67, wherein the apoptosis inhibitor is selected from the group consisting of NAIP (neuronal apoptosis inhibitory protein; BIRC1), cIAP1 and cIAP2 (cellular inhibitor of apoptosis 1 and 2; BIRC2 and BIRC3, respectively), XIAP (X-chromosome binding IAP; BIRC4), survivin (BIRC5), BRUCE (Apollon; BIRC6), livin (BIRC7) and Ts-IAP (testis-specific IAP; BIRC8).
69. The method of any of embodiments 20, 57, 63, 67, and 68, wherein the apoptosis inhibitor is emericasan.
70. The method of any of embodiments 22-28, 30-37 and 39-69, wherein the antigen presenting cells are dendritic cells, mononuclear phagocytes, B lymphocytes, endothelial cells or thymic epithelium.
71. The method of any of embodiments 22-28, 30-37 and 39-70, wherein the antigen presenting cells are dendritic cells.
72. The method of any of embodiments 22-28, 30-37 and 39-71, wherein the antigen presenting cells are autologous to the subject.
73. The method of any of embodiments 22-28, 30-37 and 39-72, wherein the one or more non-native peptide comprises an individual peptide or a pool of peptides.
74. The method of any of embodiments 22-28, 30-37 and 39-73, wherein the one or more non-native peptides are loaded on antigen presenting cells by transfection of in vitro transcribed synthesized minigene constructs encoding for the one or more non-native peptides in tandem, wherein the transcribed minigene constructs generate individual peptides.
75. The method of any of embodiments 22-28, 30-37 and 39-74, where the one or more non-native peptides are loaded on antigen presenting cells by peptide pulse, optionally by electroporation.
76. The method of embodiment 75, wherein the one or more non-native peptide is 5-30 amino acids, optionally 12-25 amino acids, optionally at or about 25 amino acids in length.
77. The method of any of embodiments 75 or 76, wherein:
   the one or more non-native peptides are a pool of peptides and the concentration of peptides in the pool of peptides for the peptide pulse is between at or about 0.001 µg/mL and at or about 40 µg/mL, 0.01 µg/mL and at or about 40 µg/mL, at or about 0.1 µg/mL and at or about 40 µg/mL, at or about 1 µg/mL and at or about 40 µg/mL, at or about 0.01 µg/mL and at or about 10 µg/mL or at or about 1 µg/mL and at or about 10 µg/mL; or
   the one or more non-native peptides is an individual peptide and the concentration of individual peptides for the peptide pulse is between at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.0001 µg/mL and at or about 1 µg/mL, at or about 0.0001 µg/mL and at or about 0.1 µg/mL, at or about 0.0001 µg/mL and at or about 0.1 µg/mL or at or about 0.0001 µg/mL and at or about 0.01 µg/mL.
78. The method of any of embodiments 75-77, wherein the concentration of individual peptides of the one or more non-native peptide, on average, is from at or about 0.00001 µg/mL to at or about 0.01 µg/mL.
79. The method of any of embodiments 75-78, wherein the concentration of individual peptide of the one or more non-native peptide, on average, is from at or about 0.0001 µg/mL and at or about 0.001 µg/mL.
80. The method of any of embodiments 22-28, 30-37 and 39-79, wherein the co-culture ratio of antigen presenting cells to T Cells is between 20:1 and 1:1, between 15:1 and 1:1, between 10:1 and 1:1, between 5:1 and 1:1, between 2.5:1 and 1:1, between 1:20 and 1:1, between 1:15 and 1:1, between 1:10 and 1:1, between 1:5 and 1:1, or between 1:2.5 and 1:1.
81. The method of any of embodiments 22-28, 30-37 and 39-80, wherein the co-culture ratio of dendritic cells to T Cells is between 5:1 and 1:5 or is between 3:1 and 1:3, optionally is or is about 1:1.
82. The method of any of embodiments 22-28, 30-37 and 39-81, wherein the co-culture ratio of antigen presenting cells to T cells is or is about 1:1.
83. The method of any of embodiments 22-28, 30-37 and 39-82, wherein the co-culturing is for 2 hours to 24 hours.
84. The method of any of embodiments 22-28, 30-37 and 39-83, wherein the co-culturing is for at or about 6 hours.
85. The method of any of embodiments 1-84, where the selecting cells is performed using a florescence based cell sorter.
86. The method of embodiment 85, wherein the fluorescence based cell sorter is an automated high-throughput flow cytometry sorter, optionally FX500 cell sorter or Miltenyi Tyto cell sorter.
87. The method of embodiment 85 or 86, wherein the selection is by 1 run, 2 runs, 3 runs or 4 runs by the fluorescence based cell sorter.
88. The method of any of embodiments 85-87, wherein the selection is performed at rate between 10,000 and 100,000 cells/ second using a florescent based disposable fluidics cell sorter.
89. The method of any of embodiments 1-20, wherein the culturing for expansion is for 7 to 35 days.
90. The method of any of embodiments 1-20 and 89, wherein the culturing for expansion is 7 to 21 days, optionally 7 to 14 days.
91. The method of any of embodiments 1-90, wherein the culturing is carried out in a closed system.
92. The method of any of embodiments 21-88, wherein the culturing for the first expansion is for 7 to 21 days, optionally 7 to 14 days.
93. The method of any of embodiments 21-88 and 92, wherein the culturing for the first expansion is carried out in a closed system using a gas permeable culture vessel.
94. The method of any of embodiments 21-88 and 92, wherein the culturing for the first expansion is carried out in a closed system using a bioreactor.
95. The method of any embodiments 21-88 and 92-94, wherein the culturing for the second expansion is for 7 to 21 days, optionally 7 to 14 days.
96. The method of any of embodiments 21-88 and 92-95, wherein the culturing for the second expansion is performed in a gas permeable culture vessel.
97. The method of any of embodiments 21-88 and 92-96, wherein the culturing for the second expansion is performed using a bioreactor.
98. The method of any of embodiments 1-97, wherein the tumor is a tumor of an epithelial cancer.
99. The method of any of embodiments 1-98, wherein the tumor is a tumor of a melanoma, lung squamous, lung adenocarcinoma, bladder cancer, lung small cell cancer, esophageal cancer, colorectal cancer (CRC), cervical cancer, head and neck cancer, stomach cancer or uterine cancer.
100. The method of any of embodiments 1-99, wherein the tumor is a melanoma.
101. The method of any of embodiments 1-99, wherein the tumor is a colorectal cancer (CRC).
102. The method of any of embodiments 1-98, wherein the tumor is a tumor of a non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.
103. The method of any of embodiments 1-102, further comprising harvesting cells produced by the method for formulation as the therapeutic composition.
104. The method of embodiment 103, comprising formulating the harvested cells with a cryoprotectant.
105. A composition comprising tumor-reactive T cells produced by the method of any of embodiments 1-104.
106. The composition of embodiment 105, wherein the composition comprises a cryoprotectant.

### VIII. EXAMPLES

The following examples are included for illustrative purposes only and are not intended to limit the scope of the invention.

### Example 1 Assessment of Tumor Processing Methodologies in Obtaining a Population of Tumor-derived T-Cells

Tumors from patients with colorectal cancer (CRC) or melanoma were processed as described below and resultant infiltrating T-cell populations were analyzed for cell count viability.

### A. Colorectal Cancer

Tumors were sourced from primary tumors in patients with CRC and shipped overnight in HypoThermosol at 4 °C. Tumors were either processed as fragment or single cell suspension (SCS) cultures.

For fragment cultures, tumors were minced into fragments 1 - 8 mm in diameter, and each 1 - 8 mm fragment was placed into a well of culture vessel, either a gas-permeable 24-well culture plate or conventional 6-well plate, in the presence of Roswell Park Memorial Institute (RPMI) containing 5% human serum or serum free OpTmizer medium (ThermoFisher). Media was supplemented with either 300 or 6000 IU/mL recombinant IL-2, and also contained gentamicin at 10 µg/ml, Immune Cell Serum Replacement (ICSR, ThermoFisher) at between 2% and 5% according to manufacturer's recommendations, and a final concentration of 2.0 mM of a L-alanyl-L-glutamine dipeptide form of glutamine (GlutaMAX Supplement; Thermofisher). Fragment cultures were maintained and monitored visually until a cell count was performed between approximately day 5 and 11 of culture.

For SCS cultures, tumors were also minced into fragments 1 - 8 mm in diameter. Fragments were then homogenized in a closed system using the Miltenyi GentleMACS in the presence or absence of an enzyme to digest the tumor, either an enzyme cocktail from the Miltenyi Tumor Dissociation Kit, human (part 130-095-929) used according to the manufacturer's recommendation, Collagenase I/II blend (Nordmark, Collagenase NB 4G Proved Grade, part: S1746503) or Collagenase IV (Worthington Biomedical part: LS004130) at 1 mg/ml or 5 mg/ml. Fragments designated for SCS with homogenization and enzyme digestion were incubated with the enzyme cocktail or collagenase for a total of 60 minutes. Immediately following the generation of SCS, cell counts and viability assessments were performed on the NC-200 Automated Cell Counter (ChemoMetec).

As shown in **FIG. 3A****,** SCS cultures with or without enzymatic digestion yielded more total viable cells (TVC) than was obtained following culture from CRC tumor fragments. Depicted in **FIG. 3B** the percent of viable cells was similar in cultures generated from fragments or SCS generated by homogenization in the presence of enzymes.

### B. Melanoma

Tumors were sourced from primary tumors in patients with melanoma and shipped overnight in HypoThermosol at 4 °C. Cells were cultured similarly as described above.

Briefly, for fragment cultures tumors were minced into fragments 1 - 8 mm in diameter, and each 1-8 mm fragment was cultured in a well of a gas-permeable 24-well culture plate or conventional 6-well plate in the presence of RPMI containing 5% human serum or serum free OpTmizer medium supplemented with recombinant IL-2 at a concentration of 300 IU/ml or 6000 IU/ml. The media also contained gentamicin at 10 µg/ml, and a final concentration of 2.0 mM of a L-alanyl-L-glutamine dipeptide form of glutamine (GlutaMAX Supplement; Thermofisher). Fragment cultures were maintained and monitored visually until a cell count was performed between day 5 and 9 of culture.

To generate SCS cultures, tumor fragments were homogenized using the Miltenyi GentleMACS in the presence or absence of an enzyme, which included Collagenase IV at a concentration of 1 mg/mL or 5 mg/mL or Collagenase I/II blend at 1 mg/mL or 5 mg/mL (Nordmark, Collagenase NB 4G Proved Grade, part: S 1746503). As above, cell count and viability assessments were performed immediately following the generation of SCS using the NC-200 Automated Cell Counter (Chemometec).

Depicted in **FIG. 4A****,** cultures generated from melanoma tumor fragments yielded more total viable cells than SCS generated by homogenization and dissociation with enzymes. As shown in **FIG. 4B****,** fragment cultures also had a higher percent viable cells than cells from SCS cultures, irrespective of enzymatic homogenization.

### Example 2 Assessment of T Cell Expansion Kinetics of Tumor-Derived Cells

Tumors were processed as described in Example 1 to generate 1-8 mm in diameter fragments or SCS cultures, which were then incubated under conditions to expand T cell populations present within the tumor. Cultures were grown under various tested conditions in the presence of recombinant IL-2 as described below in order to assess cellular expansion. Among the conditions that were tested were the effect of the type of culture plate, culture media, and concentration of IL-2 on cell expansion.

### A. Culture Conditions

Single cell suspensions (SCSs) were obtained by homogenization and enzyme digestion of primary tumors from donor patients with CRC or melanoma. As described in Example 1, cells were cultured in a conventional 6-well plate or a gas-permeable 24-well culture plate. Where possible, multiple conditions from each donor were initiated and averaged (error bars represent ± standard deviation). For 6-well plates, cells were seeded between 250,000 and 1,000,000 cells/mL and for gas-permeable 24-well plates cells were seeded between 5,000 and 750,000 cells/mL. In both cases, the cells were seeded in either RPMI containing 5% human serum or serum free OpTmizer medium with recombinant IL-2 supplemented at a concentration of IL-2 of 300 IU/mL or 6000 IU/mL. The media also contained gentamicin at 10 µg/ml and a final concentration of 2.0 mM of a L-alanyl-L-glutamine dipeptide form of glutamine (GlutaMAX Supplement; Thermofisher). The cells were incubated for up to 31 days, typically 14 to 21 days, wherein 50% of cellular media was exchanged every other day beginning on the 5th day of culture.

For expansion from tumor fragments, an individual 1 - 8 mm tumor fragment, obtained as described in Example 1 from primary tumors of donor patients with CRC or melanoma, was placed in a well of a gas-permeable 24-well culture plate or a 6-well plate, and cultured in either RPMI containing 5% human serum or serum free OpTmizer medium with recombinant IL-2 supplemented at a concentration of 300 IU/mL or 6000 IU/mL. The media also contained gentamicin at 10 µg/ml, and a final concentration of 2.0 mM of a L-alanyl-L-glutamine dipeptide form of glutamine (GlutaMAX Supplement; Thermofisher). The cells were incubated for up to 31 days, such as typically 14 to 21 days, wherein 50% of cellular media was exchanged every other day beginning on the 5th day of culture.

For all conditions, cell counts were performed approximately every other day using the NC-200 Automated Cell Counter (Chemometec) and samples were collected for fluorescence-activated cell sorting (FACS). After completion of the expansion phase (e.g. day 14 - 31), cells were washed in PBS then cryopreserved in the presence of a cryoprotectant. Cryopreservation was carried out using CoolCell devices (Corning) or the VIA Freeze (GE Healthcare).

### B. Results

### 1. Growth Curves

Growth curves of expansion of SCS obtained from tumor fragments from CRC donor patients following expansion culture in different culture vessels are shown in **FIGS. 5A and 5B****.** The results shown are from cultures incubated with recombinant IL-2 at both 300 IU/mL or 6000 IU/mL concentrations in either media type, but are separated based on the source of starting cells. As shown, it was possible to expand tumor-derived cells from SCS from tumors of CRC donors under these conditions. In some donors, expansion greater than 2-fold and even as high as 10-fold or more was observed in this initial expansion phase of cells obtained directly from CRC tumors.

Expansion achieved from SCS compared to tumor fragments from CRC tumor biopsy products were assessed. As shown in **FIGS. 5C and 5D**, it was possible to expand cells from CRC tumors under these conditions whether extracted and cultured as fragments or as SCS. However, in general, a greater expansion was achieved in CRC cultures extracted as SCS, as evidenced by higher total cell numbers (**FIG. 5C**) and fold expansion (**FIG. 5D**) compared to culturing cells extracted via fragments.

Growth curves of expansion of cells cultured as extracted tumor fragments, or as SCSs from tumor fragments, from different melanoma donors following expansion culture in different culture vessels are shown in **FIGS. 6A and 6B****.** The results shown are from cultures incubated with recombinant IL-2 at both 300 IU/mL or 6000 IU/mL concentrations and in either media type, but are separated based on the source of starting cells. As shown, substantial expansion was observed in melanoma cultures extracted as tumor fragments in either culture vessel, whereas less expansion was observed for melanoma cells cultured as SCS.

Consistent with previous observations, tumor cells from certain donors were not amenable to expansion, irrespective of tumor type. This is indicative of inherent variability in expansion potential between donors and furthermore between tumor fragments of the same donor tumor. Larger scale methods in which tumor fragments from a donor patient are pooled during culture would be expected to mitigate against intra-tumor variability by combining tumor fragments from the same donor tumor.

### 2. Growth Assessment by Cellular Media

Expanded cultures generated as described above in RPMI media containing either 5% human serum or a serum replacement formulation (OpTmizer media) were compared after expansion for between 14 and 21 days. The results shown are from cultures incubated with recombinant IL-2 at both 300 IU/mL or 6000 IU/mL concentrations, and in either type of culture vessel, but are separated based on the type of media. The results for CRC tumors are from culture of SCS obtained from tumor fragments (**FIG. 7A and 7B**), while the results from melanoma tumors are from culture of tumor fragments (**FIG. 8A and 8B**).

For both tumor types, an increase in total cell number (**FIG. 7A** **and** **FIG. 8A**) and fold-expansion (**FIG. 7B** **and** **FIG. 8B**) was observed by culture in either the 5% human serum or serum replacement media for both tumor types. In the samples tested, there was a trend to improved expansion using OpTmizer media, as evidenced by higher overall cell number at the end of the initial expansion phase (**FIG. 7A** **and** **FIG. 8A**).

### 3. IL-2 Concentration

The effect of different IL-2 concentrations during expansion from the different tumor types was compared. The cultures were expanded as described above in RPMI media containing either 5% human serum or a serum replacement formulation (OpTmizer media) for between 14 and 31 days, such as between 14 and 21 days, in either 300 IU/mL or 6000 IU/mL recombinant IL-2. The results shown are from cultures incubated in the presence of either media type, and in either type of culture vessel, but are separated based on the IL-2 concentration. The results for CRC tumors are from culture of SCS obtained from tumor fragments (**FIG. 9A and 9B**), while the results from melanoma tumors are from culture of tumor fragments (**FIG. 10A and 10B**).

For both tumor types, the results showed similar expansion of cells grown in either a high or low concentration of IL-2, as evidenced by similar total cell numbers after expansion (**FIG. 9A** **and** **FIG. 10A**) as well as fold expansion (**FIG. 9B** **and** **FIG. 10B**). These data support the observation that doses of IL-2 of about 300 IU/mL support expansion, and that a high dose of IL-2, such as 6000 IU/mL, is not necessary for cellular expansion for either CRC or melanoma cultures.

Together, the results show that while expansion can be donor and moreover, tumor sample dependent, CRC tumor infiltrating T-cells were grown successfully from SCS cultures and melanoma infiltrating T-cells from fragment cultures across multiple donors. It was similarly observed that for both melanoma and CRC derived T-cell cultures, that the addition of high concentrations of IL-2 did not result in an appreciably distinct expansion response when compared with a lower dose.

### Example 3 Assessment of Anti-CD3 Stimulation on Expansion of Tumor-Derived Cells

Cells processed from melanoma tumor fragments as described in Examples 1 and 2, were cultured in the presence or absence of 50 ng/mL OKT3, a human anti-CD3 monoclonal antibody. Cell cultures were carried out to between 14 and 31 days, such as between 14 and 21 days, in either a conventional 6-well plate or a gas permeable culture plate with RPMI or OpTmizer media. The cultures also were supplemented with 300 or 6000 IU/mL recombinant IL-2, 10 µg/mL gentamicin and a final concentration of 2.0 mM of a L-alanyl-L-glutamine dipeptide form of glutamine (GlutaMAX Supplement; Thermofisher). About 50% of cellular media was exchanged every other day beginning on the 5th day of culture as described previously. Cells were then counted using the NC-200 Automated Cell Counter (Chemometec).

The results shown are from cultures incubated with recombinant IL-2 at both 300 IU/mL or 6000 IU/mL concentrations, different medias, and in either type of culture vessel, but are separated based on the presence of absence of anti-CD3 stimulation. Cells from Donor 6 were tested in both the presence or absence of anti-CD3 stimulation, and demonstrated 2-4 fold expansion in all conditions, with a 13-fold expansion observed in OpTmizer media supplemented with 300 IU/mL IL-2 with incubation in the absence of anti-CD3 stimulation (-OKT3). The results shown in **FIGS. 11A-11B** demonstrate that CD3 stimulation via OKT3 antibody supported T cell expansion, but did not substantially impact total cell number (**FIG. 11A**) or fold expansion (**FIG. 11B**). These data are consistent with a finding that anti-CD3 stimulation (e.g. via OKT3 antibody) may not be necessary for the expansion of cells from tumor cultures.

### Example 4 Assessment of Post-Stimulation CD4+ and CD8+ Activation Markers

T-cells from three healthy donors were thawed, rested overnight in OpTmizer media supplemented with 300 IU/mL recombinant IL-2, and then activated using 50 ng/mL OKT3, a human anti-CD3 monoclonal antibody. Specific markers of activation on the CD4+ and CD8+ cell populations were measured using flow cytometry over a time course of 3 - 48 hours. Specifically, the following markers were assessed: CD38 and CD39 (**FIG. 12A** **and** **FIG. 13A**), CD134 and CD137 (**FIG. 12B** **and** **FIG. 13B**), and CD69 and CD90 (**FIG. 12C** **and** **FIG. 13C**).

Results for expression of activation markers on the surface of CD8+ cells are shown in **FIGS. 12A-12C**, which demonstrates the kinetics of upregulation of markers on CD8+ T cells in the 48 hours following CD3 stimulation with OKT3, compared to culture in the absence of OKT3. In some cases, some basal level of the markers can be seen on day 0 before stimulation. As shown, all assessed markers were upregulated to some extent during this time course, with the highest percentage of cells being upregulated for markers CD38 (**FIG. 12A**), CD134 (**FIG. 12B**) and CD69 (**FIG. 12C**) during this study.

Results for expression of activation markers on the surface of CD4+ cells are shown in **FIGS. 13A-13C**, which demonstrates the kinetics of upregulation of markers on CD4+ T cells in the first 48 hours following CD3 stimulation with OKT3 compared to the culture in the absence of OKT3 . As shown, all assessed markers were upregulated to some extent during this time course, with the highest percentage of cells being upregulated for markers CD38 (**FIG. 13A**), CD137 (**FIG. 13B**) and CD69 (**FIG. 13C**) during this study.

Taken together, these data support that expression of the above markers can be used as upregulation markers to select for T cells that have been activated, including under conditions of activation that would be expected to stimulate signaling via the TCR-CD3 complex as would occur following co-culture with antigen presenting cells presenting neoantigenic peptides.

### Example 5 Determination of Donor Cell Phenotype and Cellular Viability

T cells were sourced from primary tumors in patients with melanoma or CRC as described in Example 1. Cells from the tumors were extracted either as tumor fragments or as a SCS as described in Example 1, and then were assessed for T cell phenotype by flow cytometry.

For tumor fragments, each 1-8 mm fragment was placed into a well of culture vessel, either a gas-permeable 24-well culture plate or conventional 6-well plate, and incubated for between 5 and 11 days in the presence of RPMI containing 5% human serum or serum free OpTmizer medium (ThermoFisher). Media was supplemented with either 300 or 6000 IU/mL recombinant IL-2, and also contained gentamicin at 10 µg/ml, and a final concentration of 2.0 mM of a L-alanyl-L-glutamine dipeptide form of glutamine (GlutaMAX Supplement; Thermofisher). The incubation also was carried out with or without 50 ng/mL of anti-CD3 antibody OKT3. Fragment cultures were monitored visually until it was determined that a cell count could be performed (typically between day 5 and 9 of culture), and then cells were stained ad analyzed by flow cytometry for T cell markers.

Alternatively, for SCS cultures, tumors were minced into fragments 1 - 8 mm in diameter, then homogenized in the presence or absence of Collagenase IV (Worthington Biomedical part: LS004130) at 1 mg/ml or 5 mg/ml or Collagenase NB4G Proved Grade (Nordmark Biomedicals; Catalog No. S 1746503) at 1 mg/mL. After incubation with enzyme for about 90 minutes, cells were immediately stained and analyzed by flow cytometry for T cell markers.

The gating hierarchies for flow cytometric analysis were designed as follows: first, the percentage of CD3+ cells from a parent population of total cell events were recorded, followed by the percentage of viable CD4+ cells from the CD3+ parent populations and next the percentage of viable CD8+ cells from the same parent CD3+ population. The memory T-cell populations (Tern) were then calculated based on their respective CD4+ and CD8+ parent populations. Thus, CD4/Tem was determined from the parent population of viable CD4+ cells, while CD8/Tem was determined from the parent populations of viable CD8+ cells. Thus, the results, as depicted in **FIG. 12****,** are the percentage of CD3+ cells from a parent population of total cell events recorded, which were sorted in a hierarchy into subpopulations as a percentage of the respective parent population in the hierarchy. **FIG. 14** depicts the percentage of viable cells positive for select T cell markers in single cell suspensions immediately after extraction of tumor fragments by homogenization and enzyme digestion from an exemplary CRC donor (donor 1).

The percentage of CD3+ cells was compared in SCS samples that had been extracted by homogenization only (no collagenase) or by homogenization following digestion with a low concentration (1 mg/mL) or a high concentration (5 mg/mL) of collagenase. Results from a second CRC and a melanoma patient are shown in **FIG. 15A** and **FIG. 15B****,** respectively. As shown in **FIG. 15A****,** the results demonstrate an increased recovery of CD3+ T cells in SCSs from a CRC donor following homogenization and digestion with a low concentration of collagenase. Although the percent of CD3+ cells in SCSs from a melanoma door was lower, the results also demonstrate that homogenization and digestion with a low concentration of collagenase yielded the highest percentage of CD3+ T cells (**FIG. 15B**). Taken together, these observations demonstrate relative high purity of cells from SCS from melanoma tumors can be achieved and may support that SCS is a viable source of melanoma derived CD3+ cells.

The percentage of CD3+ cells in SCSs extracted from tumors of an additional exemplary CRC donor also was assessed. In addition, in this same donor, the percentage of CD3+ T cells in the SCSs immediately following homogenization and digestion was compared to (1) the percentage of CD3+ cells after culture of SCSs with 300 IU/mL IL-2 (low) or 6000 IU/mL IL-2 (high) for 6 days, or (2) the percentage of CD3+ cells following culture of individual tumor fragments for up to 6 days with 300 IU/mL IL-2 (low) or 6000 IU/mL (high) in the presence of absence of CD3 stimulation (OKT3 antibody). As shown in **FIG. 15C****,** the percent of CD3 cells in baseline (day 0) SCS was substantially higher than the percent of CD3+ cells in cultures obtained following culture of tumor fragments with IL-2 or OKT3 for 6 days. Similar results from culture of tumor fragments from two additional donors was observed, in which the percentage of CD3+ cells in cultures obtained following culture of CRC-derived tumor fragments with IL-2 and/or OKT3 for 11 days (**FIG. 15D**) or 9 days (**FIG. 15E**) also generally showed a low yield when extracting tumor cells from CRC tumor fragments under various assessed conditions. These results are consistent with a finding that SCSs from tumor biopsies of CRC patients may be more capable of providing an increased number of T cells for expansion than cells obtained from culture of tumor fragments.

In contrast to the results from culture of tumor fragments for CRC patients, **FIG. 16** shows that a high percentage of CD3+ T cells can be obtained from culture of melanoma tumor fragments under various conditions, such as presence of low (300 IU/mL) or high (6000 IU/mL) concentrations of IL-2, presence of absence of CD3 stimulation (OKT3) or different media. The results depicted in **FIG. 16** are from a Day 0 culture. These results are consistent with a finding that culture of tumor fragments from melanoma patients may be more capable of providing an increased number of T cells for expansion than cells obtained from SCSs of tumor biopsies.

### Example 6 Quantification of Activation of Tumor Derived T Cells Following Co-Culture with Antigen Presenting Cells

T cells were sourced from primary tumors in patients with melanoma or CRC as tumor fragments, as described in Example 1. Following 5 days of culture in serum free OpTmizer medium (ThermoFisher) supplemented with 300 IU/mL recombinant IL-2, gentamicin at 10 µg/ml, Immune Cell Serum Replacement (ThermoFisher) at between 2% and 5% according to manufacturer's recommendations, and a final concentration of 2.0 mM of a L-alanyl-L-glutamine dipeptide form of glutamine (GlutaMAX Supplement; Thermofisher), tumor derived cells were washed with OpTmizer medium before being centrifuged at 300 × g for 5 minutes and suspended at 2 × 10⁶ cells/mL. Cells were then seeded into a conventional 6-well culture plate at 10,000,000 cells/well.

In a parallel culture, antigen presenting Dendritic Cells (DCs) were differentiated from PBMCs obtained from the same patient (autologous) as sourced T cells. Cryovials of frozen PBMC isolated from apheresed donors were thawed from liquid nitrogen storage in a ten-fold volume of 1X DPBS (Gibco) and counted (NucleoCounter NC200). After washing, cells were immediately used for CD14 microbead positive selection (MACS Miltenyi) according to manufacturer kit instructions. Purified CD14 (monocyte) cells were counted, the cells were resuspended in DendriMACs (MACS Miltenyi) and seeded at a density of 0.5-2 × 10⁶ cells per mL in the appropriate culture flask. GM-CSF (100 ng/mL) and IL-4 (20 ng/mL) were added to cultures to promote differentiation into immature dendritic cells. Monocytes were cultured and differentiated for a total of 5 days, with a 50% addition of medium equal to 50% of the starting amount of medium on day 2.

Coding transcripts of tumor specific peptides were identified autologously for each patient from whole exome sequencing and RNA sequencing as described in Parkhurst, Maria R., et al. "Unique neoantigens arise from somatic mutations in patients with gastrointestinal cancers." Cancer discovery 9.8 (2019): 1022-1035. Whole-exome sequencing (WES) of patient samples was performed on snap-frozen, unfixed, tumor tissue and normal peripheral blood cells (normal source). Alignments of sequences from tumor vs. normal samples were performed using novoalign MPI from novocraft (http://www.novocraft.com/) to human genome build hg19. Duplicates were marked using Picard's MarkDuplicates tool. Insertion deletion realignment and base recalibration was carried out according to the GATK best practices workflow (https://www.broadinstitute.org/gatk/). Post cleanup of data, pileup files were created using samtools mpileup (http://samtools.sourceforge.net) and Varscan2, (http://varscan.sourceforge.net), SomaticSniper (http://gmt.genome.wustl.edu/packages/somatic-sniper/), Strelka (https://sites.google.com/site/strelkasomaticvariantcaller/), and Mutect (https://www.broadinstitute.org/gatk/). VCF files were merged using GATK Combine Variants tools and annotated using Annovar (http://annovar.openbioinformatics.org). Variants (mutations) present in patient tumors were then annotated using Annovar (http://annovar.openbioinformatics.org).

The following filters were used to generate an initial list of putative mutations for evaluation: (1) a tumor and normal coverage of greater than 10, (2) a variant allele frequency (VAF) of 7% or above, (3) variant read counts of 4 or above, (4) and two of the four callers identifying mutations. For insertions and deletions, the same cutoffs were used except only a single caller identifying the mutation was required to pass filters, as these were only called by varscan and strelka. Tables of amino acid sequences corresponding the mutant residue joined to the 12 amino acids encoded by regions upstream and downstream of single nucleotide variants (SNVs) (Nmers) were generated for those variants which passed the four filters. For frame-shifted transcripts, sequences were translated until a stop codon was generated in either the normal coding region or in the 3' un-translated region. The Integrative Genomics Viewer (IGV, Broad Institute), which allows mapped alignments to be visualized, was then used to carry out manual curation of the variant calls. Changes were made to the sequences of Nmers when manual curation revealed non-synonymous changes resulting from additional somatic variants or germline variants present within transcripts encoding the Nmers. Variants inferred from reads containing multiple mis-matched nucleotides, insertion/deletions mapping to different locations in different reads, and variants corresponding to frequent SNPs were flagged for removal.

Variants that were detected in more than one patients tumor but in fewer than 2.5% of total tumors were flagged but included in the list of variants that passed. Variant transcripts only annotated in the ENSEMBL database generally represent unverified coding regions and were also removed. Variants flagged as being known single nucleotide polymorphisms or were present in multiple tumors were not automatically removed but were further evaluated using IGV, as removing potential false positives, which are unlikely to encode products recognized by T cells, was less critical than removing candidates that could represent false negatives.

These sequencing data were then used to generate a peptide pool representing mutated peptide associated with the tumor and wild-type peptide associated with non-diseased peripheral blood cells.

Synthetic peptides were synthesized via Fmoc chemistry. For indels, 25 amino acid peptides were synthesized overlapping by 10 amino acids based on the translation of the frame-shifted sequence until the next stop codon. In some cases, peptides of minimal epitopes were synthesized. Peptides were dissolved in DMSO and mixed in equal volumes.

Differentiated DCs were loaded with a varying number and concentration of peptides from a peptide pool identified as described above before they were added to the tumor derived culture at several ratios of Tumor Cells: DC. DCs and tumor derived cells were then co-cultured at 37°C for 6 hours at 5% CO₂ before the culture was gently agitated and cells in suspension recovered. Recovered cells were then sorted via flow cytometry for activated T-cells using markers of T cell activation 4-1BB and OX40.

**FIG. 17A and 17B** show tumor derived T cell activation over a peptide range of 20 to 0.1 ng/mL. As shown in **FIG. 17A****,** T cell co-culture with DCs loaded with each of three peptide concentrations tested resulted in readily detectable levels of 4-1BB/OX40+ T cells, including as high as approximately 80% at 1 ng/mL peptide. Increase in T cell activation marker expression as compared to cells cultured with unloaded DCs is shown in **FIG. 17B****,** where 0.1 ng/mL peptide resulted in the largest delta but all three concentrations of peptide resulted in a positive fold change. These data demonstrate that lower peptide concentrations less than 20 ng/mL may result in increased upregulation of T cell activation markers (upregulation markers) following co-culture.

**FIG. 18** similarly depicts tumor derived T cell activation as a function of 41BB/OX40 expression in studies in which DCs were pulsed with one or two peptides for surface presentation during co-culture. Shown in **FIG. 18A** and again in **FIG. 18B** as fold change, DCs which were loaded with only one peptide were more markedly more efficient at activating T cells in co-culture.

As shown in FIG. 19, markers of T cell activation 41BB and OX40 were substantially upregulated when tumor derived T cells were co-cultured with DCs at a ratio of 1:2 (T cells: DC) as compared to 1:1.

### Example 7 Enrichment and Recovery of Activated T-Cells via Cell Sorting

T-cells from a healthy donor were isolated by immunoaffinity-based selection and then cryopreserved. The T cells were thawed and rested overnight, and then were activated with 50 ng/mL OKT3 for 24 - 48 hours prior to staining with anti-CD4 FITC (BD), anti-CD8 PerCPCy.5.5 (BD), anti-CD134 (Beckman Couleter), and anti-CD137 (MACs Miltenyi). Cells were brought to a concentration of approximately 20 × 10⁶ cells/mL and sorted using the BD FACSAriaII at a sort rate of approximately 15,000 events per second. A gate was drawn around cells expressing CD134, CD137, or both CD134 and CD137 and sorted into a single population. This was the positive sorted population. Cells lacking both CD134 and CD137 expression were sorted into a separate population. This was the negative sorted population. After sorting, cells from the positive and negative sorted populations and the unsorted population were analyzed on an alternative flow cytometer to verify purity and assess recovery rates.

As shown in **FIG. 20****,** the unsorted tumor derived T cell population (pre-sort) were compared to the positive sorted population which was collected after co-culture with mutant peptide loaded autologous dendritic cells as described previously in Example 6 and sorted into 41BB/OX40 positive populations. It was observed that this gating strategy resulted in an increased enrichment for percent reactive TCR for three donors (**FIG. 20A**) and average Class I reactivity (**FIG. 20B**).

Total cell recovery from cell sorting is shown with respect to total cell input in **FIG. 21A**. Similarly seen in **FIG. 21B**, percent recovery from two independent runs was approximately 80%. The results demonstrate that it is possible to obtain a high recovery of cells after selection and sorting of cells positive for upregulation markers.

**FIG. 22** depicts CD4+ population purity via flow cytometry of healthy donor T cells activated with OKT3 and stained as described above. Cells were first gated on CD4+, then the population expressing the highest intensity of CD134+ was next gated and the outputs displayed showing CD4+ vs. CD8+ and CD137+ vs. CD134+. These data support the use of these markers for gating a high purity population of tumor infiltrating T cells.

### Example 8 Post-Sort Expansion of Activated Tumor Derived T Cells

T cells sourced from a primary CRC tumor were processed as described in Example 1, and then were co-cultured with peptide presenting dendritic cells, using methods as described in Example 6. Briefly, isolated tumor infiltrating lymphocytes were cultured with autologous DCs that were loaded to express peptide associated with healthy tissue (Wild-type, WT), peptide associated with tumor tissue (Mutant), or were not loaded with peptide at all (No peptide). A control subpopulation of T cells were cultured without DCs (Unactivated). After the co-culture, the cells were sorted via the fluorescence enabled Sony FX500 based on surface expression of activation markers 4-1BB and OX40.

Cells were then seeded into a gas permeable 24 well culture plate at 250,000-1,000,000 cells/cm2 in serum free OpTmizer medium supplemented with recombinant IL-2 at a concentration of 300 IU/mL, gentamicin at 10 µg/mL, and 2.0 mM of a L-alanyl-L-glutamine dipeptide form of glutamine (GlutaMAX Supplement; Thermofisher). Cells were incubated for a total of 7 days with 50% of the media exchanged every other day beginning on culture day 5. Cell counts were performed using NC-200 Automated Cell Counter (Chemometec) on each culture day.

As shown in **FIG. 23A** and again in **FIG. 23B** as fold expansion, each tumor infiltrating lymphocyte (TIL) T cell population tested underwent measurable expansion between culture days 3 and 5 and continued to trend upwards at the 7 day conclusion of the culture period. Tumor infiltrating T cells cultured with DCs loaded with mutant tumor-associated peptide reached the highest total cell number over the course of the experiment.

Using the data above, a theoretical mathematical model shown in **FIG. 23C** was created to predict the relationship between the number of cells recovered after sorting and the expected number of cells present in culture following the expansion phase.

### Example 9 Monte Carlo Modeling Ex Vivo Expansion of Tumor Derived T Cells

In complement to the deterministic point analysis in Example 8, a first probabilistic Monte Carlo simulation was designed to forecast the number of tumor infiltrating lymphocytes resultant from a first expansion as described in Example 2. Monte Carlo simulations of likely total viable and total reactive T cell numbers post extraction and a first expansion were run by substituting a probability distribution for two factors of inherent uncertainty, recovery efficiency and fold expansion capacity. The results were iteratively calculated tens of thousands of times as a normal distribution wherein a mean value of cells recovered was defined for low and mid recovery, and a mean value for fold change in expansion was defined for low, mid, and high expansion potentials. Distributions were then calculated for total viable T cell number as well as total reactive T cells.

For the initial Monte Carlo simulations, wherein probable T cell outputs are calculated for a first expansion, test cases were run to model low recovery/low expansion, mid recovery/low expansion, mid recovery/mid expansion, and mid recovery/high expansion conditions. Values for the mean and standard deviation for both recovery and expansion variables were assigned as follows: (1) low recovery was defined as culturing a total of 20 million viable cells from the processed tumor at a standard deviation of 6 million, (2) mid recovery was defined as 50 or 60 million cells with a standard deviation of 15 million, (3) a low first fold expansion was defined as 50 fold with a standard deviation of 11, (4) mid expansion was defined as 75 fold with a standard deviation of 15, and (5) high expansion was defined as 500 fold with a standard deviation of 160.

Data for each test case from the first Monte Carlo simulations are displayed in **Table E1** below.

| **Table E1: Monte Carlo Simulations for a first expansion** | | | | |
|---|---|---|---|---|
| | | ***Total Viable Tumor Infiltrating T Cells*** | | ***Total Reactive Tumor Infiltrating T Cells*** |
| **Test Conditions** | **Assumptions** | **Statistics** | **Forecast Values** | **Forecast Values** |
| Low Recovery/ Low Expansion | Mean = 20 | Mean | 1.99 ×10⁷ | 1.59 ×10⁶ |
| | Std. Dev = 6 | Median | 1.99 ×10⁷ | 1.51 ×10⁶ |
| | Mean = 50 | Probability Distribution (10-90^{th} Percentile) | 1.22 ×10⁷ -2.76 ×10⁷ | 7.48 ×10⁵ -2.53 ×10⁶ |
| | Std Dev = 11 | | | |
| Mid Recovery/Low Expansion | Mean = 50 | Mean | 4.99 ×10⁷ | 3.99 ×10⁶ |
| | Std. Dev = 15 | Median | 5.00 ×10⁷ | 3.81 ×10⁶ |
| | Mean = 50 | Probability Distribution (10-90^{th} Percentile) | 3.05 ×10⁷ -6.93 ×10⁷ | 1.85 ×10⁶ -6.34 ×10⁶ |
| | Std Dev = 11 | | | |
| Mid Recovery/Mid Expansion | Mean = 60 | Mean | 6.00 ×10⁷ | 4.81 ×10⁶ |
| | Std. Dev = 15 | Median | 5.99 ×10⁷ | 4.63 ×10⁶ |
| | Mean = 75 | Probability Distribution (10-90^{th} Percentile) | 4.07 ×10⁷ - 7.91 ×10⁷ | 4.07 ×10⁷ - 7.91 ×10⁷ |
| | Std Dev = 15 | | | |
| Mid Recovery/High Expansion | Mean = 60 | Mean | 4.78 ×10⁷ | 5.99 ×10⁷ |
| | Std. Dev = 15 | Median | 4.60 ×10⁷ | 6.00 ×10⁷ |
| | Mean = 500 | Probability Distribution (10-90^{th} Percentile) | 4.07 ×10⁷ - 7.94 ×10⁷ | 2.43 ×10⁶ -7.40 ×10⁶ |
| | Std Dev = 160 | | | |

Using these data, a second set of Monte Carlo simulations were designed to predict the final number of reactive tumor infiltrating lymphocytes following co-culture with APCs, sorting via flow cytometry, and a second expansion as described in Example 8. A fixed value for percentage of reactive T cells present in the population of total T cells cultured from either tumor fragments or SCS was assigned to a mean of 8% and a standard variation of 2.50. Following tens of thousands of iterative calculations, data for each test case from the second Monte Carlo simulations are depicted in Table E2 below.

| **Table E2: Monte Carlo Simulations for a second and final expansion** | | | |
|---|---|---|---|
| | | ***Total Reactive Tumor Infiltrating T Cells*** | |
| **Test Conditions** | **Assumptions** | **Statistics** | **Forecast Values** |
| Low Recovery/ Low Expansion | Mean = 20 | Mean | 7.96 ×10⁷ |
| | Std. Dev = 6 | Median | 7.34 ×10⁷ |
| | Mean = 50 | Probability Distribution (10-90^{th} Percentile) | 3.41 ×10⁷ -1.34 ×10⁸ |
| | Std Dev = 11 | | |
| Mid Recovery/Low Expansion | Mean = 50 | Mean | 2.00 ×10⁸ |
| | Std. Dev = 15 | Median | 1.85 ×10⁸ |
| | Mean = 50 | Probability Distribution (10-90^{th} Percentile) | 8.44 ×10⁷ -3.33 ×10⁸ |
| | Std Dev = 11 | | |
| Mid Recovery/Mid Expansion | Mean = 60 | Mean | 3.61 ×10⁸ |
| | Std. Dev = 15 | Median | 3.39 ×10⁸ |
| | Mean = 75 | Probability Distribution (10-90^{th} Percentile) | 1.68 ×10⁸ -5.80 ×10⁸ |
| | Std Dev = 15 | | |
| Mid Recovery/High Expansion | Mean = 60 | Mean | 2.39 ×10⁹ |
| | Std. Dev = 15 | Median | 2.19 ×10⁹ |
| | Mean = 500 | Probability Distribution (10-90^{th} Percentile) | 9.57 ×10⁸ -4.06 ×10⁹ |
| | Std Dev = 160 | | |

Recovery and expansion potential of tumor infiltrating reactive T cells from a first expansion following tumor processing or a second expansion following downstream co-culture with APCs are factors which are inherently variable across donors and within a tumor cell population. The expected range of T cell numbers generated by the process described here is contained within the 10^{th} and 90^{th} percentiles. Cell numbers below the 10^{th} percentile are unlikely to be generated and will likely not result in a usable drug product. Therefore, the observations from the Monte Carlo simulations in **Tables E1 and E2** support that in all scenarios between the 10^{th} and 90^{th} percentiles, given a range of levels of variability for expansion potentials, the method described herein will likely provide a robust T cell output that is approximate to the number of cells which would be required for therapeutic dosing.

### Example 10 Assessment of Tumor-reactive TCR Enrichment by IFN-gamma production and TCR clonality

T cells sourced from primary tumor of patients with ovarian cancer (Sample A), CRC (Sample B), or melanoma (Sample C), were processed from tumor fragments as described in Example 1. Following the initial expansion, T cells were then co-cultured with peptide presenting, autologous dendritic cells for 6 hours using methods substantially as described in Example 6. For the co-culture, autologous DCs were loaded with either a mutant single long peptide (e.g. 25 mer) unique to the patient tumor, or a wild-type single long peptide that was not-mutated compared to normal sample from the patient. After the co-culture, tumor-reactive T cells were enriched by staining the cells for expression of 4-1BB (CD137) and/or OX40 (CD134) and sorting the cells by fluorescence-activated cell sorting (FACS). Cells that were positive for either or both of 41BB and OX40 were collected as the "positive" population (also called "mutant enriched" population) and cells that were double negative for 41BB and OX40 were collected as the "negative" population (also called "wild-type unenriched" population).

The mutant and wild-type T cell populations were then cultured for 16 hours in media alone or under conditions to stimulate IFN-gamma secretion. Unsorted, unenriched T cells (bulk T cells) from the co-culture that had not been sorted based on 41BB and OX40 expression were included as a pre-selection control and were similarly stimulated. Culture supernatant was collected and IFN-gamma secretion levels were determined by ELISA.

The percentage of T cells in the sorted population expressing a TCR reactive to the peptide neo-epitope was determined by single cell TCR sequencing . TCR clonality in the T cell populations also was determined by single cell RNA- sequencing for the TCR-beta and TCR-alpha chains.

### 1. Sample A (Ovarian Cancer)

The mutant and wild-type enriched T cell populations, or control bulk T cells, produced from Sample A tumor cells were cultured for 16 hours in media alone or were stimulated by culture with anti-CD28 and anti-CD49d antibodies along with either the minimal peptide epitope (8mer) corresponding to the mutant peptide (neo-epitope) or the wild-type peptide from the respective patient tumor. As shown in **FIG. 24A****,** bulk T cells exhibited improved reactivity, as evidenced by increased IFN-gamma secretion, following culture with the neo-epitope compared to culture media alone. The ability to produce IFN-gamma was further increased in the mutant enriched T cell population that was stimulated with the neo-epitope, but no difference was observed in the wild-type enriched T cell population following stimulation in media alone versus stimulation with the neo-epitope conditions. Additionally, the wild-type unenriched T cell population still included some degree of neo-antigen reactive T cells, as evidenced by their upregulation of IFN-gamma secretion compared to media alone. This data indicates that the bulk T cells following co-culture contain a neoantigen reactive population, which is enriched by sorting based on expression of 41BB and OX40. Further, the results also demonstrate the specificity of neo-antigen enrichment.

Analysis of neoepitope-specific TCRs by RNA sequencing and flow cytometry showed an enrichment of TCR "A" neoantigen-specific TCRs in the mutant enriched T cell populations with 17% neoantigen-specific TCRs compared to 2% in the initial bulk T cell population or 0.1% in the wild-type enriched T cell population **(****FIG. 24B****).** The TCR clonality of T cells in the unselected population (wild-type enriched T cell population) compared to selected population (mutant enriched T cell population) is shown in **FIG. 24C****,** which shows that the incoming TCR diversity is high the unsorted T cell population and that enrichment of unique TCR clones is achieved in the selected population. **FIG. 24D** demonstrates that the Pre- (bulk) and post-sort cell populations contain CD4 and CD8 cells, indicating that class I and class II reactive cells are present in the enriched population,

### 2. Sample B (CRC Patient)

The mutant and wild-type enriched T cell populations, or control bulk T cells, produced from Sample B tumor cells were cultured for 16 hours in media alone or were stimulated in response to a general TCR stimulation using an anti-CD3 antibody (OKT3). As shown in **FIG. 25A****,** all T cell populations displayed functionality (i.e. IFNγ production) after coculture and sorting in response to the general TCR stimulation.

Analysis of neoepitope-specific TCRs showed an enrichment of neoantigen "B"-specific TCRs in the mutant enriched T cell populations with 71% neoantigen-specific TCRs compared to 42% in the initial bulk T cell population or 17% in the wild-type enriched T cell population **(****FIG. 25B****).** Compared to the bulk T cells after co-culture, this represents an approximate 1.7-fold enrichment in the tumor-reactive T cells in the sorted T cell population, and an approximate 2.5-fold reduction in tumor-reactive T cells in the non-sorted T cell population. The TCR clonality of T cells in the unselected population (wild-type enriched T cell population) compared to selected population (mutant enriched T cell population) is shown in **FIG. 25C****,** which shows that the incoming TCR diversity is high in the unsorted T cell population (807 unique TCR clones) and that enrichment of unique TCR clone is achieved in the selected population (64 unique TCR clones). **FIG. 25D** demonstrates that the pre- (bulk) and post-sort cell populations contain CD4 and CD8 cells, indicating that class I and class II reactive cells are present in the enriched population.

### 3. Sample C (Melanoma Patient)

T cells in the mutant and wild-type enriched T cell populations, or control bulk T cells, produced from Sample C tumor cells were assessed for neoepitope-specific TCRs by RNA sequencing and flow cytometry and TCR clonality. The results showed an enrichment of neoantigen "C"-specific TCRs in the mutant enriched T cell populations with 33% neoantigen-specific TCRs compared to 5% in the initial bulk T cell population or 4% in the wild-type enriched T cell population **(****FIG. 26A****).** Compared to the bulk T cells after co-culture, this represents an approximate 7-fold enrichment in the tumor-reactive T cells in the sorted T cell population, and no enrichment in tumor-reactive T cells in the non-sorted T cell population. The TCR clonality of T cells in the unselected population (wild-type enriched T cell population) compared to selected population (mutant enriched T cell population) is shown in **FIG. 26B****,** which shows that the incoming TCR diversity is high in the unsorted T cell population (182 unique TCR clones) and that enrichment of unique TCR clone is achieved in the selected population (15 unique TCR clones). **FIG. 26C** demonstrates that the pre- (bulk) and post-sort cell populations contain CD4 and CD8 cells, indicating that class I and class II reactive cells are present in the enriched population.

### 4. Conclusion

Together, the results show that the incoming TCR diversity is high in the unsorted T cell population (e.g. 100-900 TCRs). This unsorted population produces a low level of IFNgamma (e.g. 5-25 pg/mL). After sorting of the TCR population based on activation markers, e.g. OX40/41BB, the TCR population is enriched in a reactive population of TCR's (e.g. 15-64 TCRs) that produce higher IFNgamma (e.g. 65.3-98.6 pg/mL) than the unsorted and negative sorted population (5 pg/mL) of TCRs. The results indicate that this is specific activation consistent with enrichment of tumor-reactive T cells as it is not seen in the wild-type, unsorted co-cultures.

### Example 11 Assessment of Effect of T cell Adjuvants on T cell Viability

Cells were expanded from PBMC derived from Ficoll gradient separation from three healthy donor's apheresis material. PBMCs were seeded at 2 x 10⁶ cells/ml in OpTmizer cell culture media supplemented with 300 IU/mL recombinant IL-2, gentamicin at 10 µg/ml, Immune Cell Serum Replacement (ThermoFisher) at between 2 and 5%, and a final concentration of 2.0 mM of a L-alanyl-L-glutamine dipeptide form of glutamine (GlutaMAX Supplement; Thermofisher) and activated with human anti-CD3 antibody OKT3 antibody for 48 hours. Because the studies were carried out in healthy donors, stimulation of the T cells was carried out with anti-CD3 (OKT3) stimulation in order to mimic conditions present in the tumor microenvironment (TME).

Cells were next seeded into gas permeable 100M culture vessels and expanded for 7-14 days to achieve a large bank of T cells, and cryopreserved. Previously expanded human T cells from three healthy donors were thawed and then seeded into 96-well culture plates to a final cell density of 5 × 10⁵ cells/mL with a test adjuvant agent at a range of concentrations in OpTmizer cell culture media supplemented with 300 IU/mL recombinant IL-2, gentamicin at 10 µg/ml, Immune Cell Serum Replacement (ThermoFisher) at between 2 and 5%, and a final concentration of 2.0 mM of a L-alanyl-L-glutamine dipeptide form of glutamine (GlutaMAX Supplement; Thermofisher). Half of wells were additionally supplemented with 50 ng/mL OKT3, a human anti-CD3 monoclonal antibody. In total, 15 test adjuvant agents were tested for their impact on cell viability in the presence and absence of anti-CD3 activation (see **Table E3).** Cells were cultured for 6 days total, including a 50% medium exchange on culture day 3, and monitored for total viable CD3+ cell count.

| **Table E3: Compounds and concentrations used in high throughput screen** | |
|---|---|
| Compound | Concentration |
| ***Antibodies*** | |
| Tavolixizumab, Oxelumab, Ipilimumab, Tocilizumab, Urelumab, Pembrolizumab, Varlilumab, anti-GITR MK-1248 | 0.625 µg/mL - 10 µg/mL |
| FasL (Human anti-FasL antibody) | 0.625 µg/mL - 10 µg/mL |

| ***Small Molecule Inhibitors*** | |
|---|---|
| Z-VAD-FMK | 1.5625 µM - 25 µM |
| NVP-HSP990 | 15.625 nM-250 nM |

| ***Cytokines*** | |
|---|---|
| IL-7 | 62.5 IU/ml - 1000 lU/ml |
| IL-15 | 62.5 IU/ml - 1000 IU/ml |
| IL-21 | 62.5 IU/ml - 1000 lU/ml |
| IL-23 | 62.5 ng/ml - 1000 ng/ml |
| IL-25 | 62.5 ng/ml - 1000 ng/ml |
| IL-27 | 62.5 ng/ml - 1000 ng/ml |
| IL-35 | 62.5 ng/ml - 1000 ng/ml |
| *DMSO (Control)* | *0% - 1% v*/*v* |

Total viable CD3+ cell count for cells grown in the absence and presence of OKT3 stimulation are shown in **FIGS. 27A-C** and **FIGS 28A-C**, respectively. The results shown are for the following concentrations of adjuvant: 10 µg/mL for tested antibodies (Tavolixizumab, Oxelumab, Ipilimumab, Tocilizumab, Urelumab, Pembrolizumab, Varlilumab, anti-GITR MK-1248, anto-human FasL); 25 µM for Z-VAD-FMK pan-caspase inhibitor; 250 nM for HSP inhibitor NVP-HSP990; and 1000 IU/mL for cytokine (IL-7, IL-15, IL-21, IL-23, IL-25, IL-27, or IL-35). ).

Overall, toxicity was not observed for any of the compounds tested, indicating these compounds would not be detrimental to TIL manufacturing. While inherent donor variability was observed, treatment with anti-PD1 antibody Pembrolizumab, anti-OX40L antibody Oxelumab, and pan-caspase inhibitor Z-VAD-FMK resulted in consistently higher viable cell counts than the DMSO treatment control, irrespective of activation status.

Dose response curves for IL-7 and IL-15, shown in **FIG. 29A** and **FIG. 29B**, respectively, showed a dose dependent response, wherein cell number increased with increasing concentration. These data support that IL-7 and IL-15 at this range of tested concentrations may be beneficial for potentiating total T cell number during culture.

### Example 12 T Cell Expansion with Fas Ligand or Caspase Inhibition

Apoptotic inhibitors directed against Fas- and caspase-mediated pathways were assessed to determine effects on tumor reactive T cells during manufacturing. The studies were carried out in healthy donors, and thus, stimulation of the T cells were carried out with anti-CD3 (OKT3) or anti-CD3/anti-CD28 stimulation in order to mimic conditions present in the tumor microenvironment (TME). Constant activation signals present in the tumor microenvironment, as can be stimulated by anti-CD3 or anti-CD3/anti-CD28 activation can be detrimental to T cell growth. Cellular viability and projected cell numbers were used to compare the impact of modulation of apoptotic pathways in both a transient and a continuous activation assay, the latter of which more closely recapitulates the tumor microenvironment.

### A. Anti-CD3 Stimulation

PBMC from three healthy donors were thawed and washed with OpTmizer cell culture media supplemented with 300 IU/mL recombinant IL-2, gentamicin at 10 µg/ml, Immune Cell Serum Replacement (ThermoFisher) at 5%, and a final concentration of 2.0 mM of a L-alanyl-L-glutamine dipeptide form of glutamine (GlutaMAX Supplement; Thermofisher). Cells were then seeded into a 24-well gas permeable cell culture plate at a density of 2.14 x 10⁵ cells/mL (7.5 x 10⁵ cells/cm²). Cells were activated for 48 hours using 50 ng/ml OKT3, a human anti-CD3 monoclonal antibody, and were additionally treated with agents as described below.

Culture wells were assigned to one of five treatment groups, as follows: (1) a no inhibitor culture control which contained only cellular media as described with no additional apoptotic modulator; (2) 2 µM of the pan-caspase inhibitor Z-VAD-FMK added to the media only on culture day 0 (transient); (3) 2 µM of the pan-caspase inhibitor Z-VAD-FMK added on culture day 0 and additionally replenished at the same concentration with each media exchange (continuous); (4). 500ng/ml of Fas ligand (FasL) blocking antibody NOK-1 (BioLegend) added to the culture media only on day 0 (transient); or (5) 500ng/ml of Fas ligand (FasL) blocking antibody NOK-1 (BioLegend) added to the culture media only on day 0 and additionally replenished each media exchange (continuous inhibition).

Cultures were maintained for at least 13 days with a 50% media exchange every other day beginning on culture day 2. Cell counts and viability were monitored every other day. When cells reached 3 x 10⁶ cells/ml, 1.5 x 10⁶ cells were sub-cultured into a new well of a 24-well gas permeable culture plate with 7 mL final volume of medium and the culture continued as described above.

Total cell number and cell viability for each of the three donors are shown in **FIGS. 30A-30B** (donor 1), **FIGS. 31A-31B** (donor 2) and **FIGs. 32A-32B** (donor 3). Viability remained high for all of the treatment conditions throughout the culture period, however the condition with the continuous FasL blockade showed nominally lower viability than the rest. These cells also grew the slowest and their growth plateaued before any of the other conditions, across all donors. Cell cultures with the caspase inhibitor present continuously in the media showed the greatest cell growth across donors, while the control and transient treatment conditions grew similarly. Transient treatment with FasL blocking antibody NOK-1 also resulted in considerable T cell expansion.

These results show that the use of a caspase inhibitor may be useful during the expansion steps of TIL manufacturing to maximize expansion and maintain viability, particularly in high density cultures. Transient use of FasL blockade during conditions of T cell activation, coculture, or processing in the presence of other cell types, especially tumor cells, may also be useful for blocking Fas signaling in a pro-apoptotic environment.

### B. Anti-CD3/anti-CD28 Stimulation

PBMC from two healthy donors were thawed and washed with OpTmizer cell culture media supplemented with 300 IU/mL recombinant IL-2, gentamicin at 10 µg/ml, Immune Cell Serum Replacement (ThermoFisher) at 5%, and a final concentration of 2.0 mM of a L-alanyl-L-glutamine dipeptide form of glutamine (GlutaMAX Supplement; Thermofisher). Cells were then seeded into a 24-well gas permeable cell culture plate at a density of 1.5 × 10⁵ cells/mL (5.0 x 10⁵ cells/cm²).

Culture wells were assigned to one of two treatment groups, transient or continuous activation. For transient activation, anti-CD3/anti-CD28 paramagnetic beads (Dyanbeads^{™}) were added to the culture media at a ratio of 1 bead per cell starting on culture day 0, and then were removed during media exchange on day 2. For continuous activation, anti-CD3/anti-CD28 paramagnetic beads (Dyanbeads^{™}) were added to the culture media at a ratio of 1 bead per cell starting on culture day 0, and then added again on Day 4, and again on Day 6 at a ratio of 1 bead per cell. The anti-CD3/anti-CD28 paramagnetic beads were not removed on Day 2, 4 or 6.

For both the continuous and transient activation, culture wells were assigned to one of five treatment groups as described above for 10 conditions total per donor.

Cell counts and viability were monitored every other day. When cells reached 3 x 10⁶ cells/ml, 1.5 x 10⁶ cells were sub-cultured into a new well of a 24-well gas permeable culture plate with 7 mL final volume of medium and the culture continued as described above.

Cellular viability for the single activation with anti-CD3/anti-CD28 (transient activation) treatment groups are shown in **FIG. 33A** (donor 1) and **FIG. 33B** (donor 2), and total cell number for the same treatments are shown in **FIG. 34A** (donor 1) and **FIG. 34B** (donor 2), respectively. While inherent donor variability was observed, viability remained high for all of the treatment conditions exposed to the transient activation (single activation) stimulus. Viability remained high for all of the treatment conditions except for that of the conditions with continuous blockade of FasL, where both viability and total viable cell number declined over time (FIGS. 33A-B).

Cellular viability for the continuous activation with anti-CD3/anti-CD28 treatment groups are shown in **FIG 35A** (donor 1) and **FIG. 35B** (donor 2), and total cell number for the same treatments are shown in **FIG. 36A** (donor 1) and **FIG. 36B** (donor 2).. When exposed to a continuous activation with anti-CD3/anti-CD28, resembling the native tumor microenvironment, both total viable cell number and viability differed more between treatment conditions than conditions involving only a transient activation event. In the continuously activated populations, it appeared that the cells exposed to the caspase inhibitor, both transiently and continuously, outperformed the other conditions, with the continuous caspase inhibition outperforming the transient condition. Additionally, the cells exposed to FasL blockade showed the greatest decline in both total cell number and viability, while the cells transiently exposed to FasL blockade and no additional treatment performed similarly.

These results indicate that use of caspase inhibition during culture can improve the ability of cells to perform in environments that may be hostile to normal T cell growth, such as when cells are processed directly from the tumor which may be constitutively presenting activation signals similarly to this assay system. These results also indicate that continuous blockade of FasL signaling may be detrimental to T cell growth in conditions of both transient and continuous T cell activation, and that blockade of FasL does not affect T cell growth as strongly when it is provided transiently.

### Example 13 Assessment of Caspase Inhibition in Tumor Processing

A CRC tumor from a donor was processed as described in Example 1 using a Collagenase I/II blend (Nordmark, Collagenase NB 4G Proved Grade, part: S 1746503) to generate fragment or SCS cultures. Both tumor fragment and SCS cultures were maintained in gas permeable 24-well culture plates using OpTmizer media supplemented with 300 IU/mL recombinant IL-2, gentamicin at 10 µg/ml, Immune Cell Serum Replacement (ThermoFisher) at between 5%, and a final concentration of 2.0 mM of a L-alanyl-L-glutamine dipeptide form of glutamine (GlutaMAX Supplement; Thermofisher). Half of cultures contained media additionally supplemented with 2 µM of pan-caspase inhibitor Z-VAD-FMK which was replenished at each media exchange. Cultures were incubated at about 37° C for a minimum of 18 days with a 50% medium exchange occurring every 2-3 days following culture day 5. Cell counts were performed on day 5 and at each medium exchange using the NC-200 Automated Cell Counter (ChemoMetec).

**FIG. 37A-C**shows the fold expansion (**FIG. 37A**), total viable cells (**FIG. 37B**) and percent viability (**FIG. 37C**), of both SCS and tumor fragment derived cultures grown in the presence or absence of Z-VAD-FMK. **FIG. 37A** and **FIG. 37B** demonstrate that the outgrowth of cells was superior in the tumor fragment-derived condition containing the pan-caspase inhibitor. Additionally, as seen in **FIG. 37C****,** cell viability for this condition was also high. Cell viability was similarly high for those cells cultured as a SCS in the presence caspase inhibition, even though outgrowth of T cells was not observed for the SCS conditions. These data indicate that caspase inhibition can be a mechanism to maintain high viability and outgrowth for T cells grown from tumors.

### Example 14 Evaluation of Checkpoint Modulators and Costimulatory Agonist Antibodies on T Cell Phenotype

PBMC from two healthy donors were thawed and washed with OpTmizer cell culture media supplemented with 300 IU/mL recombinant IL-2, gentamicin at 10 µg/ml, Immune Cell Serum Replacement (ThermoFisher) at 5%, and a final concentration of 2.0 mM of a L-alanyl-L-glutamine dipeptide form of glutamine (GlutaMAX Supplement; Thermofisher). Cells were then seeded into a 24-well gas permeable cell culture plate at a density of 5.0 x 10⁵ cells/cm². Cells were activated for 48 hours using 50 ng/ml OKT3, a human anti-CD3 monoclonal antibody, and were additionally treated with agents as described below. Following 48 hours in culture, cells were analyzed for phenotype.

Cells were divided into 6 treatment groups, as follows: Ipilimumab (anti-CTLA4), Pembrolizumab (anti-PD1), Tavolixizumab (anti-TNFRSF4), Urelumab (anti-CD137), and Varlilumab (anti-CD27), as well as a no added agent control. For all test groups, cells in each treatment group were cultured in the presence of the monoclonal antibodies at 0.5, 1, 10, or 20 µg/mL.

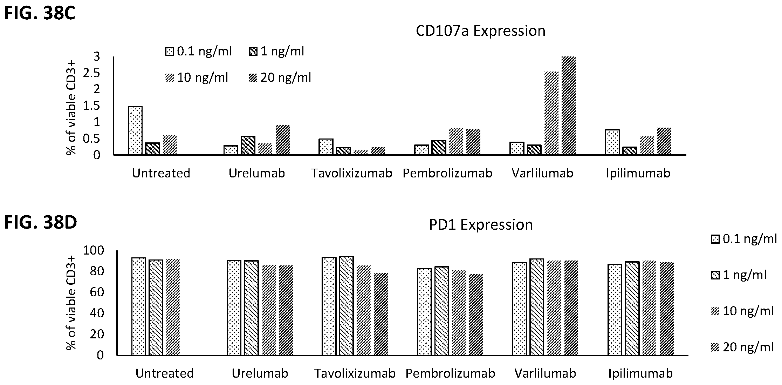

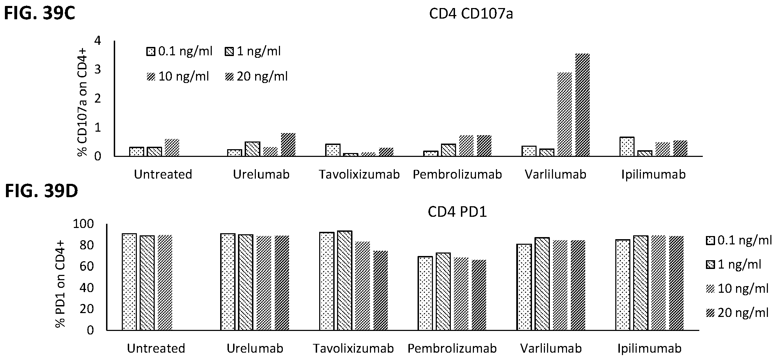

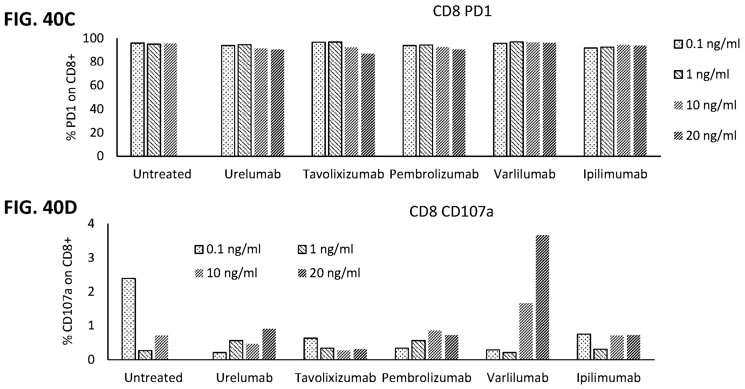

None of the tested antibodies appeared to affect the memory differentiation state of the T cells. T cell phenotype was assessed for CD4+ and CD8+ cells independently via flow cytometry for markers of activation OX40, 41BB, CD107a, and PD1. Results are shown in **FIG. 38** (CD3+), **FIG 39** (CD4+) and **FIG. 40** (CD8+). Higher concentrations of Varlilumab, an agonist anti-CD27 antibody, promoted 41BB and CD107a expression on CD3+ T cells **(****FIG. 38B** **and** **38C****),** CD4+ T cells **(****FIG. 39B** **and** **39C****)** and CD8+ T cells **(****FIG. 40B** **and** **40C****).** Urelumab, an agonist CD137 receptor antibody, promoted 41BB expression on CD4 T cells **(****FIG. 39B****)** and CD8+ T cells **(****FIG. 40B****).** Pembrolizumab, an anti-PD-1 antagonist, decreased PD1 expression on CD4 T cells **(****FIG. 39D****)**

These data support that the use of monoclonal antibody modulators for use in T cell expansion as a means of moderating cellular activation status.

### Example 15 Evaluation of Cytokines, Modulators, and Agonist Antibodies on T Cell Number, Memory Phenotype, and T cell Exhaustion

PBMC from three healthy donors were activated, expanded, and cryopreserved as described in Example 11. Cells were washed for 24 hours with OpTmizer cell culture media supplemented with 300 IU/mL recombinant IL-2, gentamicin at 10 µg/ml, Immune Cell Serum Replacement (ThermoFisher) at 5%, and a final concentration of 2.0 mM of a L-alanyl-L-glutamine dipeptide form of glutamine (GlutaMAX Supplement; Thermofisher). As previously described, stimulation of the healthy donor T cells prior to cryopreservation were carried out with anti-CD3 (OKT3) stimulation in order to mimic conditions present in the tumor microenvironment (TME).

Cells were next seeded into gas permeable 100M culture vessels and expanded for 7-14 days to achieve a large bank of T cells, and cryopreserved. Previously expanded human T cells from the three healthy donors were thawed and then seeded into 96-well culture plates to a final cell density of 5 × 10⁵ cells/mL with a test adjuvant agent at a range of concentrations in OpTmizer cell culture media supplemented with 300 IU/mL recombinant IL-2, gentamicin at 10 µg/ml, Immune Cell Serum Replacement (ThermoFisher) at between 2 and 5%, and a final concentration of 2.0 mM of a L-alanyl-L-glutamine dipeptide form of glutamine (GlutaMAX Supplement; Thermofisher).

**Table E4** shows the agents and concentrations assessed in these studies. Cells were maintained in culture for 6 days, with a 50% medium exchange occurring at 3 days post culture initiation.

At the end of the culture period for all culture conditions, cells were assessed for cell count and sub-phenotypes of naive and central memory T cells by flow cytometry by staining with CD45RA and CCR7 (naive, CD45RA+ CCR7+; "central" memory, CD45RA-CCR7+).

| **Table E4: Compounds and concentrations used in combination with IL-2** | |
|---|---|
| Compound | Concentration |
| ***Antibodies*** | |
| Anti-OX40L (e.g. Oxelumab), anti-GITR (e.g. MK-1248) | 0.2 µg/mL - 50 µg/mL |

| ***Small Molecule Inhibitors*** | |
|---|---|
| Z-VAD-FMK | 0.2 µg/mL - 50 µg/mL |

| ***Cytokines*** | |
|---|---|
| IL-23 | 3.9 ng/ml - 1000 ng/ml |
| IL-21 | 3.9 IU/ml - 1000 IU/ml |
| IL-27 | 3.9 ng/ml - 1000 ng/ml |
| IL-35 | 3.9 ng/ml - 1000 ng/ml |
| IL-7 | 3.9 IU/ml - 1000 IU/ml |
| IL-15 | 3.9 IU/ml - 1000 IU/ml |

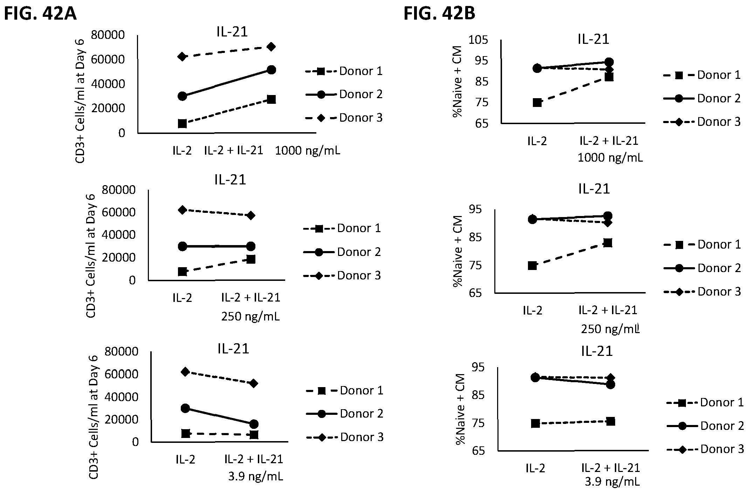

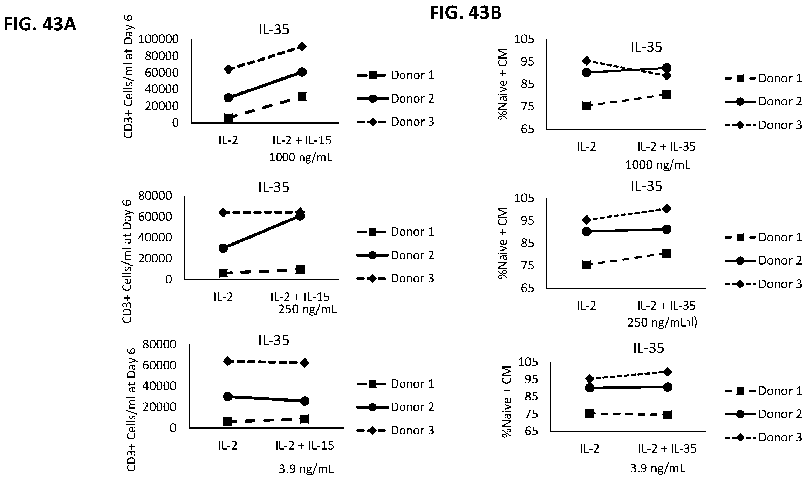

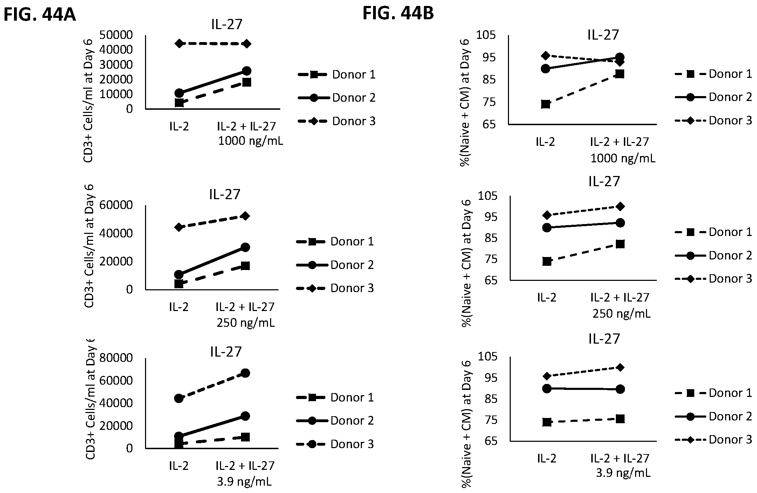

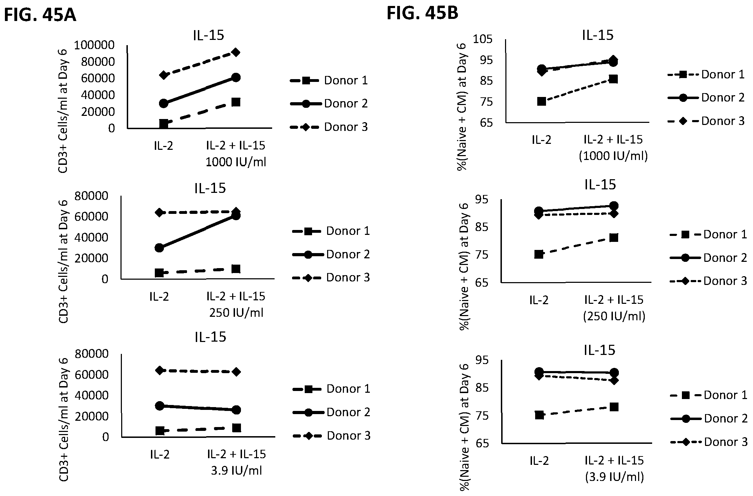

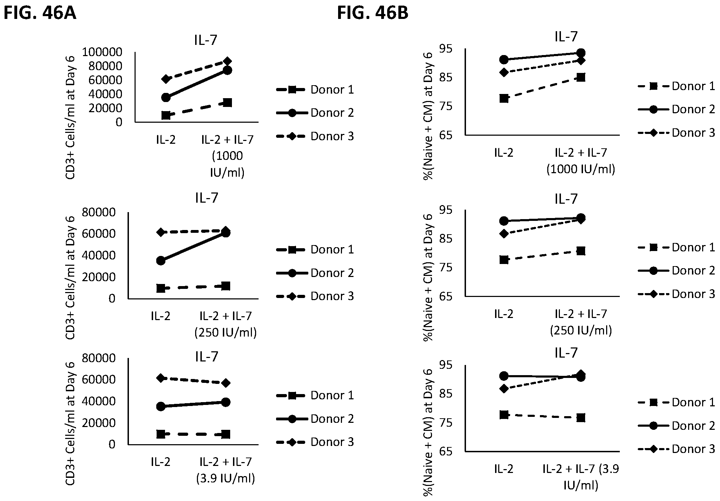

Each of the cytokines tested resulted in an increase in CD3+ cells/mL on Day 6 compared to cultures expanded just with IL-2 alone. In some cases, the greatest increase in the number of cells at Day 6 was at the highest concentration of cytokine tested. Results are shown in **FIG. 41A** (IL-23), **FIG. 42A** (IL-21), **FIG. 43A** (IL-35), **FIG. 44A** (IL-27), **FIG. 45A** (IL-15), and **FIG. 46A** (IL-7).

In addition to cell number, cytokines IL-23 **(****FIG. 41B****),** IL-21 **(****FIG. 42B****),** IL-35 (FIG. 43B), IL-27 **(****FIG. 44B****),** IL-15 **(IL-45B),** and IL-7 **(****FIG. 46B****)** also resulted in a measured increase in the percent naive and central memory T cells present in the expanded population at Day 6. In particular, an increase in the percent of naive and central memory T cells, which are T cells with a less exhausted phenotype, was observed following incubation at several of the tested concentrations of IL-23 and IL-27. For example as shown in **FIG. 44B****,** IL-27 resulted in a significant increase in CD3+ cell number as well as percent naive and central memory T cells present in the population at each of three tested concentrations (3.9, 250, and 1000 lU/mL).

The addition of either the human anti-GITR antibody or the anti-OX40L antibody resulted in an increase in CD3+ cells/mL on Day 6 at the highest concentration tested, 50 µg/mL, compared to cultures expanded with just IL-2 alone. Results are shown in **FIG. 47A** (Human anti-GITR MK-1248) and **FIG. 48B** (Oxelumab). The anti-GITR antibodyMK-1248 at the highest concentration tested additionally resulted in a significant increase in the percent naive and central memory T cells present in the population at culture Day 6 **(****FIG. 47A****),** while little effect on the percent of naive and central memory T cells was observed with the anti-OX40L antibody compared to cultures expanded just with IL-2.

The small molecule caspase inhibitor Z-VAD-FMK also substantially increased the number of CD3+ cells/mL on Day 6 at concentrations higher than 0.2 µg/mL compared to cultures expanded with just IL-2 alone **(****FIG. 49A****).** The Z-VAD-FMK compound had no effect on the percent of naive and central memory T cells was observed with the anti-OX40L antibody compared to cultures expanded just with IL-2.

### Example 16 Evaluation of Cytokines, Modulators, and Agonist Antibodies on CD4+/CD8+ T Cell Ratios

Cytokines, modulators, and agonist antibodies were assayed for impact on the ratio of CD4+ to CD8+ T cells present in the resultant population. Briefly, PBMC from three healthy donors were activated, expanded, and cryopreserved before being thawed, washed, and rested for 24 hours with OpTmizer cell culture media as described in Example 15. Previously expanded human T cells from the three healthy donors were then seeded into 96-well culture plates to a final cell density of 5 x 105 cells/mL with a test adjuvant agent at a range of concentrations in OpTmizer cell culture media supplemented with 300 IU/mL recombinant IL-2, gentamicin at 10 µg/ml, Immune Cell Serum Replacement (ThermoFisher) at between 2 and 5%, and a final concentration of 2.0 mM of a L-alanyl-L-glutamine dipeptide form of glutamine (GlutaMAX Supplement; Thermofisher).

**Table E5** shows the agents and concentrations assessed in these studies. Cells were maintained in culture for 6 days, with a 50% medium exchange occurring at 3 days post culture initiation. At the end of the culture period, cells were assessed for sub-types of T cells by flow cytometry by staining for CD4 and CD8. Representative results for one donor are shown in **FIG. 50****.**

| **Table E5: Compounds and concentrations used in high throughput screen** | |
|---|---|
| Compound | Concentration |
| ***Antibodies*** | |
| Tavolixizumab, Oxelumab, Ipilimumab, Tocilizumab, Urelumab, Pembrolizumab, Varlilumab, anti-GITR MK-1248 | 0.2 µg/mL - 50 µg/mL |
| FasL (Human anti-FasL antibody) | 0.2 µg/mL - 50 µg/mL |

| ***Small Molecule Inhibitors*** | |
|---|---|
| Z-VAD-FMK | 0.4 µg/mL - 100 µg/mL |
| NVP-HSP990 | 4.0 µg/mL - 1000 µg/mL |

| ***Cytokines*** | |
|---|---|
| IL-23 | 3.9 ng/ml - 1000 ng/ml |
| IL-21 | 3.9 IU/ml - 1000 IU/ml |
| IL-27 | 3.9 ng/ml - 1000 ng/ml |
| IL-35 | 3.9 ng/ml - 1000 ng/ml |
| IL-7 | 3.9 IU/ml - 1000 IU/ml |
| IL-15 | 3.9 IU/ml - 1000 IU/ml |
| IL-21 | 3.9 ng/ml - 1000 ng/ml |
| *DMSO (Control)* | 0.2 µg/mL - 50 µg/mL |

While some dose dependency was observed, none of the tested antibodies **(****FIG. 50A****),** cytokines **(****FIG. 50B****),** nor small molecule inhibitors **(****FIG. 50C****),** significantly altered the CD4+/CD8+ T cell ratio observed with IL-2 alone (far left bar). These data support that these agents can be used to modulate T cell number, phenotype, and exhaustion state in combination with IL-2 without significantly altering the balance of T cell subtypes present in the population.

### Example 17 Use of CD39, PD-1, and TIGIT in Direct Selection for Obtaining a Population in Tumor-derived Cells

T cells sourced from primary tumor of patients with colorectal cancer (CRC) or melanoma were processed from tumor fragments. Briefly, the tissue was minced into 1-8 mm pieces and then digested using the automated GentleMACS (Miltenyi) at approximately 1-2 grams of tissue per Gentle MACS C tube. The cells after automated processing were placed into a gas permeable culture vessel, G-Rex 10, in the presence of Roswell Park Memorial Institute (RPMI) containing 5% serum free OpTmizer medium (ThermoFisher). Media was supplemented with 300 IU/mL recombinant IL-2, and also contained gentamicin at 10 µg/ml, Immune Cell Serum Replacement (ICSR, ThermoFisher) at between 2% and 5% according to manufacturer's recommendations, and a final concentration of 2.0 mM of a L-alanyl-L-glutamine dipeptide form of glutamine (GlutaMAX Supplement; Thermofisher). Fragment cultures were maintained in culture for up to 30 days.

Cells from the tumor fragment cultures were then stained with a viable dye and with anti-CD39-APC, anti-PD-1-BV421, anti-TIGIT-PE, anti-CD4-BV510 and anti-CD8-PE-Cy7 staining antibodies at 1 µl per million cells in suspension for each. The percent of cells expressing markers and combinations thereof was then assessed by multi-color flow cytometry. Results are depicted in **Table E6.** As shown, a high percentage of cells were CD39+, and a subset of these cells also were positive for PD-1, TIGIT, or PD-1 and TIGIT. These results are consistent with a finding that cells positive for one or more of CD39, PD-1 and TIGIT are present in tumors and support the use of such markers for selection of T cells expected to be enriched in tumor reactive T cells.

| **Table E6: Exemplary Sort Summary for Two Donors** | | | | | | |
|---|---|---|---|---|---|---|
| TIL Sample | CD4 or CD8 (%) | CD39+ (%) | PD1+ TIGIT-(%) | PD1+ TIGIT+ (%) | PD1-TIGIT-(%) | PD1-TIGIT+ (%) |
| Melanoma | 79.5 | 90.1 | 8.8 | 39.7 | 23.2 | 18.7 |
| CRC | 77.5 | 71.9 | 4.7 | 28.1 | 53.1 | 10.9 |

### Example 18 Monte Carlo Modeling of Ex Vivo Expansion of Directly Selected Tumor Derived T Cells

Monte Carlo simulations were designed to forecast the number of tumor infiltrating lymphocytes obtained from a direct selection of tumor cells using markers CD39, PD-1, and TIGIT followed by the expansion of the selected cells. Monte Carlo simulations allow accurate modeling with respect to manufacture of cell therapy product for patients, without subjecting high numbers of patients to experimental procedures. These methodologies effectively allow for predictive modeling, forecasting, simulation, and optimization which in turn gives insight into the critical factors affecting risk.

The provided embodiments are based on processes for producing a TIL therapy in which a selection step for CD39, PD-1 and/or TIGIT will enrich for tumor reactive T cells. Without wishing to be bound by theory, tumor derived T cells that have recognized the tumor will upregulate activation markers (such as CD39, PD-1, TIGIT, and others) as well as exhaustion markers because they have recognized neoantigens present on the tumor. The upregulation of CD39, PD1, and TIGIT can each range between 1-80% depending on the number of T cells that have trafficked to the tumor that was collected. During the selection/sorting step, TIL or T cells that recognize mutant peptides and express one, two or all three of the markers CD39, PD1, TIGIT are isolated and each can be individually expressed on approximately 3-80% of the cells. The selected/sorted cells are then further expanded to obtain a TIL therapeutic dose.

The simulations were modeled for three different processes: (1) Tumor > Digest > Expansion 1> Selection/Sort > Expansion 2> Therapeutic Dose; (2) Tumor > Digest > Selection/Sort > Expansion 1> Expansion 2> Therapeutic Dose; and (3) Tumor > Digest > Selection/Sort > Expansion 1> Therapeutic Dose.

For each simulation, the simulation was modeled based on a process that includes collecting tumor tissue from a patient with a mass ranging from approximately 0.5 g to 10g, which would then be digested into single cell suspension using an automated system to create a population of tumor derived cells. The recovery after digest can range from 20% to 99% recovery of T cells. The cells are then processed in accord with the procedures outlined below for each process.

### (1) Process Embodiment 1: Tumor > Digest > Expansion 1 > Selection/Sort > Expansion 2

In a first embodiment of a process for preparing a TIL autologous T cell therapy, after obtaining a single cell suspension, approximately 10 x 10⁶ TIL or T cells per gram to 100 x 10⁶ TIL or T cells per gram of the original tumor collection are cultured with cytokines for a short period of time. Specifically, the cells are first minimally expanded between 1-14 days in the presence of one of several cytokines from among IL-2, IL-15, IL-7, IL-21, IL-23, IL-21, IL-27 and IL-35, generally including at least IL-2 at an amount of about 300 IU/mL. In some embodiments, a first or initial minimal expansion culture step is carried out for a protracted so that activation markers (such as CD39, PD-1 and TIGIT) as upregulated in the tumor remain present during the selection/sorting step, and have not been subsequently temporally downregulated by the culture. For instance, without wishing to be bound by theory, if the cells are cultured for too long of a period, it is possible the cells may downregulate or possibly stop expressing these markers (including CD39, PD1 and TIGIT) such that sorting using these would not be possible.

A cell count is performed on approximately 0-14 days using an automated cell counter to determine the yield of the expansion step. TIL are then stained by multi-color flow cytometry using GMP or analyte specific reagent (ASR) antibodies and sorted based on upregulation of markers CD39, PD1 and TIGIT. The sort step can isolate approximately 5-60% of the total cells. After sorting, the cells are expanded in closed culture systems for approximately 14 days in the presence of one or more cytokines from among IL-2, IL-15, IL-7, IL-21, IL-23, IL-21, IL-27 and IL-35, generally including at least IL-2 at an amount of about 300 IU/mL, and optionally one or more additional T cell stimulatory reagent or modulatory reagent as described herein. The culture is carried out to yield a sufficient number of cells for a therapeutic dose, typically > 500 × 10⁶ total T cells, which are T cells that have been expanded and would produce cytokines in the presence of autologous tumor.

An exemplary depiction of the embodiment (1) process is set forth in Table E7.

| **Table E7: Process steps in Embodiment (1)** | | | | | | | | | | | |
|---|---|---|---|---|---|---|---|---|---|---|---|
| **Process Step** | Tu mor | > | Dig est | > | PreRep(Ex pansion 1) | > | S or t | > | Rep (Expa nsion 2) | > | Therap eutic Dose |
| **Populat ion #** | | 1 | | 2 | | 3 | | 4 | | 5 | |
| **Duratio n** | | 0-1 day | | 0-1 day | | 1-14 days | | 0-1 day | | 1-14 days | |

For an initial Monte Carlo simulation, test cases were run to model likely total cells prior to sorting by substituting a probability distribution for factors of inherent uncertainty: tumor mass, percent digest recovery, and total cells per gram after a first expansion. The results were iteratively calculated by stimulating 250,000 patients and manufacturing runs using the parameters as set forth in **Table E8** wherein a mean value of cells recovered was defined across a lognormal distribution of tumor mass, a normal distribution of digest recovery, and for a lognormal distribution of total cells per gram after the first expansion, and log normal distribution of reactive cells prior to sort. Data for each test case from the first Monte Carlo simulations are also displayed in **Table E8** below. Based on the variability of each test condition within the assigned distribution the forecasted results of the total cells after the sort was calculated. Without wishing to be bound by theory, it was considered that for this first embodiment, in view of a first expansion step as described above, that a high recovery would be observed as result of digest methodology (i.e., "lighter", or gentler on the cells as a result of a lesser concentration of enzyme needed to digest the sample, such as between 1 and 5 mg/mL, such as 1 mg/mL ).

| **Table E8: Monte Carlo Simulations likely total cells after sort for Embodiment (1)** | | | | |
|---|---|---|---|---|
| | | ***Total Cells After Sort*** | | |
| **Test Conditions** | **Assumptions** | **Statistics** | **Forecast Values** | |
| Digest Recovery (%) | Mean = 90.00 | Mean: 8.95 ×10⁶ | 0% | 518,865.83 |
| | Std. Dev = 2.00 | Median : 5.47 ×10⁶ | 10% | 2,210,092.62 |
| | Normal distribution | | 20% | 2,935,427.59 |
| Reactive Cells Prior to Sort (%) | Mean = 25.00 | | 30% | 3,663,314.31 |
| | Std. Dev = 10.00 | | 40% | 4,479,771.85 |
| | | | 50% | 5,468,988.75 |
| | Location = 5.00 | | 60% | 6,731,800.27 |
| | Lognormal distribution | | 70% | 8,535,879.64 |
| | | | 80% | 11,467,180.90 |
| | | | 90% | 17,701,417.23 |
| Total Cells/gram (after expansion 1) | Mean = 20.00 | | | |
| | Std. Dev = 20.00 | | 100% | 784,104,584.93 |
| | Location = 10.00 | | | |
| | Lognormal distribution | | | |
| Tumor Mass | Mean = 2.00 | | | |
| | Std. Dev = 01.50 | | | |
| | Location = 00.50 | | | |
| | Lognormal distribution | | | |
| CD39 (%) | Min = 0.03 | | | |
| | Max - 0.50 | | | |
| PD-1 (%) | Min = 0.20 | | | |
| | Max - 0.80 | | | |
| TIGIT (%) | Min = 0.20 | | | |
| | Max - 0.80 | | | |

Monte Carlo simulations of likely total cells for a therapeutic dose following sort were run by substituting a normal distribution the expansion 2 distribution for fold expansion according to the assumptions set forth below using the post-sort forecast data from Table E8. The results were iteratively calculated for the 250,000 patient stimulations, and distributions were then calculated for total cell number as shown below in Table E9.

| **Table E9: Monte Carlo Simulations likely total cells after final expansion for Embodiment (1)** | | | | |
|---|---|---|---|---|
| | | ***Total Cells for After Sort and Expansion*** | | |
| **Test Conditions** | **Assumptions** | **Statistics** | **Forecast Values** | |
| Fold Expansion 2 | Mean = 750.00 | Mean = 6.71×10⁹ | 0% | 1,295,675,671.31 |
| | Std. Dev = 200.00 | Max = 4.00 ×10⁹ | 10% | 1,486,065,198.60 |
| | Normal distribution | | 20% | 2,041,844,493.57 |
| | | | 30% | 2,603,747,895.37 |
| | | | 40% | 3,237,519,054.02 |
| | | | 50% | 3,999,406,936.98 |
| | | | 60% | 4,981,888,632.10 |
| | | | 70% | 6,363,031,134.08 |
| | | | 80% | 8,628,338,887.85 |
| | | | 90% | 13,480,329,792.97 |
| | | | 100% | 717,174,624,245.60 |

By simulating 250,000 patients and manufacturing runs using parameters in this process, greater than 97% of these cell therapy products for therapeutic use to treat cancer will be able to be manufactured with greater than 1 billion cells as the active dose.

### (2) Process Embodiment 2: Tumor > Digest > Selection/Sort > Expansion 1 > Expansion 2> Therapeutic Dose

In a second embodiment of a process for preparing a TIL autologous T cell therapy, after obtaining a single cell suspension, approximately 10 × 10⁶ TIL or T cells per gram to 100 x 10⁶ TIL or T cells per gram of the original tumor collection, the TIL are then sorted based on upregulation of markers CD39, PD1 and TIGIT. For sorting, TIL are stained by multi-color flow cytometry using GMP or analyte specific reagent (ASR) antibodies and sorted based on upregulation of markers CD39, PD1 and TIGIT. The sort step can isolate approximately 5-60% of the total cells. After sorting, the cells are expanded in closed culture systems for 1-14 days in the presence of one or more cytokines from among IL-2, IL-15, IL-7, IL-21, IL-23, IL-21, IL-27 and IL-35, generally including at least IL-2 at an amount of about 300 IU/mL, and optionally one or more additional T cell stimulatory reagent or modulatory reagent as described herein. This expansion is carried out to yield an approximate fold expansion of 2-20 fold. A cell count is performed on approximately 0-14 days using an automated cell counter to determine the yield of the expansion 1 step. After the first expansion, the cells are further expanded (second expansion) in closed culture systems for approximately 14 days in the presence of one or more cytokines from among IL-2, IL-15, IL-7, IL-21, IL-23, IL-21, IL-27 and IL-35, generally including at least IL-2 at an amount of about 300 IU/mL, and optionally one or more additional T cell stimulatory reagent or modulatory reagent as described herein. This expansion is carried out to yield an approximate 100-3000 fold expansion. The culture is carried out to yield a sufficient number of cells for a therapeutic dose, typically > 500 10⁶ total T cells, which are T cells that have been expanded and would produce cytokines in the presence of autologous tumor.

An exemplary depiction of the embodiment (2) process is set forth in **Table E10.**

| **Table E10: Process steps in Embodiment (2)** | | | | | | | | | | | |
|---|---|---|---|---|---|---|---|---|---|---|---|
| **Process Step** | Tu mor | | Dig est | > | S or t | > | PreRep(Ex pansion 1) | > | Rep (Expa nsion 2) | > | Therapeut ic Dose |
| **Popula tion #** | | 1 | | 2 | | 3 | | 4 | | 5 | |
| **Durati on** | | 0-1 day | | 0-1 day | | 0-1 day | | 1-14 days | | 1-14 days | |

For an initial Monte Carlo simulation, test cases were run to model likely total cells prior to sorting by substituting a probability distribution for factors of inherent uncertainty: tumor mass, percent digest recovery, and total cells per gram. The results were iteratively calculated by stimulating 250,000 patients and manufacturing runs using the parameters as set forth in Table E11 wherein a mean value of cells recovered was defined across a lognormal distribution of tumor mass, a normal distribution of digest recovery, , and log normal distribution of reactive cells prior to sort. Data for each test case from the first Monte Carlo simulations are displayed in **Table E11** below. Based on the variability of each test condition within the assigned distribution the forecasted results of the total cells after the sort was calculated.

| **Table E11: Monte Carlo Simulations likely total cells after sort for Embodiment (2)** | | | | |
|---|---|---|---|---|
| | ***Total Cells After Sort*** | | | |
| **Test Conditions** | **Assumptions** | **Statistics** | **Forecast Values** | |
| Digest Recovery (%) | Mean = 40.00 | Mean: 2.01 ×10⁶ | 0% | -238,313.04 |
| | Std. Dev = 10.00 | Median : 1.32 ×10⁶ | 10% | 478,149.86 |
| | Normal distribution | | 20% | 664,335.43 |
| | | | 30% | 853,957.87 |
| Reactive Cells Prior to Sort (%) | Mean = 25.00 | | | |
| | Std. Dev = 10.00 | | 40% | 1,064,442.57 |
| | Location = 5.00 | | 50% | 1,316,139.18 |
| | Lognormal distribution | | 60% | 1,633,252.56 |
| | | | 70% | 2,072,304.31 |
| Total Cells/gram (after digest) | Mean = 10.00 | | 80% | 2,751,880.64 |
| | Std. Dev = 5.00 | | 90% | 4,142,666.83 |
| | Location = 2.00 | | 100% | 121,152,894.27 |
| | Lognormal distribution | | | |
| Tumor Mass | Mean = 2.00 | | | |
| | Std. Dev = 01.50 | | | |
| | Location = 00.50 | | | |
| | Lognormal distribution | | | |
| CD39 (%) | Min = 0.03 | | | |
| | Max - 0.50 | | | |
| PD-1 (%) | Min = 0.20 | | | |
| | Max - 0.80 | | | |
| TIGIT (%) | Min = 0.20 | | | |
| | Max - 0.80 | | | |

Monte Carlo simulations of likely total cells for a therapeutic dose following sort and then expansion were run by substituting a normal distribution of the expansion 2 distribution for fold expansion according to the assumptions set forth below using the post-sort forecast data from Table E10. The results were iteratively calculated for the 250,000 patient stimulations, and distributions were then calculated for total cell number as shown below in Table E11.

| **Table E11: Monte Carlo Simulations likely total cells after final expansion for Embodiment (2)** | | | | |
|---|---|---|---|---|
| | | ***Total Cells After Sort and Expansion*** | | |
| **Test Conditions** | **Assumptions** | **Statistics** | **Forecast Values** | |
| Fold Expansion (Expansion 1) | Mean = 6.00 | Mean: 9.04 ×10⁹ | 0% | -2,413,384,406.49 |
| | Std. Dev = 1.50 | Median : 5.58 ×10⁹ | 10% | 1,749,645,301.65 |
| | Normal distribution | | 20% | 2,581,218,329.18 |
| Fold Expansion (Expansion 2) | Mean = 750.00 | | 30% | 3,434,870,328.97 |
| | Std. Dev = 200.00 | | 40% | 4,408,919,595.70 |
| | Normal distribution | | 50% | 5,578,888,455.27 |
| Total Cells/gram (after expansion 1) | Mean = 10.00 | | 60% | 7,067,403,487.56 |
| | Std. Dev = 5.00 | | 70% | 9,140,674,828.29 |
| | Location = 2.00 | | 80% | 12,447,118,686.08 |
| | Lognormal distribution | | 90% | 19,134,097,493.43 |
| | | | 100% | 845,476,158,545.17 |

The final fifth population contains greater than 500 million total T cells that produce cytokines in the presence of autologous tumor, with greater than 97.1% of cell products manufactured using this method generating greater than 1 billion cells for therapeutic dose.

### (3) Process Embodiment 3: Tumor > Digest > Selection/Sort > Expansion 1 >

### Therapeutic Dose.

In a third embodiment of a process for preparing a TIL autologous T cell therapy, after obtaining a single cell suspension, approximately 10 x 10⁶ TIL or T cells per gram to 100 x 10⁶ TIL or T cells per gram, the TIL are then sorted based on upregulation of markers CD39, PD1 and TIGIT. For sorting, TIL are stained by multi-color flow cytometry using GMP or analyte specific reagent (ASR) antibodies and sorted based on upregulation of markers CD39, PD1 and TIGIT. The sort step can isolate approximately 5-60% of the total cells. After sorting, the cells are expanded for approximately 1-28 days in the presence of one or more cytokines from among IL-2, IL-15, IL-7, IL-21, IL-23, IL-21, IL-27 and IL-35, generally including at least IL-2 at an amount of about 300 IU/mL, and optionally one or more additional T cell stimulatory reagent or modulatory reagent as described herein. The culture is carried out to yield a sufficient number of cells for a therapeutic dose, typically > 500 10⁶ total T cells, which are T cells that have been expanded and would produce cytokines in the presence of autologous tumor.

An exemplary depiction of the embodiment (3) process is set forth in **Table E12.**

| **Table E12: Process steps in Embodiment (3)** | | | | | | | | | |
|---|---|---|---|---|---|---|---|---|---|
| **Process Step** | Turn or | | Dige st | > | So rt | > | Rep(Expans ion 1) | > | Therapeutic Dose |
| **Populatio n#** | | 1 | | 2 | | 3 | | 4 | |
| **Duration** | | 0-1 day | | 0-1 day | | 0-1 day | | 1-28 days | |

For an initial Monte Carlo simulation, test cases were run to model likely total cells prior to sorting by substituting a probability distribution for factors of inherent uncertainty: tumor mass, percent digest recovery, and total cells per gram after digest. The results were iteratively calculated by stimulating 250,000 patients and manufacturing runs using the parameters as set forth in Table E13 wherein a mean value of cells recovered was defined across a lognormal distribution of tumor mass, a normal distribution of digest recovery, and for a lognormal distribution of total cells per gram after digest, and log normal distribution of reactive cells prior to sort. Data for each test case from the first Monte Carlo simulations are displayed in Table E13 below. Without wishing to be bound by theory, it was considered that embodiment 2 and 3 were similar in lower recovery because of a stronger digest needed to dissolve the tumor at the start of the protocol to allow for immediate or close to immediate cell sorting. A higher concentration of enzyme is needed to completely digest the sample, such as between 1 and 5 mg/mL, such as 5 mg/mL. Based on the variability of each test condition within the assigned distribution the forecasted results of the total cells after the sort was calculated.

| **Table E13: Monte Carlo Simulations likely total cells after sort for Embodiment (3)** | | | | |
|---|---|---|---|---|
| | | ***Total Cells After Sort*** | | |
| **Test Conditions** | **Assumptions** | **Statistics** | **Forecast Values** | |
| Digest Recovery (%) | Mean = 50.00 | Mean: 2.50 ×10⁶ | 0% | 98,927.91 |
| | Std. Dev = 10.00 | Median : 1.66 ×10⁶ | 10% | 618,136.94 |
| | Normal distribution | | 20% | 855,954.59 |
| Reactive Cells Prior to Sort (%) | Mean = 25.00 | | 30% | 1,090,069.69 |
| | Std. Dev = 10.00 | | 40% | 1,351,091.06 |
| | Location = 5.00 | | 50% | 1,662,404.79 |
| | Lognormal distribution | | 60% | 2,053,550.59 |
| | | | 70% | 2,591,913.26 |
| Total Cells/gram (after digest) | Mean = 10.00 | | 80% | 3,434,214.94 |
| | Std. Dev = 5.00 | | 90% | 5,128,492.76 |
| | Location = 2.00 | | 100% | 472,924,420.72 |
| | Lognormal distribution | | | |
| Tumor Mass | Mean = 2.00 | | | |
| | Std. Dev = 1.50 | | | |
| | Location = 0.50 | | | |
| | Lognormal distribution | | | |
| CD39 (%) | Min = 0.03 | | | |
| | Max - 0.50 | | | |
| PD-1 (%) | Min = 0.20 | | | |
| | Max - 0.80 | | | |
| TIGIT (%) | Min = 0.20 | | | |
| | Max - 0.80 | | | |

Monte Carlo simulations of likely total cells for a therapeutic dose following expansion of the sorted/selected cells were run by substituting a normal distribution of fold expansion for fold expansion according to the assumptions set forth below using the post-sort forecast data from Table E13. The results were iteratively calculated for the 250,000 patient stimulations, and distributions were then calculated for total cell number as shown below in Table E14.

| **Table E14: Monte Carlo Simulations likely total cells after expansion for Embodiment (3)** | | | | |
|---|---|---|---|---|
| | | ***Total Cells After Sort and Expansion*** | | |
| **Test Conditions** | **Assumptions** | **Statistics** | **Forecast Values** | |
| Fold Expansion | Mean = 1500.00 | Mean: 3.75 ×10⁹ | 0% | -761,064,618.77 |
| | Std. Dev = 400.00 | Median : 2.41 ×10⁹ | 10% | 828,107,890.54 |
| | Normal distribution | | 20% | 1,186,909,754.04 |
| | | | 30% | 1,544,361,203.00 |
| | | | 40% | 1,941,230,593.39 |
| | | | 50% | 2,414,573,107.82 |
| | | | 60% | 3,018,115,043.27 |
| | | | 70% | 3,857,187,705.90 |
| | | | 80% | 5,157,994,271.81 |
| | | | 90% | 7,824,998,288.06 |
| | | | 100% | 935,380,498,046.68 |

The fourth final population contains greater than 500 million total T cells that produce cytokines in the presence of autologous tumor with greater than 97.1% of cell products manufactured using this method generating greater than 500 million cells for therapeutic dose.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

### IX. SEQUENCES

| SEQ ID | Sequence | Annotation |
|---|---|---|
| 1 | RAVPGGSSPAWTQCQQLSQKLCTLAWSAHPLVGHMDLREEGDEE TTNDVPHIQCGDGCDPQGLRDNSQFCLQRIHQGLIFYEKLLGSDIFT GEPSLLPDSPVGQLHASLLGLSQLLQPEGHHWETQQIPSLSPSQPW QRLLLRFKILRSLQAFVAVAARVFAHGAATLSP | P19, UniProt Q9NPF7 20-189 |
| 2 | IWELKKDVYVVELDWYPDAPGEMVVLTCDTPEEDGITWTLDQSSE VLGSGKTLTIQVKEFGDAGQYTCHKGGEVLSHSLLLLHKKEDGIW STDILKDQKEPKNKTFLRCEAKNYSGRFTCWWLTTISTDLTFSVKS SRGSSDPQGVTCGAATLSAERVRGDNKEYEYSVECQEDSACPAAE ESLPIEVMVDAVHKLKYENYTSSFFIRDIIKPDPPKNLQLKPLKNSR QVEVSWEYPDTWSTPHSYFSLTFCVQVQGKSKREKKDRVFTDKTS ATVICRKNASISVRAQDRYYSSSWSEWASVPCS | P40, UniProt P29460 23-328 |
| 3 | YSHWPSCCPSKGQDTSEELLRWSTVPVPPLEPARPNRHPESCRASE DGPLNSRAISPWRYELDRDLNRLPQDLYHARCLCPHCVSLQTGSH MDPRGNSELLYHNQTVFYRRPCHGEKGTHKGYCLERRLYRVSLA CVCVRPRVMG | IL-25, UniProt Q9H293 33-177 |
| 4 | FPRPPGRPQL SLQELRREFT VSLHLARKLL SEVRGQAHRF AESHLPGVNL YLLPLGEQLP DVSLTFQAWR RLSDPERLCF ISTTLQPFHA LLGGLGTQGR WTNMERMQLW AMRLDLRDLQ RHLRFQVLAA GFNLPEEEEE EEEEEEEERK GLLPGALGSA LQGPAQVSWP QLLSTYRLLH SLELVLSRAV RELLLLSKAG HSVWPLGFPT LSPQP | P28 (IL27A) |
| 5 | RKGPP AALTLPRVQC RASRYPIAVD CSWTLPPAPN STSPVSFIAT YRLGMAARGH SWPCLQQTPT STSCTITDVQ LFSMAPYVLN VTAVHPWGSS SSFVPFITEH IIKPDPPEGV RLSPLAERQL QVQWEPPGSW PFPEIFSLKY WIRYKRQGAA RFHRVGPIEA TSFILRAVRP RARYYVQVAA QDLTDYGELS DWSLPATATM SLGK | EB13 (IL27B) |
| 6 | RNLPVATPDP GMFPCLHHSQ NLLRAVSNML QKARQTLEFY PCTSEEIDHE DITKDKTSTV EACLPLELTK NESCLNSRET SFITNGSCLA SRKTSFMMAL CLSSIYEDLK MYQVEFKTMN AKLLMDPKRQ IFLDQNMLAV IDELMQALNF NSETVPQKSS LEEPDFYKTK IKLCILLHAF RIRAVTIDRV MSYLNAS | P35 (IL-12α) |
| 7 | QVTDINSKGLELRKTVTTVETQNLEGLHHDGQFCHKPCPPGERKA RDCTVNGDEPDCVPCQEGKEYTDKAHFSSKCRRCRLCDEGHGLE VEINCTRTQNTKCRCKPNFFCNSTVCEHCDPCTKCEHGIIKECTLTS NTKCKEEGSRSN | CD95 (AA 26-173) |

### NUMBERED EMBODIMENTS:

1. A method for manufacturing tumor-reactive T cells, the method comprising:
   a. selecting cells surface positive for activation markers PD-1, CD39 and TIGIT from an input sample comprising T cells from a subject that has a tumor to obtain selected cells from the sample; and
   b. performing an expansion by culture of the selected cells with one or more T-cell stimulating agent of lymphocytes under conditions to produce a population of expanded T cells.
2. A method for manufacturing tumor-reactive T cells, the method comprising:
   a. selecting cells surface positive at least two activation markers from the group consisting of PD-1, CD39 and TIGIT from an input sample comprising T cells from a subject that has a tumor to obtain selected cells from the sample; and
   b. performing an expansion by culture of the selected cells with one or more T-cell stimulating agent of lymphocytes under conditions to produce a population of expanded T cells.
3. The method of embodiment 1, wherein the selecting further comprises selecting cell surface positive for one or more further marker selected from CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3 or TIGIT, wherein the selecting for cells surface positive for the activation markers and the selecting cells surface positive for the further marker is carried out simultaneously or sequentially in any order to obtain the selected cells.
4. The method of any embodiments 1-3, further comprising selecting, optionally by positive selection or negative selection, T cells surface positive for a T cell marker selected from CD3, CD4 or CD8, wherein the selecting cells surface positive for the T cell marker and the selecting cells surface positive for the activation markers is carried out simultaneously or sequentially in any order to obtain the selected cells.
5. The method of any of embodiments 1-11, wherein the input sample comprising T cells is from the peripheral blood or from a tumor.
6. The method of any of embodiments 1-5, wherein the input sample comprises tumor infiltrating lymphocytes.
7. The method of any of embodiments 1-6, wherein the input sample comprising T cells is derived from a resected tumor.
8. The method of embodiment 7, wherein the input sample comprising T cells is a single cell suspension processed by homogenization and/or enzymatic digestion of one or more tumor fragments from the resected tumor.
9. The method of embodiment 7 or embodiment 8, wherein the input sample comprising T cells is a single cell suspension processed by homogenization and enzymatic digestion of one or more tumor fragments from the resected tumor.
10. The method of embodiment 8 and embodiment 9, wherein the enzymatic digestion is by incubation with a collagenase, optionally collagenase IV or collagenase I/II.
11. The method of any of embodiments 1-10, wherein the input sample comprises from at or about 10 x 10⁶ T cells per gram of tumor sample from the subject to at or about 100 x 10⁶ T cells per gram of the tumor sample from the subject.
12. The method of any of embodiments 1-11, wherein the expanded T cell population is for use as a therapeutic cell composition.
13. The method of any of embodiments 1-12, wherein the performing the expansion to produce the expanded population of T cells is for 7 to 35 days.
14. The method of any of embodiments 1-13, wherein the performing the expansion to produce the expanded population of T cells is for 7 to 28 days, optionally 14 days to 28 days.
15. The method of any of embodiments 1-13, wherein the performing the expansion to produce the expanded population of T cells is for 7 to 21 days, optionally 7 to 14 days.
16. The method of any of embodiments 1-15, wherein the one or more T-cell stimulating agent is one or more first T-cell stimulating agent and the performing the expansion is a first expansion, wherein the method further comprises performing a second expansion by culture of the first expanded T cell population with one or more second T-cell stimulating agent under conditions to produce a second expanded population of T cells, wherein the second population of expanded T cells is for use as a therapeutic cell composition.
17. The method of embodiment 16, wherein the one or more T cell stimulating agent of the first expansion and the one or more T cell stimulating agent of the second expansion are the same.
18. The method of embodiment 16 or embodiment 17, wherein the performing the first expansion is for 7 to 21 days, optionally 7 to 14 days.
19. The method of any of embodiments 16-18, wherein the performing the second expansion is 7 to 21 days, optionally 7 to 14 days.
20. The method of any of embodiments 1-19, wherein the one or more T-cell stimulating agent of lymphocytes is an anti-CD3 agent (e.g. OKT3) and/or a recombinant cytokine selected from one or more of IL-2, IL-7, IL-15, IL-21, IL-25, IL-23, IL-27 and IL-35.
21. The method of any of embodiments 1-20, wherein at least one of the one or more T-cell stimulating agent is recombinant IL-2.
22. The method of any of embodiments 1-21, wherein culture with the one or more T-cell stimulating agent further comprises an apoptosis inhibitor.
23. A method for manufacturing tumor-reactive T cells, the method comprising:
   a. processing a biological sample containing T cells obtained from a donor subject that has a tumor to produce an input sample comprising T cells;
   b. performing a first expansion by culture of the sample comprising T cells with one or more first T-cell stimulating agent of lymphocytes under conditions to produce a first population of expanded T cells;
   c. selecting cells surface positive for activation markers PD-1, CD39 and TIGIT from the first population of expanded cells to produce a selected population; and
   d. performing a second expansion by culture of the selected population with one or more second T-cell stimulating agent under conditions to produce a second expanded population of T cells, wherein the second population of expanded T cells is for use as a therapeutic cell composition.
24. A method for manufacturing tumor-reactive T cells, the method comprising:
   a. processing a biological sample containing T cells obtained from a donor subject that has a tumor to produce an input sample comprising T cells;
   b. performing a first expansion by culture of the sample comprising T cells with one or more first T-cell stimulating agent of lymphocytes under conditions to produce a first population of expanded T cells;
   c. selecting cells surface positive for at least two activation markers from the group consisting of PD-1, CD39 and TIGIT from the first population of expanded cells to produce a selected population; and
   d. performing a second expansion by culture of the selected population with one or more second T-cell stimulating agent under conditions to produce a second expanded population of T cells, wherein the second population of expanded T cells is for use as a therapeutic cell composition.
25. A method for manufacturing tumor-reactive T cells, the method comprising:
   a. processing a biological sample containing T cells obtained from a donor subject that has a tumor to produce an input sample comprising T cells;
   b. performing a first expansion by culture of the sample comprising T cells with one or more first T-cell stimulating agent of lymphocytes under conditions to produce a first population of expanded T cells;
   c. selecting cells surface positive for activation markers PD-1, CD39 and TIGIT from the first population of expanded cells to produce a selected cell population; and
   d. co-culturing the selected cell population in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor;
   e. performing a second expansion by culture of the reactive T cell population with one or more second T-cell stimulating agent under conditions to produce a second expanded population of T cells, wherein the second population of expanded T cells is for use as a therapeutic cell composition.
26. A method for manufacturing tumor-reactive T cells, the method comprising:
   a. processing a biological sample containing T cells obtained from a donor subject that has a tumor to produce an input sample comprising T cells;
   b. performing a first expansion by culture of the sample comprising T cells with one or more first T-cell stimulating agent of lymphocytes under conditions to produce a first population of expanded T cells;
   c. co-culturing the first population of expanded cells in the presence of antigen presenting cells that present one or more non-native peptide on a major histocompatibility complex (MHC), said one or more non-native peptides are peptides corresponding to nonsynonymous somatic mutations associated in the tumor of a subject, to produce a reactive T cell population containing T cells comprising endogenous T cell receptors reactive to mutation encoding peptides of the tumor;
   d. selecting cells surface positive for activation markers PD-1, CD39 and TIGIT from the reactive T cell population to produce a selected cell population; and
   e. performing a second expansion by culture of the selected cell population with one or more second T-cell stimulating agent under conditions to produce a second expanded population of T cells, wherein the second population of expanded T cells is for use as a therapeutic cell composition.
27. The method of any of embodiments 23-26, wherein the selecting further comprises selecting cell surface positive for one or more further marker selected from CD107a, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), TIM-3, or LAG-3, wherein the selecting for cells surface positive for the activation markers and the selecting cells surface positive for the further marker is carried out simultaneously or sequentially in any order to produce the selected cell population
28. The method of any embodiments 23-27, further comprising selecting, optionally by positive selection or negative selection, T cells surface positive for a T cell marker selected from CD3, CD4 or CD8, wherein the selecting cells surface positive for the T cell marker and the selecting cells surface positive for the activation markers is carried out simultaneously or sequentially in any order to produce the selected cell population.
29. The method of any of embodiments 23-28, wherein the biological sample is a peripheral blood sample or a tumor sample.
30. The method of any of embodiments 23-29, wherein the input sample comprising T cells comprises tumor infiltrating lymphocytes (TILs).
31. The method of any of embodiments 23-30, wherein the input sample comprising T cells is derived from a resected tumor.
32. The method of any of embodiments 23-31, wherein the biological sample is a resected tumor from the subject and the input sample comprising T cells is one or more tumor fragments from the resected tumor.
33. The method of embodiment 32, wherein the one or more tumor fragments are 1-8 mm in diameter.
34. The method of embodiment 32 or embodiment 33, wherein the one or more tumor fragments are seeded for the first expansion at about 1 tumor fragment per 2 cm2 .
35. The method of any of embodiments 23-31, wherein the input sample comprising T cells is a single cell suspension processed by homogenization and/or enzymatic digestion of one or more tumor fragments from the resected tumor.
36. The method of any of embodiments 23-31, wherein the input sample comprising T cells is a single cell suspension processed by homogenization and enzymatic digestion of one or more tumor fragments from a resected tumor.
37. The method of embodiment 35 and embodiment 36, wherein the enzymatic digestion is by incubation with a collagenase, optionally collagenase IV or collagenase I/II.
38. The method of any of embodiments 23-37, wherein the input sample comprises from at or about 10 x 10⁶ T cells per gram of tumor sample from the subject to at or about 100 x 10⁶ T cells per gram of the tumor sample from the subject.
39. The method of any embodiments 8-11 and 35-38, wherein the input sample comprising T cells is seeded for expansion at about 5 x 105 to at or about 2 x 106 total cells per 2 cm2.
40. The method of any of embodiments 23-39, wherein performing the first expansion is for 1 to 14 days.
41. The method of any of embodiments 23-40, wherein performing the first expansion is for at or about 1 day, at or about 2 days, at or about 3 days, at or about 4 days, at or about 5 days, at or about 6 days, at or about 7 days, at or about 8 days, at or about 9 days, at or about 10 days, at or about 11 days, at or about 12 days, at or about 13 days or at or about 14 days.
42. The method of any of embodiments 23-41, wherein performing the second expansion is for 7 to 35 days.
43. The method of any of embodiments 23-42, wherein the performing the second expansion is 7 to 21 days, optionally 7 to 14 days.
44. The method of any of embodiments 23-43, wherein the one or more T cell stimulating agent of the first expansion and the one or more T cell stimulating agent of the second expansion are the same.
45. The method of any of embodiments 23-44, wherein the one or more first T-cell stimulating agent of lymphocytes for the first expansion is a recombinant cytokine selected from one or more of IL-2, IL-7, IL-15, IL-21, IL-25, IL-23, IL-27 and IL-35.
46. The method of any of embodiments 23-45, wherein at least one of the one or more first T-cell stimulating agent is recombinant IL-2.
47. The method of any of embodiments 23-46, wherein the the one or more first T cell stimulating agent does not comprise an anti-CD3 antibody (e.g. OKT3).
48. The method of any of embodiments 23-47, wherein culture with the one or more first T-cell stimulating agent further comprises an apoptosis inhibitor.
49. The method of any of embodiments 23-48, wherein the one or more second T-cell stimulating agent of lymphocytes is an anti-CD3 agent and/or a recombinant cytokine selected from one or more of IL-2, IL-7, IL-15, IL-21, IL-25, IL-23, IL-27 and IL-35.
50. The method of any of embodiments 23-49, wherein at least one of the one or more second T-cell stimulating agent is recombinant IL-2.
51. The method of any of embodiments 20, 21, 45, 46, 49 and 50, wherein the concentration of recombinant IL-2 is from 100 IU/mL to 6000 IU/mL.
52. The method of any of embodiments 20, 21, 45, 46, and 49-51, wherein the concentration of recombinant IL-2 is from 300 IU/mL to 1000 IU/mL, optionally wherein the concentration of recombinant IL-2 is at or about 300 IU/mL.
53. The method of any of embodiments 20, 21, 45, 46, and 49-52, wherein the concentration of recombinant IL-2 is at or about 300 IU/mL.
54. The method of any of embodiments 20, 21, 45, 46, and 49-52, wherein the concentration of recombinant IL-2 is at or about 1000 IU/mL.
55. The method of any of embodiments 23-54, wherein the one or more second T cell stimulating agent comprises an anti-CD3 antibody, optionally OKT3, optionally wherein the concentration of the anti-CD3 antibody is at or about 50 ng/mL.
56. The method of any of embodiments 23-55, wherein the culture with the one or more second T-cell stimulating agent further comprises an apoptosis inhibitor.
57. The method of embodiment 22, embodiment 48 or embodiment 56, wherein the apoptosis inhibitor reduces apoptosis induced by CD95 (Fas), optionally wherein the apoptosis inhibitor specifically binds CD95 (Fas) or CD95 ligand (Fas ligand).
58. The method of embodiment 57, wherein the apoptosis inhibitor is an antibody or antigen-binding fragment, optionally wherein the apoptosis inhibitor is an anti-Fas antibody or an anti-Fas ligand antibody.
59. The method of embodiment 57, wherein the apoptosis inhibitor is a fusion protein comprising the extracellular domain of CD95 (Fas) or a specific binding fragment thereof that binds to CD95 ligand (Fas ligand) fused to an Fc immunoglobulin domain, optionally wherein the apoptosis inhibitor is APG101 or CAN008.
60. The method of embodiment 22, embodiment 48 or embodiment 56, wherein the apoptosis inhibitor inhibits caspase activation or activity, optionally wherein the caspase is a caspase 2, a caspase 8, a caspase 9, a caspase 10, a caspase 3, a caspase 6 or a caspase 7, optionally wherein the caspase is a caspase 3.
61. The method of any of embodiments 22, 48, 56 or 60, wherein the apoptosis inhibitor is selected from the group consisting of Emricasan (IDN-6556, PF-03491390), NAIP (neuronal apoptosis inhibitory protein; BIRC1), cIAP1 and cIAP2 (cellular inhibitor of apoptosis 1 and 2; BIRC2 and BIRC3, respectively), XIAP (X-chromosome binding IAP; BIRC4), survivin (BIRC5), BRUCE (Apollon; BIRC6), livin (BIRC7) and Ts-IAP (testis-specific IAP; BIRC8), Wedelolactone, NS3694, NSCI and Z- fluoromethyl ketone Z-VAD-FMK or a flouromethyl ketone variant thereof.
62. The method of any of embodiments 22, 48, 56, 60 and 61, wherein the apoptosis inhibitor is emericasan.
63. The method of any of embodiments 22, 48, 56 and 60-62, wherein the apoptosis inhibitor is a pan-caspase inhibitor that inhibits activation or activity of two or more caspases.
64. The method of any of embodiments 22, 48, 56 and 60-63, wherein the apoptosis inhibitor is Z-VAD-FMK, Z-FA-FMK, Z-VAD(OH)-FMK, Z-DEVD-FMK, Z-VAD(OM2)-FMK, or Z-VDVAD-FMK.
65. The method of any of embodiments 22, 48 and 56-64, wherein the concentration of the apoptosis inhibitor is between at and about 0.5 µM and at or about 50 µM, between at or about 0.5 µM and at or about 25 µM, between at or about 0.5 µM and at or about 10 µM, between at or about 0.5 µM and at or about 5 µM, between at or about 0.5 µM and at or about 1 µM, between at or about 1 µM and at or about 100 µM, between at or about 1 µM and at or about 50 µM, between at or about 1 µM and at or about 25 µM, between at or about 1 µM and at or about 10 µM, between at or about 1 µM and at or about 5 µM, between at or about 5 µM and at or about 100 µM, between at or about 5 µM and at or about 50 µM, between at or about 5 µM and at or about 25 µM, between at or about 5 µM and at or about 10 µM, between at or about 10 µM and at or about 100 µM, between at or about 10 µM and at or about 50 µM, between at or about 10 µM and at or about 25 µM, between at or about 25 µM and at or about 100 µM, between at or about 25 µM and at or about 50 µM, or between at or about 50 µM and at or about 100 µM, each inclusive.
66. The method of any of embodiments 25-65, wherein the antigen presenting cells are dendritic cells, mononuclear phagocytes, B lymphocytes, endothelial cells or thymic epithelium.
67. The method of any of embodiments 25-66, wherein the antigen presenting cells are dendritic cells.
68. The method of any of embodiments 25-67, wherein the antigen presenting cells are autologous to the subject.
69. The method of any of embodiments 25-68, wherein the one or more non-native peptide comprises an individual peptide or a pool of peptides.
70. The method of any of embodiments 25-69, wherein the one or more non-native peptides are loaded on antigen presenting cells by transfection of in vitro transcribed synthesized minigene constructs encoding for the one or more non-native peptides in tandem, wherein the transcribed minigene constructs generate individual peptides.
71. The method of any of embodiments 25-70, where the one or more non-native peptides are loaded on antigen presenting cells by peptide pulse, optionally by electroporation.
72. The method of embodiment 71, wherein the one or more non-native peptide is 5-30 amino acids, optionally 12-25 amino acids, optionally at or about 25 amino acids in length.
73. The method of embodiment 71 or embodiment 72, wherein:
   the one or more non-native peptides are a pool of peptides and the concentration of peptides in the pool of peptides for the peptide pulse is between at or about 0.001 µg/mL and at or about 40 µg/mL, 0.01 µg/mL and at or about 40 µg/mL, at or about 0.1 µg/mL and at or about 40 µg/mL, at or about 1 µg/mL and at or about 40 µg/mL, at or about 0.01 µg/mL and at or about 10 µg/mL or at or about 1 µg/mL and at or about 10 µg/mL; or
   the one or more non-native peptides is an individual peptide and the concentration of individual peptides for the peptide pulse is between at or about 0.00001 µg/mL and at or about 1 µg/mL, at or about 0.00001 µg/mL and at or about 0.1 µg/mL, at or about 0.00001 µg/mL and at or about 0.01 µg/mL, at or about 0.0001 µg/mL and at or about 1 µg/mL, at or about 0.0001 µg/mL and at or about 0.1 µg/mL, at or about 0.0001 µg/mL and at or about 0.1 µg/mL or at or about 0.0001 µg/mL and at or about 0.01 µg/mL.
74. The method of any of embodiments 71-73, wherein the concentration of individual peptides of the one or more non-native peptide, on average, is from at or about 0.00001 µg/mL to at or about 0.01 µg/mL.
75. The method of any of embodiments 71-74, wherein the concentration of individual peptide of the one or more non-native peptide, on average, is from at or about 0.0001 µg/mL and at or about 0.001 µg/mL.
76. The method of any of embodiments 25-75, wherein the co-culture ratio of antigen presenting cells to T Cells is between 20:1 and 1:1, between 15:1 and 1:1, between 10:1 and 1:1, between 5:1 and 1:1, between 2.5:1 and 1:1, between 1:20 and 1:1, between 1:15 and 1:1, between 1:10 and 1:1, between 1:5 and 1:1, or between 1:2.5 and 1:1.
77. The method of any of embodiments 25-76, wherein the co-culture ratio of dendritic cells to T Cells is between 5:1 and 1:5 or is between 3:1 and 1:3, optionally is or is about 1:1.
78. The method of any of embodiments 25-77, wherein the co-culture ratio of antigen presenting cells to T cells is or is about 1:1.
79. The method of any of embodiments 25-78, wherein the co-culturing is for 2 hours to 24 hours.
80. The method of any of embodiments 25-79, wherein the co-culturing is for at or about 6 hours.
81. The method of any of embodiments 1-80, where the selecting cells is performed using a florescence based cell sorter.
82. The method of embodiment 81, wherein the fluorescence based cell sorter is an automated high-throughput flow cytometry sorter, optionally FX500 cell sorter or Miltenyi Tyto cell sorter.
83. The method of embodiment 81 or embodiment 82, wherein the selection is by 1 run, 2 runs, 3 runs or 4 runs by the fluorescence based cell sorter.
84. The method of any of embodiments 81-83, wherein the selection is performed at rate between 10,000 and 100,000 cells / second using a florescent based disposable fluidics cell sorter.
85. The method of any of embodiments 1-22, wherein the performing the expansion is carried out in a closed system using a gas permeable membrane.
86. The method of any of embodiments 1-22, wherein the performing the expansion is carried out in a closed system using a bioreactor.
87. The method of any of embodiments 16-84, wherein the performing the first expansion is carried out in a closed system using a gas permeable culture vessel.
88. The method of any of embodiments 16-84, wherein the performing the first expansion is carried out in a closed system using a bioreactor.
89. The method of any of embodiments 16-88, wherein the performing the second expansion is performed in a gas permeable culture vessel.
90. The method of any of embodiments 16-88, wherein the performing the second expansion is performed using a bioreactor.
91. The method of any of embodiments 1-90, wherein the tumor is a tumor of an epithelial cancer.
92. The method of any of embodiments 1-91, wherein the tumor is a tumor of a melanoma, lung squamous, lung adenocarcinoma, bladder cancer, lung small cell cancer, esophageal cancer, colorectal cancer (CRC), cervical cancer, head and neck cancer, stomach cancer or uterine cancer.
93. The method of any of embodiments 1-92, wherein the tumor is a melanoma.
94. The method of any of embodiments 1-93, wherein the tumor is a colorectal cancer (CRC).
95. The method of any of embodiments 1-94, wherein the tumor is a tumor of a non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.
96. The method of any of embodiments 1-95, wherein the method results in a fold-expansion of T cells or in a fold-expansion of tumor reactive T cells from the input sample that is at least at or about 2-fold, at least at or about 5-fold, at least at or about 10-fold, at least at or about 25-fold, at least at or about 50-fold, at least at or about 100-fold, at least at or about 250-fold, at least at or about 500-fold, at least at or about 750-fold, at least at or about 1000-fold, at least at or about 1500-fold, at least at or about 2000-fold, at least at or about 2500-fold, or at least at or about 3000-fold.
97. The method of any of embodiments 1-96, wherein the composition of tumor reactive cells produced by the method are able to produce IFNgamma at a concentration of greater than at or about 30 pg/mL, optionally greater than at or about 60 pg/mL, following antigen-specific stimulation.
98. The method of any of embodiments 1-97, further comprising harvesting cells produced by the method for formulation as the therapeutic composition.
99. The method of embodiment 98, comprising formulating the harvested cells with a cryoprotectant.
100. A composition comprising tumor-reactive T cells produced by the method of any of embodiments 1-99.
101. The composition of embodiment 100, wherein the composition comprises a cryoprotectant.
102. The composition of embodiment 100 or embodiment 101, wherein the T cells are CD3+ T cells or comprise CD4+ T cells and/or CD8+ T cells.
103. The composition of any of embodiments 100-102, wherein the T cells comprise CD4+ T cells and CD8+ T cells, wherein the ratio of CD8+ T cells to CD4+ T cells is between at or about 1: 100 and at or about 100:1, between at or about 1:50 and at or about 50:1, between at or about 1:25 and at or about 25:1, between at or about 1:10 and at or about 10:1, between at or about 1:5 and at or about 5:1, or between at or about 1:2.5 and at or about 2.5:1.
104. The composition of any of embodiments 100-103, wherein the number of tumor reactive T cells, or of viable cells thereof, in the composition is between at or about 0.5 x 10⁸ and at or about 50 x 10⁹, between at or about 0.5 x 10⁸ and at or about 30 x 10⁹, between 0.5 x 10⁸ and at or about 12 x 10⁹, between at or about 0.5 x 10⁸ and at or about 60 x 10⁸, between at or about 0.5 x 10⁸ and at or about 15 x 10⁸, between at or about 0.5 x 10⁸ and at or about 8 x 10⁸, between at or about 0.5 x 10⁸ and at or about 3.5x 10⁸, between at or about 0.5 x 10⁸ and at or about 1 x 10⁸, between 1 x 10⁸ and at or about 50 x 10⁹, between at or about 1 x 10⁸ and at or about 30 x 10⁹, between 1 x 10⁸ and at or about 12 x 10⁹, between at or about 1 x 10⁸ and at or about 60 x 10⁸, between at or about 1 x 10⁸ and at or about 15 x 10⁸, between at or about 1 x 10⁸ and at or about 8 x 10⁸, between at or about 1 x 10⁸ and at or about 3.5x 10⁸, between at or about 3.5 x 10⁸ and at or about 50 x 10⁹, between at or about 3.5 x 10⁸ and at or about 30 x 10⁹, between at or about 3.5 x 10⁸ and at or about 12 x 10⁹, between at or about 3.5 x 10⁸ and at or about 60 x 10⁸, between at or about 3.5 x 10⁸ and at or about 15 x 10⁸, between at or about 3.5 x 10⁸ and at or about 8 x 10⁸, between at or about 8 x 10⁸ and at or about 50 x 10⁹, between at or about 8 x 10⁸ and at or about 30 x 10⁹, between at or about 8 x 10⁸ and at or about 12 x 10⁹, between at or about 8 x 10⁸ and at or about 60 x 10⁸, between at or about 8 x 10⁸ and at or about 15 x 10⁸, between at or about 15 x 10⁸ and at or about 50 x 10⁹, between at or about 15 x 10⁸ and at or about 30 x 10⁹, between at or about 15 x 10⁸ and at or about 12 x 10⁹, between at or about 15 x 10⁸ and at or about 60 x 10⁸, between at or about 60 x 10⁸ and at or about 50 x 10⁹, between at or about 60 x 10⁸ and at or about 30 x 10⁹, between at or about 60 x 10⁸ and at or about 12 x 10⁹, between at or about 12 x 10⁹ and at or about 50 x 10⁹, between at or about 12 x 10⁹ and at or about 30 x 10⁹, or between at or about 30 x 10⁹ and at or about 60 x 10⁹, each inclusive.
105. The composition of any of embodiments 100-104, wherein the number of tumor reactive T cells, or of viable cells thereof, in the composition is at least at or about 5 x 10⁸.
106. The composition of any of embodiments 100-104, wherein the number of tumor reactive T cells, or of viable cells thereof, in the composition is at least at or about 1 x 10⁹.
107. The composition of any of embodiments 100-104, wherein the number of tumor reactive T cells, or of viable cells thereof, in the composition is at least at or about 10 x 10⁹.
108. The composition of any of embodiments 100-107 comprising a pharmaceutically acceptable excipient.
109. A method of treatment, comprising administering the composition of any of embodiments 100-108 to a subject having a cancer.
110. The method of embodiment 109, wherein the cells of the administered composition are autologous to the subject.
111. The method of embodiment 109 or embodiment 110, wherein the therapeutically effective dose is between 1 x 10⁸ and 10 x 10⁹ T cells or viable cells thereof.
112. The method of embodiment 109 or embodiment 110, wherein the therapeutically effective dose is between 5 x 10⁸ and 10 x 10⁹ T cells or viable cells thereof.
113. The method of embodiment 109 or embodiment 110, wherein the therapeutically effective dose is between 5 x 10⁸ and 1 x 10⁹ T cells or viable cells thereof.
114. The method of any of embodiments 109-113, wherein the cancer is an epithelial cancer.
115. The method of embodiment any of embodiments 109-114, wherein the cancer is melanoma, lung squamous, lung adenocarcinoma, bladder cancer, lung small cell cancer, esophageal cancer, colorectal cancer, cervical cancer, head and neck cancer, stomach cancer or uterine cancer.
116. The method of any of embodiments 109-115, wherein the cancer is non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.
117. A composition of any of embodiments 100-108 for use in treating a subject having a cancer.
118. Use of a composition of any of embodiments 100-108 for manufacture of a medicament for treating a subject having a cancer.
119. The composition for use of embodiment 117 or the use of embodiment 118, wherein the cells of the administered composition are autologous to the subject.
120. The composition for use or the use of any of embodiments 117-119, wherein the therapeutically effective dose is between 1 x 10⁸ and 10 x 10⁹ T cells or viable cells thereof.
121. The composition for use or the use of any of embodiments 117-119, wherein the therapeutically effective dose is between 5 x 10⁸ and 10 x 10⁹ T cells or viable cells thereof.
122. The composition for use or the use of any of embodiments 117-119, wherein the therapeutically effective dose is between 5 x 10⁸ and 1 x 10⁹ T cells or viable cells thereof.
123. The composition for use or the use of any of embodiments 117-122, wherein the cancer is an epithelial cancer.
124. The composition for use or the use of any of embodiments 117-122, wherein the cancer is melanoma, lung squamous, lung adenocarcinoma, bladder cancer, lung small cell cancer, esophageal cancer, colorectal cancer, cervical cancer, head and neck cancer, stomach cancer or uterine cancer.
125. The composition for use or the use of any of embodiments 117-124, wherein the cancer is non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.

## Claims

1. A method for manufacturing tumor-reactive T cells, the method comprising:
a. selecting cells surface positive for at least two activation markers from the group consisting of PD-1, CD39 and TIGIT from an input sample comprising T cells from a subject that has a tumor to obtain selected cells from the sample; and
b. performing an expansion by culture of the selected cells with one or more T-cell stimulating agent of lymphocytes under conditions to produce a population of expanded T cells.

2. The method of claim 1, wherein the selecting further comprises selecting cell surface positive for one or more further marker selected from CD107a, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), TIM-3, or LAG-3, wherein the selecting for cells surface positive for the activation markers and the selecting cells surface positive for the further marker is carried out simultaneously or sequentially in any order to obtain the selected cells.

3. The method of claim 1 or claim 2, further comprising selecting, optionally by positive selection or negative selection, T cells surface positive for a T cell marker selected from CD3, CD4 or CD8, wherein the selecting cells surface positive for the T cell marker and the selecting cells surface positive for the activation markers is carried out simultaneously or sequentially in any order to obtain the selected cells.

4. The method of any of claims 1-3, wherein:
(i) the input sample comprising T cells is from the peripheral blood or from a tumor;
(ii) the input sample comprises tumor infiltrating lymphocytes; and/or
(iii) the input sample comprising T cells is derived from a resected tumor, optionally wherein the input sample comprising T cells is a single cell suspension processed by homogenization and/or enzymatic digestion of one or more tumor fragments from the resected tumor, or more optionally wherein the input sample comprising T cells is a single cell suspension processed by homogenization and enzymatic digestion of one or more tumor fragments from the resected tumor.

5. The method of any of claims 1-4, wherein the performing the expansion to produce the expanded population of T cells is for: (i) 7 to 35 days; (ii) 7 to 28 days, optionally 14 days to 28 days; or (iii) 7 to 21 days, optionally 7 to 14 days.

6. The method of any of claims 1-5, wherein the one or more T-cell stimulating agent is one or more first T-cell stimulating agent and the performing the expansion is a first expansion, wherein the method further comprises performing a second expansion by culture of the first expanded T cell population with one or more second T-cell stimulating agent under conditions to produce a second expanded population of T cells, optionally wherein the one or more T cell stimulating agent of the first expansion and the one or more T cell stimulating agent of the second expansion are the same.

7. The method of any of claims 1-6, wherein:
(i) the one or more T-cell stimulating agent is an anti-CD3 agent (e.g. OKT3) and/or a recombinant cytokine selected from one or more of IL-2, IL-7, IL-15, IL-21, IL-25, IL-23, IL-27 and IL-35; and/or
(ii) at least one of the one or more T-cell stimulating agent is recombinant IL-2.

8. The method of claim 7, wherein the concentration of recombinant IL-2: (i) is from 100 IU/mL to 6000 IU/mL; (ii) is from 300 IU/mL to 1000 IU/mL; (iii) is at or about 300 IU/mL; or (iv) is at or about 1000 IU/mL.

9. The method of any of claims 1-8, where the selecting cells is performed using a florescence based cell sorter, optionally wherein the fluorescence based cell sorter is an automated high-throughput flow cytometry sorter, optionally FX500 cell sorter or Miltenyi Tyto cell sorter.

10. The method of any of claims 1-9, wherein the performing the expansion is carried out in a closed system using a gas permeable membrane or in a closed system using a bioreactor.

11. The method of any of claims 1-10, wherein the tumor:
(i) is a tumor of an epithelial cancer;
(ii) is a tumor of a melanoma, lung squamous, lung adenocarcinoma, bladder cancer, lung small cell cancer, esophageal cancer, colorectal cancer (CRC), cervical cancer, head and neck cancer, stomach cancer or uterine cancer;
(iii) is a melanoma;
(iv) is a colorectal cancer (CRC); or
(v) is a tumor of a non-small cell lung cancer (NSCLC), CRC, ovarian cancer, breast cancer, esophageal cancer, gastric cancer, pancreatic cancer, cholangiocarcinoma cancer, endometrial cancer, optionally wherein the breast cancer is HR+/Her2- breast cancer, triple negative breast cancer (TNBC) or HER2+ breast cancer.

12. The method of any of claims 1-11, further comprising harvesting cells produced by the method for formulation as the therapeutic composition, optionally comprising formulating the harvested cells with a cryoprotectant.

13. A composition comprising tumor-reactive T cells produced by a method of any of claims 1-12.

14. A composition produced by the method of any of claims 1-12 for use in treating a subject having a cancer.

15. A composition comprising tumor-reactive T cells, wherein the cells are from an input sample comprising T cells from a subject that has a tumor; and wherein the tumor-reactive T cells are positive for two or more T cell exhaustion markers, wherein the two or more markers are selected from cell surface CD107a, CD39, CD103, CD137 (4-1BB), CD59, CD90, CD36, CD38, CD30, CD154 (CD40L), CD134 (OX40), CD152 (CTLA-4), CD160, CXCR5 (CD195), CD244, CD258 (LIGHT), CD256 (APRIL), CD272 (BTLA-4), PD-1, TIM-3, LAG-3, and TIGIT, or secreted CXCL13.
